# EUROPEAN PATENT APPLICATION

(11) **EP 2 305 710 A2**
(43) Date of publication of application: **06.04.2011**
(21) Application number: 10011041.0
(22) Date of filing: 03.06.2003
(51) Int. Cl.: C07K 16/22, C07K 16/32, C07K 16/42

(54) **Synthetic antibody phage libraries**

(30) Priority: 03.06.2002 US 385338 P; 16.04.2003 US 463656 P
(62) Divisional of application: 03734379.5
(71) Applicant: Genentech, Inc., South San Francisco CA 94080-4990 (US)
(72) Inventor: Fuh, Germaine, G., Pacifica CA 94044 (US); Sidhu, Sachdev, S., San Francisco CA 94107 (US)
(74) Representative: Denison, Christopher Marcus

(57) **Abstract**

The invention provides comprising variant amino acids in CDRs of antibody variable domains. These polypeptides provide a source of great sequence diversity that can be used as a source for identifying novel antigen binding polypeptides. The invention also provides these polypeptides as fusion polypeptides to heterologous polypeptides such as at least a portion of phage or viral coat proteins, tags and linkers. Libraries comprising a plurality of these polypeptides are also provided. In addition, methods of and compositions for generating and using these polypeptides and libraries are provided.

## Description

### FIELD OF THE INVENTION

The invention generally relates to libraries of antibodies or antibody variable domains. The libraries include a plurality of different antibody variable domains generated by creating diversity in the CDR regions. In particular, diversity in CDR regions is designed to maximize the diversity while minimizing the structural perturbations of the antibody variable domain. The invention also relates to fusion polypeptides of one or more antibody variable domain and a heterologous protein such as a coat protein of a virus. The invention also relates to replicable expression vectors which include a gene encoding the fusion polypeptide, host cells containing the expression vectors, a virus which displays the fusion polypeptide on the surface of the virus, libraries of the virus displaying a plurality of different fusion polypeptides on the surface of the virus and methods of using those compositions. The methods and compositions of the invention are useful for identifying novel antibodies that can be used therapeutically or as reagents.

### BACKGROUND

Phage display technology has provided a powerful tool for generating and selecting novel proteins that bind to a ligand, such as an antigen. Using the techniques of phage display allows the generation of large libraries of protein variants that can be rapidly sorted for those sequences that bind to a target antigen with high affinity. Nucleic acids encoding variant polypeptides are fused to a nucleic acid sequence encoding a viral coat protein, such as the gene III protein or the gene VIII protein. Monovalent phage display systems where the nucleic acid sequence encoding the protein or polypeptide is fused to a nucleic acid sequence encoding a portion of the gene III protein have been developed. (Bass, S., Proteins, 8:309 (1990); Lowman and Wells, Methods: A Companion to Methods in Enzymology, 3:205 (1991)). In a monovalent phage display system, the gene fusion is expressed at low levels and wild type gene III proteins are also expressed so that infectivity of the particles is retained. Methods of generating peptide libraries and screening those libraries have been disclosed in many patents (e.g. U.S. Patent No. 5,723,286, U.S. Patent No. 5,432, 018, U.S. Patent No. 5,580,717, U.S. Patent No. 5,427,908 and U.S. Patent No. 5,498,530).

The demonstration of expression of peptides on the surface of filamentous phage and the expression of functional antibody fragments in the periplasm of *E*. *coli* was important in the development of antibody phage display libraries. (Smith et al., Science (1985), 228:1315; Skerra and Pluckthun, Science (1988), 240:1038). Libraries of antibodies or antigen binding polypeptides have been prepared in a number of ways including by altering a single gene by inserting random DNA sequences or by cloning a family of related genes. Methods for displaying antibodies or antigen binding fragments using phage display have been described in U.S. Patent Nos. 5,750,373, 5,733,743, 5,837,242, 5,969,108, 6,172,197, 5,580,717, and 5,658,727. The library is then screened for expression of antibodies or antigen binding proteins with desired characteristics.

Phage display technology has several advantages over conventional hybridoma and recombinant methods for preparing antibodies with the desired characteristics. This technology allows the development of large libraries of antibodies with diverse sequences in less time and without the use of animals. Preparation of hybridomas or preparation of humanized antibodies can easily require several months of preparation. In addition, since no immunization is required, phage antibody libraries can be generated for antigens which are toxic or have low antigenicity (Hogenboom, Immunotechniques (1988), 4:1-20). Phage antibody libraries can also be used to generate and identify novel human antibodies.

Human antibodies have become very useful as therapeutic agents for a wide variety of conditions. For example, humanized antibodies to HER-2, a tumor antigen, are useful in the diagnosis and treatment of cancer. Other antibodies, such as anti-INF-γ antibody, are useful in treating inflammatory conditions such as Crohn's disease. Phage display libraries have been used to generate human antibodies from immunized, non-immunized humans, germ line sequences, or naïve B cell Ig repertories (Barbas & Burton, Trends Biotech (1996), 14:230; Griffiths et al., EMBO J. (1994), 13:3245; Vaughan et al., Nat. Biotech. (1996), 14:309; Winter EP 0368 684 B1). Naïve, or nonimmune, antigen binding libraries have been generated using a variety of lymphoidal tissues. Some of these libraries are commercially available, such as those developed by Cambridge Antibody Technology and Morphosys (Vaughan et al., Nature Biotech 14:309 (1996); Knappik et al., J. Mol. Biol. 296:57 (1999)). However, many of these libraries have limited diversity.

The ability to identify and isolate high affinity antibodies from a phage display library is important in isolating novel human antibodies for therapeutic use. Isolation of high affinity antibodies from a library is dependent on the size of the library, the efficiency of production in bacterial cells and the diversity of the library. See, for e.g., Knappik et al., J. Mol. Biol. (1999), 296:57. The size of the library is decreased by inefficiency of production due to improper folding of the antibody or antigen binding protein and the presence of stop codons. Expression in bacterial cells can be inhibited if the antibody or antigen binding domain is not properly folded. Expression can be improved by mutating residues in turns at the surface of the variable/constant interface, or at selected CDR residues. (Deng et al., J. Biol. Chem. (1994), 269:9533, Ulrich et al., PNAS (1995), 92:11907-11911; Forsberg et al., J. Biol. Chem. (1997), 272 :12430). The sequence of the framework region is a factor in providing for proper folding when antibody phage libraries are produced in bacterial cells.

Generating a diverse library of antibodies or antigen binding proteins is also important to isolation of high affinity antibodies. Libraries with diversification in limited CDRs have been generated using a variety of approaches. See, for e.g., Tomlinson, Nature Biotech. (2000), 18:989-994. CDR3 regions are of interest in part because they often are found to participate in antigen binding. CDR3 regions on the heavy chain vary greatly in size, sequence and structural conformation.

Others have also generated diversity by randomizing CDR regions of the variable heavy and light chains using all 20 amino acids at each position. It was thought that using all 20 amino acids would result in a large diversity of sequences of variant antibodies and increase the chance of identifying novel antibodies. (Barbas, PNAS 91:3809 (1994); Yelton, DE, J. Immunology, 155:1994 (1995); Jackson, J.R., J. Immunology, 154:3310 (1995) and Hawkins, RE, J. Mol. Biology, 226:889 (1992)).

There have also been attempts to create diversity by restricting the group of amino acid substitutions in some CDRs to reflect the amino acid distribution in naturally occurring antibodies. See, Garrard & Henner, Gene (1993), 128:103; Knappik et al., J. Mol. Biol. (1999), 296:57*.* However, these attempts have had varying success and have not been applied in a systematic and quantitative manner. Creating diversity in the CDR regions while minimizing the number of amino acid changes has been a challenge.

There is a need to isolate novel high affinity antibodies for clinical uses, for example therapeutic and diagnostic uses. To meet this need, there remains a need to generate a highly diverse library of antibody variable domains that can be expressed in high yield in cells. The invention described herein meets this need and provides other benefits.

### SUMMARY OF INVENTION

The present invention provides methods of systematically and efficiently generating polypeptides comprising diversified CDRs. In one embodiment, the invention provides rapid identification of high affinity binders to target antigens that can also be readily produced in high yield in cell culture. Unlike conventional methods that propose that adequate diversity of target binders can be generated only if a particular CDR(s), or all CDRs should be diversified, and unlike conventional notions that adequate diversity is dependent upon the broadest range of amino acid substitutions (generally by substitution using all or most of the 20 amino acids), the invention provides methods capable of generating high quality target binders that are not necessarily dependent upon diversifying a particular CDR(s) or a particular number of CDRs of a reference polypeptide or source antibody. The invention is based, at least in part, on the surprising and unexpected finding that highly diverse libraries of high quality can be generated by systematic and selective substitution of a minimal number of amino acid positions with a minimal number of amino acid residues. Methods of the invention are convenient, based on objective and systematic criteria, and rapid. Candidate binder polypeptides generated by the invention possess high-quality target binding characteristics and have structural characteristics that provide for high yield of production in cell culture. The invention also provides unique dimerization/multimerization techniques that further enhance library characteristics, and the binding characteristics of candidate fusion polypeptide binders therein.

In particular, fusion polypeptides comprising diversified CDR(s) and a heterologous polypeptide sequence (preferably that of at least a portion of a viral polypeptide) are generated, individually and as a plurality of unique individual polypeptides that are candidate binders to targets of interest. Compositions (such as libraries) comprising such polypeptides find use in a variety of applications, in particular as large and diverse pools of candidate immunoglobulin polypeptides (in particular, antibodies and antibody fragments) that bind to targets of interest. The invention encompasses various aspects, including polypeptides generated according to methods of the invention, and systems, kits and articles of manufacture for practicing methods of the invention, and/or using polypeptides and/or compositions of the invention.

In another aspect, the invention provides methods of generating a polypeptide and polypeptides comprising at least one, two, three, four or all variant CDRs (i.e., selected from the group consisting of CDRs L1, L2, L3, H1 and H2 wherein said polypeptide is capable of binding a target antigen of interest, and wherein said CDR is not CDRH3, said method comprising: (a) identifying at least one (or any number up to all) solvent accessible and highly diverse amino acid position in a CDR; and (b) replacing the amino acid at the solvent accessible and high diverse position with a target amino acid (as defined herein) by generating variant copies of the CDR using a non-random codon set, wherein at least about 50%, 60%, 70%, 80%, 90% or all of the amino acids encoded by the non-random codon set are target amino acids (as defined herein) for that position in known antibody and/or naturally occurring variable domains.

In another aspect, the invention provides a method of generating a polypeptide comprising at least one, two, three, four, five or all of variant CDRs selected from the group consisting of H1, H2, H3, L1, L2 and L3, wherein said polypeptide is capable of binding a target antigen of interest, said method comprising: (a) with respect to L1, L2, L3, H1 and H2, (i) identifying at least one (or any number up to all) solvent accessible and highly diverse amino acid position in a reference CDR corresponding to the variant CDR; and (ii) replacing the amino acid at the solvent accessible and high diverse position with a target amino acid by generating variant copies of the CDR using a non-random codon set, wherein at least about 50%, 60%, 70%, 80%, 90% or all of the amino acids encoded by the non-random codon set are target amino acids for that position in known antibodies or antigen binding fragments(e.g. antibody variable domains)); and (b) with respect to H3, replacing at least one (or any number up to all) position with a variant amino acid.

In another aspect, the invention provides a method of generating a polypeptide comprising at least one, two, three, four, five or all of variant CDRs selected from the group consisting of L1, L2, L3, H1, H2 and H3, said method comprising: (a) substituting at least one (or any number up to all) solvent accessible and highly diverse amino acid position in L1, L2, L3, H1 and/or H2 with a variant amino acid which is encoded by a nonrandom codon set, wherein at least 50%, 60%, 70%, 80%, 90% or all of amino acids encoded by the nonrandom codon set are target amino acids for said amino acid position in known antibodies or antigen binding fragments; and (b) substituting at least one (or any number up to all) amino acid position in H3 with a variant amino acid.

Various aspects and embodiments of methods of the invention are useful for generating and/or using a pool comprising a plurality of polypeptides of the invention, in particular for selecting and identifying candidate binders to target antigens of interest. For example, the invention provides a method of generating a composition comprising a plurality of polypeptides, each polypeptide comprising at least one, two, three, four, five or all of variant CDRs selected from the group consisting of L1, L2, L3, H1, H2 and H3, said method comprising: (a) substituting at least one (or any number up to all) solvent accessible and highly diverse amino acid position in L1, L2, L3, H1 and/or H2 with a variant amino acid which is encoded by a nonrandom codon set, wherein at least 50%, 60%, 70%, 80%, 90% or all of amino acids encoded by the nonrandom codon set are target amino acids for said amino acid position in known antibodies or antigen binding fragments; and/or (b) substituting at least one (or any number up to all) amino acid position in H3 with a variant amino acid; wherein a plurality of polypeptides are generated by amplifying a template polynucleotide with a set of oligonucleotides comprising degeneracy in the sequence encoding a variant amino acid, wherein said degeneracy reflects the multiple codon sequences of the nonrandom codon set.

In another example, the invention provides a method comprising: constructing an expression vector comprising a polynucleotide sequence which encodes a light chain, a heavy chain, or both, the light chain and the heavy chain variable domains of a source antibody comprising at least one, two, three, four, five or all CDRs selected from the group consisting of CDR L1, L2, L3, H1, H2 and H3; and mutating at least one, two, three, four, five or all CDRs of the source antibody at at least one (or any number up to all) solvent accessible and highly diverse amino acid position using a nonrandom codon set, wherein at least about 50%, 60%, 70%, 80%, 90% or all of the amino acids encoded by the non-random codon set are target amino acids for that position in known and/or naturally occurring antibodies or antigen binding fragments (for e.g. antibody variable domains).

In another example, the invention provides a method comprising: constructing a library of phage or phagemid particles displaying a plurality of polypeptides of the invention; contacting the library of particles with a target antigen under conditions suitable for binding of the particles to the target antigen; and separating the particles that bind from those that do not bind to the target antigen.

In any of the methods of the invention described herein, a solvent accessible and/or highly diverse amino acid position can be any that meet the criteria as described herein, in particular any combination of the positions as described herein, for example any combination of the positions described for the polypeptides of the invention (as described in greater detail below). Suitable variant amino acids can be any that meet the criteria as described herein, for example variant amino acids in polypeptides of the invention as described in greater detail below.

Designing diversity in CDRH3 regions involves designing diversity in the length (preferably 7 to 19 amino acids) and/or in the sequence of CDRH3. In some embodiments, a portion of CDRH3 has a length ranging from 4 to 14 amino acids and has a variant amino acid at one or more positions encoded by codon sets that encode amino acids found in CDRH3 regions in known and/or naturally occurring antibody variable domains such as *NNK, NNS, DVK, NYT, XYZ* or mixtures thereof and another portion of the CDR, preferably at the C terminal end (e.g amino acid positions X₂, X₃, X₄, X₅ and X₆ shown below), has much more limited diversity. In some embodiments, the C terminal end has an amino acid sequence YAMDY or FDY.

One aspect of the invention provides a polypeptide, such as an antibody variable domain, comprising a variant CDRH3 that comprises an amino acid sequence:

(X₁)ₙ - X₂ - " X₃ - _{X4} - X₅ -X₆

wherein X₁ is any naturally occurring amino acid encoded by a codon set *of NNK, NNS, DVK, NVT, XYZ* or is tryptophan or glycine or mixtures thereof, and n = 4 to 14;

X₂ is any naturally occurring amino acid encoded by a codon set *of KSG, DVK, DSG, NVT, KSG, NNS, XYZ, NNK or* is tyrosine, phenylalanine, serine, alanine, glycine, leucine,or tryptophan;

X₃ is missing when X₂ is phenylalanine or leucine, or if present, is alanine, valine, aspartic acid or glycine;

X₄ is missing when X₂ is phenyalanine or leucine, or if present, is methionine;

X₅ is aspartic acid, alanine, valine, or glycine;and

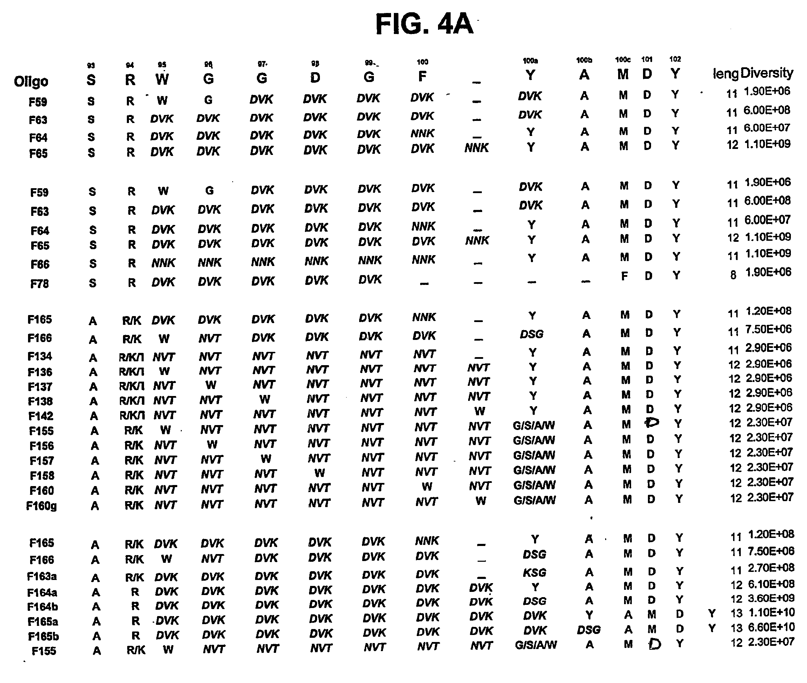

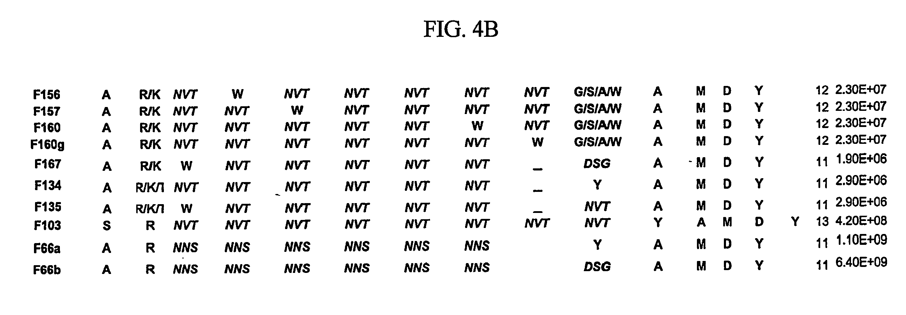

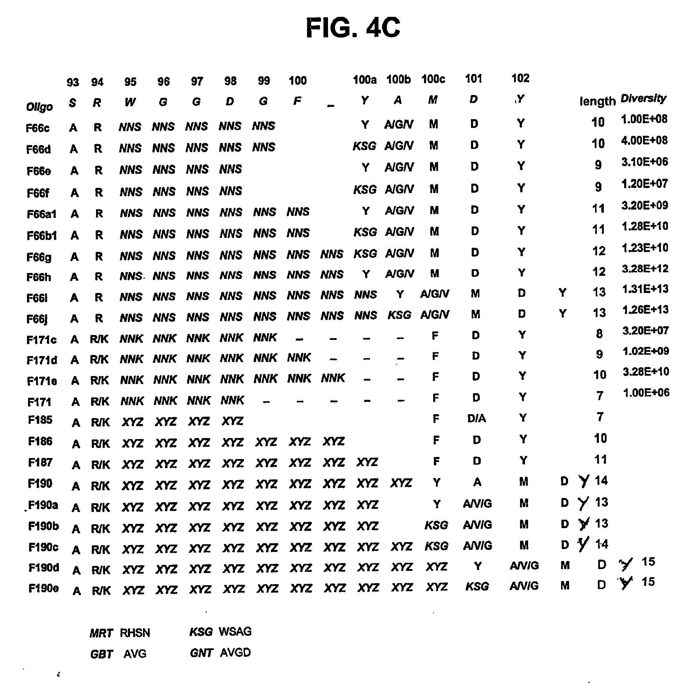

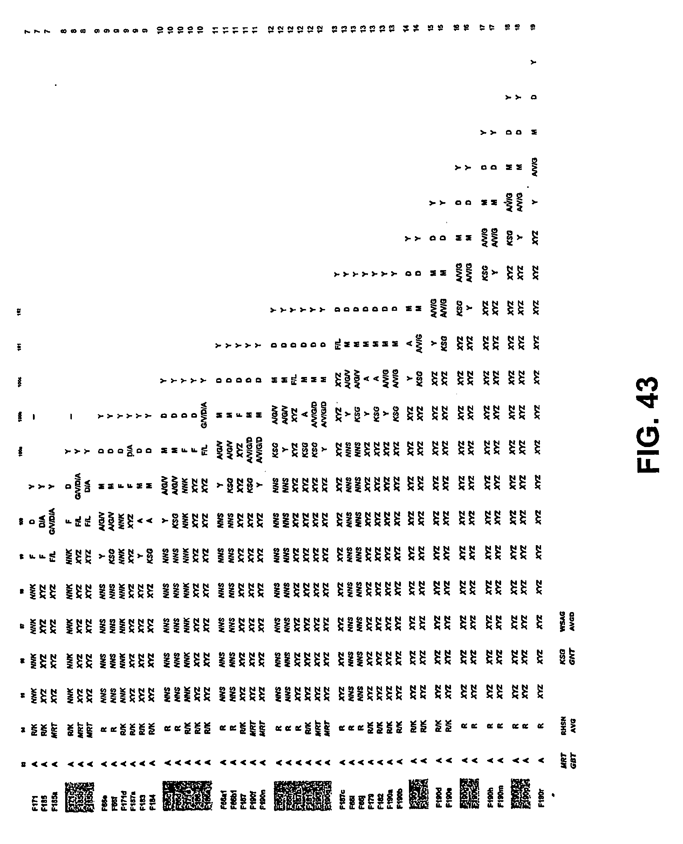

X₆ is tyrosine or valine.
A polypeptide or library of polypeptides with variant CDRH3 of the formula as shown above may be generated using any one or combination of the oligonucleotides illustrated in Figure 4 and Figure 43.

In one embodiment, CDRH3 comprises an amino acid sequence (X1)ₙ - X₂ - X₃ - X₄ - X₅ X₆, wherein X₁ is any naturally occurring amino acid encoded by a codon set *of NNK, NNS, DVK, NVT, XYZ* or tryptophan or glycine or mixtures thereof, and n = 4 to 14; X₂ is any naturally occurring amino acid encoded by a codon set *of KSG, DVK, DSG, NVT, KSG, NNS, XYZ, NNK* or is tyrosine, phenylalanine, serine, glycine,alanine, leucine,or tryptophan; X₃ is alanine, valine, aspartic acid or glycine; X₄ is methionine;X₅ is aspartic acid, alanine, valine, or glycine;and X₆ is tyrosine or valine.

In some embodiments, n is preferably 4 and X₁ is any naturally occurring amino acid encoded by codon sets *NNK, NNS or XYZ,* X₂ is any naturally occurring amino acid encoded by *KSG, NNK, XYZ* or is tyrosine, X₃ is alanine, glycine or valine, X₄ is methionine, X₅ is aspartic acid and X₆ is tyrosine. A polypeptide or plurality of polypeptides comprising variant CDRH3 region in these embodiments can be generated using at least one or more than one of oligonucleotides F66e and F66f.

In some embodiments, n is preferably 6, and X₁ is any naturally occurring amino acid encoded by codon sets *DVK, NYT, NNK, NNS or XYZ or* tryptophan or glycine or mixtures thereof, X₂ is any naturally occurring amino acid encoded by *DSG, DVK, KSG, NNK, XYZ* or is tyrosine, glycine, serine, alanine or tryptophan, X₃ is alanine, X₄ is methionine, X₅ is aspartic acid and X₆ is tyrosine. In some embodiments, X₁ corresponds to amino acid position 95, X₂ corresponds to amino acid position 100a, A is in position 100b, M is in position 100c, D is in position 101 and Y is in position 102 of CDRH3 of antibody 4D5. A polypeptide or plurality of polypeptides comprising a variant CDRH3 region in these embodiments can be generated using at least one or more than one of oligonucleotides F63, F64, F65, F66, F165, F134, F135, F163a, F166, F167, F66a, F66b, F66a1, F66b1, F190f, and F190n.

In another embodiment, CDRH3 comprises an amino acid sequence (X₁)ₙ - X₂-X₃-X₄-X₅-X₆; wherein X₁ is any naturally occurring amino acid encoded by a codon set of *NNK, NNS, DYK, NVT, XYZ* or is tryptophan or glycine or mixtures thereof, and n = 4 to 14; X₂ is leucine or phenylalanine; X₃ is missing; X₄ is missing; X₅ is aspartic acid, alanine, valine, or glycine; and X₆ is tyrosine or valine. In some embodiments, the variant CDRH3 comprises an amino acid sequence (X₁)ₙ - X₂-D-Y; wherein X₁ is any naturally occurring amino acid encoded by a codon set of *NNK, NNS, DVK, NVT, XYZ* or tryptophan or glycine or mixtures thereof, and n = 4 to 14; X₂ is leucine or phenyalanine; D is aspartic acid and Y is tyrosine. In other embodiments, n is preferably 6 and X₁ corresponds to amino acid position 95, X₂ corresponds to amino acid position 100a, X₅ is in position 101 and X₆ is in position 102 of CDRH3 of antibody 4D5. A polypeptide or plurality of polypeptides comprising a variant CDRH3 region in these embodiments can be generated using at least one or more than one of oligonucleotides F171c, F171d, F171e, F171, F185, F186, F187, F185a, F185b, F185b, F187a, F186a, F187b, and F187c.

In another aspect of the invention, methods and polypeptides, such as antibody variable domains, are provided comprising one, two or all variant CDRs selected from the group consisting of CDRH1, CDRH2 and CDRH3, wherein said variant CDRH3 comprises an amino acid sequence

(X₁)ₙ- X₂ - X₃ - X₄ - X₅-X₆

wherein X₁ is any naturally occurring amino acid encoded by a codon set *of NNK, NNS, DVK, NVT, XYZ* or is tryptophan or glycine or mixtures thereof, and n = 4 to 14;

X₂ is any naturally occurring amino acid encoded by a codon set *of KSG, DVK, DSG, NVT, KSG, NNS, XYZ*, *NNK* or is tyrosine, phenylalanine, glycine, serine, alanine, leucine,or tryptophan;

X₃ is missing when X₂ is phenylalanine or leucine, or if present, is alanine, valine, aspartic acid or glycine;

X₄ is missing when X₂ is phenyalanine or leucine, or if present, is methionine;

X₅ is aspartic acid, alanine, valine, or glycine;and

X₆ is tyrosine or valine;
and wherein the variant CDRH1 or CDRH2 or both comprise at least one variant amino acid in a solvent accessible and highly diverse amino acid position, wherein the variant amino acid is encoded by a nonrandom codon set, wherein at least about 50%, 60%, 70%, 80%, 90%, or all of the amino acids encoded by the nonrandom codon set are target amino for that position in known antibodies or antigen binding fragments (for e.g. antibody variable domains).

In some embodiments of any of the methods and polypeptides described herein, the position in H3 is any of positions 95 to 102 of antibody according to Kabat numbering system (e.g. antibody 4D5). For example, position 95 can have a variant amino acid encoded by codon set *DVK, NVT, NNS, XYZ, MVK* or is tryptophan; position 96 can be encoded *by DVK, , NVT, NNS, XYZ,* or is glycine or tryptophan; position 97 can be encoded *by DVK, NVT, NNS, XYZ,* or is tryptophan; position 98 can be encoded *by DVK, NVT, NNS, XYZ,* or is tryptophan; position 99 can be encoded by *DVK, NVT, NNS, XYZ;* position 100 can be encoded by *DVK, NVT, NNS, XYZ,* or is tryptophan; position 100a can be encoded by *DVK, DSG, KSG, NVT,NNS, XYZ,* or can be tyrosine, glycine, serine, alanine, tryptophan, aspartic acid, methionine, phenylalanine, leucine, valine; position 100b can be encoded by *DSG,KSG, XYZ* or is alanine, tyrosine, glycine, valine, aspartic acid, methionine, or phenylalanine; Position 100c can be encoded by *KSG, XYZ,* or is methionine, phenylalanine, leucine, alanine, glycine, valine, tyrosine, aspartic acid; position 101 can be encoded by *KSG,XYZ* or is aspartic acid, methionine, alanine, valine, glycine, tyrosine, phenylalanine, leucine; and position 102 can be encoded by *KSG or XYZ* or is tyrosine, aspartic acid, methionnine, alanine, valine, glycine. A polypeptide or plurality of polypeptides comprising a variant CDRH3 region in these embodiments can be generated using at least one or more than one of oligonucleotides illustrated in Figure 4 and Figure 43.

In another embodiment, methods and polypeptides, such as antibody variable domains, are provided that comprise at least one or all variant CDRL1, CDRL2 or CDRL3, wherein the variant CDR1, CDRL2 or CDRL3 or mixtures thereof comprise at least one variant amino acid in a solvent accessible and highly diverse amino acid position, wherein the variant amino acid is encoded by a nonrandom codon set, wherein at least about 50%, 60%, 70%, 80%, 90% or all of the amino acids encoded by the nonrandom codon are target amino acids for that position in known antibodies or antigen binding fragements(for e.g. antibody variable domains).

Polypeptides of the invention can be in a variety of forms as long as the target binding function of the polypeptides is retained. In some embodiments, a polypeptide of the invention is a fusion polypeptide (ie. a fusion of two or more sequences from heterologous polypeptides). Polypeptides with diversified CDRs according to the invention can be prepared as fusion polypeptides to at least a portion of a viral coat protein for use in phage display. Viral coat proteins that can be used for display of the polypeptides of the invention comprise protein p III, major coat protein pVIII, Soc (T4 phage), Hoc (T4 phage), gpD (lambda phage), pVI or variants thereof.

In some embodiments, in which the polypeptide with diversified CDRs is one or more antibody variable domains, the antibody variable domains can be displayed on the surface of the virus in a variety of formats including ScFv, Fab, S_{C}F_{V2}, F(ab')₂ and F(ab)₂. For display of the polypeptides in bivalent manner, the fusion protein preferably includes a dimerization domain. The dimerization domain can comprise at least one of a dimerization sequence, and/or sequence comprising one or more cysteine residues. The dimerization domain is preferably linked to the C-terminal end of a heavy chain variable or constant domain. The structure of the dimerization domain can be varied depending on whether the antibody variable domain is produced as a fusion protein component with the viral coat protein component(without an amber stop codon after dimerization domain) or whether the antibody variable domain is produced predominantly without viral coat protein component (eg. with an amber stop codon after dimerization domain). When the antibody variable domain is produced predominantly as a fusion protein with viral coat protein component, one or more disulfide bonds and/or a single dimerization sequence provides for bivalent display. For antibody variable domains predominantly produced without being fused to a viral coat protein component (eg. with amber stop), it is preferable to have a dimerization domain comprising both a cysteine residue and a dimerization sequence.

In addition, optionally, a fusion polypeptide can comprise a tag that may be useful in purification, detection and screening such as FLAG, poly-his, gD tag, c-myc, fluorescence protein or B-galactosidase. In one embodiment, a fusion polypeptide comprises a light chain variable or constant domain fused to a polypeptide tag.

In another aspect of the invention, a polypeptide such as an antibody variable domain is obtained from a single source or template molecule. The source or template molecule is preferably selected or designed for characteristics such as good yield and stability when produced in prokaryotic or eukaryotic cell culture, and/or to accommodate CDRH3 regions of varying lengths. The sequence of the template molecule can be altered to improve folding and/or display of the variable domain when presented as a fusion protein with a phage coat protein component. For example, a source antibody may comprise the amino acid sequence of the variable domains of humanized antibody 4D5 (light chain variable domain, SEQ ID NO: 1; heavy chain variable domain, SEQ ID NO: 2). For example, in an antibody variable domain of a heavy or light chain, framework region residues can be modified or altered from the source or template molecule to improve folding, yield, display or affinity of the antibody variable domain. In some embodiments, framework residues are selected to be modified from the source or template molecule when the amino acid in the framework position of the source molecule is different from the amino acid or amino acids commonly found at that position in naturally occurring antibodies or in a subgroup consensus sequence. The amino acids at those positions can be changed to the amino acids most commonly found in the naturally occurring antibodies or in a subgroup consensus sequence at that position. In one embodiment, framework residue 71 of the heavy chain may be R, V or A. In another example, framework residue 93 of the heavy chain may be S or A. In yet another example, framework residue 94 may be R, K or T. In yet another example, framework residue 49 in the heavy chain may be alanine or glycine. In the light chain, the amino acid at position 66 may be arginine or glycine.

Methods of the invention are capable of generating a large variety of polypeptides comprising a diverse set of CDR sequences. For example, in one embodiment, the invention provides a polypeptide comprising at least one, two, three, four, five or all of variant CDRs selected from the group consisting of CDR L1, CDR L2, CDR L3, CDR H1, CDR H2 and CDR H3; wherein (i) each of CDRs L1, L2, L3, H1 and H2 has a variant amino acid in at least one (or any number up to all) solvent accessible and highly diverse amino acid position, wherein the variant amino acid is encoded by a non-random codon set, and wherein at least about 50%, 60%, 70%, 80%, 90% or all of the amino acids encoded by the non-random codon set are target amino acids for that position in known antibodies or antigen binding fragments; and (ii) variant CDR H3 has a variant amino acid in at least one (or any number up to all) amino acid position.

In another embodiment, the invention provides a polypeptide comprising: (a) at least one, two, three, four or all of variant CDRs selected from the group consisting of CDR L1, CDR L2, CDR L3, CDR H1 and CDR H2; wherein each of CDRs L1, L2, L3, H1 and H2 has a variant amino acid in at least one (or any number up to all) solvent accessible and highly diverse amino acid position, wherein the variant amino acid is encoded by a non-random codon set, and wherein at least about 50%, 60%, 70%, 80%, 90% or all of the amino acids encoded by the non-random codon set are target amino acids for that position in known antibodies or antigen binding fragments; and (b) a variant CDR H3 comprising a variant amino acid in at least one (or any number up to all) amino acid position.

In one embodiment, the invention provides a polypeptide comprising at least one, two, three, four, five or all of CDRs selected from the group consisting of CDRL1, CDRL2, CDRL3, CDRH1, CDRH2 and CDRH3, wherein: (a) CDRL1 comprises a variant amino acid in at least one, two, three, four or all of amino acid positions 28, 29, 30, 31 and 32; (b) CDRL2 comprises a variant amino acid in at least one or both of amino acid positions 50 and 53; (c) CDRL3 comprises a variant amino acid in at least one, two, three, four or all of amino acid positions 91, 92, 93, 94 and 96; (d) CDRH1 comprises a variant amino acid in at least one, two, three, four or all of amino acid positions 28, 30, 31, 32 and 33; (e) CDRH2 comprises a variant amino acid in at least one, two, three, four, five or all of amino acid positions 50, 52, 53, 54, 56 and 58; and (f) CDRH3 comprises a variant amino acid in at least one, two, three, four, five, six or all of amino acid positions corresponding to amino acid positions 95, 96, 97, 98, 99, 100, and 100a in antibody 4D5; wherein the amino acid positions correspond to the Kabat numbering system; and wherein each variant amino acid of (a) to (e) is encoded by a non-random codon set, and wherein at least about 50%, 60%, 70%, 80%, 90% or all of the amino acids encoded by the non-random codon set are target amino acids for that amino acid position in known antibodies or antibody fragments. In some embodiments of these polypeptides, at least one variant amino acid of (f) is encoded by codon set *NNK, NNS, DVK, XYZ or NVT.* In some embodiments, the polypeptide comprises an antibody heavy and/or light chain variable domain. In some embodiments, CDRH3 amino acid positions 100b, 100c, 101 and 102 are also varied.

In some embodiments, the invention provides a polypeptide comprising antibody light chain and heavy chain variable domains, wherein: (a) CDR L1 comprises a variant amino acid in at least one, two, three, four or all of amino acid positions 28, 29, 30, 31 and 32; (b) CDR L2 comprises a variant amino acid in at least one or both of amino acid positions 50 and 53; (c) CDR L3 comprises a variant amino acid in at least one, two, three, four or all of amino acid positions 91, 92, 93, 94 and 96; (d) CDR H1 comprises a variant amino acid in at least one, two, three, four or all of amino acid positions 28, 30, 31, 32 and 33; (e) CDR H2 comprises a variant amino acid in at least one, two, three, four, five or all of amino acid positions 50, 52, 53, 54, 56 and 58; and (f) CDR H3 comprises a variant amino acid in at least one, two, three, four, five, six or all of amino acid positions corresponding to amino acid positions 95, 96, 97, 98, 99, 100 and 100a in antibody 4D5; wherein the amino acid positions correspond to the Kabat numbering system; and wherein each variant amino acid of (a) to (e) is encoded by a non-random codon set, and wherein at least about 50%, 60%, 70%, 80%, 90% or all of the amino acids encoded by the non-random codon set are target amino acids for that amino acid position in known antibodies or antibody fragments. In some embodiments, the variant amino acid of (f) is encoded by codon set *NNK, NNS, DYK, XYZ or NVT or* is tryptophan or glycine or mixtures thereof.

In some embodiments, the invention provides a polypeptide comprising at least one, two, three, four, five or all of CDRs selected from the group consisting of CDR L1, CDR L2, CDR L3, CDR H1, CDR H2 and CDR H3, wherein: (a) CDR L1 comprises a variant amino acid in amino acid positions 28, 29, 30, 31 and 32; (b) CDR L2 comprises a variant amino acid in amino acid positions 50 and 53; (c)

CDR L3 comprises a variant amino acid in amino acid positions 91, 92, 93, 94 and 96; (d) CDR H1 comprises a variant amino acid in amino acid positions 28, 30, 31, 32 and 33; (e) CDR H2 comprises a variant amino acid in amino acid positions 50, 52, 53, 54, 56 and 58; and (f) CDR H3 comprises a variant amino acid in amino acid positions corresponding to amino acid positions 95, 96, 97, 98, 99, 100 and 100a in antibody 4D5; wherein the amino acid positions correspond to the Kabat numbering system; and wherein each variant amino acid of (a) to (e) is encoded by a non-random codon set, and wherein at least about 50%, 60%, 70%, 80%, 90% or all of the amino acids encoded by the non-random codon set are target amino acids for that amino acid position in known antibodies or antibody fragments. In some embodiments, the variant amino acid of (e) is encoded by codon set *NNK, NNS, DVK, XYZ or NVT* or tryptophan or glycine or mixtures thereof.

In another embodiment, the invention provides a polypeptide comprising at least one, two, three, four, five or all of CDRs selected from the group consisting of CDRL1, CDRL2, CDRL3, CDRH1, CDRH2 and CDRH3, wherein: (a) CDRL1 comprises a variant amino acid in amino acid positions 28, 29, 30, 31 and 32, wherein: (i) the variant amino acid at position 28 is encoded by codon set *RDT;* (ii) the variant amino acid at position 29 is encoded by codon set *RKT or RTT;* (iii) the variant amino acid at position 30 is encoded by codon set *RVW;* (iv) the variant amino acid at position 31 is encoded by codon set *RVW or ANW;* (v) the variant amino acid at position 32 is encoded by codon set *DHT or THT;* (b) CDRL2 comprises a variant amino acid in amino acid positions 50 and 53, wherein: (i) the variant amino acid at position 50 is encoded by codon set *KBG;* (ii) the variant amino acid at position 53 is encoded by codon set *AVC;* (c) CDRL3 comprises a variant amino acid in amino acid positions 91, 92, 93, 94 and 96, wherein: (i) the variant amino acid at position 91 is encoded by codon set *KMT or TMT or* the combination of codon sets *TMT and SRT;* (ii) the variant amino acid at position 92 is encoded by codon set *DHT or DMC;* (iii) the variant amino acid at position 93 is encoded by codon set *RVT or DHT;* (iv) the variant amino acid at position 94 is encoded by codon set *NHT or WHT;* (v) the variant amino acid at position 96 is encoded by codon set *YHT, HWT, HTT or TDK* or the combination of codon sets *YKG and TWT;* (d) CDRH1 comprises a variant amino acid in amino acid positions 28, 30, 31, 32 and 33, wherein: (i) the variant amino acid at position 28 is encoded by codon set *AVT or WCC* or is threonine; (ii) the variant amino acid at position 30 is encoded by codon *set RVM or AVT;* (iii) the variant amino acid at position 31 is encoded by codon set *RVM, RVT or RRT;* (iv) the variant amino acid at position 32 is encoded by codon set *WMY;* (v) the variant amino acid at position 33 is encoded by codon set *KVK, RNT, DMT, KMT, KGG* or the combination of codon sets *KMT and KGG;* (e) CDRH2 comprises a variant amino acid in amino acid positions 50, 52, 53, 54, 56 and 58, wherein: (i) the variant amino acid at position 50 is encoded by codon set *KDK or DBG* or the combination of codon sets *DGG and DHT;* (ii) the variant amino acid at position 52 is encoded by codon set *DHT or DMT;* (iii) the variant amino acid at position 53 is encoded by codon set *NMT or DMT;* (iv) the variant amino acid at position 54 is encoded by codon set *DMK, DMT or RRC;* (v) the variant amino acid at position 56 is encoded by codon set *DMK or DMT;* (vi) the variant amino acid at position 58 is encoded by codon *set DMT or DAC;* and (f) CDRH3 comprises a variant amino acid in amino acid positions 95, 96, 97, 98, 99, 100 and 100a, wherein: the variant amino acid at each of positions 95, 96, 97, 98, 99, 100 and 100a is encoded by codon set *NNK, NNS, XYZ, DVK or NVT* or tryptophan or glycine or mixtures thereof; wherein the amino acid positions correspond to the Kabat numbering system. In some embodiments, the polypeptide comprises an antibody heavy and/or light chain variable domain.

In some embodiments of polypeptides comprise a variant CDRH3, the variant CDRH3 has a variant amino acid in at least one, two, three, four, five, six or all of amino acid positions corresponding to amino acid position 95 to 100a of antibody 4D5 wherein amino acid positions correspond to the Kabat numbering system. In some embodiments, the variant amino acid of variant CDRH3 is an amino acid encoded by codon set *NNK, XYZ, NNS, D VK or NVT* or tryptophan or glycine or mixtures thereof. In some embodiments of polypeptides of the invention, the variant amino acid at position 100a of CDRH3 is encoded by *NNS, NVT, DVK, XYZ,* or is tyrosine, phenylalanine, glycine, serine, alanine, tryptophan, aspartic acid, methionine, or valine. In some embodiments, CDRH3 has a variant amino acid in amino acid positions corresponding to 100b, 100c, 101 and 102 of antibody 4D5.

In various embodiments of polypeptides of the invention, amino acids encoded by a non-random codon set preferably include (generally are) amino acids found at the corresponding position in preferably at least about 50%, 60% or 70% of known antibodies or antigen binding fragments. In some embodiments, said known antibodies or antigen binding fragments are as in "Sequences of Proteins of Immunological Interest" (5th edition) published by the U.S. National Institutes of Health. In some embodiments, said known antibodies or antigen binding fragments are as in the database of Kabat at http:immuno/bme/nwu/edu.

In some embodiments of polypeptides of the invention, a nonrandom codon set does not encode cysteine. In some embodiments, a non-random codon set does not include a stop codon.

As described herein, a variant CDR refers to a CDR with a sequence variance as compared to the corresponding CDR of a single reference polypeptide/source antibody. Accordingly, the CDRs of a single polypeptide of the invention preferably correspond to the set of CDRs of a single reference polypeptide or source antibody.

Polypeptides of the invention can be in a variety of forms as long as the target binding function of the polypeptides are retained. In some embodiments, a polypeptide of the invention is a fusion polypeptide (i.e., a fusion of two or more sequences from heterologous polypeptides). In some embodiments, the fusion polypeptide is fused to at least a portion of a viral coat protein, such as a viral coat protein selected from the group consisting of pIII, pVIII, Soc, Hoc, gpD, pVI, and variants thereof.

In some embodiments, a polypeptide of the invention comprises a light chain and a heavy chain antibody variable domain, wherein the light chain variable domain comprises at least 1, 2 or 3 variant CDRs selected from the group consisting of CDR L1, L2 and L3, and the heavy chain variable domain comprises at least 1, 2 or 3 variant CDRs selected from the group consisting of CDR H1, H2 and H3.

In some embodiments, a polypeptide of the invention is an ScFv. In some embodiments, it is a Fab fragment. In some embodiments, it is a F(ab)₂ or F(ab')₂. Accordingly, in some embodiments, a polypeptide of the invention further comprises a dimerization domain. In some embodiments, the dimerization domain is located between an antibody heavy chain or light chain variable domain and at least a portion of a viral coat protein. The dimerization domain can comprise at least one of a dimerization sequence, and/or sequence comprising one or more cysteine residues. The dimerization domain is preferably linked to the C-terminal end of a heavy chain variable or constant domain. The structure of the dimerization domain can be varied depending on whether the antibody variable domain is produced as a fusion protein component with the viral coat protein component (without an amber stop codon after dimerization domain) or whether the antibody variable domain is produced predominantly without viral coat protein component (eg. with an amber stop codon after dimerization domain). When the antibody variable domain is produced predominantly as a fusion protein with viral coat protein component, one or more disulfide bond and/or a single dimerization sequence provides for bivalent display. For antibody variable domains predominantly produced without being fused to a viral coat protein component (eg. with amber stop), it is preferable to have a dimerization domain comprising both a cysteine residue and a dimerization sequence. In some embodiments, heavy chains of the F(ab)₂ dimerize at a dimerization domain not including a hinge region. The dimerization domain may comprise a leucine zipper sequence (for example, a GCN4 sequence as depicted in SEQ ID NO: 3).

In some embodiments, a polypeptide of the invention further comprises a light chain constant domain fused to a light chain variable domain, which in some embodiments comprises at least one, two or three variant CDRs. In some embodiments of polypeptides of the invention, the polypeptide comprises a heavy chain constant domain fused to a heavy chain variable domain, which in some embodimentscomprises at least one, two or three variant CDRs.

In some embodiments, a polypeptide of the invention comprises an antibody light chain variable domain, wherein the variant CDR is CDR L1 and the amino acid positions that are diversified are those positions that correspond to amino acid positions 28, 29, 30, 31 and 32. In some embodiments, the variant amino acid at position 28 is encoded by codon set *RDT,* the variant amino acid at position 29 is encoded by codon set *RKT* or *RTT,* the variant amino acid at position 30 is encoded by codon set *RVW,* the variant amino acid at position 31 is encoded by codon set *RVW or ANW,* and the variant amino acid at position 32 is encoded by codon set *DHT or THT.* In one embodiment, the amino acid at position 30 is asparagine or serine.

In another embodiment, a polypeptide of the invention comprises an antibody light chain variable domain, wherein the variant CDR is CDR L2, and the amino acid positions that are diversified are those that correspond to amino acid positions 50 and 53. In some embodiments, the variant amino acid at position 50 is encoded by KBG codon set, and the variant amino acid at position 53 is encoded by codon set *AVC.*

In another embodiment, a polypeptide of the invention comprises an antibody light chain variable domain, wherein the variant CDR is CDR L3, and the amino acid positions that are diversified are selected from those that correspond to amino acid positions 91, 92, 93, 94, or 96. In some embodiments, the variant amino acid at position 91 is encoded by codon set *KMT, TMT or* the combination of codon sets *TMT and SRT,* the variant amino acid at position 92 is encoded by codon set *DHT or DMC,* the variant amino acid at position 93 is encoded by codon set *RVT or DHT,* the variant amino acid at position 94 is encoded by codon set *NHT or WHT,* and the variant amino acid at position 96 is encoded by codon set *YHT, HWT; HTT* or the combination of codon sets *YKG* and *TWT.* In one embodiment, amino acid position 91 is histidine or serine.

In another embodiment, a polypeptide of the invention comprises a heavy chain variable domain, the variant CDR is CDR H1, and the amino acid positions that are diversified are those selected from amino acids positions corresponding to amino acids 28, 30, 31, 32 or 33. In some embodiments, the variant amino acid at position 28 is encoded by codon set *AVT, WCC* or is threonine, the variant amino acid at position 30 is encoded by codon set *RVM or AVT,* the variant amino acid at position 31 is encoded by codon set *RVM, RVT or RRT,* the variant amino acid at position 32 is encoded by codon set WMY, and the variant amino acid at position 33 is encoded by codon set *KVK, RNT, DMT, KMT or* the combination of codon sets *KMT and KGG.*

In another embodiment, a polypeptide of the invention comprises a heavy chain variable domain, the variant CDR is CDR H2, and the amino acid positions that are diversified are those selected from amino acid positions corresponding to amino acids 50, 52, 53, 54, 56 or 58. In some embodiments, the variant amino acid at position 50 is encoded by codon set *KDK, DBG* or the combination of codon sets *DGG and DHT,* the variant amino acid at position 52 is encoded by codon set *DHT or DMT,* the variant amino acid at position 53 is encoded by codon set *NMT or DMT,* the variant amino acid at position 54 is encoded by codon set *DMK, DMT or RRC,* the variant amino acid at position 56 is encoded by codon set *DMK or DMT,* and the variant amino acid at position 58 is encoded by codon set *DMT or DAC.*

In another embodiment, a polypeptide of the invention comprises a heavy chain variable domain, and the variant CDR is CDR H3 comprising a variant amino acid in at least one, two, three, four, five, six or all of amino acid positions 95 to 100a, wherein the variant amino acids is encoded by codon set *NNK, NNS, XYZ, DVK or NVT* or tryptophan or glycine or mixtures thereof.

In some instances, it may be preferable to mutate a framework residue such that it is variant with respect to a reference polypeptide or source antibody. For example, framework residue 71 of the heavy chain may be amino acid R, V or A. In another example, framework residue 93 of the heavy chain may be amino acid S or A. In yet another example, framework residue 94 of the heavy chain may be amino acid R, K or T or encoded by *MRT.* In yet another example, framework residue 49 of the heavy chain may be amino acid A or G. Framework residues in the light chain may also be mutated. For example, framework residue 66 in the light chain may be amino acid R or G.

As described herein, a variant CDR refers to a CDR with a sequence variance as compared to the corresponding CDR of a single reference polypeptide/source antibody. Accordingly, the CDRs of a single polypeptide of the invention preferably correspond to the set of CDRs of a single reference polypeptide or source antibody. Polypeptides of the invention may comprise any one or combinations of variant CDRs. For example, a polypeptide of the invention may comprise a variant CDRH1 and CDRH2. A polypeptide of the invention may comprise a variant CDRH1, variant CDRH2 and a variant CDRH3. In another example, a polypeptide of the invention may comprise a variant CDRH1, H2, H3 and L3. In another example, a polypeptide of the invention comprises a variant CDRL1, L2 and L3. Any polypeptide of the invention may further comprise a variant CDRL3. Any polypeptide of the invention may further comprise a variant CDRH3.

Polypeptides of the invention may be in a complex with one another. For example, the invention provides a polypeptide complex comprising two polypeptides, wherein each polypeptide is a polypeptide of the invention, and wherein one of said polypeptides comprises at least one, two or all of variant CDRs H1, H2 and H3, and the other polypeptide comprises a variant CDR L3. A polypeptide complex may comprise a first and a second polypeptide (wherein the first and second polypeptides are polypeptides of the invention), wherein the first polypeptide comprises at least one, two or three variant light chain CDRs, and the second polypeptide comprises at least one, two or three variant heavy chain CDRs. The invention also provides complexes of polypeptides that comprise the same variant CDR sequences. Complexing can be mediated by any suitable technique, including by dimerization/multimerization at a dimerization/multimerization domain such as those described herein or covalent interactions (such as through a disulfide linkage) (which in some constexts is part of a dimerization domain, for e.g. a dimerization domain may contain a leucine zipper sequence and a cysteine).

In another aspect, the invention provides compositions comprising polypeptides and/or polynucleotides of the invention. For example, the invention provides a composition comprising a plurality of any of the polypeptides of the invention described herein. Said plurality may comprise polypeptides encoded by a plurality of polynucleotides generated using a set of oligonucleotides comprising degeneracy in the sequence encoding a variant amino acid, wherein said degeneracy is that of the multiple codon sequences of the nonrandom codon set encoding the variant amino acid.

In one aspect, the invention provides a polynucleotide encoding a polypeptide of the invention as described herein. A polynucleotide of the invention may be a replicable expression vector comprising a sequence encoding a polypeptide of the invention.

In another aspect, the invention provides a library comprising a plurality of vectors of the invention, wherein the plurality of vectors encode a plurality of polypeptides.

The invention also provides a host cell comprising any of the polynucleotides and/or vectors of the invention described herein.

In another aspect, the invention provides a virus or viral particle(such as phage or phagemid particles) displaying a polypeptide of the invention on its surface. The invention also provides a library comprising a plurality of the viruses or viral particles of the invention, each virus or virus particle displaying a polypeptide of the invention. A library of the invention may comprise any number of distinct polypeptides (sequences), preferably at least about 1X10⁸, preferably at least about 1X10⁹, preferably at least about 1X10¹⁰ distinct sequences.

The invention also provides libraries containing a plurality of polypeptides, wherein each type of polypeptide is a polypeptide of the invention as described herein.

In another aspect of the invention provides methods for selecting for high affinity binders to specific target antigens such as growth hormone, bovine growth hormone, insulin like growth factors, human growth hormone including n-methionyl human growth hormone, parathyroid hormone, thyroxine, insulin, proinsulin, amylin, relaxin, prorelaxin, glycoprotein hormones such as follicle stimulating hormone(FSH), leutinizing hormone (LH), hemapoietic growth factor, fibroblast growth factor, prolactin, placental lactogen, tumor necrosis factors, mullerian inhibiting substance, mouse gonadotropin -associated polypeptide, inhibin, activin, vascular endothelial growth factors, integrin, nerve growth factors such as NGF-beta, insulin- like growth factor- I and II, erythropoietin, osteoinductive factors, interferons, colony stimulating factors, interleukins, bone morphogenetic proteins, LIF,SCF,FLT-3 ligand and kit-ligand.

The methods of the invention provide for libraries of polypeptides (eg. antibody variable domains) with one or more diversified CDR regions. These libraries are sorted (selected) and/or screened to identify high affinity binders to a target antigen. In one aspect, polypeptide binders from the library are selected for binding to target antigens, and for affinity. The polypeptide binders selected using one or more of these selection strategies, then, may be screened for affinity and/or for specificity (binding only to target antigen and not to non-target antigens).

A method comprises generating a plurality of polypeptides with one or more diversified CDR regions, sorting the plurality of polypeptides for binders to a target antigen by contacting the plurality of polypeptides with a target antigen under conditions suitable for binding; separating the binders to the target antigen from those that do not bind; isolating the binders; and identifying the high affinity binders. The affinity of the binders that bind to the target antigen can be determined using competition ELISA such as described herein. Optionally, the polypeptides can be fused to a polypeptide tag such as gD, poly his or FLAG which can be used to sort binders in combination with sorting for the target antigen.

Another embodiment provides a method of selecting for an antibody variable domain that binds to a target antigen from a library of antibody variable domain comprising:a) generating a library of replicable expression vectors comprising a plurality of polypeptides of any of claims 1 to 9 and 17 to 44; b)isolating polypeptide binders to a target antigen from the library by contacting the library with an immobilized target antigen under conditions suitable for binding;c)separating the polypeptide binders in the library from the nonbinders and eluting the binders from the target antigen;d) amplifying the replicable expression vectors having the polypeptide binders; and e)optionally, repeating steps a-d at least twice.

The method may further comprise: f) incubating the amplified replicable expression vectors comprising polypeptide binders with a concentration of labelled target antigen in the range of 0.1 nM to 1000 nM under conditions suitable for binding to form a mixture; g) contacting the mixture with an immobilized agent that binds to the label on the target antigen; h) separating the polypeptide binders bound to labelled target antigen and eluting the polypeptide binders from the labelled target antigen; i) amplifying replicable expression vectors comprising the polypeptide binders; and j) optionally, repeating steps f) to i) at least twice, using a lower concentration of labelled target antigen each time. Optionally, the method may comprise adding an excess of unlabelled target antigen to the mixture and incubating for a period of time sufficient to elute low affinity binders from the labelled target antigen.

Another embodiment provides a method of isolating or selecting for high affinity binders to a target antigen from a library of replicable expression vectors comprising: a) generating a library of replicable expression vectors comprising a plurality of polypeptides of any of claims 1-9 and 17-44; b) contacting the library with a target antigen in a concentration of at least about 0.1 nM to 1000 nM to isolate polypeptide binders to the target antigen; c) separating the polypeptide binders from the target antigen and amplifying the replicable expression vector comprising the polypeptide binders; d) optionally, repeating steps a-c at least twice, each time with a lower concentration of target antigen to isolate polypeptide binders that bind to lowest concentration of target antigen; e) selecting the polypeptide binder that binds to the lowest concentration of the target antigen for high affinity by incubating the polypeptide binders with several diffent dilutions of the target antigen and determining the IC50 of the polypeptide binder; and f) identifying a polypeptide binder that has an affinity for the target antigen of about 0.1 nM to 200 nM.

Another embodiment provides an assay for selecting polypeptide binders from a library of replicable expression vectors comprising a plurality of polypeptides of any of claims 1 to 9 and 17 to 44 comprising:a)contacting the library with a concentration of labelled target antigen in a concentration range of 0.1 nM to 1000 nM, under conditions suitable for binding to form a complex of a polypeptide binders and the labelled target antigen;b)isolating the complexes and separating the polypeptide binders from the labelled target antigen;c)amplifying the replicable expression vector comprising the polypeptide binders;d)optionally, repeating steps a-c at least twice, each time using a lower concentration of target antigen. Optionally, the method may further comprise adding an excess of unlabelled target antigen to the complex of the polypeptide binder and target antigen. In a preferred embodiment, the steps of the method are repeated twice and the concentrations of target in the first round of selection is about 100 nM to 250 nM, and in the second round of selection is about 25 nM to 100 nM, and in the third round of slection is about 0.1 nM to 25 nM.

The invention also includes a method of screening a library of replicable expression vectors comprising a plurality of polypeptides of any of claims 1 to 9 and 17-44 comprising: a) incubating first a sample of the library with a concentration of a target antigen under conditions suitable for binding of the polypeptides to the target antigen; b) incubating a second sample of the library without a target antigen; c) contacting each of the first and second sample with immobilized target antigen under conditions suitable for binding of the polypeptide to the immobilized target antigen; d) detecting the amount of the bound polypeptides to immobilized target antigen for each sample; e) determining the affinity of the polypeptide for the targ et antigen by calculating the ratio of the amounts of bound polypeptide from the first sample over the amount of bound polypeptide from the second sample.

The libraries generated as described herein may also be screened for binding to a specific target and for lack of binding to nontarget antigens. In one aspect, another embodiment provides a method of screening for an antibody variable domain that binds to a specific target antigen from a library of antibody variable domain comprising: a) generating a library of replicable expression vectors comprising a plurality of polypeptides of any of claims 1 to 9 and 17 to 44; b) contacting the library with a target antigen and at least one nontarget antigen under conditions suitable for binding; c) separating the polypeptide binders in the library from the nonbinders; d) identifying the binders that bind to the target antigen and do not bind to the nontarget antigen; e) eluting the binders from the target antigen;and f) amplifying the replicable expression vectors comprising the polypeptide binder that bind to a specific antigen.

Combinations of any of the sorting/ selection methods described above may be combined with the screening methods. For example, in one embodiment, polypeptide binders are first selected for binding to immobilized target antigen. Polypeptide binders that bind to the immobilized target antigen can then be amplified and screened for binding to the target antigen and for lack of binding to nontarget antigens. Polypeptide binders that bind specifically to the target antigen are amplified. These polypeptide binders can then selected for higher affinity by contact with a concentration of a labelled target antigen to form a complex, wherein the concentration range of labelled target antigen is from about 0.1 nM to about 1000 nM, the complexes are isolated by contact with an agent that binds to the label on the target antigen. The polypeptide binders are then eluted from the labled target antigen and optionally, the rounds of selection are repeated, each time a lower concentration of labelled target antigen is used. The high affinity polypeptide binders isolated using this selection method can then be screened for high affinity using for example, the solution phase ELISA assay as described in Example 8 or the spot competition ELISA assay as described in Example 9.

### BRIEF DESCRIPTION OF THE FIGURES

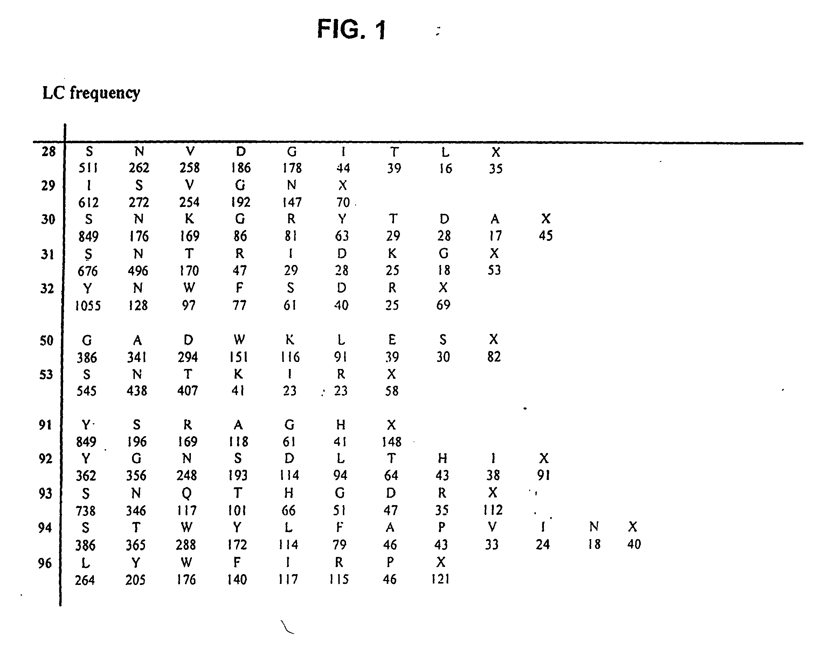

Figure 1 shows the frequency of amino acids (identified by single letter code) in human antibody light chain CDR sequences from the Kabat database. The frequency of each amino acid at a particular amino acid position is shown starting with the most frequent amino acid at that position at the left and continuing on to the right to the least frequent amino acid. The number below the amino acid represents the number of naturally occurring sequences in the Kabat database that have that amino acid in that position.

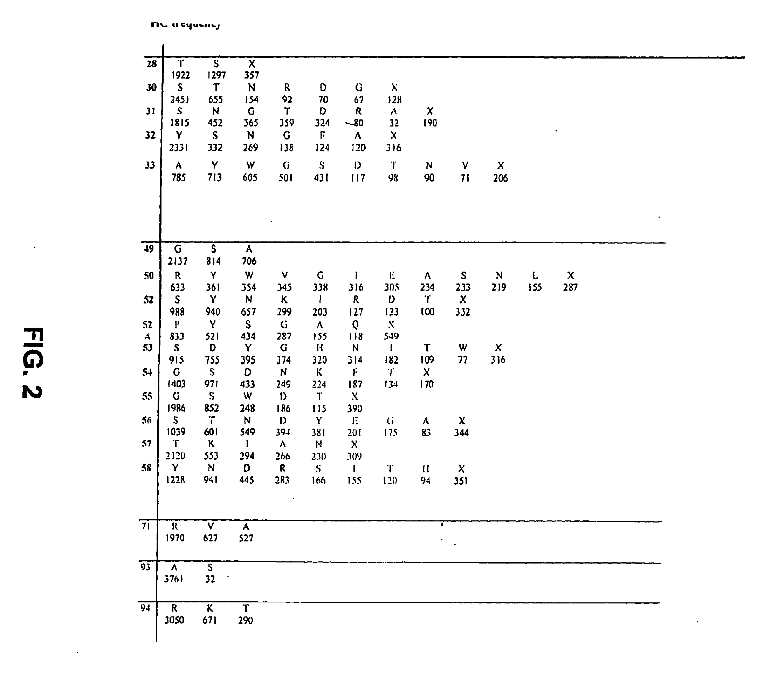

Figure 2 shows the frequency of amino acids (identified by single letter code) in human antibody heavy chain CDR sequences from the Kabat database. The frequency of each amino acid at a particular amino acid position is shown starting with the most frequent amino acid at that position at the left and continuing on to the right to the least frequent amino acid. The number below the amino acid represents the number of naturally occurring sequences in the Kabat database that have that amino acid in that position. Framework amino acid positions 71, 93 and 94 are also shown.

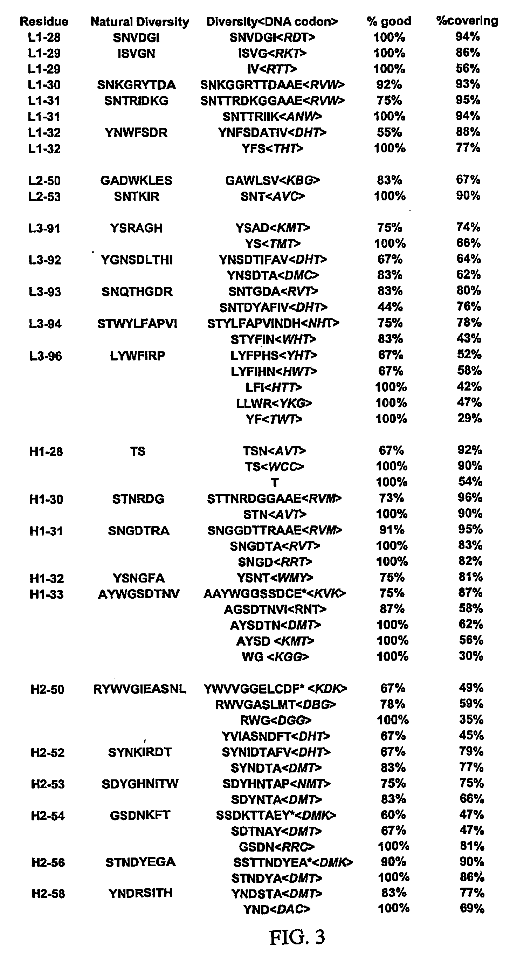

Figure 3 shows illustrative embodiments of suitable codon set design for amino acid positions in CDRL1, CDRL2, CDRL3, CDRH1 and CDRH2. The codon sets are identified by three capital letters in italics and are bracketed by < and >, e.g. <*RDT>* The amino acids encoded by that codon set are indicated by single letter code under the column labelled Diversity<DNA . The column labelled Natural Diversity shows the most commonly occuring amino acids at those positions in the naturally occurring antibody variable domains in the Kabat database. The % good is the % of amino acids that are encoded by the codon set that are target amino acids for that position. The % covering is the % of natural occurring antibodies in the Kabat database that have the amino acids encoded by the codon set at that position.
Figures 4A & B &C show illustrative embodiments of designed diversity in CDRH3. The different oligonucleotides encode for diversity at amino acid positions in CDRH3 as well as diversity in sequence length. The oligonucleotides are identified as F59, F63, and F64 etc in the left hand column. The amino acid sequence at each amino acid position for CDRH3 for each oligonucleotide is shown. The CDRH3 sequence in the source antibody 4D5 is shown across the top: S₉₃, R₉₄, W₉₅, G₉₆, G₉₇, D₉₈, G₉₉, F₁₀₀ , Y₁₀₀ₐ, A_{100b} , M_{100c} ,D₁₀₁ , and Y₁₀₂. Amino acid positions 93 and 94 are considered framework positions. In some embodiments, certain positions may have a fixed amino acid shown in single letter code, e.g. position 93 is S(serine); amino acid position 94 may be R/K/T ( arginine/lysine /threonine); amino acid position 100a may be G/S/A/W(glycine/serine/alanine/tryptophan). Other amino acid positions are diversified using codon sets identified by three capital letters in italics, e.g. *DVK, NVT , DSG, KSG.* The length of the CDRH3 is indicated at the right column. The lengths of the CDRH3 regions varied from 7 to 15. The diversity of the library generated with the strategy shown for each oligonucleotide is also shown on the right. A single oligonucleotide may be used or oligonucleotides may be pooled to generate a library.

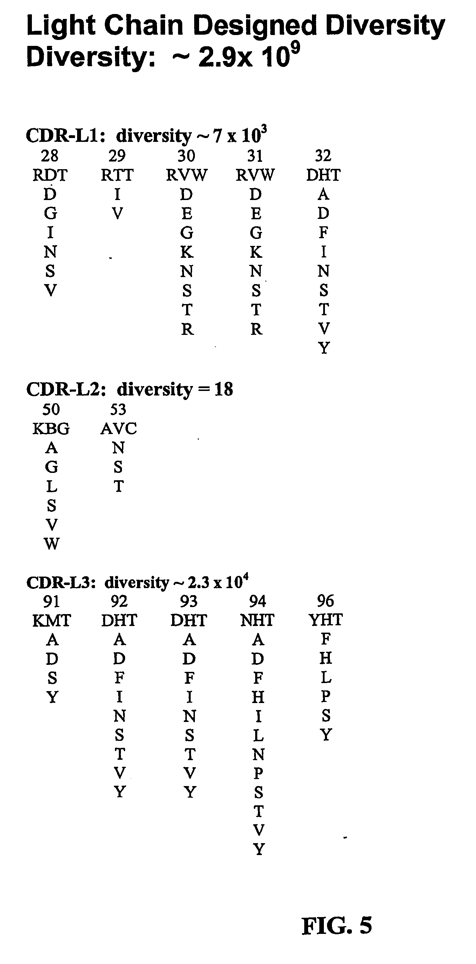

Figure 5 shows an illustrative embodiment of designed diversity for CDR's L1 and L2 and L3. The codon sets for each position is shown. The amino acids (in single letter code) encoded by the codon set at each position are shown below in a column. The diversity generated with this design results in a library with 2.9 x 10⁹ sequences.

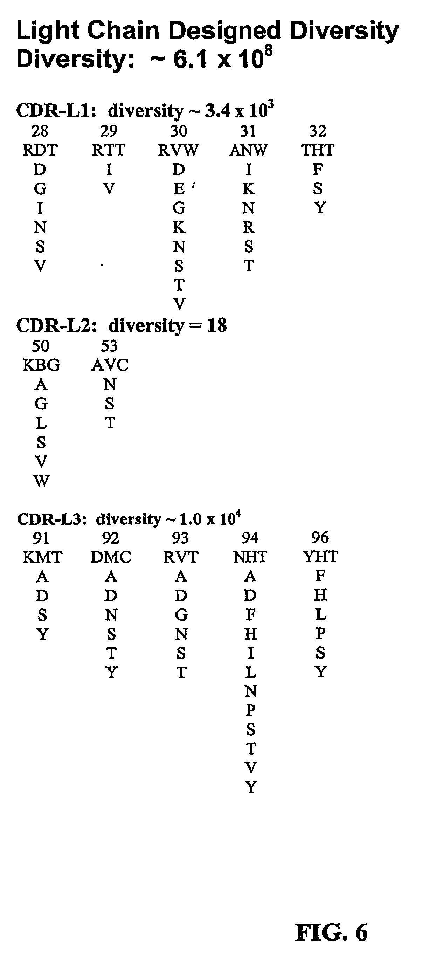

Figure 6 shows an illustrative embodiment of designed diversity using nonrandom codon sets for amino acid positions in CDRL1, L2 and L3. The amino acids (in single letter code) encoded by the codon set at each position are shown below in a column. The diversity generated with this design results in 6.1 x 10⁸ sequences.

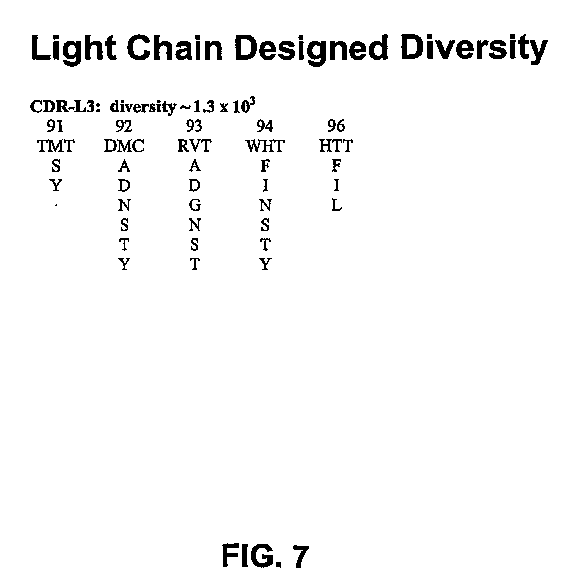

Figure 7 shows an illustrative embodiment of designed diversity using nonrandom codon sets at amino acid positions in CDRL3. The amino acids (in single letter code) encoded by the codon set at each position are shown below in a column.

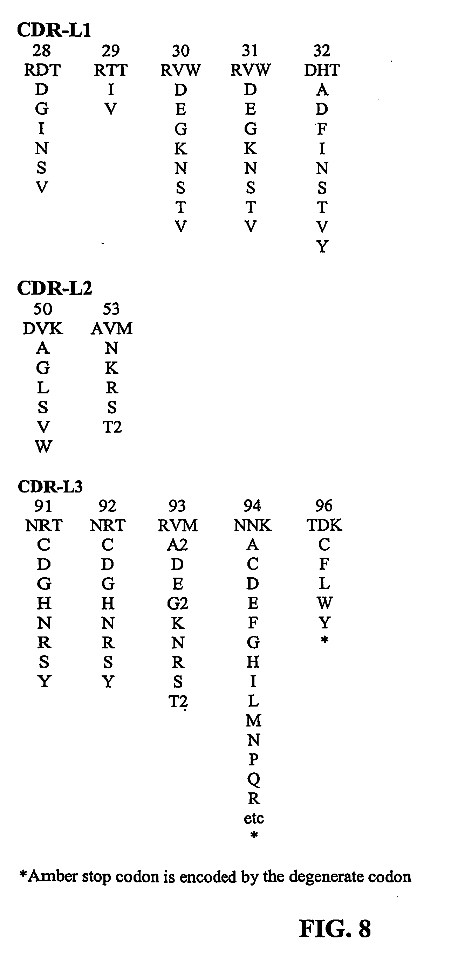

Figure 8 shows an illustrative embodiment of designed diversity using nonrandom codon sets for CDRs L1, L2 and L3. At some positions, the codon set may encode an increased proportion of one or more amino acids. For example, at position 93 in CDRL3, codon set *RVM* encodes an increased proportion of alanine (A2), glycine (G2) and threonine (T2). The amino acids (in single letter code) encoded by the codon set at each position are shown below in a column.

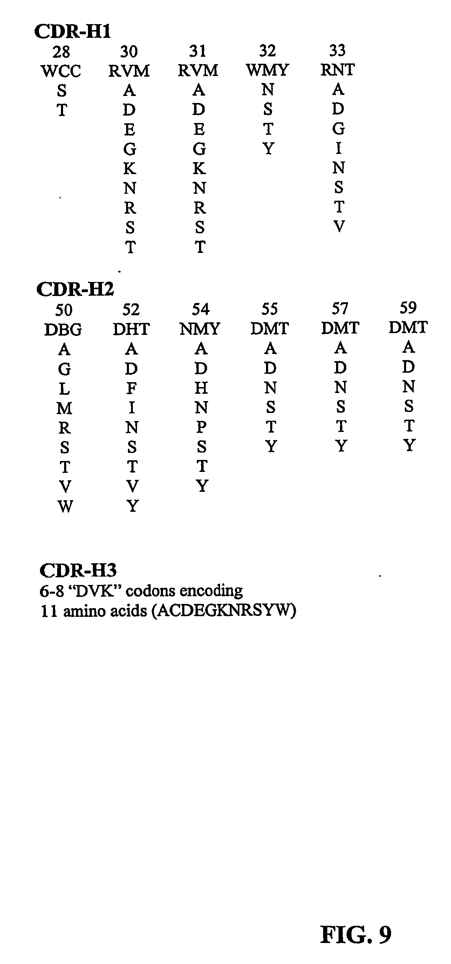

Figure 9 shows an illustrative embodiment of designed diversity using nonrandom codon sets at amino acid positions in CDRH1, H2 and H3. The amino acids (in single letter code) encoded by the codon set at each position are shown below in a column.

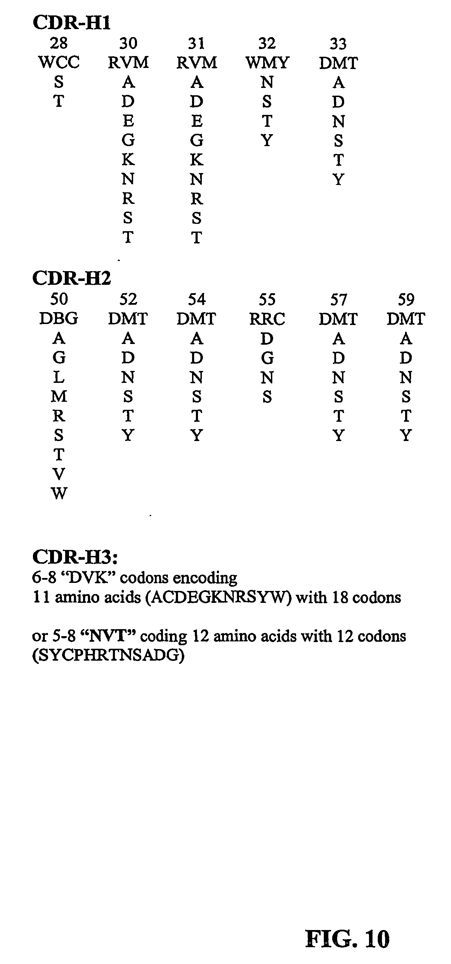

Figure 10 shows an illustrative embodiment of designed diversity using nonrandom codon set at amino acid positions in CDRs H1, H2 and H3. The amino acids (in single letter code) encoded by the codon set at each position are shown below in a column.

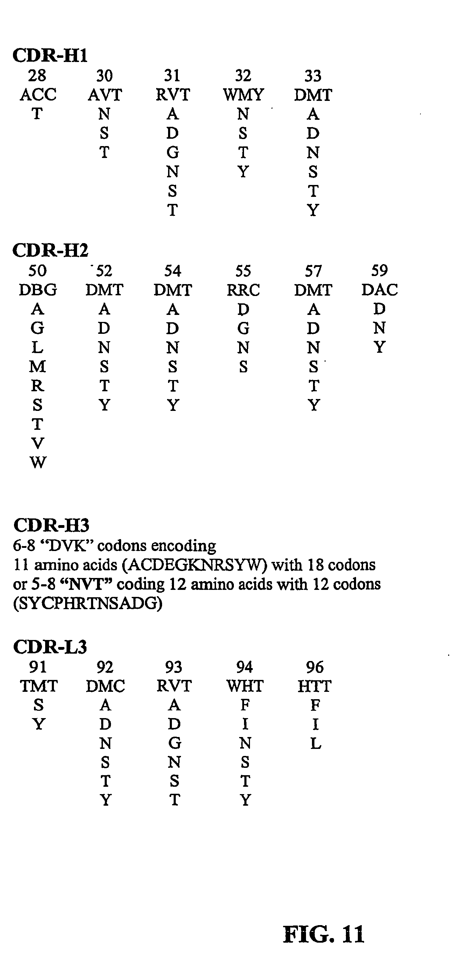

Figure 11 shows an illustrative embodiment of designed diversity using nonrandom codon sets at amino acid positions in CDRs in H1, H2, H3 and L3. The amino acids (in single letter code) encoded by the codon set at each position are shown below in a column.

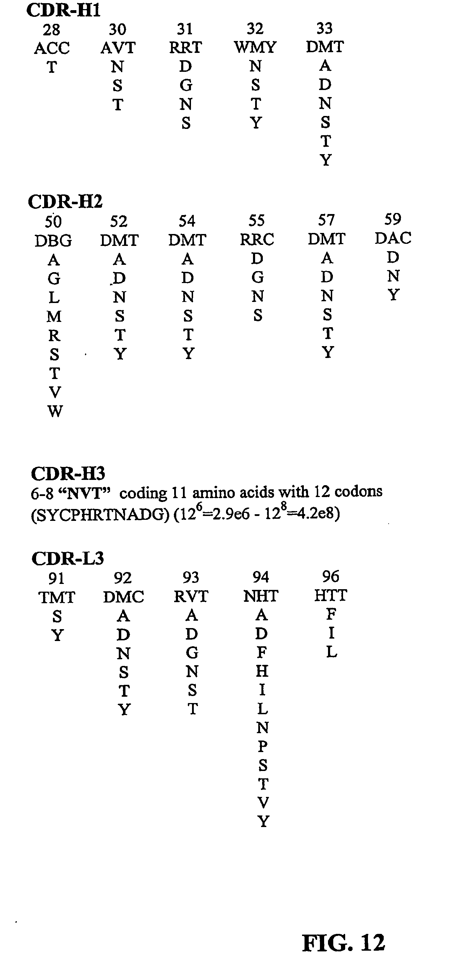

Figure 12 shows an illustrative embodiment of designed diversity using nonrandom codon sets at amino acid positions in CDRs in H1, H2, H3 and L3. The amino acids(in single letter code) encoded by the codon set at each position are shown below in a column.

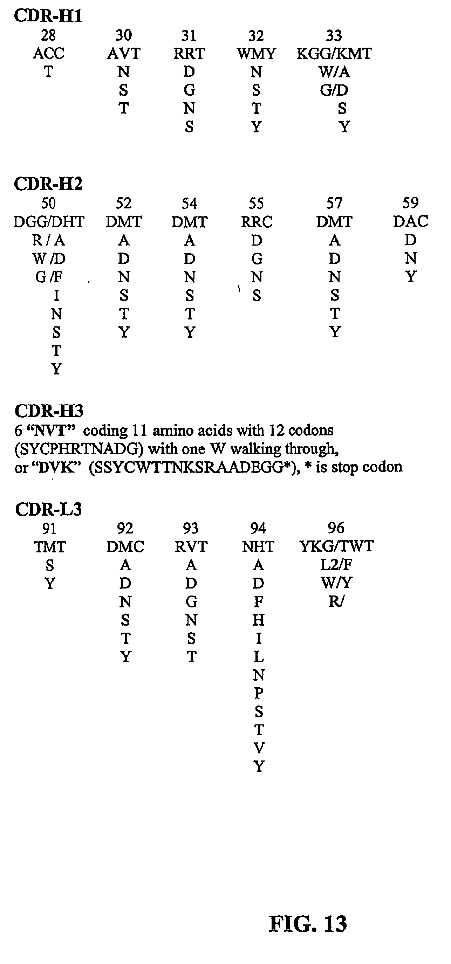

Figure 13 shows an illustrative embodiment of designed diversity using nonrandom codon sets at amino acid positions in CDRs H1, H2, H3 and L3. The amino acids (in single letter code) encoded by the codon set at each position are shown below in a column.

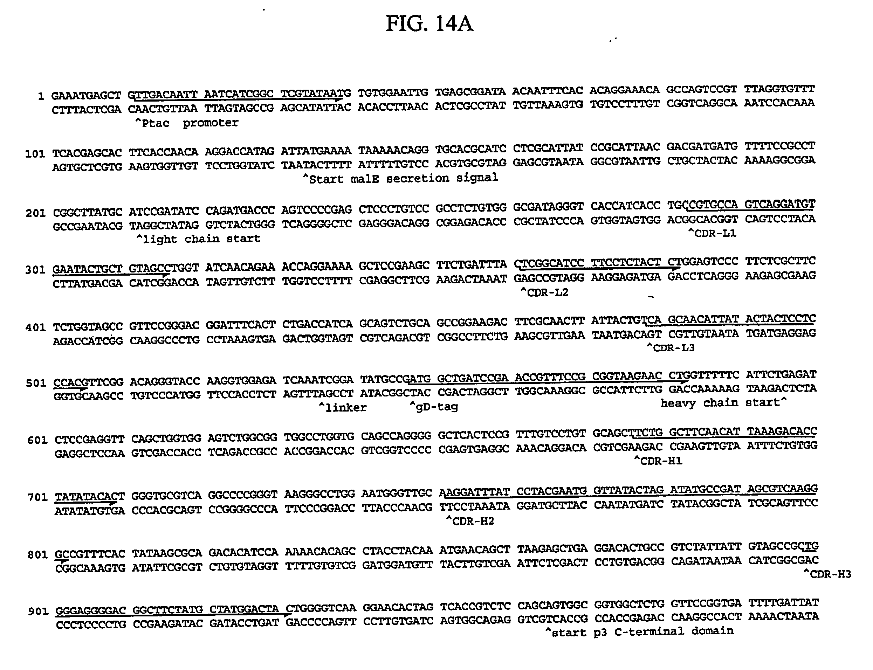

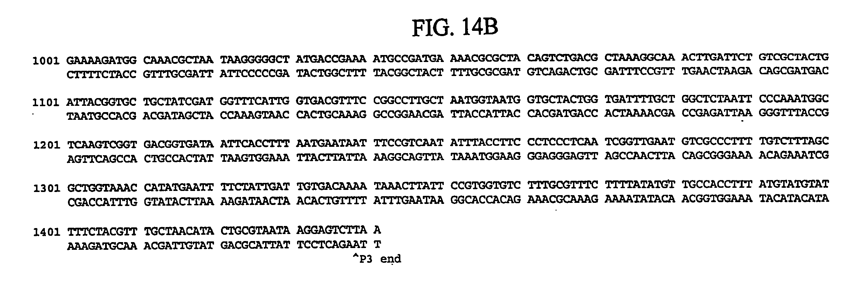

Figures 14 A & B shows nucleotide sequence of Ptac promoter driver cassette for display of ScFv (SEQ ID NO: 23). Sequences encoding *malE* secretion signal, humanized antibody 4D5 light chain variable domain, linker, gD tag, humanized 4D5 heavy chain variable domain, and C-terminal domain of p3 (cP3) are indicated.

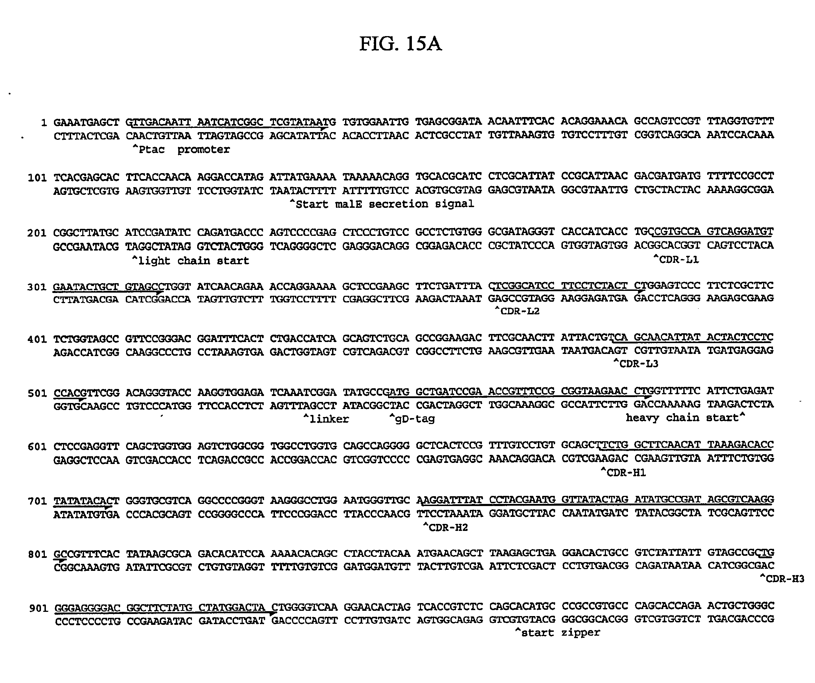

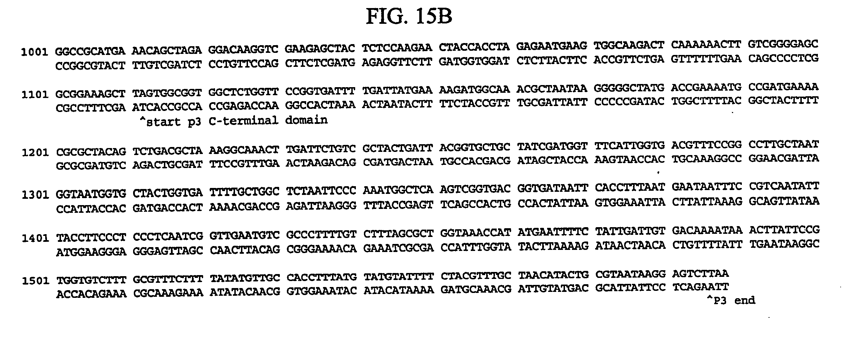

Figures 15 A & B shows the DNA sequence of the Ptac promoter driver cassette for display ScFv-zip (SEQ ID NO: 24). Sequences encoding *malE* secretion signal, humanized 4D5 light chain variable domain, linker, gD tag, humanized 4D5 heavy chain variable domain, zipper sequence, and C-terminal domain p3 (cP3) are indicated.

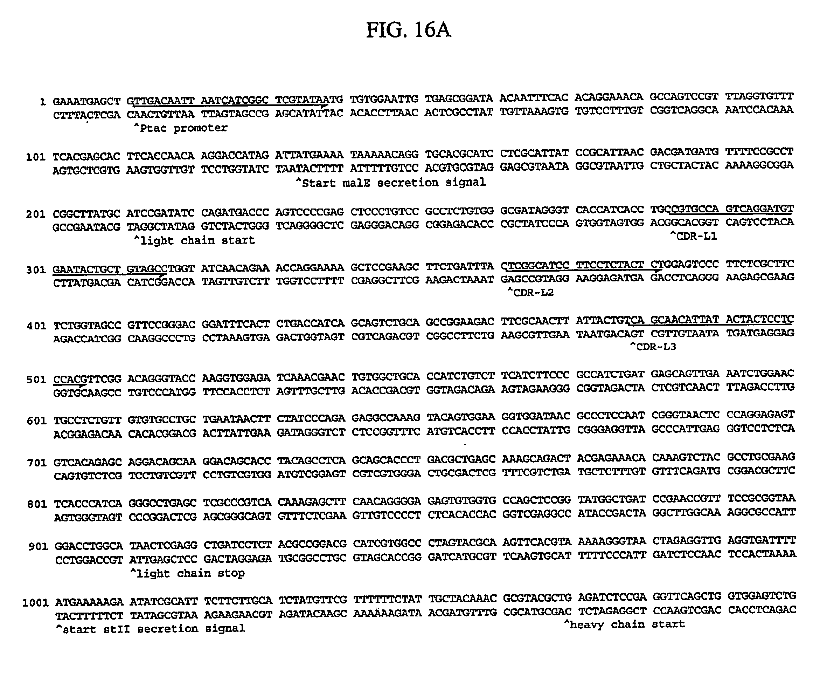

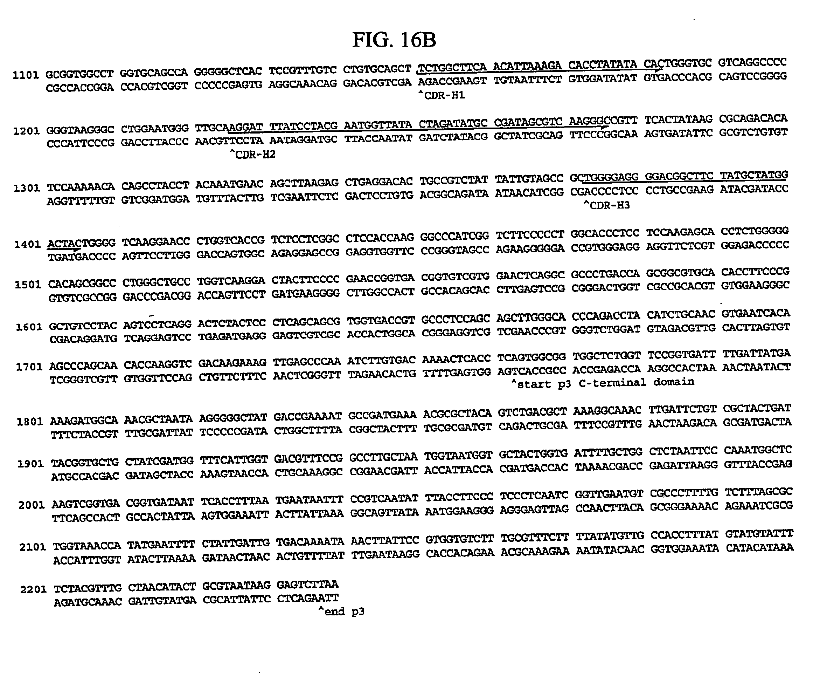

Figures 16 A & B shows DNA sequence of the Ptac promoter driven cassette for display of Fab (SEQ ID NO: 25). Two open reading frames are indicated. The first open reading frame encodes a *malE* secretion signal, humanized 4D5 light chain variable and constant domain. The second open reading frame encodes stII secretion signal, humanized heavy chain variable domain, humanized 4D5 heavy chain first constant region (CH1) and C-terminal domain of p3.

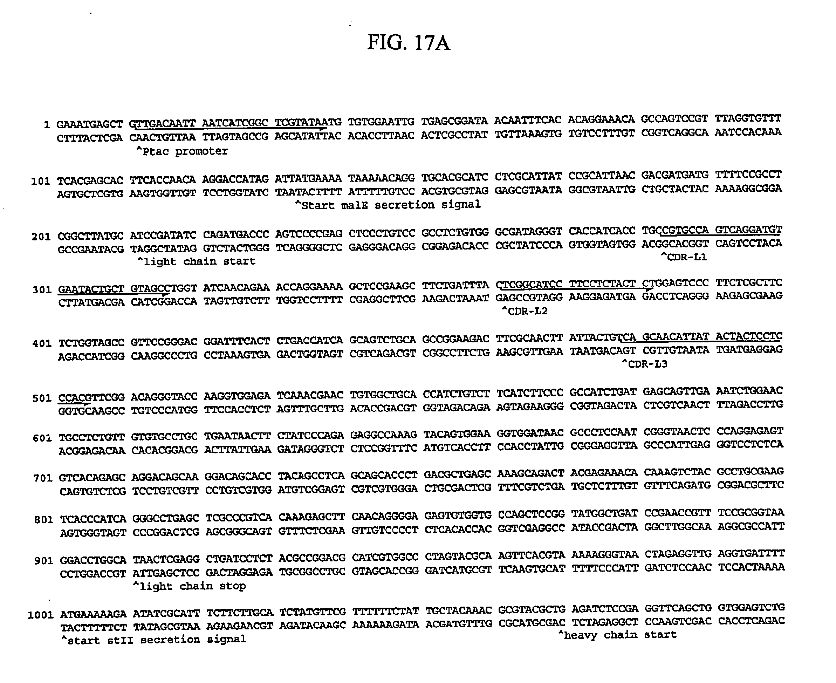

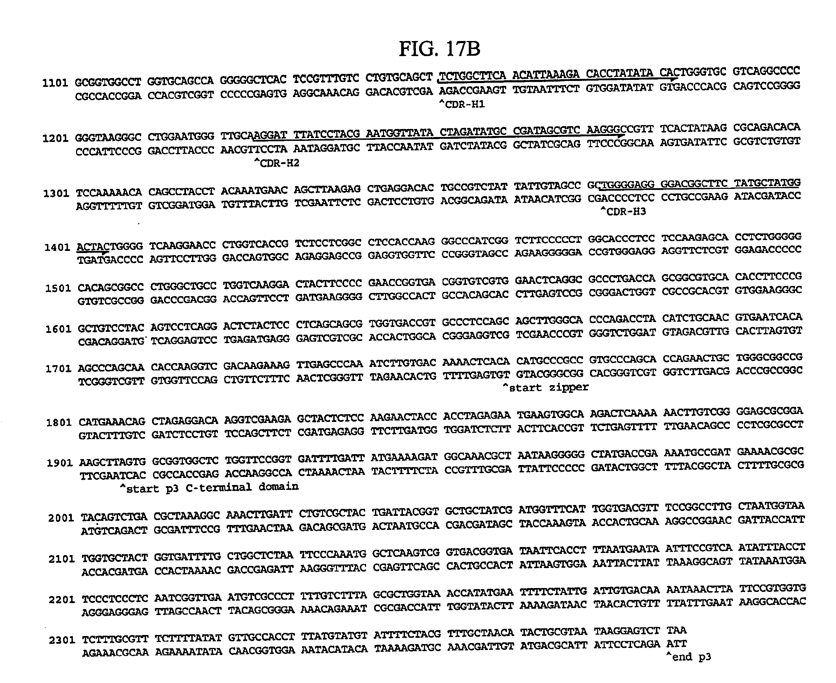

Figures 17 A & B shows the DNA sequence of Ptac promoter driven cassette for display of Fab-zip (SEQ ID NO: 26). Two open reading frames are indicated. The first open reading frame encodes a *malE* secretion signal, humanized 4D5 light chain variable and constant domain. The second open reading frame encodes a stII secretion signal, humanized 4D5 heavy chain variable domain, humanized 4D5 heavy chain first constant domain (CH1), zipper sequence, and C-terminal of p3 (cP3).

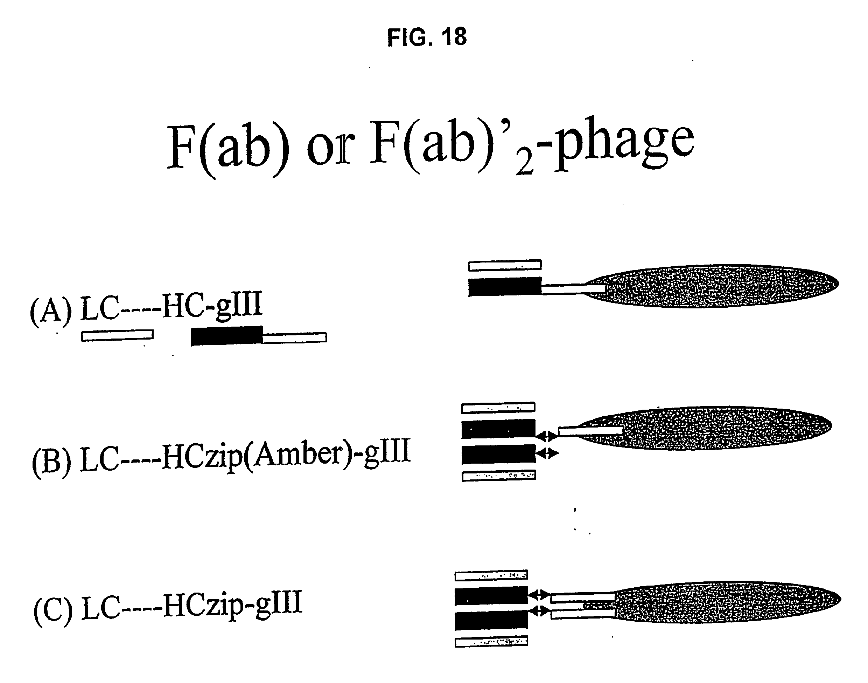

Figure 18 shows a schematic representation of display of different constructs including F(ab) and F(ab')₂. (A) shows a Fab with a light chain, and a heavy chain variable and CH1 domain fused to at least a portion of a viral coat protein; (B) shows a F(ab')2 with two light chains, and one heavy chain with a dimerization domain(zip) fused to at least a portion of the viral coat protein; an amber stop codon is present after the dimerization domain and (C) shows a F(ab')2 with two light chains, and both heavy chain variable and CH1 domains, each with a dimerization domain, and each fused to at least a portion of the viral coat protein

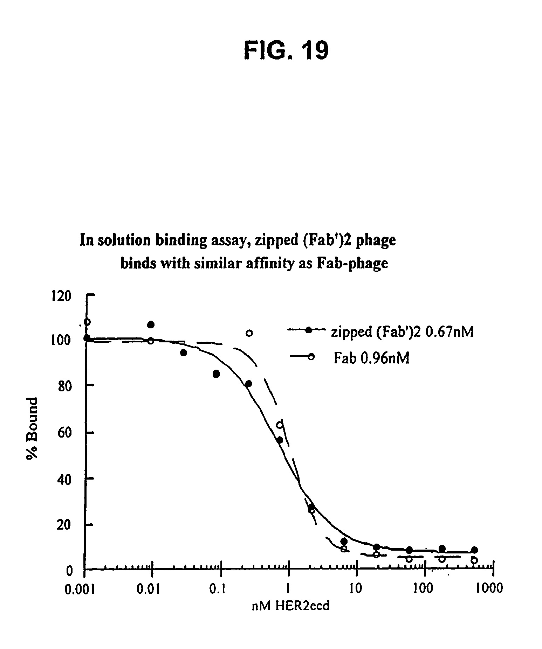

Figure 19 shows a graph of the % bound of Fab phage constructs in the presence of increasing amounts of HER-2ecd (target antigen). The constructs are Fab phage (-o-) or zipped F(ab')₂ phage (-•-). The F(ab) or zipped F(ab')₂ phage, each was incubated with increasing concentrations of Her-2ECD (0.001 to 1000 nM) in solution for 5 hours at 37°C. The unbound phage was captured with plates coated with Her-2ECD and measured with HRP-anti-M13 conjugate.

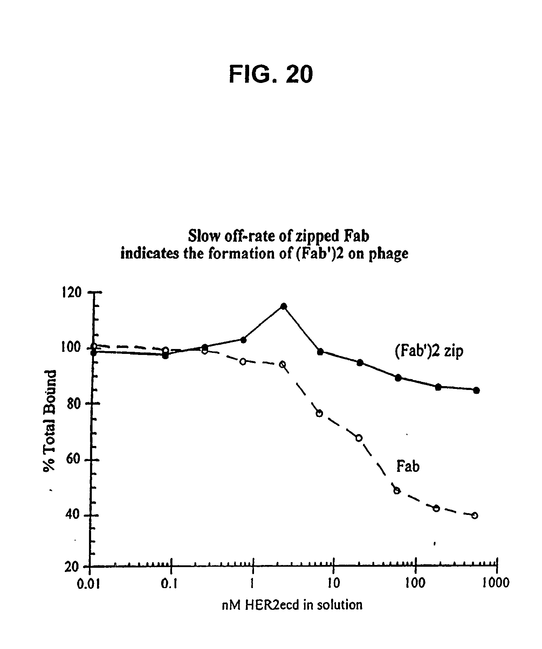

Figure 20 shows the differences in off rate between Fab (-o-) or zipped F(ab')₂
   (-•-) phage. Serial dilutions of Her-2ECD (0.01 nm to 1000 nM) were added to Fab or zipped F(ab')₂ phage bound to Her-2ECD coated wells. The phage remaining bound to the plate was quantified using HRP-anti-M13 conjugate. The relative proportion of remaining phage bound as a percentage was calculated by dividing OD at a particular Her-2ECD concentration with OD in absence of Her-2ECD.

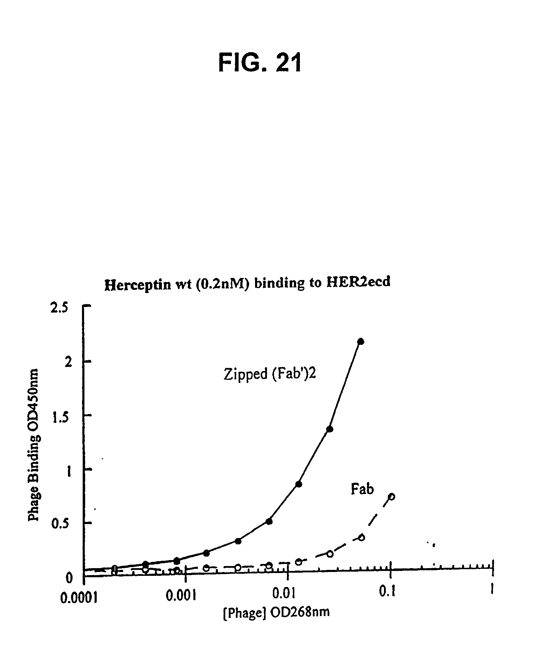

Figure 21 shows the differences in the amount of phage F(ab) phage (-o-) or zipped F(ab')₂ (-•-) that is required to give detectable binding on a ligand coated support by standard phage ELISA. Differing concentrations of phage were diluted and the binding signal on Her-2ECD coated plates was detected with HRPanti-M13 measured at an O.D.of 450nm.

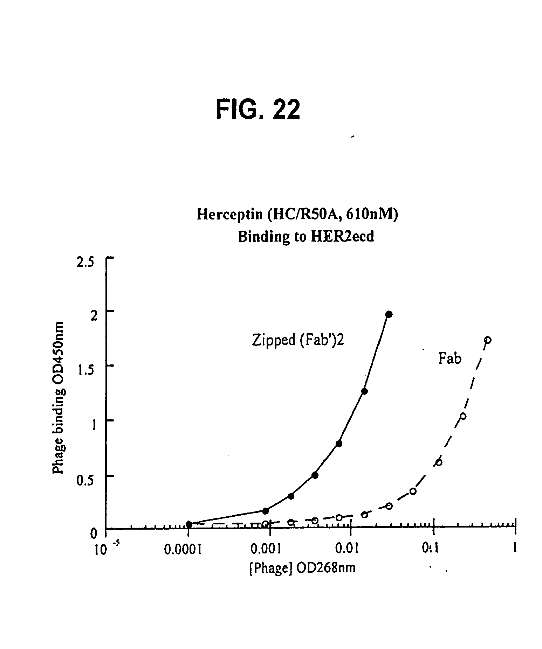

Figure 22 shows the ability to detect a low affinity binder using divalent display. A humanized 4D5 mutant was prepared with arginine 50 changed to alanine (R50A) in both F(ab) phage (-o-) or zipped F(ab')₂ (-•-) format. The phage was diluted and the binding on Her-2ECD coated plates was detected with HRP anti-M13.

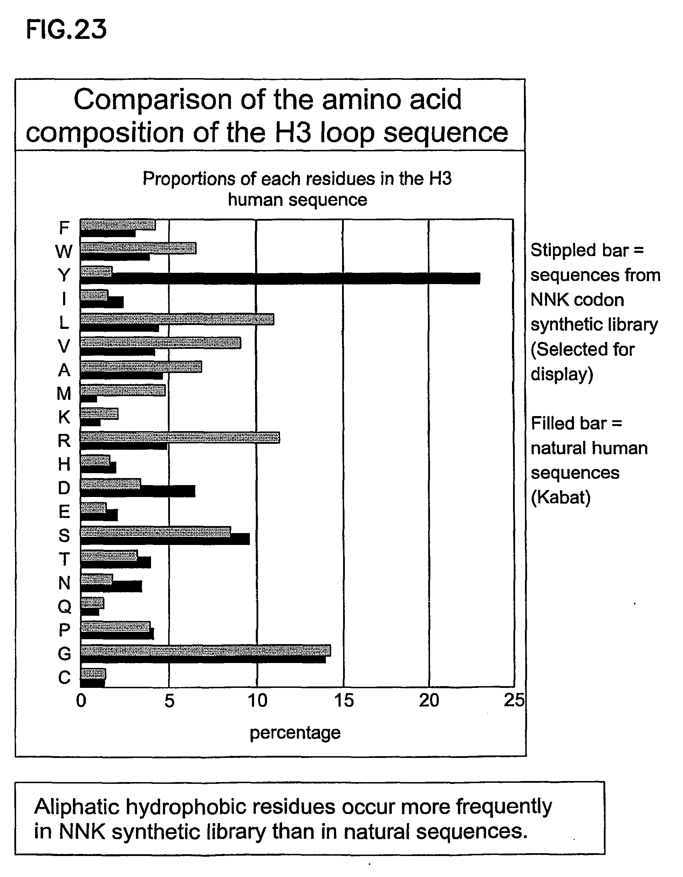

Figure 23 shows the comparison of the frequency of amino acid types in CDRH3 regions in naturally occurring antibodies (solid bar) and in antibody variable domains with diversity generated with *NNK* codon sets (stippled bar). Amino acids are grouped as follows: phenyalanine (F),trytophan (W) and tyrosine (Y) are aromatic amino acids; isoleucine (I) , leucine (L), valine (V), alanine(A), and methionine (M) are aliphatic, lysine (K), arginine(R), and histidine (H) are basic; aspartic acid (D) and glutamic acid (E) are acidic; serine (S), threonine(T), asparagine (N), and glutamine (Q) are polar; and proline (P), glycine (G), and cysteine (C) are conformational.

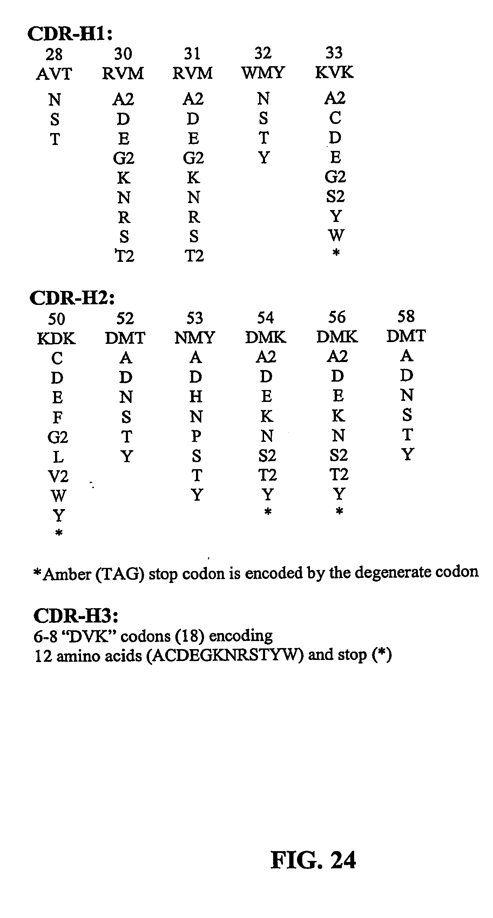

Figure 24 shows an illustrative embodiment of designed diversity using nonrandom codon sets for amino acid positions in CDRs H1, H2 and H3. The amino acids(in single letter code) encoded by the codon set at that position are shown below in a column.

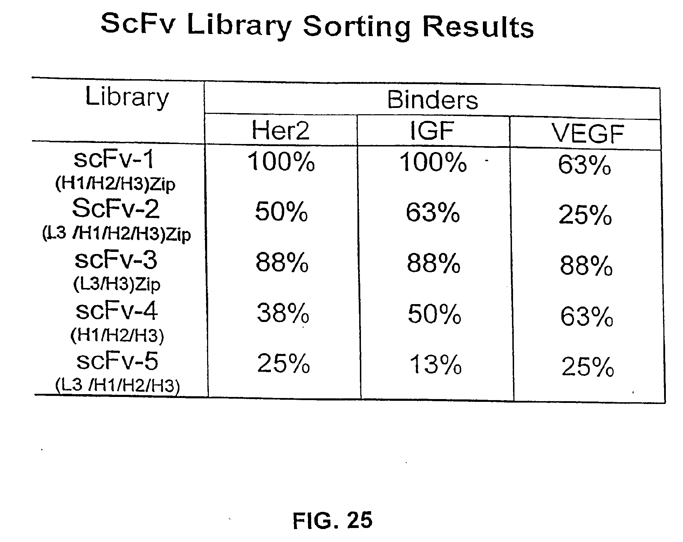

Figure 25 shows the results from sorting ScFv libraries for binding to target antigens Her-2, IGF-1 and mVEGF. The ScFv-1 library was generated with a vector having a zipper sequence and with diversity in CDRH1, H2 and H3. The ScFv-2 was generated with a vector having a zipper sequence and with diversity in H1, H2, H3 and L3. The ScFv-3 was generated with a vector with a zipper sequence and with diversity in H3 and L3. The ScFv-4 has no zipper region and the CDR diversity was generated in CDR H1, H2 and H3. The ScFv-5 was generated with no zipper sequence and with CDR diversity in CDR's H1, H2, H3 and L3. The results for each library after three rounds of sorting are shown as a % of clones binding to a target antigen.

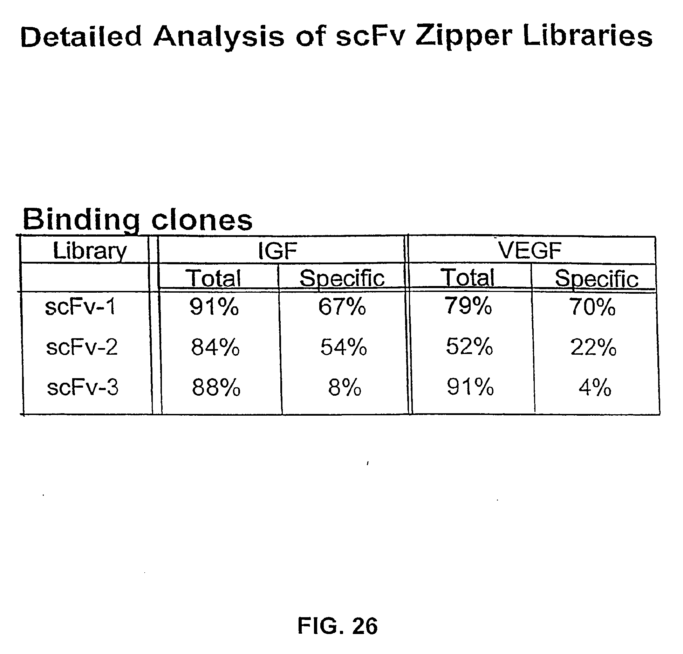

Figure 26 shows the results of specific binders isolated from ScFv-1, ScFv-2 and ScFv-3 libraries. Phage clones from 3 rounds of selection against IGF-1 or mVEGF were analyzed for specific binding by ELISA assays using both IGF1 and mVEGF. Clones that bound to the target for which they were selected and not to the other antigen were identified as specific. The percentage of clones from each selection that bound targets (Total) and the percentage of clones that bound only the target against which they were selected (specific) are shown.

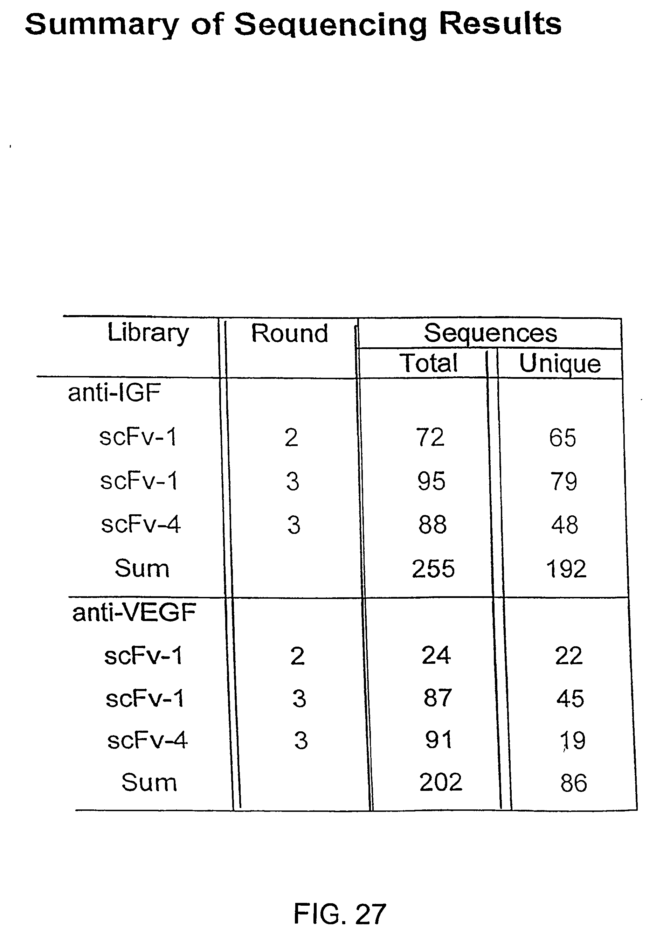

Figure 27 shows the total number of sequences and of those sequences the number of unique sequences of anti-VEGF or anti-IGF antibody variable domains identified from each library of scFv-1 and scFv-4 after two or three rounds of sorting.

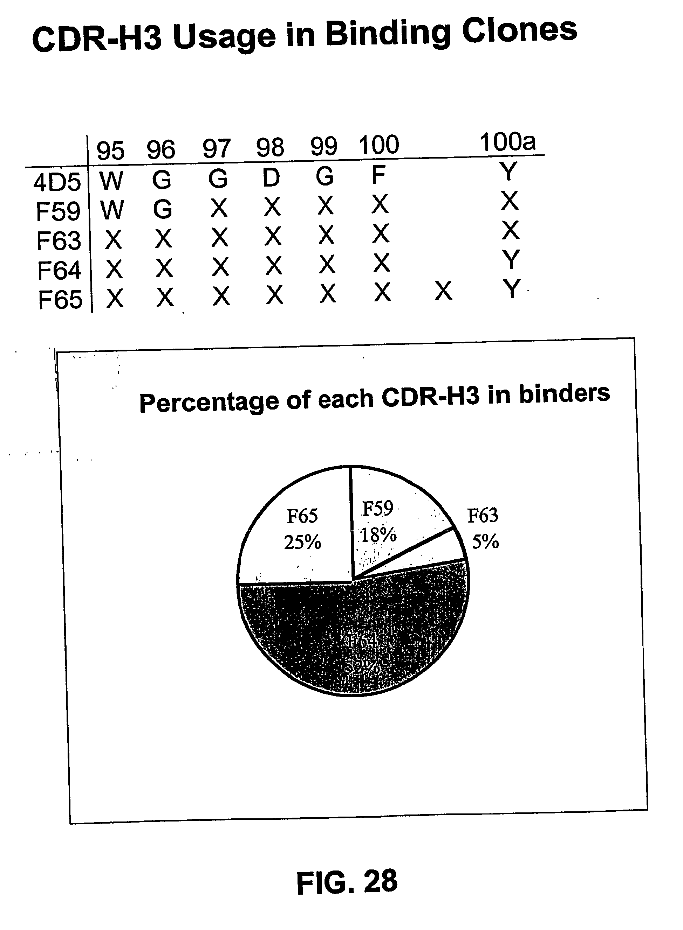

Figure 28 shows an example of CDRH3 codon/amino acid usage distribution in one set of binders. "X" denotes codon set usage as shown for each oligonucleotides in Figure 4. The percentage of the CDR-H3 design of each oligonucleotides in the sequences of binders isolated from the library are shown.

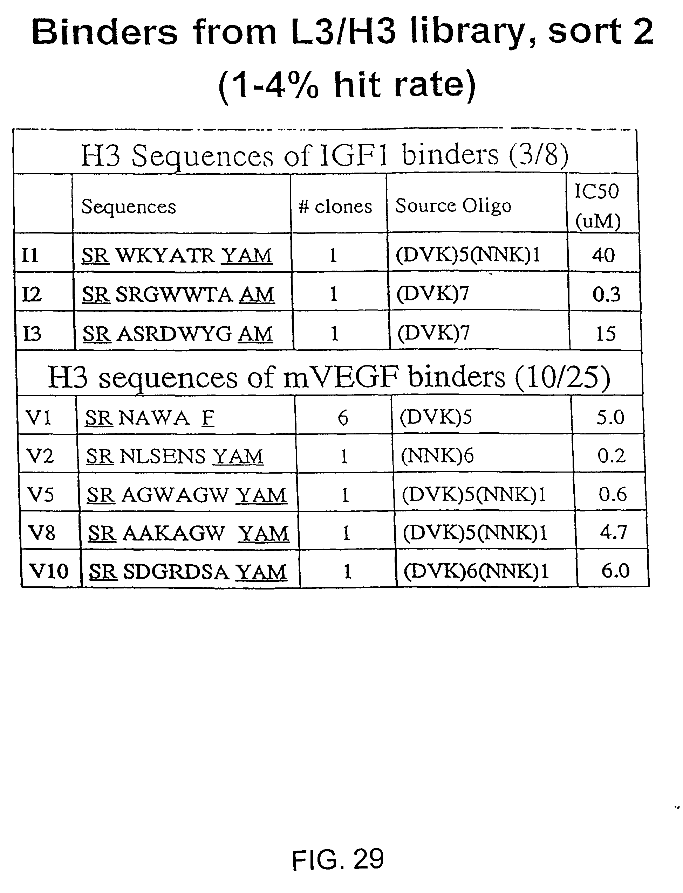

Figure 29 shows H3 sequences and affinities of some anti-IGF1 and anti-VEGF binders from a F(ab')₂ L3/H3 library. Underlined residues represent residues that were fixed in the source library of the clones.

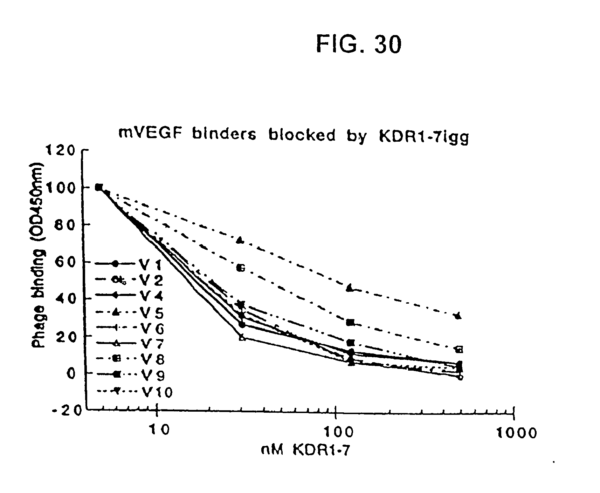

Figure 30 shows the identity of the epitope bound by some of the clones. Murine VEGF was coated on plate and phage clones competitively inhibited in the present of KDR-7igg were identified. Clones V1 (-•-), V2(-o-), V4 (-◆ -), V5 (-▲-), V6 (-+ -), V7 (- △ -), V8 (- -), V9 (-■-), V10 (-▼-) were tested.

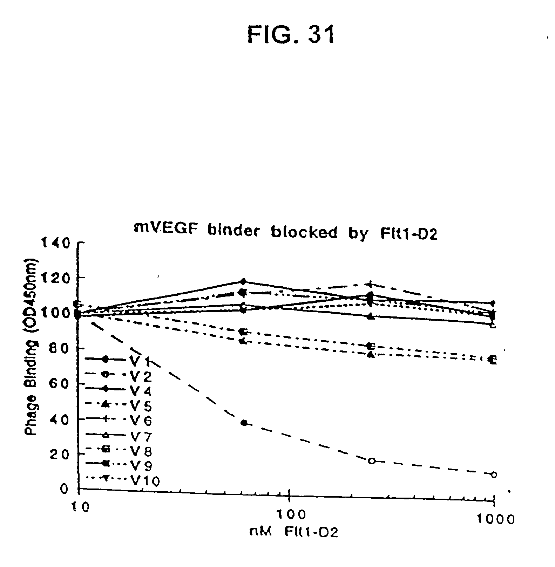

Figure 31 shows the identity of the epitope bound by some of the clones. Murine VEGF was coated on a plate and phage clones competitively inhibited in the present of F1t1-D2 were identified. Clones Vl (-•-), V2(-o-), V4 (- ◆ -), V5 (- ▲ -), V6 (-+-), V7 (- △ -), V8 (- -), V9 (-■-), V10 (-▼-) were tested.

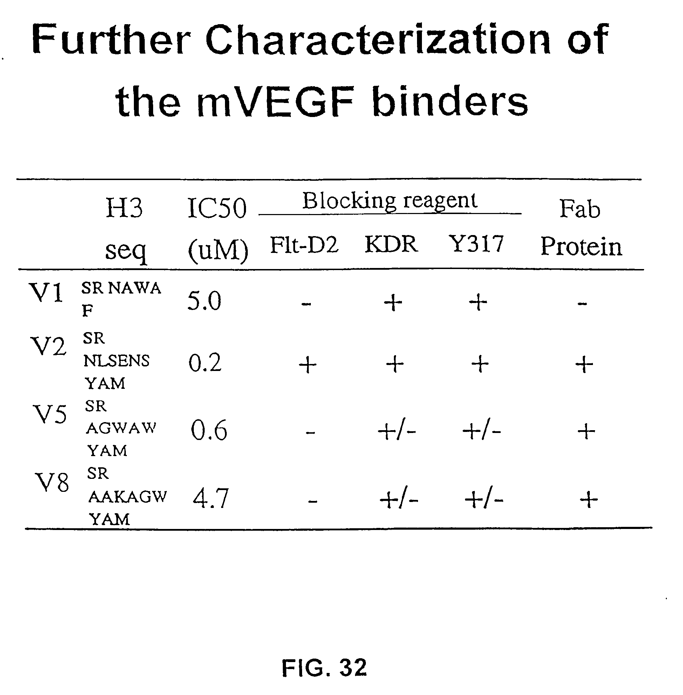

Figure 32 shows the Fab polypeptide phage CDRH3 amino acid sequences, affinities, epitope specificity and production of Fab in cell culture for clones V1, V2, V3, and V8.

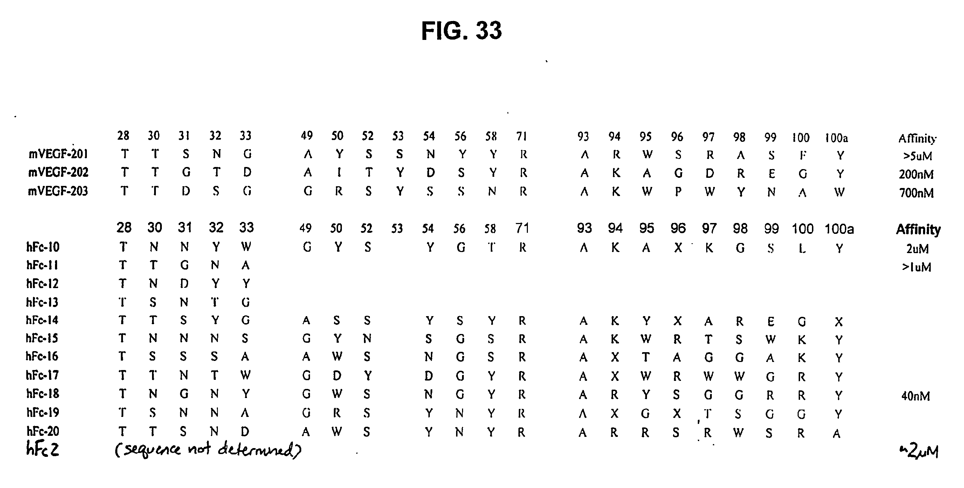

Figure 33 shows the heavy chain variable domain CDR amino acid sequences and affinities of binders to mVEGF and human Fc receptor from a F(ab) or F(ab')₂ library. The amino acid sequence of heavy chain framework positions 49, 71, 93 and 94 are also shown.

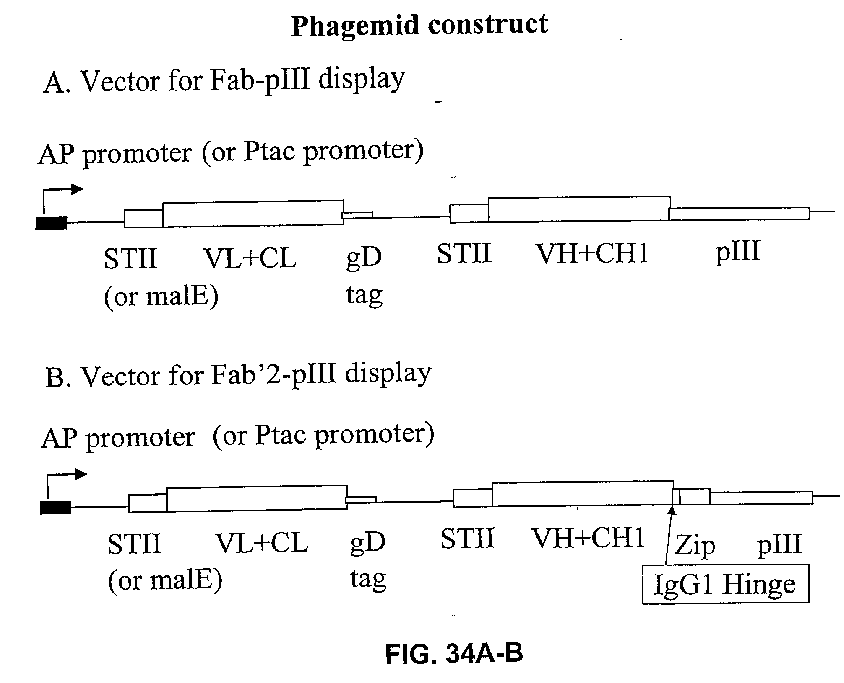

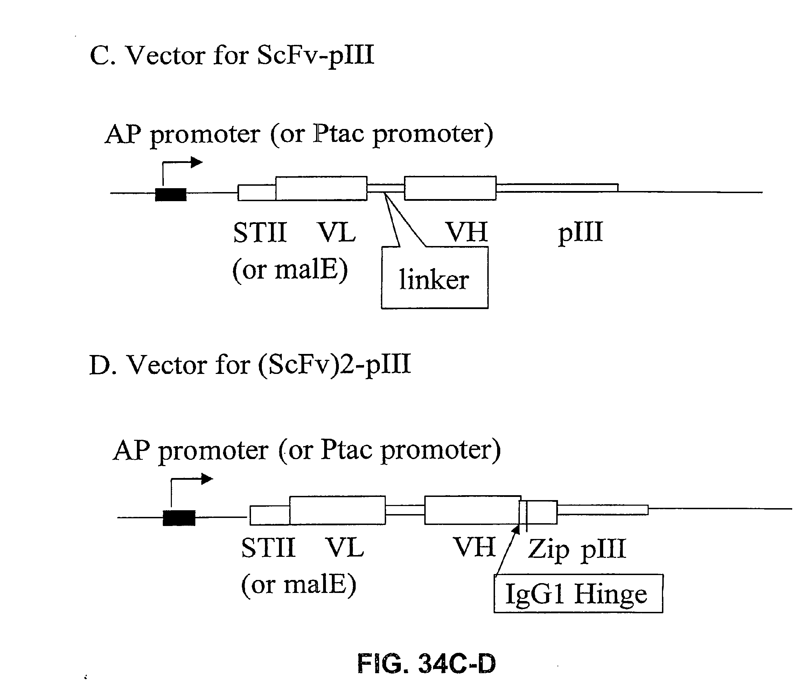

Figures 34 A - D are a schematic illustration of phagemid constructs. Figure 34A shows a bicistronic vector allowing expression of separate transcripts for display of Fab. A suitable promoter, such as Ptac or PhoA promoter drives expression of the bicistronic message. The first cistron encodes a *malE* or heat stable enterotoxin II (stII) secretion signal connected to a sequence encoding a light chain variable and constant domain and a gD tag. The second cistron encodes a secretion signal sequence, a heavy chain variable domain and constant domain 1 (CH1) and at least a portion of a viral coat protein. Figure 34B shows a bicistronic message for display of F(ab')₂. A suitable promoter drives expression of the first and second cistron. The first cistron encodes a secretion signal sequence *(malE* or stII), a light chain variable and constant domain and a gD tag. The second cistron encodes a secretion signal, a sequence encoding heavy chain variable domain and constant domain 1 (CH1) and dimerization domain and at least a portion of the viral coat protein. Figure 34C is a monocistronic vector for display of ScFv. A suitable promotor drives expression of V_{L} and V_{H} domains linked by a peptide linker. The cistronic sequence is connected at the 5' end to a secretion signal sequence and at 3' end to at least a portion of a viral coat protein (pIII). Figure 34D shows a vector for diplay of ScFv₂. The vector is similar to Figure 34C, but comprises a dimerization domain between V_{H} and the coat protein.

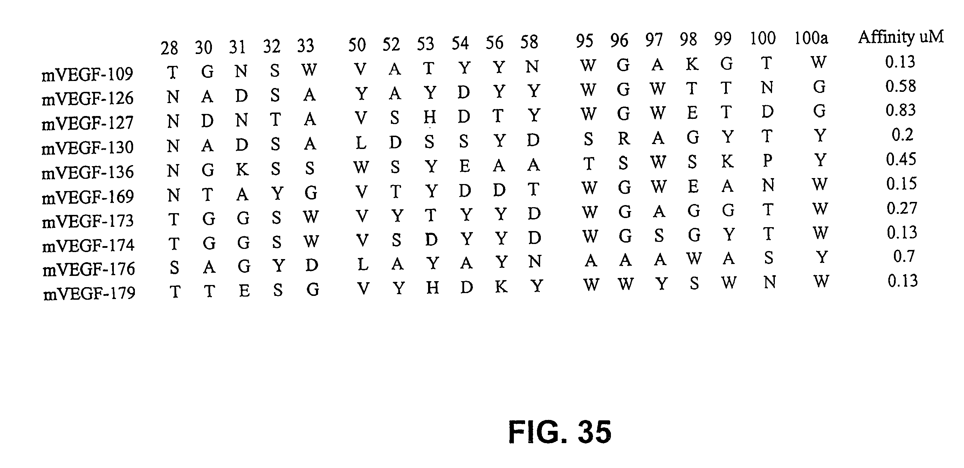

Figure 35 shows the amino acid sequences for heavy chain variable CDR sequences and affinities of anti-VEGF binders from a ScFv and ScFv₂ library.

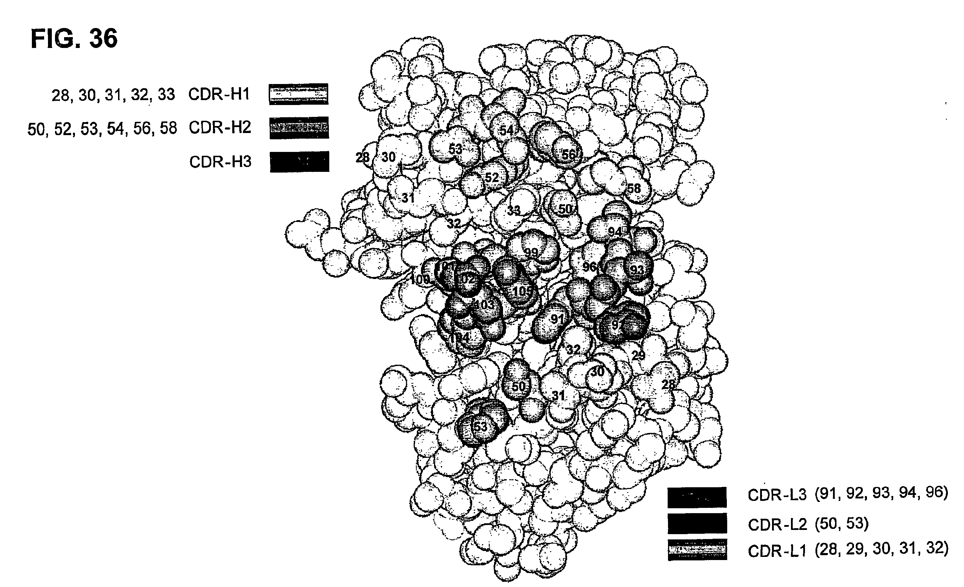

Figure 36 shows a 3-D modeled structure of humanized 4D5 showing CDR residues that form contiguous patches. Contiguous patches are formed by amino acid residues 28, 29,30,31 and 32 in CDRL1; amino acids residues 50 and 53 of CDRL2; amino acid residues 91,92, 93, 94 and 96 of CDRL3; amino acid residues 28, 30, 31, 32,33 in CDRH1; and amino acid residues 50,52,53,54,56, and 58 in CDRH2.

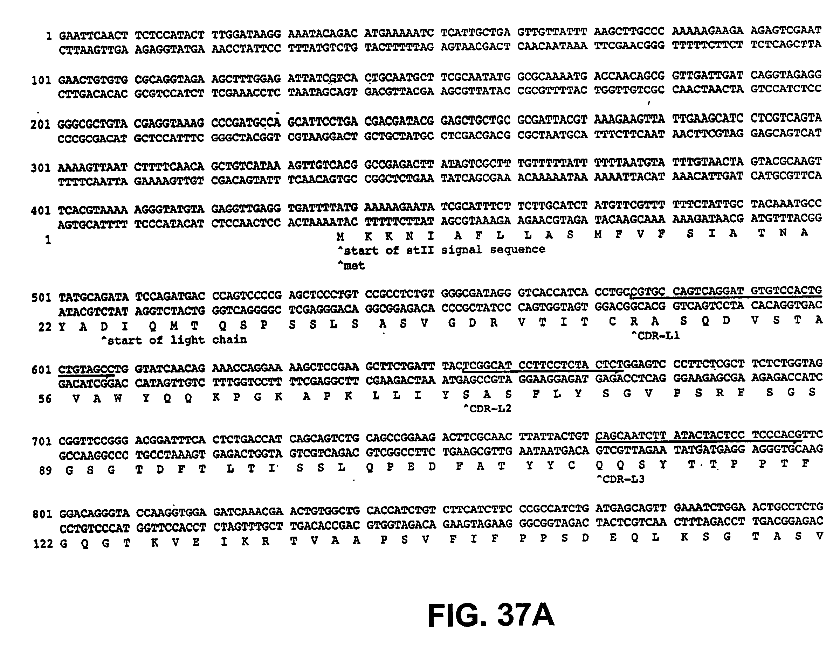

Figure 37 A-F shows the DNA (SEQ ID NO: 28) and amino acid sequence of pV3050-2b vector. The sequence shows stII secretion signal sequence, light chain variable and constant domain of 4D5, and a gD tag, another stII secretion signal sequence heavy chain variable domain sequence of 4D5, CHI heavy chain constant domain, and C-terminal domain of p3.

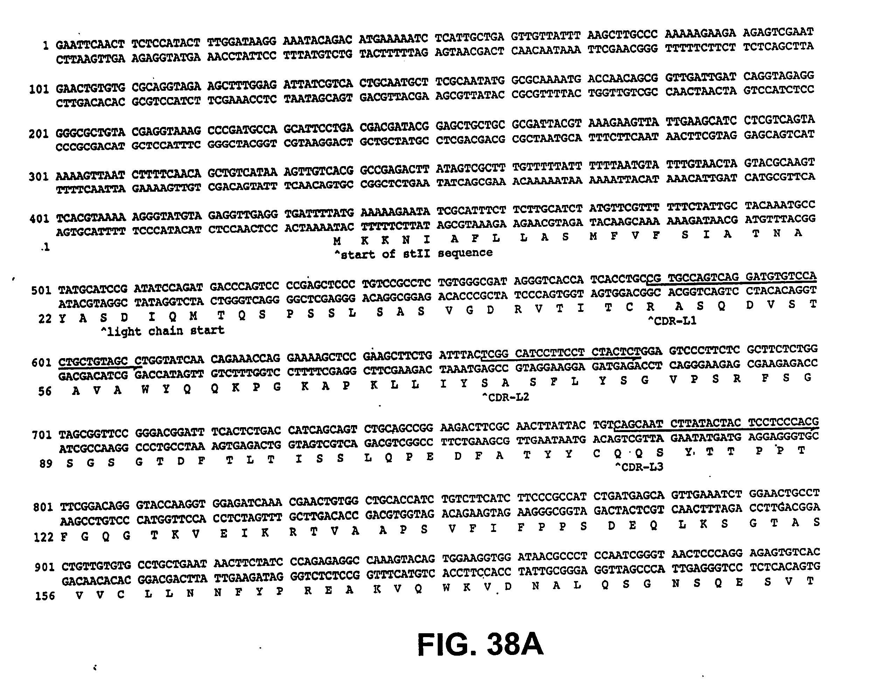

Figure 38 A-F shows the DNA (SEQ ID NO: 29) and amino acid sequence of vector pV-0350-4. The pV0350-2b vector was modified by inserting a dimerization domain between heavy chain constant CH1 domain and p3 sequences.

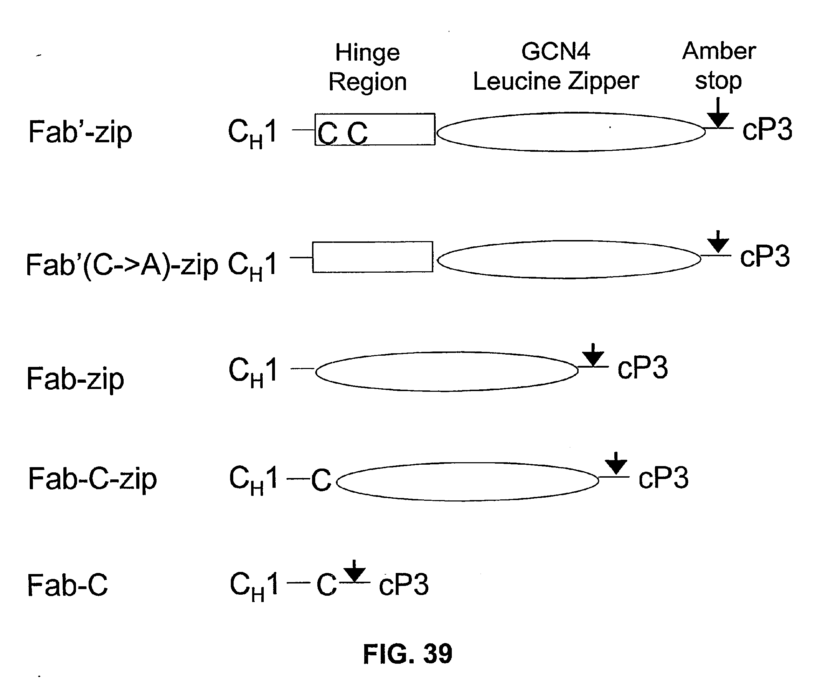

Figure 39 shows a schematic illustration of constructs with different dimerization domains: Fab'-zip with hinge region and leucine zipper, cysteine-free hinge plus zipper (Fab'(C→A)-zip), zipper alone (Fab-zip), one cysteine plus zipper (Fab-C-zip) or just one cysteine (Fab-C) by itself between Fab and cP3.

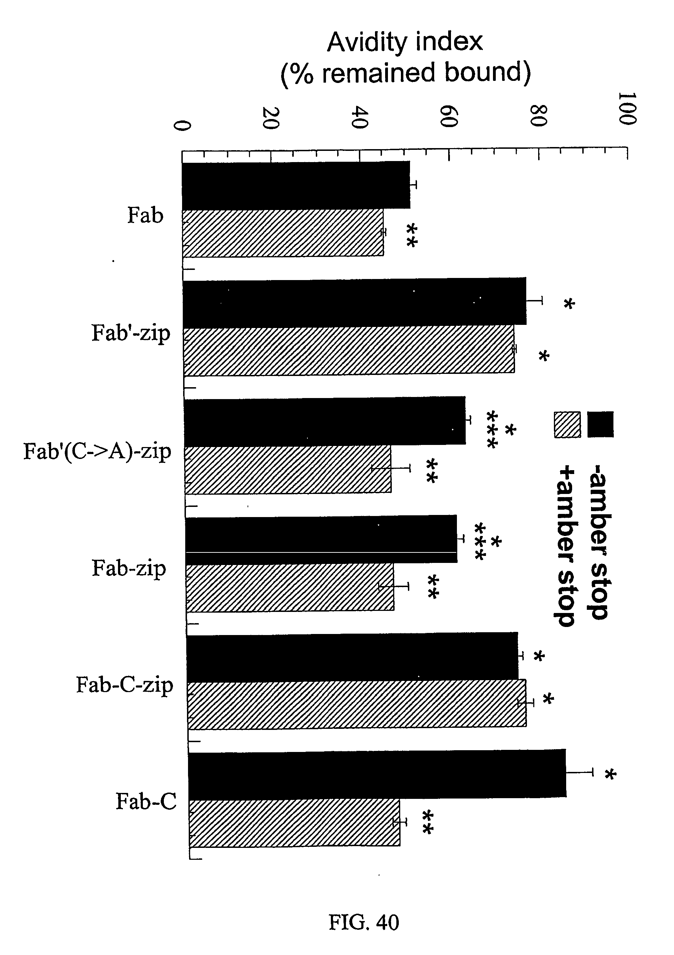

Figure 40 shows the avidity index of variants with variations in dimerization domains in the presence of amber stop codons (cross hatch) or in the absence of an amber stop codon (solid bar) after the dimerization domain. Assays were performed as in Fig.20 in triplicate and the avidity index is the percentage of phage remained bound after 500 nM ErbB2-ECD incubation relative to phage bound in a sample incubated without ErbB2-ECD. The significance of the differences were analysed with t-test. One asterisk (*) represents significant difference compared to Fab phage of equal amber stop status (p-values <0.001, except that Fab' A)-zip and Fab-zip (-amber) have p value as 0.001-0.003). Two asterisks (**) represent significant difference comparing between with and without the amber stop codon (p<0.01). Three asterisks (***) indicate significant difference comparing amber-less constructs of Fab' (C->A)-zip and Fab-zip against Fab'-zip phage (p= 0.003-0.007).

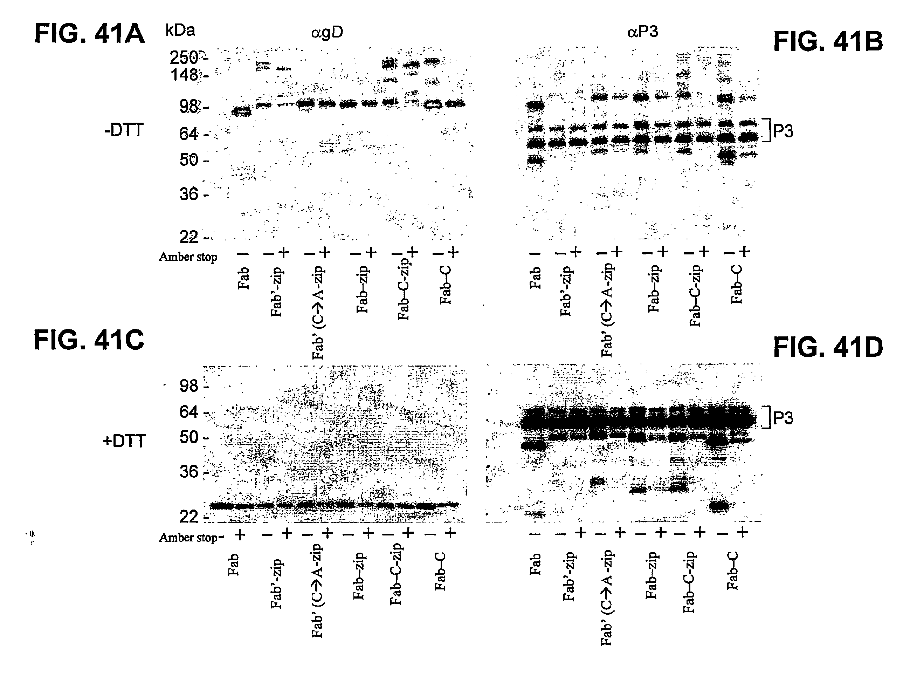

Figure 41 shows western blot of constructs with different dimerization domains under reducing conditions (Panels C and D) and non-reducing conditions (Panels A and B). Phage samples are labelled at the bottom of each lane and MW markers are labelled at the left. All constructs contained gD-tag fused to the C terminus of the light chain.
   Western blot samples were run under non-reducing (-DTT; Panels A and B) or reducing (+DTT; Panels C and D) conditions, and proteins were detected with an anti-gD-tag antibody (Panels A and C) or anti-P3 antibody (Panels B and D). Phage infected XL-1Blue cells were grown at 30 °C overnight in 2YT medium and phage were purified by precipitation twice with PEG/NaCl and resuspended in 1 mL of 10 mM TRIS, 1.0 mM EDTA, pH 8.0. Phage samples (3X10¹¹, 1OD₂₆₈ =1.3X10¹³) were incubated at 95 °C for 5 min in SDS-PAGE sample buffer (2% SDS, 20mM Tris pH 6.8, 10% glycerol) with or without 2mM DTT. The denatured samples were run on a 4-20% Tris-Glycine gel and then electro-transferred to a PVDF filter. The PVDF filter was blocked with 2% milk and 0.1% Tween20 in Tris, pH 7.5, O.15M NaCl (blocking buffer) for 2 h and then incubated with 2nM of anti-gD-tag (left) or anti-P3 (right) antibody in blocking buffer for 1 h. The filter was washed with PBS, 0.05% Tween20, incubated with horse radish peroxidase/rabbit anti-mouse Fab conjugate for 30 min and visualized with ECL™ western blotting detection reagent (Amersham Life Sciences).

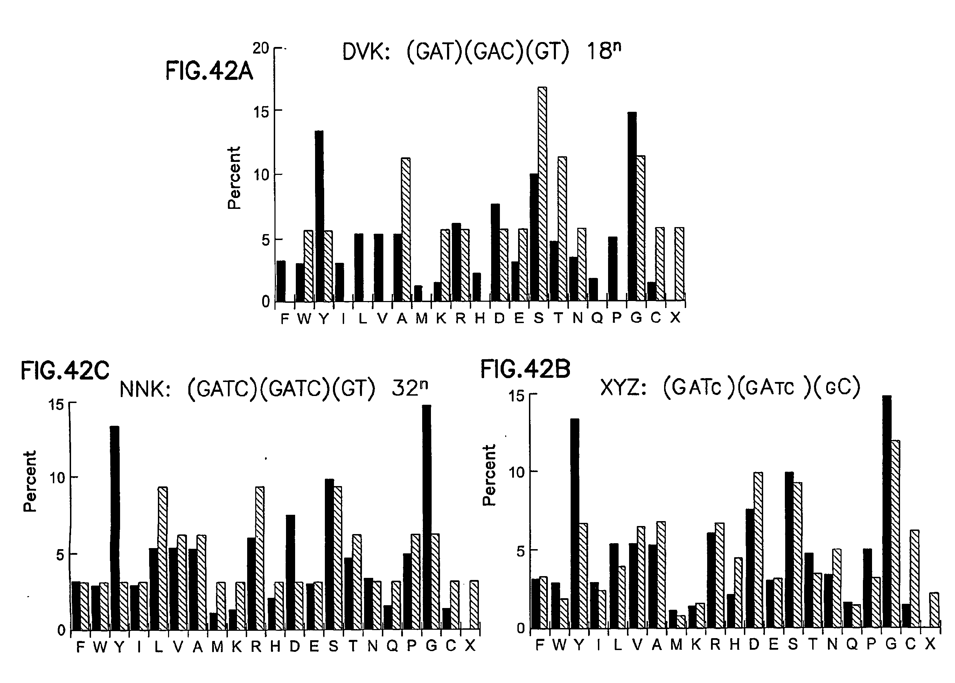

Figure 42 shows a comparison of frequency of amino acids in CDRH3 in naturally occurring antibodies (solid bar); amino acids encoded by codon set (A) *DVK* (cross hatch bar); (B) *XYZ* ( cross hatch bar); and (C) *NNK* ( cross hatch bar). Amino acids are grouped as follows: phenyalanine (F), trytophan (W) and tyrosine (Y) are aromatic amino acids; isoleucine (I) , leucine (L), valine (V), alanine(A), and methionine (M) are aliphatic, lysine (K), arginine(R), and histidine (H) are basic; aspartic acid (D) and glutamic acid (E) are acidic; serine (S), threonine(T), asparagine (N), and glutamine (Q) are polar; and proline (P), glycine (G), and cysteine (C) are conformational.
Figure 43 shows embodiments of oligonucleotides encoding diversity in sequence and in length of CDRH3. The oligonucleotides are identified as F171, F185, F185a etc. in the left hand column. The amino acid sequence at each amino acid position for CDRH3 using each oligonucleotide is shown. The CDRH3 sequence in the source antibody 4D5 is : S₉₃, R₉₄, W_{95,}G₉₆, G₉₇, D₉₈, G₉₉ , F₁₀₀, Y₁₀₀ₐ, A_{100b} , M_{100c},D₁₀₁ , and Y_{102.} Amino acid positions 93 and 94 are considered framework positions. In some embodiments, certain positions may have a fixed amino acid shown in single letter code, e.g. position 93 is A(alanine); amino acid position 94 may be R/K (arginine /lysine); amino acid position 100a may be A/G/V/D (alanine/glycine/ valine/aspartic acid). Other amino acid positions are diversified using codon sets identified the three capital letters in italics, e.g.*NNK, XYZ, NNS ,KSG.* The length of the CDRH3 is indicated on the right column.

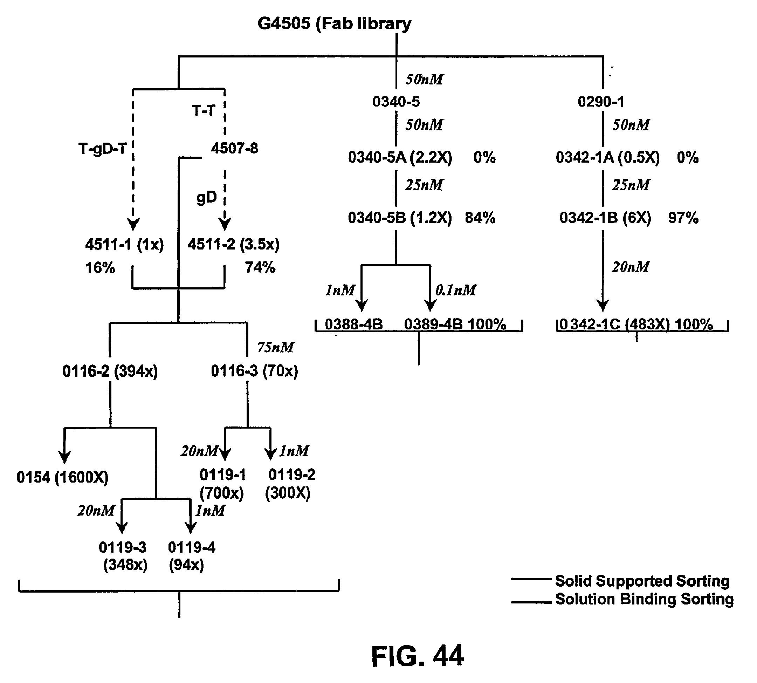

Figure 44 shows schematic illustration of sorting strategies to select for high affinity anti-VEGF antibody variable domains form libraries using solid support bound target (---) or solution phase target binding as described in Example 9.

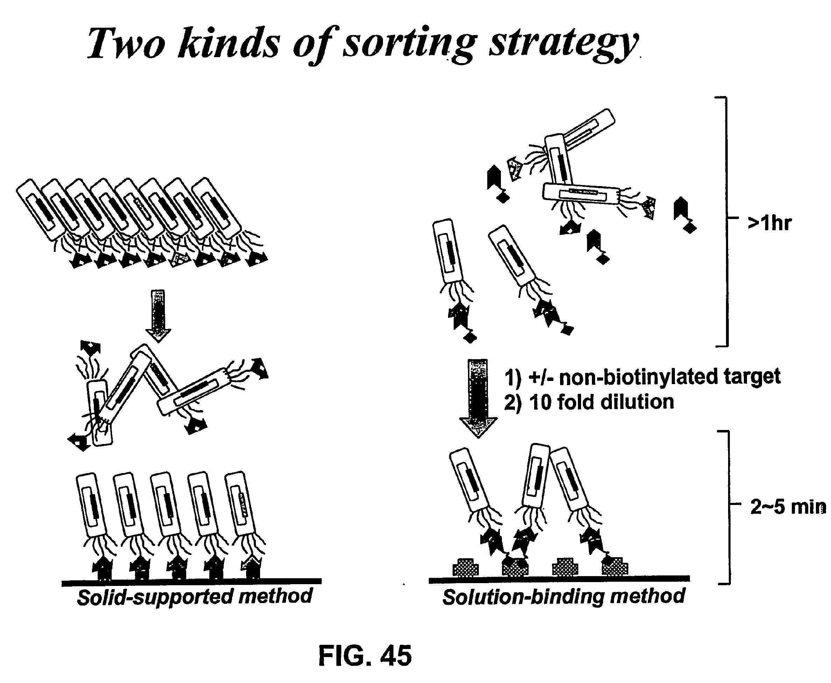

Figure 45 shows a schematic depiction of two kinds of sorting strategies for selecting high affinity antibody variable domains. The sorting or selection includes sorting against target bound to solid substrate and/or sorting against solution phase target antigens as described in Example 9.

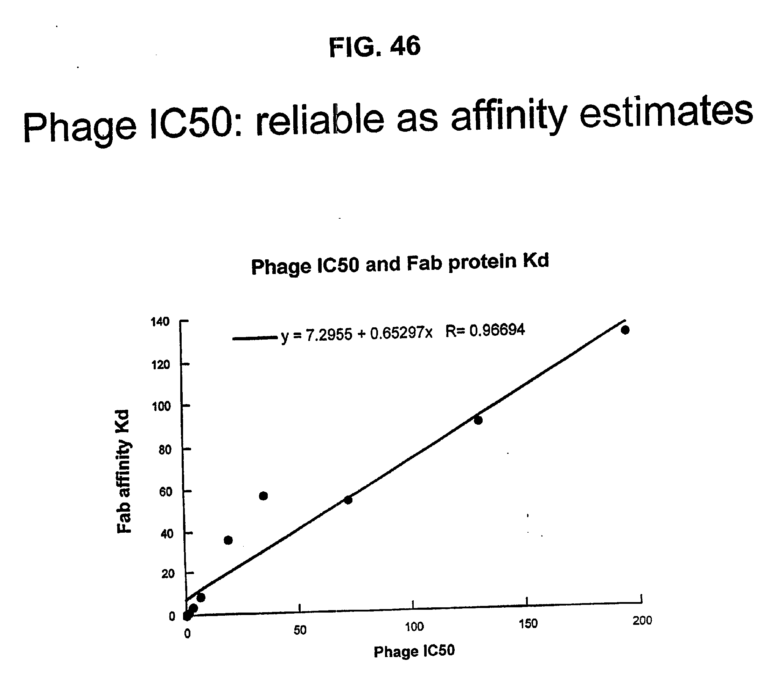

Figure 46 shows the correlation of affinity determination based on IC50 from a competition ELISA as described in Example 8 with KD of the same clone.

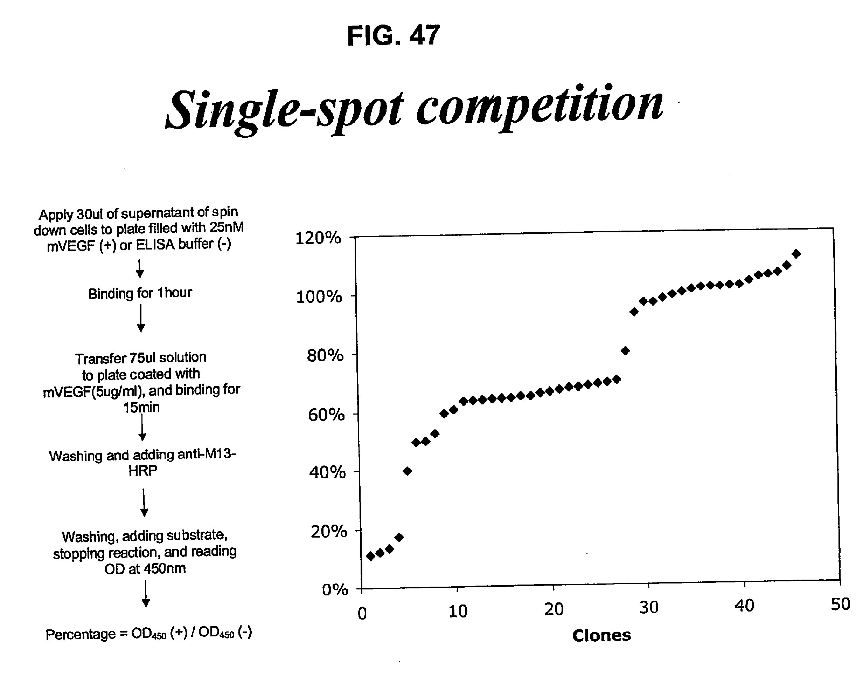

Figure 47 shows a schematic representation of single spot competition ELISA for binders. The graph shows the data from screening about 40 clones. The % inhibition of binding was calculated by calculating the ratio of OD of samples plus target over samples minus target. The lower the %, the higher the potential affinity. The results show, that of the 40 clones, there was a range of affinities. About 10% of the clones were of very high affinity.

| **SEQ ID NO:** | **Name** | **Sequence** | **Page** |
|---|---|---|---|
| 1 | 4D5 light chain variable domain | Asp Ile Gln Met Thr Gln Ser Pro Ser Ser Leu Ser Ala Ser Val Gly Asp Arg Val Thr Ile Thr Cys Arg Ala Ser Gln Asp Val Asn Thr Ala Val Ala Trp Tyr Gln Gln Lys Pro Gly Lys Ala Pro Lys Leu Leu Ile Tyr Ser Ala Ser Phe Leu Glu Ser Gly Val Pro Ser Arg Phe Ser Gly Ser Arg Ser Gly Thr Asp - #Phe Thr Leu Thr Ile Ser Ser Leu Gln Pro Glu Asp Phe Ala Thr Tyr Tyr Cys Gln Gln His Tyr Thr Thr Pro Pro Thr Phe Gly Gln Gly Thr Lys Val Glu Ile Lys Arg Thr | 12 |
| 2 | 4D5 heavy chain variable domain | Glu Val Gln Leu Val Glu Ser Gly Gly Gly Leu Val Gln Pro Gly Gly Ser Leu Arg Leu Ser Cys Ala Ala Ser Gly Phe Asn Ile Lys Asp Thr Tyr Ile His Trp Val Arg Gln Ala Pro Gly Lys Gly Leu Glu Trp Val Ala Arg Ile Tyr Pro Thr Asn Gly Tyr Thr Arg Tyr Ala Asp Ser Val Lys Gly Arg Phe Thr Ile Ser Ala Asp Thr Ser Lys Asn Thr Ala Tyr Leu Gln Met Asn Ser Leu Arg Ala Glu Asp Thr Ala Val Tyr Tyr Cys Ser Arg Trp Gly Gly Asp Gly Phe Tyr Ala Met Asp Val Trp Gly Gln Gly Thr Leu Val Thr Val Ser Ser | 12 |
| 3 | GNC4 leucine zipper | GRMKQLEDKVEELLSKNYHLENE VARLKKLVGERG | 18 |
| 4 | C-terminal of CDRH3 | YAMDY | 7 |
| 5 | heavy chain CDR3 | SRNAWAF | 89 |
| 6 | heavy chain CDR3 | SRNLSENSYAM | 89 |
| 7 | heavy chain CDR3 | SRAGWAGWYAM | 89 |
| 8 | heavy chain CDR3 | SRAAKAGWYAM | 89 |
| 9 | heavy chain CDR3 | SRSDGRDSAYAM | 89 |
| 10 | F63 | SRXXXXXXXAMDY | Figure 4 |
| 11 | F65 | SRXXXXXXXYAMDY | Figure 4 |
| 12 | F64 | SRXXXXXXYAMDY | Figure 4 |
| 13 | F66 | SRXXXXXXYAMDY | Figure 4 |
| 14 | oligonucleotide F151 | gca gct tct ggc ttc acc att ant nnt nnn nnt ata cac tgg gtg cgt cag | 92 |
| 15 | oligonucleotide F152 | gca gct tct ggc ttc acc att ant nnt nnn ngg ata cac tgg gtg cgt cag 92 | 92 |
| 16 | oligonucleotide F175 | gca gct tct ggc ttc acc att ant nnn nnn nnt ata cac tgg gtg cgt cag | 92 |
| 17 | oligonucleotide F176 | gca gct tct ggc ttc acc att ant nnn nnn ngg ata cac tgg gtg cgt cag | 92 |
| 18 | oligonucleotide F153 | aag ggc ctg gaa tgg gtt gnt ngg att nnt cct nnt nnc ggc nnt act nac tat gcc gat agc gtc aag ggc | 92 |
| 19 | oligonucleotide F154 | aag ggc ctg gaa tgg gtt gnt nnt att nnt cct nnt nnc ggc nnt act nac tat gcc gat agc gtc aag ggc | 92 |
| 20 | oligonucleotide F173 | agg gcc ctg gaa tgg gtt gnt ngg att nnt cct nnt nnc ggc nnt act nac tat gcc gat agc gtc aag ggc | 92 |
| 21 | oligonucleotide F174 | aag ggc ctg gaa tgg gtt gnt nnt att nnt cct nnt nnc ggc nnt act nac tat gcc gat agc gtc aag ggc | 92 |
| 22 | oligonucleotide F125 | cgn ttc act ata agc cgt gac aca tcc aaa aac | 92 |
| 23 | single chain Fv | | Figure 14 |
| 24 | single chain Fv with zipper domain | | Figure 15 |
| 25 | Fab fragment | | Figure 16 |
| 26 | Fab fragment with zipper domain | | Figure 17 |
| 27 | hinge sequence | TCPPCPAPELLG | 97 |
| 28 | Fab phagemid vector pV0350-2b | | Figure 37 |
| 29 | F(ab)'₂ phagemid vector pV0350-4 | | Figure 38 |
| 30 | oligonucleotide F61 | gca act tat tac tgt cag caa nnt nnt nnn nnn cct tnn acg ttc gga cag ggt acc | 120 |
| 31 | F59 | SRWGGDGFYAMDY | Figure 4 |
| 32 | F78 | SRXXXXXFDY | Figure 4 |
| 33 | F165 | AXXXXXXXYAMDY | Figure 4 |
| 34 | F166 | AXWXXXXXXAMDY | Figure 4 |
| 35 | F134 | AXXXXXXXYAMDY | Figure 4 |
| 36 | F136 | AXWXXXXXXYAMDY | Figure 4 |
| 37 | F137 | AXXWXXXXXYAMDY | Figure 4 |
| 38 | F138 | AXXXWXXXXYAMDY | Figure 4 |
| 39 | F142 | AXXXXXXWYAMDY | Figure 4 |
| 40 | F155 | AXWXXXXXXXXAMY | Figure 4 |
| 41 | F156 | AXXWXXXXXXAMDY | Figure 4 |
| 42 | F157 | AXXXWXXXXXAMDY | Figure 4 |
| 43 | F158 | AXXXXWXXXXAMDY | Figure 4 |
| 44 | F160 | AXXXXXXWXXAMDY | Figure 4 |
| 45 | F160g | AXXXXXXXWXAMDY | Figure 4 |
| 46 | F163a | AXXXXXXXXAMDY | Figure 4 |
| 47 | F164a | ARXXXXXXXYAMDY | Figure 4 |
| 48 | F164b | ARXXXXXXXXAMDY | Figure 4 |
| 49 | F165a | ARXXXXXXXXYAMDY | Figure 4 |
| 50 | F165b | ARXXXXXXXXXAMDY | Figure 4 |
| 51 | F155 | AXWXXXXXXXAMY | Figure 4 |
| 52 | F167 | AXWXXXXXXAMDY | Figure 4 |
| 53 | F135 | AXWXXXXXXAMDY | Figure 4 |
| 54 | F103 | SRXXXXXXXXYAMDY | Figure 4 |
| 55 | F66a | ARXXXXXXYAMDY | Figure 4 |
| 56 | F66b | ARXXXXXXXAMDY | Figure 4 |
| 57 | F66c | ARXXXXXYXMDY | Figure 4 |
| 58 | F66d | ARXXXXXXXMDY | Figure 4 |
| 59 | F66e | ARXXXXYXMDY | Figure 4 |
| 60 | F66f | ARXXXXXXMDY | Figure 4 |
| 61 | F66a1 | ARXXXXXXYXMDY | Figure 4 |
| 62 | F66b1 | ARXXXXXXXXMDY | Figure 4 |
| 63 | F66g | ARXXXXXXXXXMDY | Figure 4 |
| 64 | F66h | ARXXXXXXXYXMDY | Figure 4 |
| 65 | F66i | ARXXXXXXXXYXMDY | Figure 4 |
| 66 | F66j | AKXXXXXXXXXXMDY | Figure 4 |
| 67 | F171c | AXXXXXXFDY | Figure 4 |
| 68 | F171d | AXXXXXXXFDY | Figure 4 |
| 69 | F171e | AXXXXXXXXFDY | Figure 4 |
| 70 | F171 | AXXXXXFDY | Figure 4 |
| 71 | F186 | AXXXXXXXXFDY | Figure 4 |
| 72 | F187 | AXXXXXXXXXFDY | Figure 4 |
| 73 | F190 | AXXXXXXXXXXYAMD | Figure 4 |
| 74 | F190a | AXXXXXXXXXYXMD | Figure 4 |
| 75 | F190d | AXXXXXXXXXXXYXMD | Figure 4 |
| 76 | 4D5 CDRH3 | WGGDGFY | Figure 4 |
| 77 | CDRH3 | SRWKYATRYAM | Figure 29 |
| 78 | CDRH3 | SRSRGWWTAAM | Figure 29 |
| 79 | CDRH3 | SRASRDWYGAM | Figure 29 |
| 80 | mVEGF-201 CDRH1 | TTSNG | Figure 33 |
| 81 | mVEGF-201 CDRH2 | AYSSNYYR | Figure 33 |
| 82 | mVEGF-201 CDRH3 | ARWSRASFY | Figure 33 |
| 83 | mVEGF-202 CDRH1 | TTGTD | Figure 33 |
| 84 | mVEGF-202 CDRH2 | AITYDSYR | Figure 33 |
| 85 | mVEGF-202 CDRH3 | AKAGDREGY | Figure 33 |
| 86 | mVEGF-203 CDRH1 | TTDSG | Figure 33 |
| 87 | mVEGF-203 CDRH2 | GRSYSSNR | Figure 33 |
| 88 | mVEGF-203 CDRH3 | AKWPWYNAW | Figure 33 |
| 89 | hFc-10 CDRH1 | TNNYW | Figure 33 |
| 90 | hFc-10 CDRH2 | GYSYGTR | Figure 33 |
| 91 | hFc-10 CDRH3 | AKAXKGSLY | Figure 33 |
| 92 | hFc-11 CDRH1 | TTGNA | Figure 33 |
| 93 | hFc-12 CDRH1 | TNDYY | Figure 33 |
| 94 | hFc-13 CDRH1 | TSNTG | Figure 33 |
| 95 | hFc-14 CDRH1 | TTSYG | Figure 33 |
| 96 | hFc-14 CDRH2 | ASSYSYR | Figure 33 |
| 97 | hFc-14 CDRH3 | AKYXAREGX | Figure 33 |
| 98 | hFc-15 CDRH1 | TNNNS | Figure 33 |
| 99 | hFc-15 CDR2 | GYNSGSR | Figure 33 |
| 100 | hFc-15 CDRH3 | AKWRTSWKY | Figure 33 |
| 101 | hFc-16 CDRH1 | TSSSA | Figure 33 |
| 102 | hFc-16 CDRH2 | AWSNGSR | Figure 33 |
| 103 | hFc-16 CDRH3 | AXTAGGAKY | Figure 33 |
| 104 | hFc-17 CDRH1 | TTNTW | Figure 33 |
| 105 | hFc-17 CDRH2 | GDYDGYR | Figure 33 |
| 106 | hFc-17 CDRH3 | AXWRWWGRY | Figure 33 |
| 107 | hFc-18 CDRH1 | TNGNY | Figure 33 |
| 108 | hFc-18 CDRH2 | GWSNGYR | Figure 33 |
| 109 | hFc-18 CDRH3 | ARYSGGRRY | Figure 33 |
| 110 | hFc-19 CDRH1 | TSNNA | Figure 33 |
| 111 | hFc-19 CDRH2 | GRSYNYR | Figure 33 |
| 112 | hFc-19 CDRH3 | AXGXTSGGY | Figure 33 |
| 113 | hFc-20 CDRH1 | TTSND | Figure 33 |
| 114 | hFc-20 CDRH2 | AWSYNYR | Figure 33 |
| 115 | hFc-20 CDRH3 | ARRSRWSRA | Figure 33 |
| 116 | mVEGF-109 CDRH1 | TGNSW | Figure 35 |
| 117 | mVEGF-109 CDRH2 | VATYYN | Figure 35 |
| 118 | mVEGF-109 CDRH3 | WGAKGTW | Figure 35 |
| 119 | mVEGF-126 CDRH1 | NADSA | Figure 35 |
| 120 | mVEGF-126 CDRH2 | YAYDYY | Figure 35 |
| 121 | mVEGF-126 CDRH3 | WGWTTNG | Figure 35 |
| 122 | mVEGF-127 CDRH1 | NDNTA | Figure 35 |
| 123 | mVEGF-127 CDRH2 | VSHDTY | Figure 35 |
| 124 | mVEGF-127 CDRH3 | WGWETDG | Figure 35 |
| 125 | mVEGF-130 CDRH2 | LDSSYD | Figure 35 |
| 126 | mVEGF-130 CDRH3 | SRAGYTY | Figure 35 |
| 127 | mVEGF-136 CDRH1 | NGKSS | Figure 35 |
| 128 | mVEGF-136 CDRH2 | WSYEAA | Figure 35 |
| 129 | mVEGF-136 CDRH3 | TSWSKPY | Figure 35 |
| 130 | mVEGF-169 CDRH1 | NTAYG | Figure 35 |
| 131 | mVEGF-169 CDRH2 | VTYDDT | Figure 35 |
| 132 | mVEGF-169 CDRH3 | WGWEANW | Figure 35 |
| 133 | mVEGF-173 CDRH1 | TGGSW | Figure 35 |
| 134 | mVEGF-173 CDRH2 | VYTYYD | Figure 35 |
| 135 | mVEGF-173 CDRH3 | WGAGGTW | Figure 35 |
| 136 | mVEGF-174 CDRH2 | VSDYYD | Figure 35 |
| 137 | mVEGF-174 CDRH3 | WGSGYTW | Figure 35 |
| 138 | mVEGF-176 CDRH1 | SAGYD | Figure 35 |
| 139 | mVEGF-176 CDRH2 | LAYAYN | Figure 35 |
| 140 | mVEGF-176 CDRH3 | AAAWASY | Figure 35 |
| 141 | mVEGF-179 CDRH1 | TTESG | Figure 35 |
| 142 | mVEGF-179 CDRH2 | VYHDKY | Figure 35 |
| 143 | mVEGF-179 CDRH3 | WWYSWNW | Figure 35 |
| 144 | F183 | AXXXXXYAMDY | Figure 43 |
| 145 | F184 | AXXXXXXAMDY | Figure 43 |
| 146 | F190f | AXXXXXXXXXMDY | Figure 43 |
| 147 | F190n | AXXXXXXXYXMDY | Figure 43 |
| 148 | F187b | ARXXXXXXXXXXDY | Figure 43 |
| 149 | F181 | AXXXXXXXXXAMDY | Figure 43 |
| 150 | F190j | AXXXXXXXXXXMDY | Figure 43 |
| 151 | F190q | AXXXXXXXXYXMDY | Figure 43 |
| 152 | F187c | ARXXXXXXXXXXXDY | Figure 43 |
| 153 | F179 | AXXXXXXXXXYAMDY | Figure 43 |
| 154 | F182 | AXXXXXXXXXXAMDY | Figure 43 |
| 155 | F190g | ARXXXXXXXXXXXXXMDY | Figure 43 |
| 156 | F190k | ARXXXXXXXXXXXYXMDY | Figure 43 |
| 157 | F190h | ARXXXXXXXXXXXXXXMDY | Figure 43 |
| 158 | F190m | ARXXXXXXXXXXXXYXMDY | Figure 43 |
| 159 | F190i | ARXXXXXXXXXXXXXXXMDY | Figure 43 |
| 160 | F190p | ARXXXXXXXXXXXXXYXMDY | Figure 43 |
| 161 | F190r | ARXXXXXXXXXXXXXXYXMDY | Figure 43 |
| 162 | F59 | SRWCXXXXXAMDY | Figure 4 |
| 163 | C-terminal of CDRH3 | YAMDX | 64 |
| 164 | CDRH3 | (X)ₙXAMDY where n = 4 to 14 | 72 |

### DETAILED DESCRIPTION OF THE INVENTION

The invention provides novel and systematic methods for diversifying antibody variable domain sequences, and libraries comprising a multiplicity, generally a great multiplicity of diversified antibody variable domain sequences. Such libraries provide combinatorial libraries useful for, for example, selecting and/or screening for synthetic antibody clones with desirable activities such as binding affinities and avidities. These libraries are useful for identifying immunoglobulin polypeptide sequences that are capable of interacting with any of a wide variety of target antigens. For example, libraries comprising diversified immunoglobulin polypeptides of the invention expressed as phage displays are particularly useful for, and provide a high throughput, efficient and automatable systems of, selecting and/or screening for antigen binding molecules of interest. The methods of the invention are designed to provide high affinity binders to target antigens with minimal changes to a source or template molecule and provide for good production yields when the antibody or antigens binding fragments are produced in cell culture.

### DEFINITIONS

Amino acids are represented herein as either a single letter code or as the three letter code or both.

The term "affinity purification" means the purification of a molecule based on a specific attraction or binding of the molecule to a chemical or binding partner to form a combination or complex which allows the molecule to be separated from impurities while remaining bound or attracted to the partner moiety.

The term "antibody" is used in the broadest sense and specifically covers single monoclonal antibodies (including agonist and antagonist antibodies), antibody compositions with polyepitopic specificity, affinity matured antibodies, humanized antibodies, chimeric antibodies, as well as antigen binding fragments (e.g., Fab, F(ab')₂, scFv and Fv), so long as they exhibit the desired biological activity.

As used herein, "antibody variable domain" refers to the portions of the light and heavy chains of antibody molecules that include amino acid sequences of Complementarity Determining Regions (CDRs; ie., CDR1, CDR2, and CDR3), and Framework Regions (FRs). V_{H} refers to the variable domain of the heavy chain. V_{L} refers to the variable domain of the light chain. According to the methods used in this invention, the amino acid positions assigned to CDRs and FRs may be defined according to Kabat (Sequences of Proteins of Immunological Interest (National Institutes of Health, Bethesda, Md., 1987 and 1991)). Amino acid numbering of antibodies or antigen binding fragments is also according to that of Kabat.

As used herein, the term "Complementarity Determining Regions (CDRs; ie., CDR1, CDR2, and CDR3) refers to the amino acid residues of an antibody variable domain the presence of which are necessary for antigen binding. Each variable domain typically has three CDR regions identified as CDR1, CDR2 and CDR3. Each complementarity determining region may comprise amino acid residues from a "complementarity determining region" as defined by Kabat (i.e. about residues 24-34 (L1), 50-56 (L2) and 89-97 (L3) in the light chain variable domain and 31-35 (H1), 50-65 (H2) and 95-102 (H3) in the heavy chain variable domain; Kabat et al., Sequences of Proteins of Immunological Interest, 5th Ed. Public Health Service, National Institutes of Health, Bethesda, MD. (1991)) and/or those residues from a "hypervariable loop" (*i.e.* about residues 26-32 (L1), 50-52 (L2) and 91-96 (L3) in the light chain variable domain and 26-32 (H1), 53-55 (H2) and 96-101 (H3) in the heavy chain variable domain; Chothia and Lesk J. Mo/. Biol. 196:901-917 (1987)). In some instances, a complementarity determining region can include amino acids from both a CDR region defined according to Kabat and a hypervariable loop. For example, the CDRH1 of the heavy chain of antibody 4D5 includes amino acids 26 to 35.

"Framework regions" (hereinafter FR) are those variable domain residues other than the CDR residues. Each variable domain typically has four FRs identified as FR1, FR2, FR3 and FR4. If the CDRs are defined according to Kabat, the light chain FR residues are positioned at about residues 1-23 (LCFR1), 35-49 (LCFR2), 57-88 (LCFR3), and 98-107 (LCFR4) and the heavy chain FR residues are positioned about at residues 1-30 (HCFR1), 36-49 (HCFR2), 66-94 (HCFR3), and 103-113 (HCFR4) in the heavy chain residues. If the CDRs comprise amino acid residues from hypervariable loops, the light chain FR residues are positioned about at residues 1-25 (LCFR1), 33-49 (LCFR2), 53-90 (LCFR3), and 97-107 (LCFR4) in the light chain and the heavy chain FR residues are positioned about at residues 1-25 (HCFR1), 33-52 (HCFR2), 56-95 (HCFR3), and 102-113 (HCFR4) in the heavy chain residues. In some instances, when the CDR comprises amino acids from both a CDR as defined by Kabat and those of a hypervariable loop, the FR residues will be adjusted accordingly. For example, when CDRH1 includes amino acids H26-H35, the heavy chain FR1 residues are at positions 1-25 and the FR2 residues are at positions 36-49.

As used herein, "codon set" refers to a set of different nucleotide triplet sequences used to encode desired variant amino acids. A set of oligonucleotides can be synthesized, for example, by solid phase synthesis, including sequences that represent all possible combinations of nucleotide triplets provided by the codon set and that will encode the desired group of amino acids. A standard form of codon designation is that of the IUB code, which is known in the art and described herein. A codon set typically is represented by 3 capital letters in italics, eg. *NNK, NNS, XYZ, DVK* and the like. A "non-random codon set", as used herein, thus refers to a codon set that encodes select amino acids that fulfill partially, preferably completely, the criteria for amino acid selection as described herein. Synthesis of oligonucleotides with selected nucleotide "degeneracy" at certain positions is well known in that art, for example the TRIM approach (Knappek et al.; J. Mol. Biol. (1999), 296:57-86); Garrard & Henner, Gene (1993), 128:103). Such sets of oligonucleotides having certain codon sets can be synthesized using commercial nucleic acid synthesizers (available from, for example, Applied Biosystems, Foster City, CA), or can be obtained commercially (for example, from Life Technologies, Rockville, MD). Therefore, a set of oligonucleotides synthesized having a particular codon set will typically include a plurality of oligonucleotides with different sequences, the differences established by the codon set within the overall sequence. Oligonucleotides, as used according to the invention, have sequences that allow for hybridization to a variable domain nucleic acid template and also can, but does not necessarily, include restriction enzyme sites useful for, for example, cloning purposes.

An "Fv" fragment is an antibody fragment which contains a complete antigen recognition and binding site. This region consists of a dimer of one heavy and one light chain variable domain in tight association, which can be covalent in nature, for example in scFv. It is in this configuration that the three CDRs of each variable domain interact to define an antigen binding site on the surface of the V_{H}-V_{L} dimer. Collectively, the six CDRs or a subset thereof confer antigen binding specificity to the antibody. However, even a single variable domain (or half of an Fv comprising only three CDRs specific for an antigen) has the ability to recognize and bind antigen, although usually at a lower affinity than the entire binding site.

The "Fab" fragment contains a variable and constant domain of the light chain and a variable domain and the first constant domain (CHl) of the heavy chain. F(ab')₂ antibody fragments comprise a pair of Fab fragments which are generally covalently linked near their carboxy termini by hinge cysteines between them. Other chemical couplings of antibody fragments are also known in the art.

"Single-chain Fv" or "scFv" antibody fragments comprise the V_{H} and V_{L} domains of antibody, wherein these domains are present in a single polypeptide chain. Generally the Fv polypeptide further comprises a polypeptide linker between the V_{H} and V_{L} domains, which enables the scFv to form the desired structure for antigen binding. For a review of scFv, see Pluckthun in The Pharmacology of Monoclonal Antibodies, Vol 113, Rosenburg and Moore eds. Springer-Verlag, New York, pp. 269-315 (1994).

The term "diabodies" refers to small antibody fragments with two antigen-binding sites, which fragments comprise a heavy chain variable domain (V_{H}) connected to a light chain variable domain (V_{L}) in the same polypeptide chain (V_{H} and V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

The expression "linear antibodies" refers to the antibodies described in Zapata et al., Protein Eng., 8(10):1057-1062 (1995). Briefly, these antibodies comprise a pair of tandem Fd segments (V_{H}-C_{H}1-V_{H}-C_{H}1) which, together with complementary light chain polypeptides, form a pair of antigen binding regions. Linear antibodies can be bispecific or monospecific.

"Cell", "cell line", and "cell culture" are used interchangeably herein and such designations include all progeny of a cell or cell line. Thus, for example, terms like "transformants" and "transformed cells" include the primary subject cell and cultures derived therefrom without regard for the number of transfers. It is also understood that all progeny may not be precisely identical in DNA content, due to deliberate or inadvertent mutations. Mutant progeny that have the same function or biological activity as screened for in the originally transformed cell are included. Where distinct designations are intended, it will be clear from the context.

"Control sequences" when referring to expression means DNA sequences necessary for the expression of an operably linked coding sequence in a particular host organism. The control sequences that are suitable for prokaryotes, for example, include a promoter, optionally an operator sequence, a ribosome binding site, and possibly, other as yet poorly understood sequences. Eukaryotic cells are known to utilize promoters, polyadenylation signals, and enhancers.

The term "coat protein" means a protein, at least a portion of which is present on the surface of the virus particle. From a functional perspective, a coat protein is any protein which associates with a virus particle during the viral assembly process in a host cell, and remains associated with the assembled virus until it infects another host cell. The coat protein may be the major coat protein or may be a minor coat protein. A "major" coat protein is generally a coat protein which is present in the viral coat at preferably at least about 5, more preferably at least about 7, even more preferably at least about 10 copies of the protein or more. A major coat protein may be present in tens, hundreds or even thousands of copies per virion. An example of a major coat protein is the p8 protein of filamentous phage.

The "detection limit" for a chemical entity in a particular assay is the minimum concentration of that entity which can be detected above the background level for that assay. For example, in the phage ELISA, the "detection limit" for a particular phage displaying a particular antigen binding fragment is the phage concentration at which the particular phage produces an ELISA signal above that produced by a control phage not displaying the antigen binding fragment.

A "fusion protein" and a "fusion polypeptide" refers to a polypeptide having two portions covalently linked together, where each of the portions is a polypeptide having a different property. The property may be a biological property, such as activity *in vitro* or *in vivo.* The property may also be a simple chemical or physical property, such as binding to a target antigen, catalysis of a reaction, etc. The two portions may be linked directly by a single peptide bond or through a peptide linker containing one or more amino acid residues. Generally, the two portions and the linker will be in reading frame with each other. Preferably, the two portions of the polypeptide are obtained from heterologous or different polypeptides.

"Heterologous DNA" is any DNA that is introduced into a host cell. The DNA may be derived from a variety of sources including genomic DNA, cDNA, synthetic DNA and fusions or combinations of these. The DNA may include DNA from the same cell or cell type as the host or recipient cell or DNA from a different cell type, for example, from a mammal or plant. The DNA may, optionally, include marker or selection genes, for example, antibiotic resistance genes, temperature resistance genes, etc.

As used herein, "highly diverse position" refers to a position of an amino acid located in the variable regions of the light and heavy chains that have a number of different amino acid represented at the position when the amino acid sequences of known and/or naturally occurring antibodies or antigen binding fragments are compared. The highly diverse positions are typically in the CDR regions. In one aspect, the ability to determine highly diverse positions in known and/or naturally occurring antibodies is facilitated by the data provided by Kabat, Sequences of Proteins of Immunological Interest (National Institutes of Health, Bethesda, Md., 1987 and 1991). An internet-based database located at http:/immuno/bme/nwu/edu provides an extensive collection and alignment of human light and heavy chain sequences and facilitates determination of highly diverse positions in these sequences. According to the invention, an amino acid position is highly diverse if it has preferably from about 2 to about 11, preferably from about 4 to about 9, and preferably from about 5 to about 7 different possible amino acid residue variations at that position. In some embodiments, an amino acid position is highly diverse if it has preferably at least about 2, preferably at least about 4, preferably at least about 6, and preferably at least about 8 different possible amino acid residue variations at that position.

As used herein, "library" refers to a plurality of antibody or antibody fragment sequences (for example, polypeptides of the invention), or the nucleic acids that encode these sequences, the sequences being different in the combination of variant amino acids that are introduced into these sequences according to the methods of the invention.

"Ligation" is the process of forming phosphodiester bonds between two nucleic acid fragments. For ligation of the two fragments, the ends of the fragments must be compatible with each other. In some cases, the ends will be directly compatible after endonuclease digestion. However, it may be necessary first to convert the staggered ends commonly produced after endonuclease digestion to blunt ends to make them compatible for ligation. For blunting the ends, the DNA is treated in a suitable buffer for at least 15 minutes at 15°C with about 10 units of the Klenow fragment of DNA polymerase I or T4 DNA polymerase in the presence of the four deoxyribonucleotide triphosphates. The DNA is then purified by phenolchloroform extraction and ethanol precipitation or by silica purification. The DNA fragments that are to be ligated together are put in solution in about equimolar amounts. The solution will also contain ATP, ligase buffer, and a ligase such as T4 DNA ligase at about 10 units per 0.5 *µ*g of DNA. If the DNA is to be ligated into a vector, the vector is first linearized by digestion with the appropriate restriction endonuclease(s). The linearized fragment is then treated with bacterial alkaline phosphatase or calf intestinal phosphatase to prevent self-ligation during the ligation step.

A "mutation" is a deletion, insertion, or substitution of a nucleotide(s) relative to a reference nucleotide sequence, such as a wild type sequence.

As used herein, "natural" or "naturally occurring" antibodies, refers to antibodies identified from a nonsynthetic source, for example, from a differentiated antigen-specific B cell obtained *ex vivo,* or its corresponding hybridoma cell line, or from antibodies obtained from the serum of an animal. These antibodies can include antibodies generated in any type of immune response, either natural or otherwise induced. Natural antibodies include the amino acid sequences, and the nucleotide sequences that constitute or encode these antibodies, for example, as identified in the Kabat database. As used herein, natural antibodies are different than "synthetic antibodies", synthetic antibodies referring to antibody sequences that have been changed from a source or template sequence, for example, by the replacement, deletion, or addition, of an amino acid, or more than one amino acid, at a certain position with a different amino acid, the different amino acid providing an antibody sequence different from the source antibody sequence.

"Operably linked" when referring to nucleic acids means that the nucleic acids are placed in a functional relationship with another nucleic acid sequence. For example, DNA for a presequence or secretory leader is operably linked to DNA for a polypeptide if it is expressed as a preprotein that participates in the secretion of the polypeptide; a promotor or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous and, in the case of a secretory leader, contingent and in reading frame. However, enhancers do not have to be contiguous. Linking is accomplished by ligation at convenient restriction sites. If such sites do not exist, the synthetic oligonucleotide adapters or linkers are used in accord with conventional practice.

"Phage display" is a technique by which variant polypeptides are displayed as fusion proteins to at least a portion of coat protein on the surface of phage, e.g., filamentous phage, particles. A utility of phage display lies in the fact that large libraries of randomized protein variants can be rapidly and efficiently sorted for those sequences that bind to a target antigen with high affinity. Display of peptide and protein libraries on phage has been used for screening millions of polypeptides for ones with specific binding properties. Polyvalent phage display methods have been used for displaying small random peptides and small proteins through fusions to either gene III or gene VIII of filamentous phage. Wells and Lowman, Curr. Opin. Struct. Biol., 3:355-362 (1992), and references cited therein. In monovalent phage display, a protein or peptide library is fused to a gene III or a portion thereof, and expressed at low levels in the presence of wild type gene III protein so that phage particles display one copy or none of the fusion proteins. Avidity effects are reduced relative to polyvalent phage so that sorting is on the basis of intrinsic ligand affinity, and phagemid vectors are used, which simplify DNA manipulations. Lowman and Wells, Methods: A companion to Methods in Enzymology, 3:205-0216 (1991).

A "phagemid" is a plasmid vector having a bacterial origin of replication, e.g., Co1E1,and a copy of an intergenic region of a bacteriophage. The phagemid may be used on any known bacteriophage, including filamentous bacteriophage and lambdoid bacteriophage. The plasmid will also generally contain a selectable marker for antibiotic resistance. Segments of DNA cloned into these vectors can be propagated as plasmids. When cells harboring these vectors are provided with all genes necessary for the production of phage particles, the mode of replication of the plasmid changes to rolling circle replication to generate copies of one strand of the plasmid DNA and package phage particles. The phagemid may form infectious or non-infectious phage particles. This term includes phagemids which contain a phage coat protein gene or fragment thereof linked to a heterologous polypeptide gene as a gene fusion such that the heterologous polypeptide is displayed on the surface of the phage particle.

The term "phage vector" means a double stranded replicative form of a bacteriophage containing a heterologous gene and capable of replication. The phage vector has a phage origin of replication allowing phage replication and phage particle formation. The phage is preferably a filamentous bacteriophage, such as an M13, f1, fd, Pf3 phage or a derivative thereof, or a lambdoid phage, such as lambda, 21, phi80, phi81, 82, 424, 434, etc., or a derivative thereof.

"Oligonucleotides" are short-length, single- or double-stranded polydeoxynucleotides that are chemically synthesized by known methods (such as phosphotriester, phosphite, or phosphoramidite chemistry, using solid-phase techniques such as described in EP 266,032 published 4 May 1988, or via deoxynucloside H-phosphonate intermediates as described by Froeshler et al., Nucl. Acids, Res., 14:5399-5407 (1986)). Further methods include the polymerase chain reaction defined below and other autoprimer methods and oligonucleotide syntheses on solid supports. All of these methods are described in Engels et al., Agnew. Chem. Int. Ed. Engl., 28:716-734 (1989). These methods are used if the entire nucleic acid sequence of the gene is known, or the sequence of the nucleic acid complementary to the coding strand is available. Alternatively, if the target amino acid sequence is known, one may infer potential nucleic acid sequences using known and preferred coding residues for each amino acid residue. The oligonucleotides can be purified on polyacrylamide gels or molecular sizing columns or by precipitation.

DNA is "purified" when the DNA is separated from non-nucleic acid impurities. The impurities may be polar, non-polar, ionic, etc.

A "source antibody", as used herein, refers to an antibody or antigen binding fragment whose antigen binding sequence serves as the template sequence upon which diversification according to the criteria described herein is performed. An antigen binding sequence generally includes an antibody variable region, preferably at least one CDR, preferably including framework regions.

As used herein, "solvent accessible position" refers to a position of an amino acid residue in the variable regions of the heavy and light chains of a source antibody or antigen binding fragment that is determined, based on structure, ensemble of structures and/or modeled structure of the antibody or antigen binding fragment, as potentially available for solvent access and/or contact with a molecule, such as an antibody-specific antigen. These positions are typically found in the CDRs and on the exterior of the protein. The solvent accessible positions of an antibody or antigen binding fragment, as defined herein, can be determined using any of a number of algorithms known in the art. Preferably, solvent accessible positions are determined using coordinates from a 3-dimensional model of an antibody, preferably using a computer program such as the InsightII program (Accelrys, San Diego, CA). Solvent accessible positions can also be determined using algorithms known in the art (e.g., Lee and Richards, J. Mol. Biol. 55, 379 (1971) and Connolly, J. Appl. Cryst. 16, 548 (1983)). Determination of solvent accessible positions can be performed using software suitable for protein modeling and 3-dimensional structural information obtained from an antibody. Software that can be utilized for these purposes includes SYBYL Biopolymer Module software (Tripos Associates). Generally and preferably, where an algorithm (program) requires a user input size parameter, the "size" of a probe which is used in the calculation is set at about 1.4 Angstrom or smaller in radius. In addition, determination of solvent accessible regions and area methods using software for personal computers has been described by Pacios ((1994) "ARVOMOL/CONTOUR: molecular surface areas and volumes on Personal Computers." Comput. Chem. 18(4): 377-386; and (1995). "Variations of Surface Areas and Volumes in Distinct Molecular Surfaces of Biomolecules." J. Mol. Model. 1: 46-53.)

As used herein, "target amino acid" refers to an amino acid that belongs to the group of amino acids that are collectively the most commonly occurring amino acids found at a particular position of known and/or natural occurring antibodies or antigen binding fragments. In some embodiments, the most commonly occurring amino acids" are those amino acids that are found in a particular position in preferably at least about 50%, preferably at least about 70%, preferably at least about 80%, preferably at least about 90%, preferably all of sequences of known and/or natural antibodies or antigen binding fragments. In some embodiments, the most commonly occurring amino acids" are those amino acids that are found in a particular position in preferably from about 50% to about 100%, preferably from about 60% to about 90%, preferably from about 70% to about 85%, preferably from about 80% to about 85% of the sequences of known and/or natural antibodies or antigen binding fragments. Known antibodies or antigen binding fragments are those whose sequences are available in the art, such as those available in publicly-accessible databases, such as the database of Kabat ("Sequence of Proteins of Immunological Interest, National Institutes of Health, Bethesda, Md., 1987 and 1991) and/or as located at http:/immuno/bme/nwu/edu. The amino acid position is preferably a position in the CDR region. A target group of amino acids refers to a group of target amino acids for a particular position. Preferably, a target amino acid is not a cysteine residue. For positions in the light chain CDR1, CDR2, CDR3, and for heavy chain CDR1 and CDR2, typically, a target group of amino acids can include from preferably about 2 to about 11, preferably from about 4 to about 9, preferably from about 5 to about 7, preferably about 6 amino acids at a particular highly diverse and solvent-accessible position of the source sequence.

A "transcription regulatory element" will contain one or more of the following components: an enhancer element, a promoter, an operator sequence, a repressor gene, and a transcription termination sequence. These components are well known in the art. U.S. Patent No. 5,667,780.

A "transformant" is a cell which has taken up and maintained DNA as evidenced by the expression of a phenotype associated with the DNA (e.g., antibiotic resistance conferred by a protein encoded by the DNA).

"Transformation" means a process whereby a cell takes up DNA and becomes a "transformant". The DNA uptake may be permanent or transient.

A "variant" or "mutant" of a starting or reference polypeptide (for e.g., a source antibody or its variable domain(s)/CDR(s)), such as a fusion protein (polypeptide) or a heterologous polypeptide (heterologous to a phage), is a polypeptide that 1) has an amino acid sequence different from that of the starting or reference polypeptide and 2) was derived from the starting or reference polypeptide through either natural or artificial (manmade) mutagenesis. Such variants include, for example, deletions from, and/or insertions into and/or substitutions of, residues within the amino acid sequence of the polypeptide of interest. For example, a fusion polypeptide of the invention generated using an oligonucleotide comprising a nonrandom codon set that encodes a sequence with a variant amino acid (with respect to the amino acid found at the corresponding position in a source antibody/antigen binding fragment) would be a variant polypeptide with respect to a source antibody or antigen binding fragment. Thus, a variant CDR refers to a CDR comprising a variant sequence with respect to a starting or reference polypeptide sequence (such as that of a source antibody or antigen binding fragment). A variant amino acid, in this context, refers to an amino acid different from the amino acid at the corresponding position in a starting or reference polypeptide sequence (such as that of a source antibody or antigen binding fragment). Any combination of deletion, insertion, and substitution may be made to arrive at the final variant or mutant construct, provided that the final construct possesses the desired functional characteristics. The amino acid changes also may alter post-translational processes of the polypeptide, such as changing the number or position of glycosylation sites. Methods for generating amino acid sequence variants of polypeptides are described in U.S. Patent No. 5,534,615, expressly incorporated herein by reference.

A "wild type" or "reference" sequence or the sequence of a "wild type" or "reference" protein/polypeptide, such as a coat protein, or a CDR or variable domain of a source antibody, maybe the reference sequence from which variant polypeptides are derived through the introduction of mutations. In general, the "wild type" sequence for a given protein is the sequence that is most common in nature. Similarly, a "wild type" gene sequence is the sequence for that gene which is most commonly found in nature. Mutations may be introduced into a "wild type" gene (and thus the protein it encodes) either through natural processes or through man induced means. The products of such processes are "variant" or "mutant" forms of the original "wild type" protein or gene.

A "plurality" of a substance, such as a polypeptide or polynucleotide of the invention, as used herein, generally refers to a collection of two or more types or kinds of the substance. There are two or more types or kinds of a substance if two or more of the substances differ from each other with respect to a particular characteristic, such as the variant amino acid found at a particular amino acid position. For example, there is a plurality of polypeptides of the invention if there are two or more polypeptides of the invention that are substantially the same, preferably identical, in sequence except for the sequence of a variant CDR or except for the variant amino acid at a particular solvent accessible and highly diverse amino acid position. In another example, there is a plurality of polynucleotides of the invention if there are two or more polynucleotides of the invention that are substantially the same, preferably identical, in sequence except for the sequence that encodes a variant CDR or except for the sequence that encodes a variant amino acid for a particular solvent accessible and highly diverse amino acid position.

The invention provides methods for generating and isolating novel antibodies or antigen binding fragments that preferably have a high affinity for a selected antigen. A plurality of different antibodies or antibody variable domains are prepared by mutating (diversifying) one or more selected amino acid positions in a source antibody light chain variable domain and/or heavy chain variable domain to generate a diverse library of antibody variable domains with variant amino acids at those positions. The amino acid positions are those that are solvent accessible, for example as determined by analyzing the structure of a source antibody, and/or that are highly diverse among known and/or natural occurring immunoglobulin polypeptides.

The amino acid positions that are solvent accessible and highly diverse are preferably those in the CDR regions of the antibody variable domains selected from the group consisting of CDRL1, CDRL2, CDRL3, CDRH1, CDRH2, CDRH3, and mixtures thereof. Amino acid positions are each mutated using a non-random codon set encoding the commonly occurring amino acids at each position. In some embodiments, when a solvent accessible and highly diverse position in a CDR region is to be mutated, a codon set is selected that encodes preferably at least about 50%, preferably at least about 60%, preferably at least about 70%, preferably at least about 80%, preferably at least about 90%, preferably all the target amino acids (as defined above) for that position. In some embodiments, when a solvent accessible and highly diverse position in a CDR region is to be mutated, a codon set is selected that encodes preferably from about 50% to about 100%, preferably from about 60% to about 95%, preferably from at least about 70% to about 90%, preferably from about 75% to about 90% of all the target amino acids (as defined above) for that position. For CDRH3, variant CDRH3 regions are generated that have different lengths and/or are randomized at selected positions using codons set such as *NNK, NNS, NVT, DVK, XYZ* or tryptophan or glycine or mixtures thereof.

The diversity of the library of the antibody variable domains is designed to maximize diversity while minimizing structural perturbations of the antibody variable domain to provide for increased ability to isolate high affinity antibodies and to provide for antibodies that can be produced in high yield in cell culture. The number of positions mutated in the antibody variable domain is minimized and the variant amino acids at each position are designed to include the commonly occurring amino acids at each position, while preferably (where possible) excluding uncommonly occurring amino acids. Preferably, a single antibody, including at least one CDR, is used as the source antibody. It is surprising that a library of antibody variable domains having diversity in sequences and size can be generated using a single source antibody as a template and targeting diversity to particular positions using particular amino acid substitutions.

### Design of Diversity of Antibody Variable Domains

In one aspect of the invention, high quality libraries of antibody variable domains are generated. The libraries have diversity in number of members of the library as well as in the diversity of different sequences of the antibody variable domains. The libraries include high affinity binding antibody variable domains for one or more antigens, including, for example, insulin like growth factor-1 (IGF-1), vascular endothelial growth factor (VEGF), and Her-2. The diversity in the library is designed by selecting amino acid positions that are solvent accessible and highly diverse in a single source antibody and mutating those positions in at least one CDR using nonrandom codon sets. The nonrandom codon set preferably encodes at least a subset of the commonly occurring amino acids at those positions while minimizing nontarget sequences such as cysteine and stop codons.

A preferred source antibody is humanized antibody 4D5, but the methods for diversity design can be applied to other source antibodies whose sequence is known. A source antibody can be a naturally occurring antibody, synthetic antibody, recombinant antibody, humanized antibody, germ line derived antibody, chimeric antibody, affinity matured antibody, or antigen binding fragment thereof. The antibodies can be obtained from a variety of mammalian species including humans, mice and rats. In some embodiments, a source antibody is an antibody that is obtained after one or more initial affinity screening rounds, but prior to an affinity maturation step(s). A source antibody may be selected or modified to provide for high yield and stability when produced in cell culture.

One source antibody is the humanized antibody 4D5. Antibody 4D5 is a humanized antibody specific for a cancer-associated antigen known as Her-2 (erbB2). The antibody includes variable domains having consensus framework regions; a few positions were reverted to mouse sequence during the process of increasing affinity of the humanized antibody. The sequence and crystal structure of humanized antibody 4D5 have been described in U. S. 6,054,297, Carter et al, PNAS 89:4285 (1992), the crystal structure is shown in J Mol. Biol. 229:969 (1993) and online at www/ncbi/nih/gov/structure/ mmdb(MMDB#s-990-992).

A criterion for generating diversity in antibody variable domains is to mutate residues at positions that are solvent accessible (as defined above). These positions are typically found in the CDRs, and are typically on the exterior of the protein. Preferably, solvent accessible positions are determined using coordinates from a 3-dimensional model of an antibody, using a computer program such as the InsightII program (Accelrys, San Diego, CA). Solvent accessible positions can also be determined using algorithms known in the art (e.g., Lee and Richards, J. Mol. Biol. 55, 379 (1971) and Connolly, J. Appl. Cryst. 16, 548 (1983)). Determination of solvent accessible positions can be performed using software suitable for protein modeling and 3-dimensional structural information obtained from an antibody. Software that can be utilized for these purposes includes SYBYL Biopolymer Module software (Tripos Associates). Generally and preferably, where an algorithm (program) requires a user input size parameter, the "size" of a probe which is used in the calculation is set at about 1.4 Angstrom or smaller in radius. In addition, determination of solvent accessible regions and area methods using software for personal computers has been described by Pacios ((1994) "ARVOMOL/CONTOUR: molecular surface areas and volumes on Personal Computers", Comput. Chem. 18(4): 377-386; and "Variations of Surface Areas and Volumes in Distinct Molecular Surfaces of Biomolecules." J. Mol. Model. (1995), 1: 46-53).

In some instances, selection of solvent accessible residues is further refined by choosing solvent accessible residues that collectively form a minimum contiguous patch, for example when the reference polypeptide or source antibody is in its 3-D folded structure. For example, as shown in Figure 36, a compact (minimum) contiguous patch is formed by residues selected for CDRH1/H2/H3/L1/L2/L3 of humanized 4D5. A compact (minimum) contiguous patch may comprise only a subset (for example, 2-5 CDRs) of the full range of CDRs, for example, CDRH1/H2/H3/L3. Solvent accessible residues that do not contribute to formation of such a patch may optionally be excluded from diversification. Refinement of selection by this criterion permits the practitioner to minimize, as desired, the number of residues to be diversified. For example, residue 28 in H1 can optionally be excluded in diversification since it is on the edge of the patch. However, this selection criterion can also be used, where desired, to choose residues to be diversified that may not necessarily be deemed solvent accessible. For example, a residue that is not deemed solvent accessible, but forms a contiguous patch in the 3-D folded structure with other residues that are deemed solvent accessible may be selected for diversification. An example of this is CDRL1-29. Selection of such residues would be evident to one skilled in the art, and its appropriateness can also be determined empirically and according to the needs and desires of the skilled practitioner.

The solvent accessible positions identified from the crystal structure of humanized antibody 4D5 for each CDR are as follows (residue position according to Kabat):
CDRL1: 28,30,31,32
CDRL2:50, 53
CDRL3:91, 92, 93, 94, 96
CDRH1:28, 30, 31, 32, 33
CDRH2:50, 52, 52A, 53, 54, 55, 56, 57, 58.
In addition, residue 29 of CDRL1 was also selected based on its inclusion in a contiguous patch comprising other solvent accessible residues.

Another criterion for selecting positions to be mutated are those positions which show variability in amino acid sequence when the sequences of known and/or natural antibodies are compared. A highly diverse position refers to a position of an amino acid located in the variable regions of the light or heavy chains that have a number of different amino acids represented at the position when the amino acid sequences of known and/or, natural antibodies/antigen binding fragments are compared. The highly diverse positions are preferably in the CDR regions. The positions of CDRH3 are all considered highly diverse. According to the invention, amino acid residues are highly diverse if they have preferably from about 2 to about 11 (although the numbers can range as described herein) different possible amino acid residue variations at that position.

In one aspect, identification of highly diverse positions in known and/or naturally occurring antibodies is facilitated by the data provided by Kabat, Sequences of Proteins of Immunological Interest (National Institutes of Health, Bethesda, Md., 1987 and 1991). An internet-based database located at http/immuno/bme/nwu/edu provides an extensive collection and alignment of human light and heavy chain sequences and facilitates determination of highly diverse positions in these sequences. The diversity at the solvent accessible positions of humanized antibody 4D5 in known and/or naturally occurring light and heavy chains is shown in Figures 1 and 2.

In one aspect of the invention, the highly diverse and solvent accessible residues in at least one CDR selected from the group consisting of CDRL1, CDRL2, CDRL3, CDRH1, CDRH2 and mixtures thereof are mutated (i.e., randomized using codon sets as described herein). In some embodiments, the group also includes CDRH3. For example, the solvent accessible and/or highly diverse residues in CDRL3 and CDRH3 are mutated. Accordingly, the invention provides for a large number of novel antibody sequences formed by replacing the solvent accessible and highly diverse positions of at least one CDR of the source antibody variable domain with variant amino acids.

A target group of amino acids is the group of amino acids found at each solvent accessible and highly diverse position in a CDR in preferably at least about 50%, preferably at least about 70%, preferably at least about 80%, preferably at least about 90% of antibodies when the sequences of known and/or natural antibodies/antigen binding fragments are compared. The variant amino acids are a group of amino acids that include some or all of the target amino acids and are encoded by a nonrandom codon set. Of the amino acids encoded by the nonrandom codon set, preferably at least about 70% of the amino acids are target amino acids and more preferably at least about 80% of the amino acids are target amino acids. The nonrandom codon set for each position preferably encodes at least two amino acids and does not encode cysteine. Nontarget amino acids at each position are minimized and cysteines and stop codons are generally and preferably excluded because they can adversely affect the structure of the antibody variable domain for, in particular, L1, L2, L3, H1 and H2. For positions in the light chain CDR1, CDR2, CDR3, and for heavy chain CDR1 and CDR2, typically, a set of target amino acids can include from about two to eleven amino acids at a particular highly diverse and solvent-accessible position of the source sequence.

As discussed above, the variant amino acids are encoded by nonrandom codon sets. A codon set is a set of different nucleotide triplet sequences which can be used to form a set of oligonucleotides used to encode the desired group of amino acids. A set of oligonucleotides can be synthesized, for example, by solid phase synthesis, containing sequences that represent all possible combinations of nucleotide triplets provided by the codon set and that will encode the desired group of amino acids. Synthesis of oligonucleotides with selected nucleotide "degeneracy" at certain positions is well known in that art. Such sets of nucleotides having certain codon sets can be synthesized using commercial nucleic acid synthesizers (available from, for example, Applied Biosystems, Foster City, CA), or can be obtained commercially (for example, from Life Technologies, Rockville, MD). Therefore, a set of oligonucleotides synthesized having a particular codon set will typically include a plurality of oligonucleotides with different sequences, the differences established by the codon set within the overall sequence. Oligonucleotides, as used according to the invention, have sequences that allow for hybridization to a variable domain nucleic acid template and also can include restriction enzyme sites for cloning purposes.

In one aspect, the target amino acids were identified for each solvent accessible and highly diverse position in CDRs of humanized antibody 4D5. The target amino acids were identified by identifying different amino acids at each of the solvent accessible and highly diverse positions in CDRL1, CDRL2, CDRL3, CDRH1 and CDRH2 using the sequences of known and/or naturally occurring antibodies in the Kabat database. Light chain diversity and heavy chain diversity from the Kabat database are shown in Figures 1 and 2, respectively. Based on the diversity as shown in Figures 1 and 2, the target amino acids identified at each position are shown in Figure 3.

Illustrative nonrandom codon sets encoding a group of amino acids comprising preferably at least about 50%, preferably at least about 60%, preferably at least about 70%, preferably at least about 80%, preferably at least about 90%, preferably all of the target amino acids for each position are also shown in Figure 3. The "% good" in Figure 3 represents the percentage of amino acids encoded by the nonrandom codon set that are target amino acids for that position. Most preferably, the variant amino acids encoded by the codon set include the amino acids occurring with the highest frequency in known and/or naturally occurring antibodies. The high percentage means very low nontarget amino acids and this is more important than having more of the target amino acids in the design of the nonrandom codon set. The redundancy is included in all calculations.

The "% covering" in Figure 3, represents the percentage of known and/or natural occurring antibody sequences that are encoded by the designed codons at each position. For example, for L3-91, the amino acids YSA (tyrosine, serine and alanine) are in the group of target amino acids which occur at position 91 in known and/or naturally occurring antibodies. The codon set is designed to encode YSAD (tyrosine, serine, alanine and aspartic acid), which encodes 75% of the target amino acids. These three amino acids are also found in 1190 out of 1580 natural antibody sequences at that site, which is 75% of the known and/or natural antibodies. It is preferable that codon sets are designed for each position in a CDR region to include amino acids found in those positions in at least about 50% of the known and/or naturally occurring antibodies and more preferably in at least about 60 % of the known and/or naturally occurring antibodies and most preferably in at least about 70 % of the known and/or naturally occurring antibodies.

In one embodiment, a polypeptide having a variant CDR L1, L2, L3, H1, H2 or mixtures thereof is formed, wherein at least one variant CDR comprises a variant amino acid in at least one solvent accessible and highly diverse amino acid position, wherein the variant amino acid is encoded by a nonrandom codon set, and wherein at least 70% of the amino acids encoded by the nonrandom codon set are target amino acids for that position in known antibody variable domain sequences. For example, an antibody variable domain can comprise a variant amino acid in one or more amino acid positions 28, 30, 31, 32 and/or 33 of CDRH1; and/or in one or more amino acid positions 50, 52, 53, 54, 56 and/or 58 of CDRH2; and/or in one or more amino acid positions 28, 29, 30 and/or 31 of CDRL1; and/or in one or more amino acid positions 50 and/or 53 in CDRL2; and/or in one or more amino acid positions 91, 92, 93, 94 and/or 96. The variant amino acids at these positions are preferably encoded by codon sets as exemplified in Figures 3, 5-13 and 24.

Heavy chain CDR3s (CDRH3s) in known antibodies have diverse sequences, structural conformations, and lengths. CDRH3s are often found in the middle of the antigen binding pocket and often participate in antigen contact. The design of CDRH3 is thus preferably developed separately from that of the other CDRs because it can be difficult to predict the structural conformation of CDRH3 and the amino acid diversity in this region is especially diverse in known antibodies. In accordance with the present invention, CDRH3 is designed to generate diversity at specific positions within CDRH3 and to exclude amino acids which do not occur frequently in CDRH3 sequences at any position.

To generate diversity in CDRH3, a database of known, generally natural, antibodies can be used as a guideline. In comparison to other CDRs, CDRH3 has the highest diversity in sequences and length, although the sequence diversity is not completely random (i.e., some amino acids occur more often than others). In one embodiment, a library is generated with a degenerate codon set such as *NNK,* which codes for all 20 amino acids and a stop codon. Clones that display functionally on the phage are analyzed for their sequences. Frequency of amino acids in the synthetically-generated library is then compared with the frequency of amino acids in known antibodies. Good agreement of amino acid frequency can be expected, although in some instances there may be increased frequency of certain classes of amino acids in the synthetic library compared to known antibodies. For example, a library generated with *NNK* can be expected to contain sequences that utilize more usage of aliphatic/hydrophobic amino acids. This procedure can be performed to obtain useful information on appropriate choice of amino acids, and thus codon sets, to include in generating CDRH3 diversity. In another embodiment, CDRH3 diversity is generated using the codon set *NNS. NNS and NNK* encode the same amino acid group. However, there can be individual preferences for one codon set or the other, depending on the various factors known in the art, such as efficiency of coupling in oligonucleotide synthesis chemistry.

In some embodiments, the practitioner of methods of the invention may wish to modify the amount/proportions of individual nucleotides (G, A, T, C) for a codon set, such as the N nucleotide in a codon set such as in *NNS*. This is illustratively represented as *XYZ* codons indicated in Figure 4. This can be achieved by, for example, doping different amounts of the nucleotides within a codon set instead of using a straight, equal proportion of the nucleotides for the N in the codon set. For example, a codon set *XYZ* has the following proportions of nucleotides. X is 38% G, 19% A,, 26% T and 17% C; Y is 31% G, 34% A, 17% T and 18% C, and Z is 24% G/76% C. Such modifications can be useful for various purposes depending on the circumstances and desire of the practitioner. For example, such modifications can be made to more closely reflect the amino acid bias as seen in a natural diversity profile, such as the profile of CDRH3.

In some embodiments, a diversified CDRH3 library can be generated with a codon set such as *DVK,* which encodes 11 amino acids (ACDEGKNRSYW) and one stop codon. This excludes amino acids that occur infrequently in known antibodies. A CDRH3 library may also be generated using the codon set *NVT,* which encodes 11 amino acids (ACDGHNPRSTY), but does not encode stop codons or tryptophan (Trp, W). In some embodiments, the design of a codon set, such as *NVT,* may be doped with Trp.

As discussed previously, there is great diversity in CDRH3 regions in length of the CDRH3 regions. In natural antibodies, lengths of CDRH3 can range from 2 to 24 amino acids with the most common length being 11-13 residues. In one embodiment, diversity in CDRH3 is generated by preparing a number of different libraries, each library having a different length of CDRH3 region ranging from about 7 to 19 amino acids. The libraries with different lengths of CDRH3 regions are then pooled and used to select and/or screen for high affinity binding antibody variable domains. Illustrative embodiments of oligonucleotides that can be utilized to provide for variety in CDRH3 sequence length include those shown in Figure 4 and Figure 43.

In some embodiments, the diversity in the N and C-terminal regions of CDRH3 is minimized. At the C-terminus, there may be two types of sequences in known antibodies: Y₁₀₀ₐ AMD₁₀₁ (Y/V)₁₀₂ or F₁₀₀ₐ D₁₀₁ (Y/V)₁₀₂. Limited diversity can be targeted at selected residues in the C-terminal region. For example, Y₁₀₀ₐ position can be varied using codon sets that encode fewer amino acids such as *DVK, DSG, KSG, DSG,* or may be varied with a codon set that encodes all 20 amino acids such as *NNK, NVT, or XYZ.*

One aspect of the invention provides a polypeptide, such as an antibody variable domain, comprising a variant CDRH3 that comprises an amino acid sequence:

(X₁)ₙ - X₂ - X₃ - X₄ - X_{5―}X₆

wherein X₁ is any naturally occurring amino acid encoded by a codon set of *NNK, NNS, DVK, NVT, XYZ* or is tryptophan or glycine or mixtures thereof, and n = 4 to 14;

X₂ is any naturally occurring amino acid encoded by a codon set of *KSG, DYK, DSG, NVT, KSG, NNS, XYZ, NNK* or is tyrosine, phenylalanine, serine, alanine, glycine, leucine,or tryptophan;

X₃ is missing when X₂ is phenylalanine or leucine, or if present, is alanine, valine, aspartic acid or glycine;

X₄ is missing when X₂ is phenyalanine or leucine, or if present, is methionine;

X₅ is aspartic acid, alanine, valine, or glycine;and

X₆ is tyrosine or valine.
A polypeptide or library of polypeptides with variant CDRH3 of the formula as shown above may be generated using any one or combination of the oligonucleotides illustrated in Figure 4 and Figure 43.

In one embodiment, CDRH3 comprises an amino acid sequence (X₁)ₙ - X₂ - X₃ - X₄ - X₅―X₆, wherein X₁ is any naturally occurring amino acid encoded by a codon set of *NNK, NNS, DVK, NVT, XYZ* or tryptophan or glycine or mixtures thereof, and n = 4 to 14; X₂ is any naturally occurring amino acid encoded by a codon set of *KSG, DVK, DSG, NVT, KSG, NNS, XYZ, NNK* or is tyrosine, phenylalanine, serine, glycine,alanine, leucine, or tryptophan; X₃ is alanine, valine, aspartic acid or glycine; X₄ is methionine;X₅ is aspartic acid, alanine, valine, or glycine;and X₆ is tyrosine or valine.

In some embodiments, n is preferably 4 and X₁ is any naturally occurring amino acid encoded by codon sets *NNK, NNS or XYZ,* X₂ is any naturally occurring amino acid encoded by *KSG, NNK, XYZ* or is tyrosine, X₃ is alanine, glycine or valine, X₄ is methionine, X₅ is aspartic acid and X₆ is tyrosine. A polypeptide or plurality of polypeptides comprising variant CDRH3 region in these embodiments can be generated using at least one or more than one of oligonucleotides F66e and F66f.

In some embodiments, n is preferably 6, and X₁ is any naturally occurring amino acid encoded by codon sets *DVK, NVT, NNK, NNS or XYZ* or tryptophan or glycine or mixtures thereof, X₂ is any naturally occurring amino acid encoded by *DSG, DVK, KSG, NNK, XYZ* or is tyrosine, glycine, serine, alanine or tryptophan, X₃ is alanine, X₄ is methionine, X₅ is aspartic acid and X₆ is tyrosine. In some embodiments, X₁ corresponds to amino acid position 95, X₂ corresponds to amino acid position 100a, A is in position 100b, M is in position 100c, D is in position 101 and Y is in position 102 of CDRH3 of antibody 4D5. A polypeptide or plurality of polypeptides comprising a variant CDRH3 region in these embodiments can be generated using at least one or more than one of oligonucleotides F63, F64, F65, F66, F165, F134, F135, F163a, F166, F167, F66a, F66b, F66a1, F66b1, F190f, and F190n.

In another embodiment, CDRH3 comprises an amino acid sequence (X₁)ₙ - X₂-X₃-X₄-X₅-X₆; wherein X₁ is any naturally occurring amino acid encoded by a codon set of *NNK, NNS, DYK, NYT, XYZ* or is tryptophan or glycine or mixtures thereof, and n = 4 to 14; X₂ is leucine or phenylalanine; X₃ is missing; X₄ is missing; X₅ is aspartic acid, alanine, valine, or glycine; and X₆ is tyrosine or valine. In some embodiments, the variant CDRH3 comprises an amino acid sequence (X₁)ₙ - X₂-D-Y; wherein X₁ is any naturally occurring amino acid encoded by a codon set of *NNK, NNS, DVK, NVT, XYZ* or tryptophan or glycine or mixtures thereof, and n = 4 to 14; X₂ is leucine or phenyalanine; D is aspartic acid and Y is tyrosine. In other embodiments, n is preferably 6 and X₁ corresponds to amino acid position 95, X₂ corresponds to amino acid position 100a, X₅ is in position 101 and X₆ is in position 102 of CDRH3 of antibody 4D5. A polypeptide or plurality of polypeptides comprising a variant CDRH3 region in these embodiments can be generated using at least one or more than one of oligonucleotides F171c, F171d, F171e, F171, F185, F186, F187, F185a, F185b, F185b1, F187a, F186a, F187b, and F187c.

In another aspect of the invention, methods and polypeptides, such as antibody variable domains, are provided comprising one, two or all variant CDRs selected from the group consisting of CDRH1, CDRH2 and CDRH3, wherein said variant CDRH3 comprises an amino acid sequence

(X₁)ₙ - X₂ - X₃ - X₄ - X₅-X₆

wherein X₁ is any naturally occurring amino acid encoded by a codon set of *NNK, NNS, DYK, NVT, XYZ* or is tryptophan or glycine or mixtures thereof, and n = 4 to 14;

X₂ is any naturally occurring amino acid encoded by a codon set of *KSG, DVK, DSG, NYT, KSG, NNS, XYZ, NNK* or is tyrosine, phenylalanine, glycine, serine, alanine, leucine,or tryptophan;

X₃ is missing when X₂ is phenylalanine or leucine, or if present, is alanine, valine, aspartic acid or glycine;

X₄ is missing when X₂ is phenyalanine or leucine, or if present, is methionine;

X₅ is aspartic acid, alanine, valine, glycine;and

X₆ is tyrosine or valine;
and wherein the variant CDRH1 or CDRH2 or both comprise at least one variant amino acid in a solvent accessible and highly diverse amino acid position, wherein the variant amino acid is encoded by a nonrandom codon set, wherein at least about 50%, 60%, 70%, 80%, 90%, or all of the amino acids encoded by the nonrandom codon set are target amino for that position in known antibodies or antigen binding fragments (for e.g. antibody variable domains).

In some embodiments of any of the methods and polypeptides described herein, the position in H3 is any of positions 95 to 102 of antibody according to Kabat numbering system (e.g. antibody 4D5). For example, position 95 can have a variant amino acid encoded by codon set *DVK, NVT, NNS, XYZ, NNK* or is tryptophan; position 96 can be encoded by *DVK, NVT, NNS, XYZ,* or is glycine or tryptophan; position 97 can be encoded by *DVK, NVT, NNS, XYZ,* or is tryptophan; position 98 can be encoded by *DVK, NVT, NNS, XYZ,* or is tryptophan; position 99 can be encoded by *DVK, NVT, NNS, XYZ;* position 100 can be encoded by *DVK, NVT, NNS, XYZ,* or is tryptophan; position 100a can be encoded by *DVK, DSG, KSG, NVT,NNS, XYZ,* or can be tyrosine, glycine, serine, alanine, tryptophan, aspartic acid, methionine, phenylalanine, leucine, valine; position 100b can be encoded by *DSG,KSG, XYZ* or is alanine, tyrosine, glycine, valine, aspartic acid, methionine, or phenylalanine; Position 100c can be encoded by *KSG, XYZ,* or is methionine, phenylalanine, leucine, alanine, glycine, valine, tyrosine, aspartic acid; position 101 can be encoded by *KSG,XYZ* or is aspartic acid, methionine, alanine, valine, glycine, tyrosine, phenylalanine, leucine; and position 102 can be encoded by *KSG or XYZ* or is tyrosine, aspartic acid, methionnine, alanine, valine, glycine. A polypeptide or plurality of polypeptides comprising a variant CDRH3 region in these embodiments can be generated using at least one or more than one of oligonucleotides illustrated in Figure 4 and Figure 43.

It is contemplated that the sequence diversity of libraries created by introduction of variant amino acids in CDRH3 can be increased by combining these CDRH3 variations with variations in other regions of the antibody, specifically in other CDRs of either the light or heavy chain variable sequences. It is contemplated that the nucleic acid sequences that encode members of this set can be further diversified by introduction of other variant amino acids in the CDRs of either the light or heavy chain sequences, via codon sets. Thus, for example, in one embodiment, CDRH3 sequences from fusion polypeptides that bind a target antigen can be combined with diversified CDRL3, CDRH1, or CDRH2 sequences, or any combination of diversified CDRs.

It should be noted that in some instances framework residues may be varied relative to the sequence of a source antibody or antigen binding fragment, for example, to reflect a consensus sequence or to improve stability or display. For example, framework residues 49, 93, 94 or 71 in the heavy chain may be varied. Heavy chain framework residue 93 may be serine or alanine (which is the human consensus sequence amino acid at that position.) Heavy chain framework residue 94 may be changed to reflect framework consensus sequence from threonine to arginine or lysine. Another example of a framework residue that may be altered is heavy chain framework residue 71, which is R in about 1970 polypeptides, V in about 627 polypeptides and A in about 527 polypeptides, as found in the Kabat database. Heavy chain framework residue 49 may be alanine or glycine. In addition, optionally, the 3 N-terminal amino acids of the heavy chain variable domain can be removed. In the light chain, optionally, the arginine at amino acid position 66 can be changed to glycine.

In one aspect, the invention provides vector constructs for generating fusion polypeptides that bind with significant affinity to potential ligands. These constructs comprise a dimerizable domain that when present in a fusion polypeptide provides for increased tendency for heavy chains to dimerize to form dimers of Fab or Fab' antibody fragments/portions. These dimerization domains may include, eg. a heavy chain hinge sequence that may be present in the fusion polypeptide. Dimerization domains in fusion phage polypeptides bring two sets of fusion polypeptides (LC/HC-phage protein/fragment (such as pIII)) together, thus allowing formation of suitable linkages (such as interheavy chain disulfide bridges) between the two sets of fusion polypeptide. Vector constructs containing such dimerization domains can be used to achieve divalent display of antibody variable domains, for example the diversified fusion proteins described herein, on phage. Preferably, the intrinsic affinity of each monomeric antibody fragment (fusion polypeptide) is not significantly altered by fusion to the dimerization domain. Preferably, dimerization results in divalent phage display which provides increased avidity of phage binding, with significant decrease in off-rate, which can be determined by methods known in the art and as described herein. Dimerization domain-containing vectors of the invention may or may not also include an amber stop codon after the dimerization domain. Illustrative embodiments of vectors are shown in Figure 34.

Dimerization can be varied to achieve different display characteristics. Dimerization domains can comprise a sequence comprising a cysteine residue, a hinge region from a full-length antibody, a dimerization sequence such as leucine zipper sequence or GCN4 zipper sequence or mixtures thereof. Dimerization sequences are known in the art, and include, for example, the GCN4 zipper sequence (GRMKQLEDKVEELLSKNYHLENEVARLKKLVGERG) (SEQ ID NO: 3). The dimerization domain is preferably located at the C-terminal end of the heavy chain variable or constant domain sequence and/or between the heavy chain variable or constant domain sequence and any viral coat protein component sequence. An amber stop codon may also be present at or after the C-terminal end of the dimerization domain. In one embodiment, wherein an amber stop codon is present, the dimerization domain encodes at least one cysteine and a dimerizing sequence such as leucine zipper. In another embodiment, wherein no amber stop codon is present, then the dimerization domain comprises a single cysteine residue.

The polypeptides of the invention can also be fused to other types of polypeptides in order to provide for display of the variant polypeptides or to provide for purificiation, screening or sorting, and detection of the polypeptide. For embodiment involving phage display, the polypeptides of the invention are fused to all or a portion of a viral coat protein. Examples of viral coat protein include protein PIII, major coat protein, pVIII, Soc, Hoc, gpD, pVI and variants thereof. In addition, the variant polypeptides generated according to the methods of the invention can optionally be fused to a polypeptide marker or tag such as FLAG, polyhistidine, gD, c-myc, B-galactosidase and the like.

### Methods of Generating Libraries of Randomized Variable Domains

Methods of substituting an amino acid of choice into a template nucleic acid are well established in the art, some of which are described herein. For example, libraries can be created by targeting solvent accessible and/or highly diverse positions in at least one CDR region for amino acid substitution with variant amino acids using the Kunkel method. See, for e.g., Kunkel et al., Methods Enzymol. (1987), 154:367-382. Generation of randomized sequences is also described below in the Examples.

The sequence of oligonucleotides includes one or more of the designed codon sets for different lengths of CDRH3 or for the solvent accessible and highly diverse positions in a CDR. A codon set is a set of different nucleotide triplet sequences used to encode desired variant amino acids. Codon sets can be represented using symbols to designate particular nucleotides or equimolar mixtures of nucleotides as shown in below according to the IUB code. Typically, a codon set is represented by three capital letters eg. *NNK, NNS, DVK* and the like.

### IUB CODES

- G: Guanine
- A: Adenine
- T: Thymine
- C: Cytosine
- R: (A or G)
- Y: (C or T)
- M: (A or C)
- K: (G or T)
- S: (C or G)
- W: (A or T)
- H: (A or C or T)
- B: (C or G or T)
- V: (A or C or G)
- D: (AorGorT)H
- N: (A or C or G or T)

For example, in the codon set *DVK,* D can be nucleotides A or G or T; V can be A or G or C; and K can be G or T. This codon set can present 18 different codons and can encode amino acids Ala, Trp, Tyr, Lys, Thr, Asn, Lys, Ser, Arg, Asp, Glu, Gly, and Cys.

Oligonucleotide or primer sets can be synthesized using standard methods. A set of oligonucleotides can be synthesized, for example, by solid phase synthesis, containing sequences that represent all possible combinations of nucleotide triplets provided by the codon set and that will encode the desired group of amino acids. Synthesis of oligonucleotides with selected nucleotide "degeneracy" at certain positions is well known in that art. Such sets of nucleotides having certain codon sets can be synthesized using commercial nucleic acid synthesizers (available from, for example, Applied Biosystems, Foster City, CA), or can be obtained commercially (for example, from Life Technologies, Rockville, MD). Therefore, a set of oligonucleotides synthesized having a particular codon set will typically include a plurality of oligonucleotides with different sequences, the differences established by the codon set within the overall sequence. Oligonucleotides, as used according to the invention, have sequences that allow for hybridization to a variable domain nucleic acid template and also can include restriction enzyme sites for cloning purposes.

In one method, nucleic acid sequences encoding variant amino acids can be created by oligonucleotide-mediated mutagenesis of a nucleic acid sequence encoding a source or template polypeptide such as the antibody variable domain of 4D5. This technique is well known in the art as described by Zoller et al. Nucleic Acids Res. 10:6487-6504(1987). Briefly, nucleic acid sequences encoding variant amino acids are created by hybridizing an oligonucleotide set encoding the desired codon sets to a DNA template, where the template is the single-stranded form of the plasmid containing a variable region nucleic acid template sequence. After hybridization, DNA polymerase is used to synthesize an entire second complementary strand of the template that will thus incorporate the oligonucleotide primer, and will contain the codon sets as provided by the oligonucleotide set. Nucleic acids encoding other source or template molecules are known or can be readily determined.

Generally, oligonucleotides of at least 25 nucleotides in length are used. An optimal oligonucleotide will have 12 to 15 nucleotides that are completely complementary to the template on either side of the nucleotide(s) coding for the mutation(s). This ensures that the oligonucleotide will hybridize properly to the single-stranded DNA template molecule. The oligonucleotides are readily synthesized using techniques known in the art such as that described by Crea et al., Proc. Nat'l. Acad. Sci. USA, 75:5765 (1978).

The DNA template is generated by those vectors that are either derived from bacteriophage M13 vectors (the commercially available M13mp18 and M13mp19 vectors are suitable), or those vectors that contain a single-stranded phage origin of replication as described by Viera et al., Meth. Enzymol., 153:3 (1987). Thus, the DNA that is to be mutated can be inserted into one of these vectors in order to generate single-stranded template. Production of the single-stranded template is described in sections 4.21-4.41 of Sambrook et al., above.

To alter the native DNA sequence, the oligonucleotide is hybridized to the single stranded template under suitable hybridization conditions. A DNA polymerizing enzyme, usually T7 DNA polymerase or the Klenow fragment of DNA polymerase I, is then added to synthesize the complementary strand of the template using the oligonucleotide as a primer for synthesis. A heteroduplex molecule is thus formed such that one strand of DNA encodes the mutated form of gene 1, and the other strand (the original template) encodes the native, unaltered sequence of gene 1. This heteroduplex molecule is then transformed into a suitable host cell, usually a prokaryote such as E. *coli* JM101. After growing the cells, they are plated onto agarose plates and screened using the oligonucleotide primer radiolabelled with a 32-Phosphate to identify the bacterial colonies that contain the mutated DNA.

The method described immediately above may be modified such that a homoduplex molecule is created wherein both strands of the plasmid contain the mutation(s). The modifications are as follows: The single stranded oligonucleotide is annealed to the single-stranded template as described above. A mixture of three deoxyribonucleotides, deoxyriboadenosine (dATP), deoxyriboguanosine (dGTP), and deoxyribothymidine (dTT), is combined with a modified thiodeoxyribocytosine called dCTP-(aS) (which can be obtained from Amersham). This mixture is added to the template-oligonucleotide complex. Upon addition of DNA polymerase to this mixture, a strand of DNA identical to the template except for the mutated bases is generated. In addition, this new strand of DNA will contain dCTP-(aS) instead of dCTP, which serves to protect it from restriction endonuclease digestion. After the template strand of the double-stranded heteroduplex is nicked with an appropriate restriction enzyme, the template strand can be digested with ExoIII nuclease or another appropriate nuclease past the region that contains the site(s) to be mutagenized. The reaction is then stopped to leave a molecule that is only partially single-stranded. A complete double-stranded DNA homoduplex is then formed using DNA polymerase in the presence of all four deoxyribonucleotide triphosphates, ATP, and DNA ligase. This homoduplex molecule can then be transformed into a suitable host cell.

As indicated previously the sequence of the oligonucleotide set is of sufficient length to hybridize to the template nucleic acid and may also, but does not necessarily, contain restriction sites. The DNA template can be generated by those vectors that are either derived from bacteriophage M13 vectors or vectors that contain a single-stranded phage origin of replication as described by Viera et al. ((1987) Meth. Enzymol., 153:3). Thus, the DNA that is to be mutated must be inserted into one of these vectors in order to generate single-stranded template. Production of the single-stranded template is described in sections 4.21-4.41 of Sambrook et al., supra.

According to another method, a library can be generated by providing upstream and downstream oligonucleotide sets, each set having a plurality of oligonucleotides with different sequences, the different sequences established by the codon sets provided within the sequence of the oligonucleotides. The upstream and downstream oligonucleotide sets, along with a variable domain template nucleic acid sequence, can be used in a polymerase chain reaction to generate a "library" of PCR products. The PCR products can be referred to as "nucleic acid cassettes", as they can be fused with other related or unrelated nucleic acid sequences, for example, viral coat protein components and dimerization domains, using established molecular biology techniques.

The sequence of the PCR primers includes one or more of the designed codon sets for the solvent accessible and highly diverse positions in a CDR region. As described above, a codon set is a set of different nucleotide triplet sequences used to encode desired variant amino acids.

Oligonucleotide sets can be used in a polymerase chain reaction using a variable region nucleic acid template sequence as the template to create nucleic acid cassettes. The variable region nucleic acid template sequence can be any portion of the light or heavy immunoglobulin chains containing the target nucleic acid sequences (ie., nucleic acid sequences encoding amino acids targeted for substitution). The variable region nucleic acid template sequence is a portion of a double stranded DNA molecule having a first nucleic acid strand and complementary second nucleic acid strand. The variable region nucleic acid template sequence contains at least a portion of a variable domain and has at least one CDR. In some cases, the variable region nucleic acid template sequence contains more than one CDR. An upstream portion and a downstream portion of the variable region nucleic acid template sequence can be targeted for hybridization with members of an upstream oligonucleotide set and a downstream oligonucleotide set.

A first oligonucleotide of the upstream primer set can hybridize to the first nucleic acid strand and a second oligonucleotide of the downstream primer set can hybridize to the second nucleic acid strand. The oligonucleotide primers can include one or more codon sets and be designed to hybridize to a portion of the variable region nucleic acid template sequence. Use of these oligonucleotides can introduce two or more codon sets into the PCR product (ie., the nucleic acid cassette) following PCR. The oligonucleotide primer that hybridizes to regions of the nucleic acid sequence encoding the antibody variable domain includes portions that encode CDR residues that are targeted for amino acid substitution.

The upstream and downstream oligonucleotide sets can also be synthesized to include restriction sites within the oligonucleotide sequence. These restriction sites can facilitate the insertion of the nucleic acid cassettes [ie., PCR reaction products] into an expression vector having additional antibody sequences. Preferably, the restriction sites are designed to facilitate the cloning of the nucleic acid cassettes without introducing extraneous nucleic acid sequences or removing original CDR or framework nucleic acid sequences.

Nucleic acid cassettes can be cloned into any suitable vector for expression of a portion or the entire light or heavy chain sequence containing the targeted amino acid substitutions generated. According to methods detailed in the invention, the nucleic acid cassette is cloned into a vector allowing production of a portion or the entire light or heavy chain sequence fused to all or a portion of a viral coat protein (ie., creating a fusion protein) and displayed on the surface of a particle or cell. While several types of vectors are available and may be used to practice this invention, phagemid vectors are the preferred vectors for use herein, as they may be constructed with relative ease, and can be readily amplified. Phagemid vectors generally contain a variety of components including promoters, signal sequences, phenotypic selection genes, origin of replication sites, and other necessary components as are known to those of ordinary skill in the art.

In another embodiment, wherein a particular variant amino acid combination is to be expressed, the nucleic acid cassette contains a sequence that is able to encode all or a portion of the heavy or light chain variable domain, and is able to encode the variant amino acid combinations. For production of antibodies containing these variant amino acids or combinations of variant amino acids, as in a library, the nucleic acid cassettes can be inserted into an expression vector containing additional antibody sequence, for example all or portions of the variable or constant domains of the light and heavy chain variable regions. These additional antibody sequences can also be fused to other nucleic acids sequences, such as sequences which encode viral coat protein components and therefore allow production of a fusion protein.

### Vectors

One aspect of the invention includes a replicable expression vector comprising a nucleic acid sequence encoding a gene fusion, wherein the gene fusion encodes a fusion protein comprising an antibody variable domain, or an antibody variable domain and a constant domain, fused to all or a portion of a viral coat protein. Also included is a library of diverse replicable expression vectors comprising a plurality of gene fusions encoding a plurality of different fusion proteins including a plurality of the antibody variable domains generated with diverse sequences as described above. The vectors can include a variety of components and are preferably constructed to allow for movement of antibody variable domain between different vectors and /or to provide for display of the fusion proteins in different formats.

Examples of vectors include phage vectors. The phage vector has a phage origin of replication allowing phage replication and phage particle formation. The phage is preferably a filamentous bacteriophage, such as an M13, f1, fd, Pf3 phage or a derivative thereof, or a lambdoid phage, such as lambda, 21, phi80, phi81, 82, 424, 434, etc., or a derivative thereof.

Examples of viral coat proteins include infectivity protein PIII, major coat protein PVIII, p3, Soc (T4), Hoc (T4), gpD (of bacteriophage lambda), minor bacteriophage coat protein 6 (pVI) (filamentous phage; J Immunol Methods. 1999 Dec 10;231(1-2):39-51), variants of the M13 bacteriophage major coat protein (P8) (Protein Sci 2000 Apr;9(4):647-54). The fusion protein can be displayed on the surface of a phage and suitable phage systems include M13K07 helper phage, M13R408, M13-VCS, and Phi X 174, pJuFo phage system (J Virol. 2001 Aug;75(15):7107-13.v), hyperphage (Nat Biotechnol. 2001 Jan;19(1):75-8). The preferred helper phage is M13KO7, and the preferred coat protein is the M13 Phage gene III coat protein. The preferred host is *E. coli,* and protease deficient strains of *E. coli.* Vectors, such as the fth1 vector (Nucleic Acids Res. 2001 May 15;29(10):E50-0) can be useful for the expression of the fusion protein.

The expression vector also can have a secretory signal sequence fused to the DNA encoding each subunit of the antibody or fragment thereof. This sequence is typically located immediately 5' to the gene encoding the fusion protein, and will thus be transcribed at the amino terminus of the fusion protein. However, in certain cases, the signal sequence has been demonstrated to be located at positions other than 5' to the gene encoding the protein to be secreted. This sequence targets the protein to which it is attached across the inner membrane of the bacterial cell. The DNA encoding the signal sequence may be obtained as a restriction endonuclease fragment from any gene encoding a protein that has a signal sequence. Suitable prokaryotic signal sequences may be obtained from genes encoding, for example, LamB or OmpF (Wong et al., Gene, 68:1931 (1983), Ma1E, PhoA and other genes. A preferred prokaryotic signal sequence for practicing this invention is the E. coli heat-stable enterotoxin II (STII) signal sequence as described by Chang et al., Gene 55:189 (1987), and malE.

The vector also typically includes a promoter to drive expression of the fusion protein. Promoters most commonly used in prokaryotic vectors include the lac Z promoter system, the alkaline phosphatase pho A promoter (Ap), the bacteriophage λ_{PL} promoter (a temperature sensitive promoter), the tac promoter (a hybrid trp-lac promoter that is regulated by the lac repressor), the tryptophan promoter, and the bacteriophage T7 promoter. For general descriptions of promoters, see section 17 of Sambrook et al. supra. While these are the most commonly used promoters, other suitable microbial promoters may be used as well.

The vector can also include other nucleic acid sequences, for example, sequences encoding gD tags, c-Myc epitopes, poly-histidine tags, fluorescence proteins (eg., GFP), or beta-galactosidase protein which can be useful for detection or purification of the fusion protein expressed on the surface of the phage or cell. Nucleic acid sequences encoding, for example, a gD tag, also provide for positive or negative selection of cells or virus expressing the fusion protein. In some embodiments, the gD tag is preferably fused to an antibody variable domain which is not fused to the viral coat protein component. Nucleic acid sequences encoding, for example, a polyhistidine tag, are useful for identifying fusion proteins including antibody variable domains that bind to a specific antigen using immunohistochemistry. Tags useful for detection of antigen binding can be fused to either an antibody variable domain not fused to a viral coat protein component or an antibody variable domain fused to a viral coat protein component.

Another useful component of the vectors used to practice this invention is phenotypic selection genes. Typical phenotypic selection genes are those encoding proteins that confer antibiotic resistance upon the host cell. By way of illustration, the ampicillin resistance gene (*ampr*)*,* and the tetracycline resistance gene (*tetr*) are readily employed for this purpose.

The vector can also include nucleic acid sequences containing unique restriction sites and suppressible stop codons. The unique restriction sites are useful for moving antibody variable domains between different vectors and expression systems, especially useful for production of full-length antibodies or antigen binding fragments in cell cultures. The suppressible stop codons are useful to control the level of expression of the fusion protein and to facilitate purification of soluble antibody fragments. For example, an amber stop codon can be read as Gln in a *supE* host to enable phage display, while in a non-*sup*E host it is read as a stop codon to produce soluble antibody fragments without fusion to phage coat proteins. These synthetic sequences can be fused to one or more antibody variable domains in the vector.

It is preferable to use vector systems that allow the nucleic acid encoding an antibody sequence of interest, for example a CDR having variant amino acids, to be easily removed from the vector system and placed into another vector system. For example, appropriate restriction sites can be engineered in a vector system to facilitate the removal of the nucleic acid sequence encoding an antibody or antibody variable domain having variant amino acids. The restriction sequences are usually chosen to be unique in the vectors to facilitate efficient excision and ligation into new vectors. Antibodies or antibody variable domains can then be expressed from vectors without extraneous fusion sequences, such as viral coat proteins or other sequence tags.

Between nucleic acid encoding antibody variable or constant domain (gene 1) and the viral coat protein component (gene 2), DNA encoding a termination or stop codon may be inserted, such termination codons including UAG (amber), UAA (ocher) and UGA (opel). (Microbiology, Davis et al., Harper & Row, New York, 1980, pp. 237, 245-47 and 374). The termination or stop codon expressed in a wild type host cell results in the synthesis of the gene 1 protein product without the gene 2 protein attached. However, growth in a suppressor host cell results in the synthesis of detectable quantities of fused protein. Such suppressor host cells are well known and described, such as *E. coli* suppressor strain (Bullock et al., BioTechniques 5:376-379 (1987)). Any acceptable method may be used to place such a termination codon into the mRNA encoding the fusion polypeptide.

The suppressible codon may be inserted between the first gene encoding a antibody variable or constant domain, and a second gene encoding at least a portion of a phage coat protein. Alternatively, the suppressible termination codon may be inserted adjacent to the fusion site by replacing the last amino acid triplet in the antibody variable domain or the first amino acid in the phage coat protein. The suppressible termination codon may be located at or after the C-terminal end of a dimerization domain. When the plasmid containing the suppressible codon is grown in a suppressor host cell, it results in the detectable production of a fusion polypeptide containing the polypeptide and the coat protein. When the plasmid is grown in a non-suppressor host cell, the antibody variable domain is synthesized substantially without fusion to the phage coat protein due to termination at the inserted suppressible triplet UAG, UAA, or UGA. In the non-suppressor cell the antibody variable domain is synthesized and secreted from the host cell due to the absence of the fused phage coat protein which otherwise anchored it to the host membrane.

In some embodiments, the CDR being diversified (randomized) may have a stop codon engineered in the template sequence (referred to herein as a "stop template"). This feature provides for detection and selection of successfully diversified sequences based on successful repair of the stop codon(s) in the template sequence due to incorporation of the oligonucleotide(s) comprising the sequence(s) for the variant amino acids of interest. This feature is further illustrated in the Examples below.

The light and/or heavy chain antibody variable or constant domains can also be fused to an additional peptide sequence, the additional peptide sequence providing for the interaction of one or more fusion polypeptides on the surface of the viral particle or cell. These peptide sequences are herein referred to as "dimerization domains". Dimerization domains may comprise at least one or more of a dimerization sequence, or at least one sequence comprising a cysteine residue or both. Suitable dimerization sequences include those of proteins having amphipathic alpha helices in which hydrophobic residues are regularly spaced and allow the formation of a dimer by interaction of the hydrophobic residues of each protein; such proteins and portions of proteins include, for example, leucine zipper regions. Dimerization domains can also comprise one or more cysteine residues (e.g. as provided by inclusion of an antibody hinge sequence within the dimerization domain). The cysteine residues can provide for dimerization by formation of one or more disulfide bonds. In one embodiment, wherein a stop codon is present after the dimerization domain, the dimerization domain comprises at least one cysteine residue. The dimerization domains are preferably located between the antibody variable or constant domain and the viral coat protein component.

In some cases the vector encodes a single antibody-phage polypeptide in a single chain form containing, for example, both the heavy and light chain variable regions fused to a coat protein. In these cases the vector is considered to be "monocistronic", expressing one transcript under the control of a certain promoter. Illustrative examples of such vectors are shown in Figures 34C and D. In Figure 34C, a vector is shown as utilizing the alkaline phosphatase (AP) or Tac promoter to drive expression of a monocistronic sequence encoding VL and VH domains, with a linker peptide between the VL and VH domains. This cistronic sequence is connected at the 5' end to an E. coli *malE* or heat-stable enterotoxin II (STII) signal sequence and at its 3' end to all or a portion of a viral coat protein (shown in the Figure 34 as the pIII protein). The fusion polypeptide encoded by this vector is referred to herein as "ScFv-pIII". In some embodiments, illustrated in Figure 34D, the vector may further comprise a sequence encoding a dimerization domain (such as a leucine zipper) at its 3' end, between the second variable domain sequence (VH in Figure 34D) and the viral coat protein sequence. Fusion polypeptides comprising the dimerization domain are capable of dimerizing to form a complex of two scFv polypeptides (referred to herein as "(ScFv)2-pIII)").

In other cases, the variable regions of the heavy and light chains can be expressed as separate polypeptides, the vector thus being "bicistronic", allowing the expression of separate transcripts. Examples of bicistronic vectors are schematically shown in Figures 34A and 4B. In these vectors, a suitable promoter, such as the Ptac or PhoA promoter, can be used to drive expression of a bicistronic message. A first cistron, encoding, for example, a light chain variable and constant domain, is connected at the 5' end to a E. coli *malE* or heat-stable enterotoxin II (STII) signal sequence and at the 3' end to a nucleic acid sequence encoding a gD tag. A second cistron, encoding, for example, a heavy chain variable domain and constant domain CH1, is connected at its 5' end to a E. coli *malE* or heat-stable enterotoxin II (STII) signal sequence and at the 3' end to all or a portion of a viral coat protein.

A vector which provides a bicistronic message and for display of F(ab')₂-pIII is shown in Figure 34B. In this vector, a suitable promoter, such as Ptac or PhoA (AP) promoter drives expression of first cistron encoding a light chain variable and constant domain operably linked at 5' end to a E. coli *malE* or heat stable enteroxtoxin II (STII) signal sequence and at the 3' end to a nucleic acid sequence encoding a gD tag. The second cistron encodes, for example, a heavy chain variable and constant domain operatively linked at 5' end to a E. coli *malE* or heat stable enterotoxin II (STII) signal sequence and at 3' end has a dimerization domain comprising IgG hinge sequence and a leucine zipper sequence followed by at least a portion of viral coat protein.

### Display of Fusion Polypeptides

Fusion polypeptides of an antibody variable domain can be displayed on the surface of a cell, virus, or phagemid particle in a variety of formats. These formats include single chain Fv fragment (scFv), F(ab) fragment and multivalent forms of these fragments. The multivalent forms preferably are a dimer of ScFv, Fab, or F(ab'), herein referred to as (ScFV)₂, F(ab)₂ and F(ab')₂, respectively. The multivalent forms of display are preferred in part because they have more than one antigen binding site which generally results in the identification of lower affinity clones and also allows for more efficient sorting of rare clones during the selection process.

Methods for displaying fusion polypeptides comprising antibody fragments, on the surface of bacteriophage, are well known in the art, for example as described in patent publication number WO 92/01047 and herein. Other patent publications WO 92/20791; WO 93/06213; WO 93/11236 and WO 93/19172, describe related methods and are all herein incorporated by reference. Other publications have shown the identification of antibodies with artificially rearranged V gene repertoires against a variety of antigens displayed on the surface of phage (for example, H. R Hoogenboom & G. Winter J. Mol. Biol. 227 381-388 1992; and as disclosed in WO 93/06213 and WO 93/11236).

When a vector is constructed for display in a scFv format, it includes nucleic acid sequences encoding an antibody variable light chain domain and an antibody variable heavy chain variable domain. Typically, the nucleic acid sequence encoding an antibody variable heavy chain domain is fused to a viral coat protein component. One or both of the antibody variable domains can have variant amino acids in at least one CDR region. The nucleic acid sequence encoding the antibody variable light chain is connected to the antibody variable heavy chain domain by a nucleic acid sequence encoding a peptide linker. The peptide linker typically contains about 5 to 15 amino acids. Optionally, other sequences encoding, for example, tags useful for purification or detection can be fused at the 3' end of either the nucleic acid sequence encoding the antibody variable light chain or antibody variable heavy chain domain or both.

When a vector is constructed for F(ab) display, it includes nucleic acid sequences encoding antibody variable domains and antibody constant domains. A nucleic acid encoding a variable light chain domain is fused to a nucleic acid sequence encoding a light chain constant domain. A nucleic acid sequence encoding an antibody heavy chain variable domain is fused to a nucleic acid sequence encoding a heavy chain constant CH1 domain. Typically, the nucleic acid sequence encoding the heavy chain variable and constant domains are fused to a nucleic acid sequence encoding all or part of a viral coat protein. One or both of the antibody variable light or heavy chain domains can have variant amino acids in at least one CDR. The heavy chain variable and constant domains are preferably expressed as a fusion with at least a portion of a viral coat protein and the light chain variable and constant domains are expressed separately from the heavy chain viral coat fusion protein. The heavy and light chains associate with one another, which may be by covalent or non-covalent bonds. Optionally, other sequences encoding, for example, polypeptide tags useful for purification or detection, can be fused at the 3' end of either the nucleic acid sequence encoding the antibody light chain constant domain or antibody heavy chain constant domain or both.

Preferably a bivalent moiety, for example, a F(ab)₂ dimer or F(ab')₂ dimer, is used for displaying antibody fragments with the variant amino acid substitutions on the surface of a particle. It has been found that F(ab')₂ dimers have the same affinity as F(ab) dimers in a solution phase antigen binding assay but the off rate for F(ab')₂ are reduced because of a higher avidity. Therefore the bivalent format (for example, F(ab')₂) is a particularly useful format since it can allow for the identification of lower affinity clones and also allows more efficient sorting of rare clones during the selection process.

### Introduction of Vectors into Host Cells

Vectors constructed as described in accordance with the invention are introduced into a host cell for amplification and/or expression. Vectors can be introduced into host cells using standard transformation methods including electroporation, calcium phosphate precipitation and the like. If the vector is an infectious particle such as a virus, the vector itself provides for entry into the host cell. Transfection of host cells containing a replicable expression vector which encodes the gene fusion and production of phage particles according to standard procedures provides phage particles in which the fusion protein is displayed on the surface of the phage particle.

Replicable expression vectors are introduced into host cells using a variety of methods. In one embodiment, vectors can be introduced into cells using electroporation as described in WO/00106717. Cells are grown in culture in standard culture broth, optionally for about 6-48 hours (or to OD₆₀₀ = 0.6 - 0.8) at about 37°C, and then the broth is centrifuged and the supernatant removed (e.g. decanted). Initial purification is preferably by resuspending the cell pellet in a buffer solution (e.g. 1.0 mM HEPES pH 7.4) followed by recentriguation and removal of supernatant. The resulting cell pellet is resuspended in dilute glycerol (e.g. 5-20% v/v) and again recentrifuged to form a cell pellet and the supernatant removed. The final cell concentration is obtained by resuspending the cell pellet in water or dilute glycerol to the desired concentration.

A particularly preferred recipient cell is the electroporation competent *E. coli* strain of the present invention, which is *E. coli* strain SS320 (Sidhu et al., Methods Enzymol. (2000), 328:333-363). Strain SS320 was prepared by mating MC1061 cells with XL1-BLUE cells under conditions sufficient to transfer the fertility episome (F' plasmid) or XL1-BLUE into the MC1061 cells. Strain SS320 has been deposited with the American Type Culture Collection (ATCC), 10801 University Boulevard, Manassas, Virginia USA, on June 18, 1998 and assigned Deposit Accession No. 98795. Any F' episome which enables phage replication in the strain may be used in the invention. Suitable episomes are available from strains deposited with ATCC or are commercially available (CJ236, CSH18, DHF', JM101, JM103, JM105, JM107, JM109, JM110), KS1000, XL1-BLUE, 71-18 and others).

The use of higher DNA concentrations during electroporation (about 10X) increases the transformation efficiency and increases the amount of DNA transformed into the host cells. The use of high cell concentrations also increases the efficiency (about 10X). The larger amount of transferred DNA produces larger libraries having greater diversity and representing a greater number of unique members of a combinatorial library. Transformed cells are generally selected by growth on antibiotic containing medium.

### Selection (sorting) and Screening for Binders to targets of choice

Use of phage display for identifying target antigen binders, with its various permutations and variations in methodology, are well established in the art. One approach involves constructing a family of variant replicable vectors containing a transcription regulatory element operably linked to a gene fusion encoding a fusion polypeptide, transforming suitable host cells, culturing the transformed cells to form phage particles which display the fusion polypeptide on the surface of the phage particle, followed by a process that entails selection or sorting by contacting the recombinant phage particles with a target antigen so that at least a portion of the population of particles bind to the target with the objective to increase and enrich the subsets of the particles which bind from particles relative to particles that do not bind in the process of selection. The selected pool can be amplified by infecting host cells, such as fresh XL1 -Blue cells, for another round of sorting on the same target with different or same stringency. The resulting pool of variants are then screened against the target antigens to identify novel high affinity binding proteins. These novel high affinity binding proteins can be useful as therapeutic agents as antagonists or agonists, and/or as as diagonostic and research reagents.

Fusion polypeptides such as antibody variable domains comprising the variant amino acids can be expressed on the surface of a phage, phagemid particle or a cell and then selected and/or screened for the ability of members of the group of fusion polypeptides to bind a target antigen which is typically an antigen of interest. The processes of selection for binders to target can also be include sorting on a generic protein having affinity for antibody variable domains such as protein L or a tag specific antibody which binds to antibody or antibody fragments displayed on phage, which can be used to enrich for library members that display correctly folded antibody fragments (fusion polypeptides).

Target proteins, such as receptors, may be isolated from natural sources or prepared by recombinant methods by procedures known in the art. Target antigens can include a number of molecules of therapeutic interest.

Two main strategies of selection (sorting) for affinity can be used as depicted schematically in Figure 45. The first strategy (left of Figure) is solid-support method or plate sorting or immobilized target sorting. The second strategy is a solution-binding method (right).

For the solid support method, the target protein may be attached to a suitable solid or semi solid matrix which are known in the art such as agarose beads, acrylamide beads, glass beads, cellulose, various acrylic copolymers, hydroxyalkyl methacrylate gels, polyacrylic and polymethacrylic copolymers, nylon, neutral and ionic carriers, and the like. Attachment of the target protein to the matrix may be accomplished by methods described in Methods in Enzymology, 44 (1976), or by other means known in the art.

After attachment of the target antigen to the matrix, the immobilized target is contacted with the library expressing the fusion polypeptides under conditions suitable for binding of at least a subset of the phage particle population with the immobilized target antigen. Normally, the conditions, including pH, ionic strength, temperature and the like will mimic physiological conditions. Bound particles ("binders") to the immobilized target are separated from those particles that do not bind to the target by washing. Wash conditions can be adjusted to result in removal of all but the high affinity binders. Binders may be dissociated from the immobilized target by a variety of methods. These methods include competitive dissociation using the wild-type ligand (e.g. excess target antigen), altering pH and/or ionic strength, and methods known in the art. Selection of binders typically involves elution from an affinity matrix with a suitable elution material such as acid like 0.1M HCl or ligand. Elution with increasing concentrations of ligand could elute displayed binding molecules of increasing affinity.

The binders can be isolated and then re-amplified in suitable host cells by infecting the cells with the viral particles that are binders (and helper phage if necessary, e.g.when viral particle is a phagemid particle)and the host cells are cultured under conditions suitable for amplification of the particles that display the desired fusion polypeptide. The phage particles are then collected and the selection process is repeated one or more times until binders of the target antigen are enriched in a way. any number of rounds of selection or sorting can be utilized. One of the selection or sorting procedures can involve isolating binders that bind to a generic affinity protein such as protein L or an antibody to a polypeptide tag present in a displayed polypeptide such as antibody to the gD protein or polyhistidine tag.

One aspect of the invention involves a novel selection method which is termed "solution-binding method" as schematized in Figure 45 (right panel). The invention allows solution phase sorting with much improved efficiency over the conventional solution sorting method. The solution binding method has been used for finding original binders from a random library or finding improved binders from a library that was designated to improve affinity of a particular binding clone or group of clones. The method comprises contacting a plurality of polypeptides, such as those displayed on phage or phagemid particles(library), with a target antigen labelled or fused with a tag molecule. The tag could be biotin or other moieties for which specific binders are available. The stringency of the solution phase can be varied by using decreasing concentrations of labelled target antigen in the first solution binding phase. To further increase the stringency, the first solution binding phase can be followed by a second solution phase having high concentration of unlabelled target antigen after the initial binding with the labelled target in the first solution phase. Usually, 100 to 1000 fold of unlabelled target over labelled target is used in the second phase (if included). The length of time of incubation of the first solution phase can vary from a few minutes to one to two hours or longer to reach equilibrium. Using a shorter time for binding in this first phase may bias or select for binders that have fast on-rate. The length of time and temperature of incubation in second phase can be varied to increase the stringency. This provides for a selection bias for binders that have slow rate of coming off the target (off-rate). After contacting the plurality of polypeptides (displayed on the phage/ phagemid particles) with a target antigen, the phage or phagemid particles that are bound to labelled targets are separated from phage that do not bind. The particle-target mixture from solution phase of binding is isolated by contacting it with the labelled target moiety and allowing for its binding to, a molecule that binds the labelled target moiety for a short period of time (eg. 2-5 minutes). The initial concentration of the labelled target antigen can range from about 0.1 nM to about 1000nM. The bound particles are eluted and can be propagated for next round of sorting. Multiple rounds of sorting are preferred using a lower concentration of labelled target antigen with each round of sorting.

For example, an initial sort or selection using about 100 to 250 nM labelled target antigen should be sufficient to capture a wide range of affinities, although this factor can be determined empirically and/or to suit the desire of the practitioner. In the second round of selection, about 25 to 100 nM of labelled target antigen may be used. In the third round of selection, about 0.1 to 25 nM of labled target antigen may be used. For example, to improve the affinity of a 100 nM binder, it may be desirable to start with 20 nM and then progress to 5 and 1 nM labelled target, then, followed by even lower concentrations such as about 0.1 nM labelled target antigen.

The conventional solution sorting involves use of beads like strepavidin coated beads, which is very cumbersome to use and often results in very low efficiency of phage binders recovery. The conventional solution sorting with beads takes much longer than 2-5 minutes and is less feasible to adapt to high throughput automation than the invention described above.

As described herein, combinations of solid support and solution sorting methods can be advantageously used to isolate binders having desired characteristics.After selection/sorting on target antigen for a few rounds, screening of individual clones from the selected pool generally is performed to identify specific binders with the desired properties/ characteristics. Preferably, the process of screening is carried out by automated systems to allow for high-throughput screening of library candidates.

Two major screening methods are described below. However, other methods known in the art may also be used in the methods of the invention. The first screening method comprises a phage ELISA assay with immobilized target antigen, which provides for identification of a specific binding clone from a non-binding clone. Specificity can be determined by simultaneous assay of the clone on target coated well and BSA or other non-target protein coated wells. This assay is automatable for high throughput screening.

One embodiment provides a method of selecting for an antibody variable domain that binds to a specific target antigen from a library of antibody variable domain by generating a library of replicable expression vectors comprising a plurality of polypeptides ; contacting the library with a target antigen and at least one nontarget antigen under conditions suitable for binding;separating the polypeptide binders in the library from the nonbinders; identifying the binders that bind to the target antigen and do not bind to the nontarget antigen; eluting the binders from the target antigen;and amplifying the replicable expression vectors comprising the polypeptide binder that bind to a specific antigen.

The second screening assay is an invention embodied in this application which is a affinity screening assay that provides for screening for clones that have high affinity from clones that have low affinity in a high throughput manner. In the assay, each clone is assayed with and without first incubating with target antigen of certain concentration for a period of time (for e.g 30-60 minutes) before application to target coated wells briefly (e.g.5-15 minutes). Then bound phage is measured by usual phage ELISA method, eg. using anti-M13 HRP conjugates. The ratio of binding signal of the two wells, one well having been preincubated with target and the other well not preincubated with target antigen is an indication of affinity. The selection of the concentraion of target for first incubation depends on the affinity range of interest. For example, if binders with affinity higher than 10nM are desired, 100nM of target in the first incubation is often used. See Figure 47 and example 9 for detailed description of one embodiment of the method. Once binders are found from a particular round of sorting (selection), these clones can be screened with affinity screening assay to identify binders with higher affinity.

Combinations of any of the sorting/ selection methods described above may be combined with the screening methods. For example, in one embodiment, polypeptide binders are first selected for binding to immobilized target antigen. Polypeptide binders that bind to the immobilized target antigen can then be amplified and screened for binding to the target antigen and for lack of binding to nontarget antigens. Polypeptide binders that bind specifically to the target antigen are amplified. These polypeptide binders can then selected for higher affinity by contact with a concentration of a labelled target antigen to form a complex, wherein the concentration ranges of labelled target antigen from about 0.1 nM to about 1000 nM, the complexes are isolated by contact with an agent that binds to the label on the target antigen. The polypeptide binders are then eluted from the labled target antigen and optionally, the rounds of selection are repeated, each time a lower concentration of labelled target antigen is used. The high affinity polypeptide binders isolated using this selection method can then be screened for high affinity using for example, the solution phase ELISA assay as described in Example 8 or the spot competition ELISA assay as described in Example 9.

These methods can provide for finding clones with high affinity without having to perform long and complex competition affinity assays on a large number of clones. The intensive aspect of doing complex assays of many clones often is a significant obstacle to finding best clones from a selection. This method is especially useful in affinity improvement efforts where multiple binders with similar affinity can be recovered from the selection process. Different clones may have very different efficiency of expression/display on phage or phagemid particles. Those clones more highly expressed have better chances being recovered. That is, the selection can be biased by the display or expression level of the variants. The solution-binding sorting method of the invention can improve the selection process for finding binders with high affinity. This method is an affinity screening assay that provides a significant advantage in screening for the best binders quickly and easily.

After binders are identified by binding to the target antigen, the nucleic acid can be extracted. Extracted DNA can then be used directly to transform E. coli host cells or alternatively, the encoding sequences can be amplified, for example using PCR with suitable primers, and sequenced by typical sequencing method. Variable domain DNA of the binders can be restriction enzyme digested and then inserted into a vector for protein expression.

In one embodiment, the invention provides isolation of novel antibody and antibody fragments that bind to mVEGF (murine Vascular Endothelial Growth Factor). Preferably, the antibody variable domains bind mVEGF with an IC₅₀ of less than 10 µM and more preferably less than 1 µM. In one embodiment, mVEGF- binding antibodies include members of the library created by substituting amino acid in residues in CDR-L3 and residues 95-100a of the CDRH3 region of the variable region of the heavy chain with *DVK* codon sets or a combination of *DYK* and *NNK* codon sets. It has been discovered that some members of the library, as created above, have a particularly high affinity for mVEGF.

In particular, antibodies including the heavy chain CDR3 sequences SRNAWAF (SEQ ID NO: 5; amino acid position 93-100c), SRNLSENSYAM (SEQ ID NO: 6; amino acid position 93-100c), SRAGWAGWYAM (SEQ ID NO: 7; amino acid position 93-100c), SRAAKAGWYAM (SEQ ID NO: 8; amino acid position 93-100c), and SRSDGRDSAYAM (SEQ ID NO: 9; amino acid position 93-100c) display high affinity binding to mVEGF. Novel antibody variable domains that bind to mVEGF, generated by substituting amino acids at positions in other CDRs, such as L1, L2, L3, H1 and H2 can also be generated according to the method described herein.

In some cases, novel CDRH1/H2/H3 binders for example, sequences can be combined with other sequences generated by introducing variant amino acids via codon sets into the other light chains CDRs, for example through a 2-step process. An example of a 2-step process comprises first determining binders (generally lower affinity binders) within one or more libraries generated by randomizing one or more CDRs, wherein the CDRs randomized are each library are different or, where the same CDR is randomized, it is randomized to generate different sequences. Binders from a heavy chain library can then be randomized with CDR diversity in a light chain CDRs by kunkle mutagenesis or by cloning (cut-and-paste, (eg. by ligating different CDR sequences together)) the new light chain library into the existing heavy chain binders that has only a fixed light chain. The pool can then be further sorted against target to identify binders possessing increased affinity. For example, binders (for example, low affinity binders) obtained from sorting an L3/H3, an H1/H2/H3 or an L3/H1/H2/H3 library may be fused with library of an L1/L2/H1/H2 or an L1/L2/L3 diversity to replace its original fixed L1/L2/H1/H2 or an L1/L2/L3, wherein the new libraries are then further sorted against a target of interest to obtain another set of binders (for example, high affinity binders). Novel antibody sequences can be identified that display higher binding affinity to either the IGF1 or mVEGF antigens.

In some embodiments, libraries comprising polypeptides of the invention are subjected to a plurality of sorting rounds, wherein each sorting round comprises contacting the binders obtained from the previous round with a target antigen distinct from the target antigen(s) of the previous round(s). Preferably, but not necessarily, the target antigens are homologous in sequence, for example members of a family of related but distinct polypeptides, such as, but not limited to, cytokines (for example, alpha interferon subtypes).

### Generation of Antibody Variable Domain Libraries

Antibody libraries can be generated by mutating the solvent accessible and/or highly diverse positions in at least one CDR of an antibody variable domain. Some or all of the CDRs can be mutated using the methods of the invention. In some embodiments, it may be preferable to generate diverse antibody libraries by mutating positions in CDRH1, CDRH2 and CDRH3 to form a single library or by mutating positions in CDRL3 and CDRH3 to form a single library or by mutating positions in CDRL3 and CDRH1, CDRH2 and CDRH3 to form a single library.

A library of antibody variable domains can be generated, for example, having mutations in the solvent accessible and/or highly diverse positions of CDRH1, CDRH2 and CDRH3. Another library can be generated having mutations in CDRL1, CDRL2 and CDRL3. These libraries can also be used in conjunction with each other to generate binders of desired affinities. For example, after one or more rounds of selection of heavy chain libraries for binding to a target antigen, a light chain library can be replaced into the population of heavy chain binders for further rounds of selection to increase the affinity of the binders.

In one embodiment, a library is created by substitution of original amino acids with variant amino acids in the CDRH3 region of the variable region of the heavy chain sequence. According to the invention, this library can contain a plurality of antibody sequences, wherein the sequence diversity is primarily in the CDRH3 region of the heavy chain sequence.

In one aspect, the library is created in the context of the humanized antibody 4D5 sequence, or the sequence of the framework amino acids of the humanized antibody 4D5 sequence. Preferably, the library is created by substitution of at least residues 95-100a of the heavy chain with amino acids encoded by the *DYK* codon set, wherein the *DVK* codon set is used to encode a set of variant amino acids for every one of these positions. An example of an oligonucleotide set that is useful for creating these substitutions comprises the sequence *(DVK)*₇; an example of an oligonucleotide set having this sequence is oligonucleotide (F63) (SEQ ID NO: 10). In some embodiments, a library is created by substitution of residues 95-100a with amino acids encoded by both *DVK and NNK* codon sets. An example of an oligonucleotide set that is useful for creating these substitutions comprises the sequence (*DVK*)*₆*(*NNK*); an example of an oligonucleotide set having this sequence is oligonucleotide (F65) (SEQ ID NO: 11). In another embodiment, a library is created by substitution of at least residues 95-100a with amino acids encoded by both *DVK* and *NNK* codon sets. An example of an oligonucleotide set that is useful for creating these substitutions comprises the sequence (*DVK*)₅ (*NNK*); an example of an oligonucleotide set having this sequence is oligonucleotide (F64) (SEQ ID NO: 12). Another example of an oligonucleotide set that is useful for creating these substitutions comprises the sequence *(NNK)₆;* an example of an oligonucleotide set having this sequence is oligonucleotide (F66) (SEQ ID NO: 13). Other examples of suitable oligonucleotide sequences are listed in Figure 4 and Figure 43 and can be determined by one skilled in the art according to the criteria described herein.

In another embodiment, different CDRH3 designs are utilized to isolate high affinity binders and to isolate binders for a variety of epitopes. The range of lengths of CDRH3 generated in this library is 11 to 13 amino acids, although lengths different from this can also be generated. H3 diversity can be expanded by using *NVK, DVK and NYK* codon sets, as well as more limited diversity at N and/or C-terminal. Diversity can also be generated in CDRH1 and CDRH2.

In one embodiment of a library, diversity in H1 and H2 is generated utilizing the following oligonucleotides:
The oligonucleotides used for H1 : F151 (GCA GCT TCT GGC TTC ACC ATT AVT RRT WMY KMT ATA CAC TGG GTG CGT CAG; SEQ ID NO: 14), F152 (GCA GCT TCT GGC TTC ACC ATT AVT RRT WMY KGG ATA CAC TGG GTG CGT CAG; SEQ ID NO: 15), F175 (GCA GCT TCT GGC TTC ACC ATT AVT RVM WMY KMT ATA CAC TGG GTG CGT CAG; SEQ ID NO: 16) and F176 (GCA GCT TCT GGC TTC ACC ATT AVT RVM WMY KGG ATA CAC TGG GTG CGT CAG; SEQ ID NO: 17) were pooled, and for H2, oligonucleotides F153 (AAG GGC CTG GAA TGG GTT GST DGG ATT WMT CCT DMT RRC GGC DMT ACT DAC TAT GCC GAT AGC GTC AAG GGC; SEQ ID NO: 18), F154 (AAG GGC CTG GAA TGG GTT GST DHT ATT WMT CCT DMT RRC GGC DMT ACT DAC TAT GCC GAT AGC GTC AAG GGC; SEQ ID NO: 19), F173 (AAG GGC CTG GAA TGG GTT GST DGG ATT DMT CCT NMT RRC GGC DMT ACT DAC TAT GCC GAT AGC GTC AAG GGC; SEQ ID NO: 20) and F174 (AAG GGC CTG GAA TGG GTT GST DHT ATT DMT CCT NMT RRC GGC DMT ACT DAC TAT GCC GAT AGC GTC AAG GGC; SEQ ID NO: 21) were pooled.

For diversity in CDRH3, multiple libraries can be constructed separately with different lengths of H3 and then combined to select for binders to target antigens. Optionally, in one library, DVK codon sets are utilized in the context of oligonucleotides F163a, F164a, F1646, F165 and F166 (see Figure 4), which are pooled (i.e., the oligonucleotides are used as a single pool). A second library may use the same oligonucleotides as the first library and oligonucleotide F125 (CGR TTC ACT ATA AGC CGT GAC ACA TCC AAA AAC; SEQ ID NO: 22). A third library may use oligonucleotides F165a and F1656. A fourth library can be constructed using a pool of *NVT* oligonucleotides with tryptophan doped in the sequences: F103, F134, F135, F155, F156, F157, F160, F160g and F167 pooled together. A fifth library can be constructed using codon sets *NNS* in oligonucleotides F66a and F66b.

The multiple libraries can be pooled and sorted using solid support selection and solution sorting methods as described previously and shown in Figure 44 and Figure 45. Multiple sorting satrategies may be employed. For example, one variation involves sorting on target bound to a solid, followed by sorting for a tag that may be present on the fusion polypeptide (eg. anti-gD tag) and followed by another sort on target bound to solid. Alternatively, the libraries can be sorted first on target bound to a solid surface, the eluted binders are then sorted using solution phase binding with decreasing concentrations of target antigen. Utilizing combinations of different sorting methods provides for minimization of selection of only highly expressed sequences and provides for selection of a number of different high affinity clones.

High affinity binders for target antigens can be isolated from the libraries. Limiting diversity in the H1/H2 region decreases degeneracy about 10⁴ to 10⁵ fold and allowing more H3 diversity provides for more high affinity binders. Utilizing libraries with different types of diversity in CDRH3 (eg. utilizing DVK or NVT) provides for isolation of binders that may bind to different epitopes of a target antigen.

Of the binders isolated from the pooled libraries as described above, it has been discovered that affinity may be further improved by providing limited diversity in the light chain. Light chain diversity is generated in this embodiment as follows in CDRL1: amino acid position 28 is encoded by RDT; amino acid position 29 is encoded by RKT; amino acid position 30 is encoded by RVW; amino acid position 31 is encoded by ANW; amino acid position 32 is encoded by THT; optionally, amino acid position 33 is encoded by CTG; in CDRL2: amino acid position 50 is encoded by KBG; amino acid position 53 is encoded by AVC; and optionally, amino acid position 55 is encoded by GMA ; in CDRL3: amino acid position 91 is encoded by TMT or SRT or both; amino acid position 92 is encoded by DMC; amino acid position 93 is encoded by RVT; amino acid position 94 is encoded by NHT; and amino acid position 96 is encoded by TWT or YKG or both.

In another embodiment, a library or libraries with diversity in CDRH1, CDRH2 and CDRH3 regions is generated. For diversity in CDRH1 and CDRH2, the following oligonucleotides are used: F151 (5'-GCA GCT TCT GGC TTC ACC ATT AVT RRT WMY KMT ATA CAC TGG GTG CGT CAG-3'; (SEQ ID NO: 14) and F152 (5'-GCA GCT TCT GGC TTC ACC ATT AVT RRT WMY KGG ATA CAC TGG GTG CGT CAG-3'; SEQ ID NO: 15) were pooled, and for H2, oligonucleotides 173 (5'-AAG GGC CTG GAA TGG GTT GST DGG ATT DMT CCT NMT RRC GGT DMT ACT DAC TAT GCC GAT AGC GTC AAG GGC-3'; SEQ ID NO: 20) and F174 (5'-AAG GGC CTG GAA TGG GTT GST DHT ATT DMT CCT NMT RRC GGC DMT ACT DAC TAT GCC GAT AGC GTC AAG GGC-3'; SEQ ID NO: 21) were pooled.

In this embodiment, diversity in CDRH3 is generated using a variety of lengths of H3 regions and using primarily codon sets *XYZ and NNK or NNS.* The oligonucleotides used include F66b, F66d, F66f, F66a₁, F66b₁, F66g, F66h, F66i, F66j, F171c, F171d, F171e, F171, F185, F186, F187, F190, F190a, F190b, F190c, F190d, and F190e. (See Figure 4C.) Libraries can be formed using individual oligonucleotides and pooled or oligonucleotides can be pooled to form a subset of libraries. The libraries of this embodiment can be sorted against target bound to solid. Clones isolated from multiple sorts can be screened for specificity and affinity using ELISA assays. For specificity, the clones can be screened against the desired target antigens as well as other nontarget antigens. Those binders to the desired target antigen can then be screened for affinity in solution binding competition ELISA assay as described in Example 8 or spot competition assay described in Example 9. High affinity binders for target antigens can be isolated from the library utilizing XYZ codon sets prepared as described above. These binders can be readily produced as antibodies or antigen binding fragments in high yield in cell culture.

In some embodiments, it may be desirable to generate libraries with a greater diversity in lengths of CDRH3 region. For example, it may be desirable to generate libraries with CDRH3 regions ranging from about 7 to 19 amino acids. Illustrative oligonucleotides useful for this purpose are shown in Figure 43.

High affinity binders isolated from the libraries of these embodiments are readily produced in bacterial and eukaryotic cell culture in high yield. The vectors can be designed to readily remove sequences such as gD tags, viral coat protein component sequence, and/or to add in constant region sequences to provide for production of full length antibodies or antigen binding fragments in high yield.

A library with mutations in CDRH3 can be combined with a library containing variant versions of other CDRs, for example CDRL1, CDRL2, CDRL3, CDRH1 and/or CDRH2. Thus, for example, in one embodiment, a CDRH3 library is combined with a CDRL3 library created in the context of the humanized 4D5 antibody sequence with variant amino acids at positions 28, 29, 30,31, and/or 32 using codon sets as described in Figure 3. Examples of oligonucleotides useful in creating these substitutions include those that incorporate these codon sets. In another embodiment, a library with mutations to the CDRH3 can be combined with a library comprising variant CDRH1 and/or CDRH2 heavy chain variable domains. In one embodiment, the CDRH1 library is created with the humanized antibody 4D5 sequence with variant amino acids at positions 28, 30,31, 32 and 33 using codon sets as described in Figure 3. Examples of oligonucleotide sets useful in creating these substitutions include those that incorporate these codon sets. A CDRH2 library may be created with the sequence of humanized antibody 4D5 with variant amino acids at positions 50, 52, 53, 54, 56 and 58 using the codon sets described in Figure 3. Examples of oligonucleotide sets useful in creating these substitutions include those that incorporate these codon sets.

Any combination of codon sets and CDRs can be diversified according to the amino acid position selection criteria described herein. Examples of suitable codons in various combinations of CDRs are illustrated in Figures 5-13. Figures 5-7 also include illustrative calculations of designed diversity values of libraries generated according to the choice of codon sets used in the indicated CDRs and amino acid positions.

Having generally described the invention, the same will be more readily understood by reference to the following examples, which are provided by way of illustration and are not intended as limiting.

### EXAMPLE 1

Vectors encoding fusion polypeptides comprising variant CDRs were constructed as follows.

### Antibody Phage Display Vectors based on pS1607

A vector for antibody phage display was constructed by modifying vector pS1607 (Sidhu et al., J. Mol. Biol. (2000), 296:487-495). Vector pS1607, which has pTac promoter sequence and *malE* secretion signal sequence, contained a sequence of human growth hormone fused to the C-terminal domain of the gene-3 minor coat protein (p3). The sequence encoding hGH was removed, and the resulting vector sequence served as the vector backbone for construction of vectors of the present invention that contain DNA fragments encoding the anti-her2 humanized antibody 4D5 light chain and heavy chain variable domain sequences in the form of:,
(i) single chain Fv (scFv) (SEQ ID NO: 23; Figure 14);
(ii) single chain Fv with zipper domain (scFvzip) (SEQ ID NO: 24; Figure 15);
(iii) Fab fragment (Fab) (SEQ ID NO: 25; Figure 16);
   or
(iv) Fab fragment with zipper domain (Fabzip) (SEQ ID NO: 26; Figure 17).

The humanized antibody 4D5 is an antibody which has mostly human consensus sequence framework regions in the heavy and light chains, and CDR regions from a mouse monoclonal antibody specific for Her-2. The method of making the anti-Her-2 antibody and the identity of the variable domain sequences are provided in U.S. Pat. Nos. 5,821,337 and 6,054,297. The resulting vectors (schematically illustrated in Figures 34A-D) comprise the humanized antibody 4D5 variable domains under the control of the IPTG-inducible Ptac promoter (sequences as shown in Figures 14-17) or the alkaline phosphatase phoA promoter (as described in U.S. Pat. No. 5,750,373).

### Construction of Fab-zip construct and characterization of its function in phage display

Construction of an example of a vector is described in greater illustrative detail below for Fab-zip. Inclusion of the zipper region facilitates the formation and display of dimers of ScFv and F(ab) to form scFv₂ and F(ab')₂, respectively.

Fab-zip vectors were constructed as described below and shown in Figure 34B.

### METHODS AND MATERIALS

Construction of Anti-Her2 F(ab')₂ vector: A phagemid construct comprising a sequence encoding an anti-Her2 polypeptide under the control of the Ptac promoter was generated using vector pS 1607 as the backbone, as described above. malE secretion signal sequence was first fused to the N-terminal sequence of light chain (LC) to direct the LC synthesis to the periplasm of bacteria cell. A gD tag was added at the C-terminus of LC. Following the stop codon of LC, another ribosome binding site and STII signal sequence were fused to the N-terminus of heavy chain (HC) sequence and continued with the C-terminal domain of the pIII, a minor coat protein of M13 phage.

To generate F(ab')₂ displayed on phage, the dimerizable leucine zipper GCN4 sequence was utilized. Cassette mutagenesis was performed to insert in between HC and pIII first the hinge sequence that came from full length IgG1 antibody (TCPPCPAPELLG (SEQ ID NO: 27)) followed by GCN4 sequences (GRMKQLEDKVEELLSKNYHLENEVARLKKLVGERG (SEQ ID NO: 3)). The GCN4 leucine zipper was expected to bring two sets of LC/HC-pIII fusion polypeptides together in the E. *coli* periplasm, which would allow the formation of disulfide bonds in the hinge region to secure the dimer formation before and after getting out of the E. *coli* periplasm.

Two versions of the vector schematically illustrated in Figure 34B were made. One had an Amber stop codon (TAG) after the GCN4 zipper sequence and one did not. These two constructs would theoretically produce one or both of the divalently displaying phage as depicted in Figure 18. The Amber-less construct would make only the form (C) that would have two copies out of the five copies of pIII on phage as fusion polypeptide which would be stabilized with both the hinge disulfide and GCN4 zipper. The construct with Amber after the GCN4 should be able to produce either form (B) or (C) of the phage depending on the efficiency of suppression of the Amber stop codon in XL-1 bacterial strain.

The formation of F(ab'),₂ on phage: To demonstrate the formation of F(ab')₂, or the divalent display of F(ab') on the phage, the expected function of divalent display was measured. With the avidity effect of divalent display, the phage binding to ligand-modified solid phase should demonstrate significantly decreased off-rate, the rate at which it detaches off the solid phase, if the density of the ligand on the solid support is high enough to allow divalent binding. For divalent interaction to detach off the plate, both interactions have to be broken simultaneously for the phage to come off, which predictably would occur with much less frequency.

To produce displaying phage, E. *Coli* strain XL-1 Blue (Stratagene, San Diego, CA) infected first with F(ab) or zipped F(ab')₂ phage and then VCS helper phage (Strategene, San Diego, CA) were grown in 2YT media at 37°C for 20 h and phage was harvested as described (Sidhu et al., Methods Enzymol. (2000), 328:333-363). Briefly, phage was purified by first precipitating them from the overnight culture media with polyethylene glycol, and resuspended in PBS. Phage were quantitated by spectrophotometer with its reading at 268nm (1 OD=1.13X10¹³/ml). Phage ELISA (Sidhu et al., supra) was first performed by titrating the phage in phage ELISA binding buffer (PBS with 0.5% BSA and 0.05% Tween 20) and its binding to ligand (Her-2 extracellular domain, Her-2ECD) coated on 96-well plate was quantified by HRP conjugated anti-M13 antibody followed by adding the peroxidase substrate, H202 and TMB (Kirkgaad) which can be read at wavelength 450nm. The plate was coated with Her-2ECD at 2*µ*g/ml in PBS for 2h at room temperature or 4°C overnight, which is sufficient to allow divalent binding. We blocked the plate with.0.5% BSA and then 0.2% Tween20 for 1 hour before adding phage dilutions to the wells.

For the off-rate plate binding experiments or solution binding competition ELISA, a phage concentration was used of either F(ab)or zippedF(ab')₂ which gave about 90% of maximum binding to the coated plate. To show that the F(ab')₂ phage still maintain the same binding affinity where avidity plays less of a role, competition ELISA was performed by incubating the F(ab) or zipped F(ab')₂ phage with increasing concentrations of Her-2ECD ((0.1 to 500nM) in solution for 5 hours at 37°C. The unbound phage was captured briefly (15 min.) with plates coated with HER-2ECD and measured with HRP-anti-M13 conjugate. The IC50, the concentration of Her-2ECD that inhibits 50% of the F(ab)-phage, represents the affinity (see Figure 19).

For the off-rate experiment, F(ab) or zippedF(ab')₂ phage was allowed to bind to Her-2ECD coated wells first, which were then washed to get rid of excess phage. Serial dilutions of Her-2ECD (0.1nM to 500 nM) were added to the well and incubated for 5 hours at 37°C, during which time the phage was allowed to detach off the plate and the rebinding was inhibited by the Her-2ECD in the solution. Phage that still remained on the plate was then quantified with HRP-antiM13 conjugate. The relative proportion of remaining phage was calculated by dividing the OD at the particular Her-2ECD concentration with OD in the absence of Her-2ECD and shown as % in the Figure 20.

Another way to demonstrate the divalency of F(ab'₎2 phage was to show a difference in the amount of phage that is required to give detectable binding on the ligand coated solid support by standard phage ELISA method as compared to its non-divalent counterpart. We also examined the detectability of low affinity binder. We generated a humanized antibody 4D5 mutant by Kunkel site-directed mutagenesis (Kunkle et al., 1985) in its heavy chain with Arginine 50 changed to Alanine (R50A), in both F(ab) and zipped F(ab')₂ format. Standard phage dilution versus its binding signal on Her-2ECD coated plate phage ELISA was performed (Figure 21 and Figure 22). The display level of the mutant was equivalent for these two formats judged from its binding to antibody to gD tag.

### Results

Binding properties: The zipped F(ab')₂ displaying phage has essentially indistinguishable affinity (1nM) in solution as F(ab) phage (Figure 19). This means that the insertion of hinge and GCN4 zipper to the C-terminus of HC did not perturb its binding capability. However, the avidity effect of the divalent zipped F(ab')₂ is clearly demonstrated by the significantly different behavior from the monovalent F(ab) phage in the plate binding experiments (Figure 20 and 21). In Figure 20, zipped F(ab')₂ phage of either construct with or without amber stop codon after the leucine zipper has a much slower rate of detachment from the ligand coated plate than F(ab) phage. In Figure 21, we saw a consistent 40-50 fold difference in the concentration of phage concentration to achieve the same binding signal. The binding of a low affinity R50A (650nM) binder can be detected and captured at 40-50 fold lower concentration of phage with divalent F(ab')₂ display (Figure 22). This difference is commonly seen comparing monovalent and divalent interaction, e.g. F(ab) vs. M(ab).

### Phage Display Vectors based on Vector pS0643

Construction of Anti-Her2 Fab and F(ab')2 phagemid : The phagemid vector, pS0643 (also known as phGHam-g3, e.g., United States Patent 5,688,666), contains pBR322 and f1 origins of replication, an ampicillin resistant gene, an E. coli alkaline phosphatase (phoA) promoter (Bass et al., (1990) Proteins 8:309-314), and a sequence encoding a stII secretion signal sequence fused to residues 1-191 of human growth hormone (hGH) and a sequence encoding the C-terminal residues 267-421 of protein III of M13 phage (hereinafter, cP3). The pS0643 phagemid also contains an Xbal site and an amber stop codon following residue 191 of hGH. The stII secretion signal sequence can export a protein to the periplasm of a bacteria cell (e. g., a light chain region (LC) of an antibody). The sequence encoding the human growth hormone (hGH) was removed from the pS0643 vector and replaced with a NsiI/XbaI nucleic acid fragment encoding a humanized anti-Her2 Fab fragment ("h4D5" sequence) ligated in frame with the stII secretion signal .(humAb4D5-8, see Carter et al., (1992) PNAS 89:4285-4289, Table 1 and FIGURE1 therein or U.S. Patent No. 5,821,337, for sequence).

The h4D5 antibody is a humanized antibody that specifically recognizes a cancer-associated antigen known as Her-2 (erbB2) developed as described previously. The h4d5 was obtained by polymerase chain reaction using the humAb4D5 version 8 ("humAb4D5-8") sequence and primers engineered to give rise to a 5' NsiI site and a 3' XbaI site in the PCR product (Carter et al., (1992) PNAS 89:4285-4289). The PCR product was cleaved with NsiI and XbaI and ligated into the pS0643 phagemid vector.

The pS0643 plasmid containing humanized 4D5 (version 8) was still further modified. The LC was changed at residues 30, 66 and 91 by site-directed mutagenesis. Specifically, residue 30 was changed from asparagine to serine, residue 66 was changed from arginine to glycine, and residue 91 was changed from histidine to serine. These changes were made to increase the expression or the display of Fab on phage to improve the performance of the library and to convert the framework (66) back to the human consensus framework. Finally, the amber stop codon and XbaI site was removed from C-terminal end of the heavy chain.

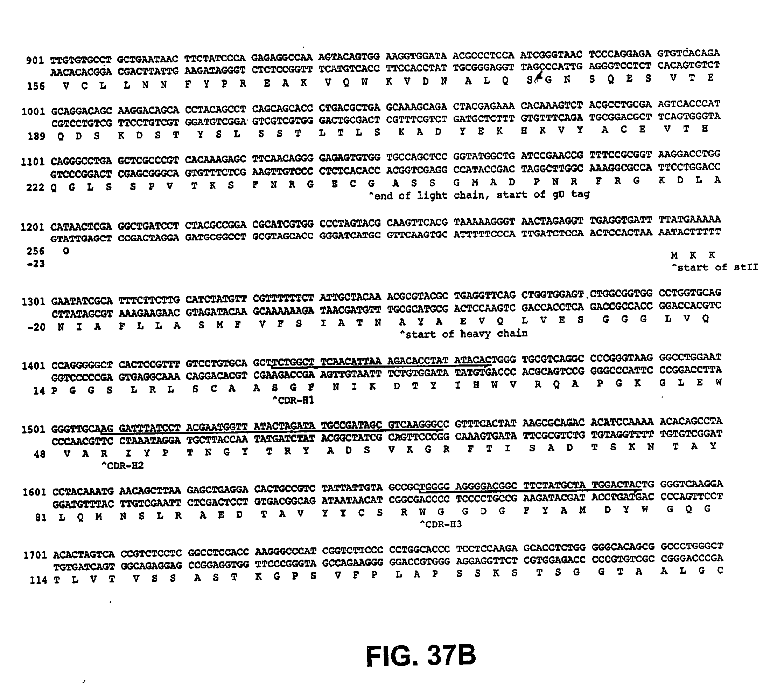

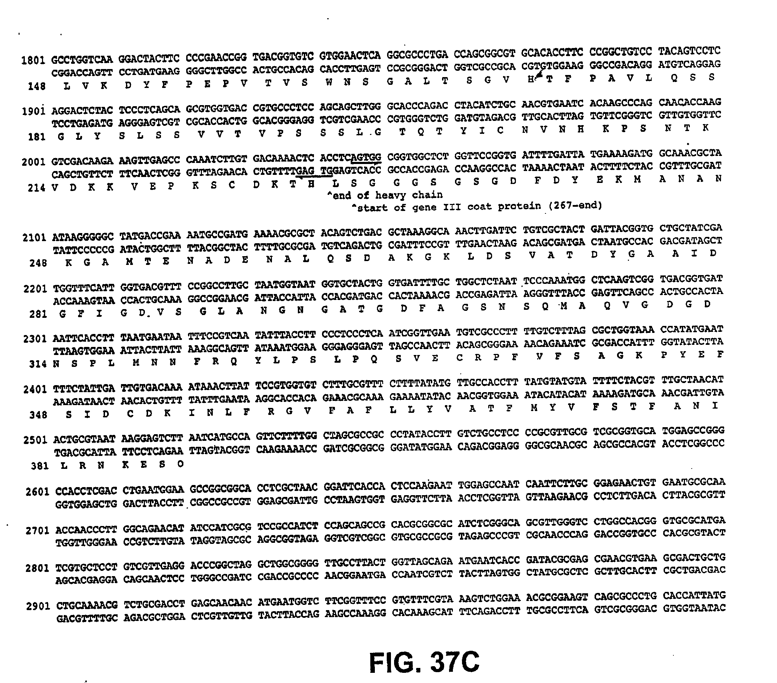

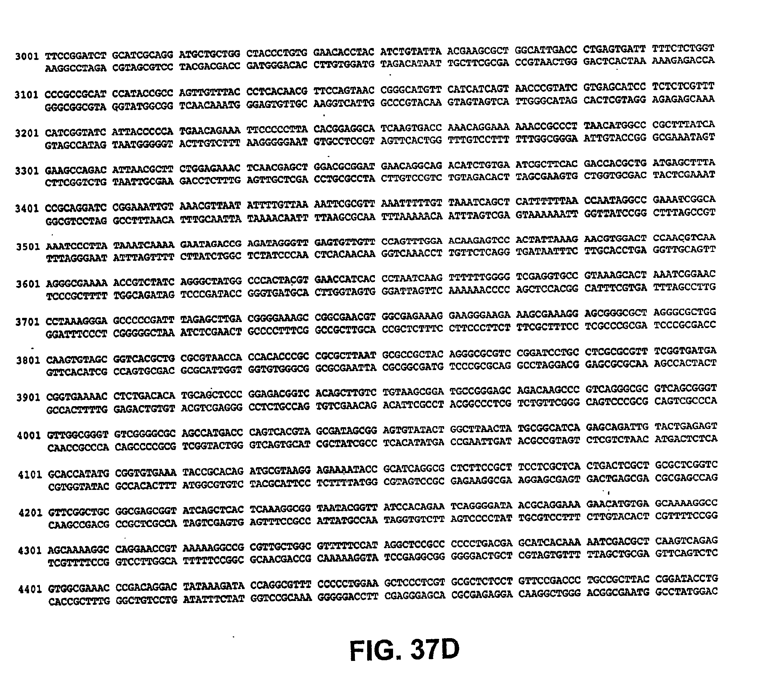

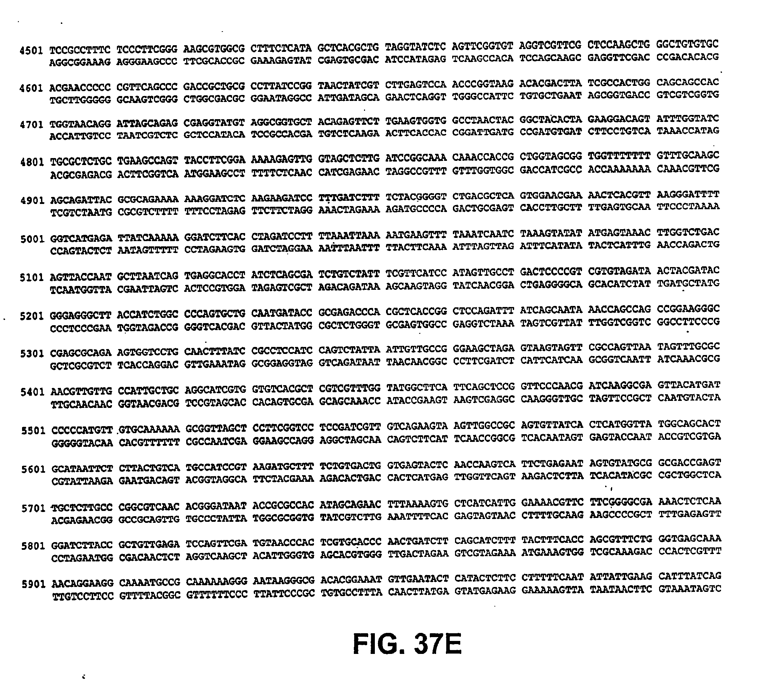

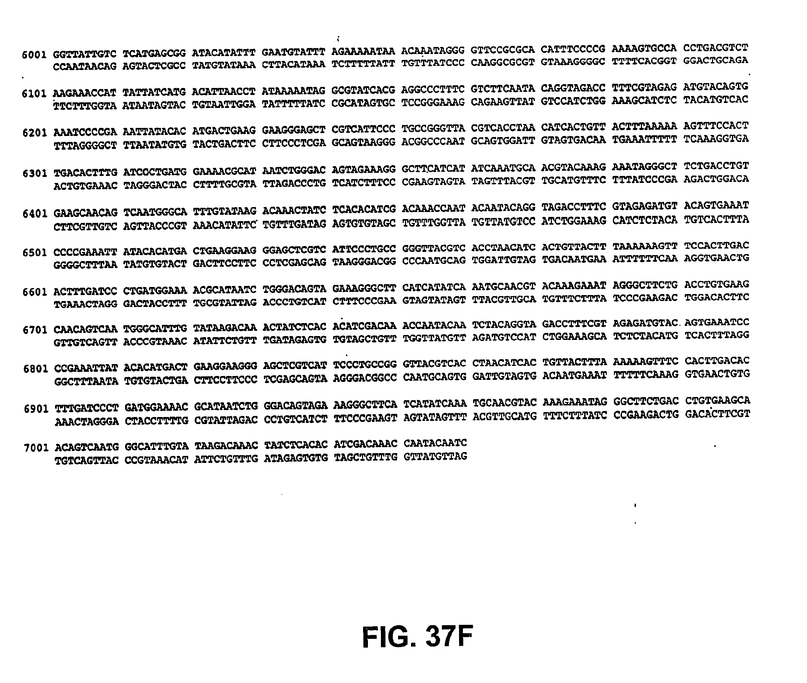

A herpes simplex virus type 1 glycoprotein D epitope tag (gD tag) was added in frame to the C-terminus of the LC using site-directed mutagenesis. Following the stop codon downstream of the LC, a ribosome binding site and nucleic acid molecule encoding a stII signal sequence were ligated to the N-terminus of the HC sequence. Consequently, the HC sequence is in frame with the C-terminal domain of the p3 (cP3), a minor coat protein of M13 phage. Thus, a Fab displayed on phage can be produced from one construct. This Fab phagemid vector is referred to as pV0350-2b. The sequence of this vector is shown in Figure 37 and has SEQ ID NO: 28.

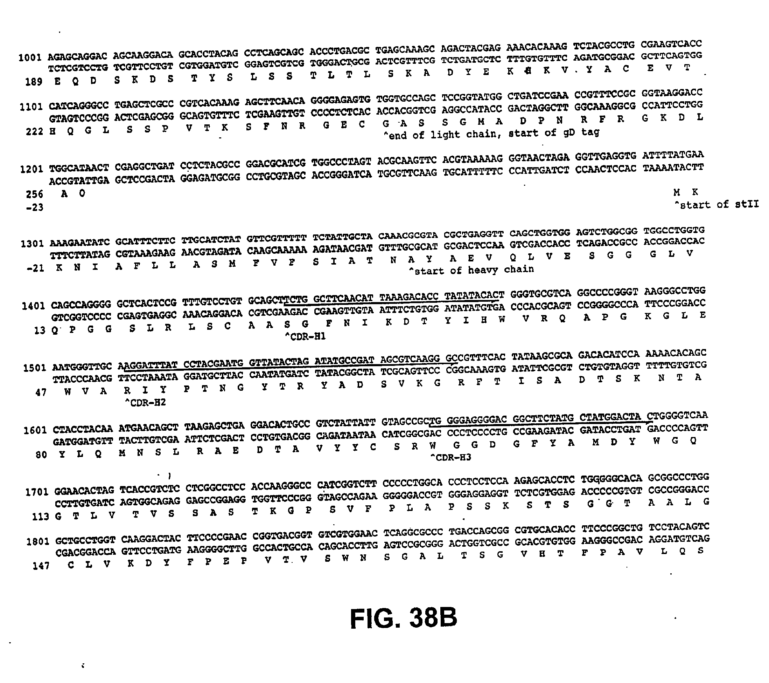

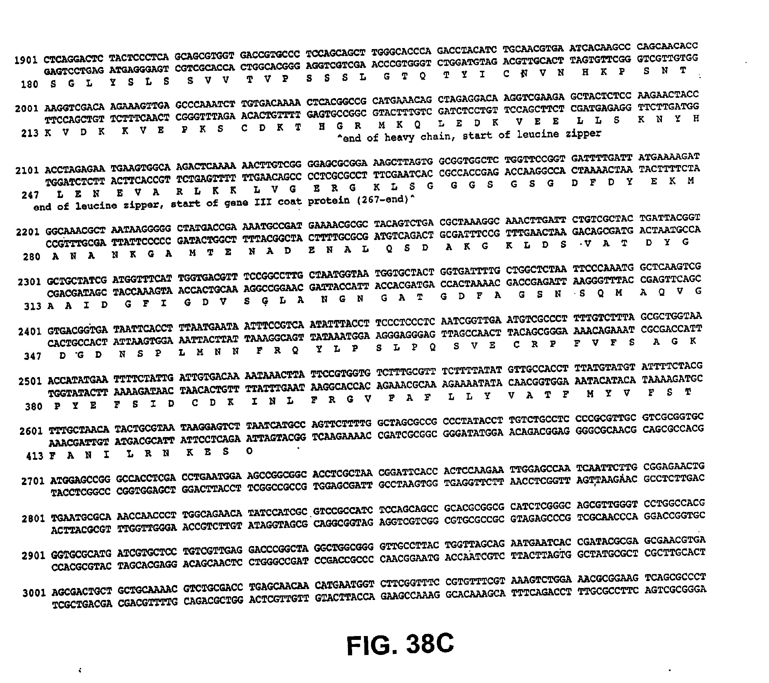

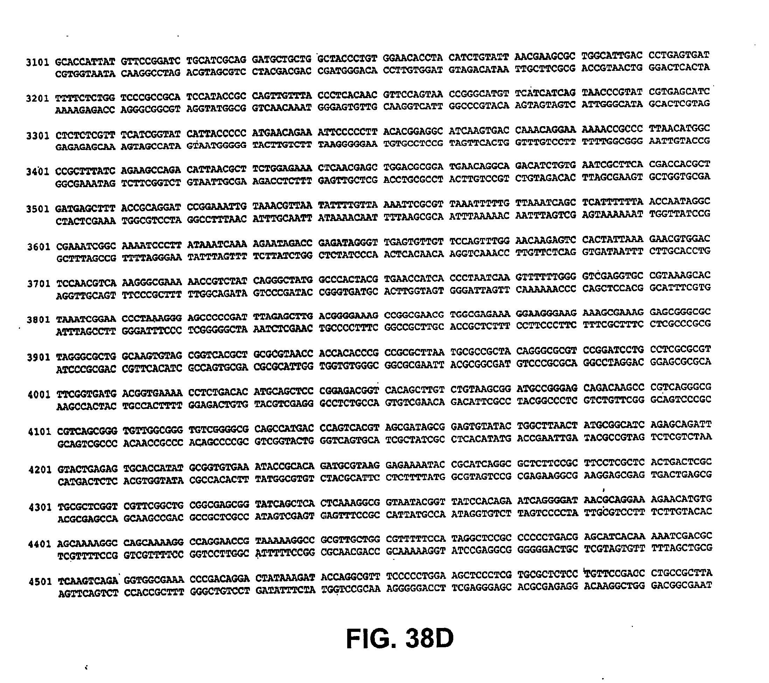

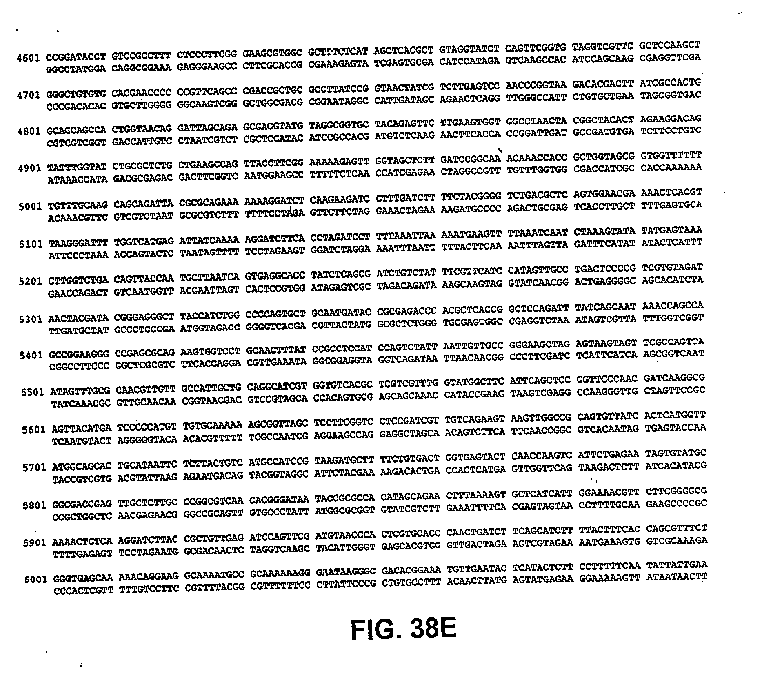

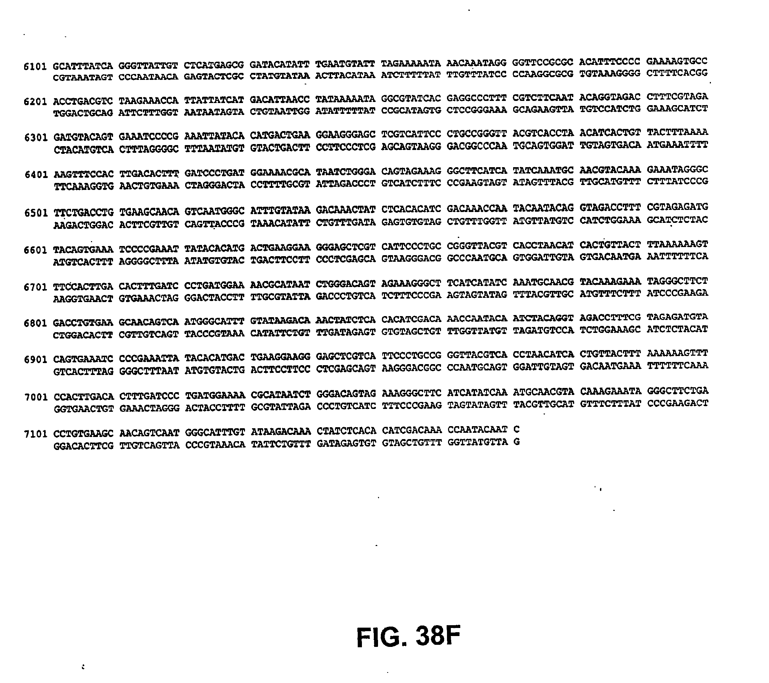

To generate F(ab')₂ displayed on phage, the PV0350-2b vector was further modified by inserting a dimerizable leucine zipper GCN4 sequence (GRMKQLEDKVEELLSKNYHLENEVARLKKLVGERG) (SEQ ID NO: 3) between the HC and cP3 sequences by cassette mutagenesis. The GCN4 leucine zipper brings two sets of LC/HC-cP3 fusion polypeptides together in the E. *coli* periplasm and presents the dimer on the surface of phage. This F(ab')₂ phagemid vector is referred to as pV0350-4. The sequence of this vector is shown in Figure 38 and has SEQ ID NO: 29.

Vectors based on either ps1607 or ps0643 have been designed including restriction sites that allow for removal of the viral P3 sequences, leucine zipper and gD tag. The variable region domains from heavy and light chain can then be combined with constant region sequences to allow for expression of the variable region domain as Fab fragments or full-length antibodies. Sources of constant region sequences are known to those of skill in the art.

### EXAMPLE 2

### Modifications to Dimerization Domains

A series of mutations were made in the dimerizing domain, which for example, include the hinge region plus GCN4 leucine zipper for constructs such as Fab-zip as described in Example 1. The mutations were made to the dimerization domain to determine if both a disulfide bond as well as the zipper region were important to the display of F(ab')₂ or ScFV₂.

A series of mutations (Fig. 39) was made in the dimerization domain, i.e. hinge region plus GCN4 leucine zipper, and their effects on avidity were examined (Fig 40). The phagemid vector phGHam.g3 with alkaline phosphatase (phoA) promoter was used as described previously since it is less toxic to bacterial cells and increases the phage yield compared to a vector with Ptac promoter (C. V. Lee, unpublished observation). The solution binding of all constructs was first examined to make certain the change in the construct did not disrupt the folding and function of the Fab. IC50s for all variants were between 0.05-0.07 nM measured at room temperature, which agreed well with Kd of h4D5 (0.1nM).

The mutations were designed to examine the function of hinge region and leucine zipper in Fab'-zip phage in its bivalency. Constructs include a construct that has 1) that has the hinge region and the leucine zipper, Fab'-zip ;2)both cysteines in hinge region mutated to alanine (Fab'(C→A)-zip), 3) has the whole hinge region deleted (Fab-zip), 4) the hinge deleted leaving the first cysteine (Fab-C-zip), or 5) has both hinge and zipper deleted leaving a single cysteine (Fab-C). All of these constructs were made with or without amber stop codon before cP3 (C- terminal region of viralcoat protein p3)since the presence or absence of stop codons allows for the examination of the dependence of avidity on the interchain covalent bond between two Fabs. Avidity assay was performed as described in Example 1. Avidity index represents a measurement of functional avidity of individual construct and is defined as the percent phage bound to the immobilized antigen after the chase of 500nM erbB2. High avidity is a functional consequence of displaying two copies of Fab per phage.

Without an amber stop codon, all constructs present Fab with significantly higher avidity than Fab-phage (Fig. 40). These results indicate that the probability of incorporating two copies of cP3 fusion on phage coat increases when the following are present: hinge plus zipper (Fab'-zip), cysteine-free hinge plus zipper (Fab'(C→A)-zip), zipper alone (Fab-zip), one cysteine plus zipper (Fab-C-zip) or just one cysteine (Fab-C) by itself between Fab and cP3. When both Fab' or Fab fusions are covalently linked to cP3 (no amber stop codon), bivalent display does not require the additional covalent links provided by the hinge region disulfides. However, the presence of an interchain covalent bond does appear to improve the frequency of incorporating two copies of fusion per phage since constructs that lack a cysteine residue such as, Fab'(C→A)-zip and Fab-zip (without amber stop), exhibited reduced avidity compared to Fab'-zip or other constructs that have disulfide bond in the linker, despite the fact that they do have significantly higher avidity than Fab-phage (no amber).

With the presence of an amber stop codon before cP3, most proteins were expressed into periplasm and associated with cP3 fusion protein via leucine zipper and/or disulfide bond since the read through of amber stop codon in the amber-suppressor strain XL-1 blue occur only at ∼10% frequency. It is expected that a dimer should be linked to phage by single copy of cP3 as depicted in Fig. 18 and the bivalency be formed by forming an interchain covalent bond because the concentration of phage exuded into bacterial growth media is in the sub-nanomolar range, and thus, the binding energy of the leucine zipper interaction would not be sufficient to maintain the dimer during overnight growth at 37°C. Indeed, it was observed that those constructs that do not have disulfide bond in the hinge (Fab'(C→A)-zip) or with hinge deleted (Fab-zip) lose the bivalent dependent avidity in the presence of an amber stop codon. The constructs that contain disulfide bonds between Fab and cP3 maintained a high avidity of bivalency in the presence of an amber stop codon.

The exception is Fab-C. It was surprising that Fab-C, with just one cysteine between Fab and cP3, exhibited such high avidity, if not higher, as compared to the original Fab'-zip phage with full hinge and leucine zipper. These results suggest that Fab-C as cP3 fusion has good probability to associate with another cP3 fusion and get incorporated in the phage coat as dimers. However, the presence of amber stop between cysteine and cP3 in Fab-C construct reduced the avidity to the level of Fab-phage since most proteins were expressed as free Fab-C and the chance of free Fab-C in the periplasmic space forming dimer with a Fab-C-cP3 fusion appears to be very low.

The minimum element for bivalent display is either leucine zipper alone (Fab-zip) or one disulfide bond (Fab-C). When Fab-C is compared to Fab-zip, the results show that the disulfide bond is more effective in some circumstances to improve avidity than leucine zipper alone.

### Western blot analysis

Western blotting was used to examine whether these constructs were displayed as covalently-linked dimers. We analyzed phage constructs Fab, Fab'-zip, Fab'(C→A)-zip, Fab-zip, Fab-C-zip, or Fab-C with or without amber stop codon. Phage samples (3X10¹¹) were denatured, subjected to SDS-PAGE and transferred to PVDF filters, which were then probed with either antibody that recognized the gD-tag, which was fused to the C-terminus of the light chain (Figure 41 A and C), or antibody to phage coat protein P3 (Mobi-Tech) (Figure 41 B and D) .

Under reducing conditions (+DTT), the disulfide bond linking the light chain to the heavy chain fusion was reduced, and anti-gD blot (Figure 41 C) showed a single band with an apparent molecular weight (MW) of ∼25 kDa, which was in good agreement with the calculated MW of a light chain plus gD-tag (26 kDa). When blotted with anti-P3, full length P3 (MW 48 kDa) supplied by helper phage and heavy chain-cP3 (MW 43-45 kDa depending on constructs) fusion in the reduced condition was expected. The results in Figure 41D showed a main band at 60 kDa representing P3 and lower band with 50-52 kDa representing the fusion, based on the comparison with K07 alone (data not shown); both are greater than the calculated MW of the two main species expected. However, P3 is known to exhibit an anomalously high apparent MW under SDS-PAGE conditions {Gray, 1981 et al. 1981 J. Mol. Biol. 146:621-627; Crissman, 1984, Virology 132:445-455}. Further, the relative position of the 50-52 kDa bands seems to correlate with the MW of the expected heavy chain-cP3 fusion of each construct with various insertions. It is interesting to note that the same 50-52 kDa band in the non-reduced gel blotted with anti-P3 was observed (Figure 41 B), which suggests that there are some heavy chain-cP3 fusion proteins that were not covalently linked with its light chain. Non-covalent linked light chain (25 kDa) was also observed in the non-reduced gel blotted with anti-gD antibody. (See Figure 41 A) In the reduced gel, the intensities of the 50-52 kDa bands from anti-P3 blot and the light chain bands from anti-gD blot indicate the level of display as Fab fusion for individual construct.

In general, adding amber stop codon reduced the display for all constructs as expected since the read-through of the amber stop codon is infrequent in amber suppressor strain. The insertion of the full dimerization domain (hinge and zipper) as in Fab'-zip reduces the display, and the culprit seems to reside in the two disulfide bonds in the hinge region since replacing the cysteine as in the Fab'(C→A)-zip or deleting the whole hinge (Fab-zip) increased most of the display to that of Fab-phage. Having just one cysteine in the dimerization domain appears to be well tolerated and displays well as in Fab-C-zip or Fab-C construct. The results show that reducing the number of disulfide bonds to one or zero increased expression of the fusion proteins.

Under non-reducing conditions (-DTT), interchain disulfide bonds were preserved and different patterns of bands for each of the constructs were observed (Figure 41 A and B). As is often the case in western blots of phage preparations(Gray, 1981 supra;Crissman, 1984, supra), multiple bands can be observed resulting from proteolysis. However, comparing the highest MW major band in each lane should indicate the different products of the constructs as the intact cP3 fusion. Fab-phage produced a band with an apparent MW of 95 kDa, which was detected with both anti-gD and anti-P3 antibodies and agrees well with the calculated MW of 75 kDa of Fab-cP3 fusion. Again, the P3 fusion protein seems to have an effect on the mobility on SDS-PAGE system. In contrast, the Fab'-zip phage (-amber stop) contained a pair of bands with apparent MWs of∼180 and ∼200 kDa which is in close agreement with the calculated MW of a dimeric Fab'-zip-cP3 fusion (155 kDa). It is interesting to note that the equivalent construct with amber stop codon produced only the 180 kDa band, which could represent the expected dimer fused to single cP3 as a monovalently displayed dimer complex depicted in Fig. 18. In any case, the covalently linked dimer is formed regardless of the presence of amber stop codon, which is expected since the leucine zipper promotes the dimer formation independent of any covalent link with phage coat in the periplasmic space and, functionally, avidity was observed with this set of constructs. Some lower MW bands were also seen which might be from proteolysis or alternatively disulfide bonded forms. There is a major band at 98 kDa which migrated slightly higher than Fab-cP3 but comigrated with the major band of Fab'(C→A)-zip construct, and would be the monomeric Fab'-zip-cP3 fusion which as expected is stained by anti-gD and anti-P3. So the expression of Fab'-zip fusion is reduced relative to Fab and there is significant portion of fusion protein that did not form disulfide bond.

With constructs Fab' A)-zip and Fab-zip, a major band that migrated slight higher than Fab-cP3 at 98 kDa or 97 kDa individually was observed. These bands are the expected product for constructs with or without amber stop because there is no possibility of covalent disulfides between the two Fab moieties. The presence of dimeric Fab on A)-zip and Fab-zip phage can not be observed in SDS-PAGE gel because of the lack of disulfide bond. Yet, the functional avidity suggests that the proportion of phage containing two copies of fusion is higher than Fab phage due to the presence of leucine zipper.

Constructs Fab-C-zip and Fab-C contain one interchain disulfide bond, and produce more covalently linked dimers as represented by the 180-200 MW band than Fab'-zip, which have two disulfide bond in the hinge linker. The increase of the covalent-linked dimers correlates with the overall increase of expression of the fusion including the monomer as shown in the 97 and 95 kDa band. It is interesting to note that the proportion of dimer is higher in Fab-C-zip than Fab-C, which suggests that leucine zipper, although not required, does improve the association of the fusion and the efficiency of forming interchain disulfide bond. Yet the net expression of Fab-C-cP3 fusion is slightly higher than Fab-C-zip construct.

Thus, western blotting suggests the presence of bivalent moieties displayed on Fab'-zip, Fab-C-zip, and Fab-C phage, mirroring what is observed functionally in avidity assays. For constructs, for which the dimer can not be observed on SDS-PAGE, like A)-zip and Fab-zip, the avidity assay is relied upon to demonstrate its bivalency. The avidity assay shows the consequences of presenting two copies of Fab per phage particle since all constructs have same solution binding affinity and the avidity is not influenced by the display level. Net expression of Fab'-zip can be significantly improved by minimizing the dimerization domain. Potentially, the different extent of dimer display and avidity of these various constructs may provide useful tools for applications that may call for different extent of display and avidity.

### EXAMPLE 3

Libraries of antibody variable domains were generated to isolate and screen for isolation of high affinity binders to particular target antigens. Diversity of antibody variable domains is preferably generated in CDR regions of the heavy and/or light chain variable domains. Generation of diversity in CDR regions of heavy chains is described below. Utilization of a single template molecule as the backbone for generation of the diversity in CDR regions is preferred because the single template can be modified to improve display and folding and may accommodate H3 regions of various sizes.

### Library Design: H1,H2

Libraries of antibody variable domains were designed to maximize diversity in the heavy chain CDR regions while minimizing structural perturbations in the antibody variable domains. Structural perturbations in antibody variable domains are generally associated with improperly folded antibody domains resulting in low yield and unstable products, for example when produced in bacterial cells. Low yields decrease the number of binders detected in screening. Improperly folded and unstable antibody structures also can result in low yields and low affinity when antibodies are produced as Fab fragments or full-length antibodies in cell cultures. Production of properly folded antibodies in high yield in cell culture is desirable especially in the case of antibodies useful therapeutically.

Diversity in the CDR regions was generated by identifying solvent accessible and highly diverse positions in each CDR for CDRs H1 and H2 of the template molecule, and designing an oligonucleotide comprising at least one tailored (i.e., non-random) codon set encoding variant amino acids for the amino acid position corresponding to the position of at least one solvent accessible residue at a highly diverse position in at least one CDR region. A tailored codon set is a degenerate nucleic acid sequence that preferably encodes the most commonly occurring amino acids at the corresponding positions of the solvent accessible residues in known, natural antibodies.

Solvent accessible residues in the CDRs were identified in the antibody variable domain template molecule by analyzing the crystal structure of the template molecule. Humanized antibody 4D5 is efficiently produced and properly folded when produced in a variety of host cells, including bacterial cell culture. The crystal structure for the humanized antibody 4D5 variable region is known and publicly available at www/rcsb/org (accession code IFVC).

The solvent accessible positions in the CDRs of the light chain and CDRH1 and CDRH2 of the heavy chain were identified using the Insight II program (Accelrys, San Diego, CA).

CDR residues were also analyzed to determine which positions in the CDRs were highly diverse. Highly diverse positions in the CDR regions for the heavy chains were identified by examining the sequences of known, naturally occurring antibodies in the Kabat database. The Kabat database is available at immuno/bme/nwu/edu. In the Kabat database, there were about 1540 sequences of the human light chain and 3600 sequences for the human heavy chain. The CDR sites were aligned and numbered as described by Kabat (see immuno/bme/nwu/edu). Highly diverse amino acid positions were identified by lining up and ranking the amino acid usage, from most frequently used to less frequently used for each CDR residue. For example, H1-30 (i.e., residue 30 of the heavy chain CDR1) was found to be S (serine) in 2451 out of 3617 antibody sequences in the Kabat database, and it is the amino acid found most frequently at this position. Next on the list of frequency is threonine (occurring in 655 sequences), followed by asparagine (154 sequences), arginine (70 sequences), glycine (67 sequences), with the remaining 128 sequences being one of the remaining amino acids. Illustrative diverse sites, with corresponding diversity list of amino acids, are shown in Figure 2 (for the heavy chain).

Amino acid residues found in a particular position that collectively constitute the most frequently occurring amino acids among the known, natural antibody sequences are selected as the basis for library design. The most frequently occurring amino acids were deemed to be those that are most commonly found in the top 90% of the list of diverse amino acids at that position (this group of amino acids is referred to herein as "target group of amino acids"). However, as described herein, the percent cutoff for a target group of amino acids can be varied, as described above, according to the circumstances and purpose of the diversity library that is to be achieved.

For humanized antibody 4D5, the positions identified as solvent accessible and highly diverse were: Heavy Chain
- CDR 1: 28,30,31,32
- CDR2: 50, 52, 53, 54, 56, 58

Examples of amino acids that occur at high frequency in natural diversity (i.e., among known, natural antibody sequences) (referred to as "target group" or "natural diversity" in Figure 3), and the designed diversity of amino acids by DNA codons ("Diversity<DNA ") for each of these positions is shown in Figure 3.

Codon sets encoding a specific group of amino acids were designed to include at least a certain percentage of the amino acids in the known, natural sequences (designated as "% covering" in Figure 3). Of the amino acids encoded by a codon set, at least about 40% of the amino acids are target amino acids identified for a particular solvent accessible and highly diverse position (designated as "% good" in Figure 3; amino acids encoded by a codon set that are target amino acids are shown in bold in column 3 of Figure 3), more preferably any one of 40 to 100%, more preferably 70%. However, as described herein, the % good value can be varied according to circumstance and objectives. The codon sets were selected such that they preferably encoded the amino acids with the highest occurrences at a particular position. The number of non-target amino acids coded by a codon set for a particular position was minimized. Effectiveness of codon set selection/design was evaluated in part based on the "% good" value. A high percentage meant very low non-target amino acids; a high value of "% good" was deemed more important than having more target amino acids among the amino acids coded by a particular codon set. Redundancy was included in calculating the "% good" value. For evaluation purposes, the "% covering" value was also calculated. This value represents the percentage of natural diversity covered by the "good" or target amino acids encoded by a particular codon set. For example, for H-30, when codon set *RMV* is used, the "good" amino acids are STNRDG, which is 73% (% good) of the amino acids encoded by the codon set. STNRDG are amino acids that cover 3489 out of 3617 known, natural antibody sequences at this amino acid position. 3489/3617 equals 96%, which is the "% covering" value. Thus, in one design using *RVM* at H-30, 73% of the library covers 96% of the natural diversity in CDRH1 at position 30.

The codon sets were also designed to exclude, when possible, cysteine and stop codons. The presence of cysteine residues tends to cause folding problems and stop codons can decrease the effective library size. In the design of the codon sets, it was also deemed desirable to minimize the number of nontarget amino acids.

The codon sets designed for each solvent accessible and highly diverse residue of humanized antibody 4D5 are shown in Figure 3. At any particular residue, one or more codon sets could be used depending on the target amino acids that were identified. For example, combining two H1 oligonucleotides, one having residue H1-33 as *KMT,* the other having H1-33 as *KGG,* results in 100% of the codons used for H1-33 covering 86% (50% + 30%) of the natural diversity at the H1-33 position. Other examples of instances where two codon sets can be beneficially used include using codons *DGG and DHT* at H2-50.

The various codon sets could be used to generate diverse libraries with diversity in one or more CDR regions, including CDRH1 and CDRH2. For example, Figures 5-13 and Figure 24 show various illustrative versions of codon set designs that can be used to generate diversity. Figures 3A and 3B provide a summary of the amino acid coverage of these designs. In general, it is preferable, but not necessary, that the designs narrow the diversity to cover more of the natural diversity and exclude as much as possible the "non-target" amino acids.

### Library Design: H3

In comparison to other CDRs, heavy chain CDR3 (H3) regions exhibit the greatest diversity in sequences and lengths, although the sequence diversity is not completely random (i.e., some amino acids were found to occur more often than other in particular amino acid positions). Because of this diversity in sequences and sequence lengths, it is difficult to design a strategy that provides for diversity in H3 region. Using a single antibody template molecule allows for a strategy that includes different lengths of H3 because the template can be selected and designed to accommodate H3 regions of varying lengths in the initial design of the library. The template is selected to accommodate different lengths of H3 region while maintaining good structural characteristics of folding and stability. The diversity in the H3 region is preferably tailored to mimic natural diversity, although different libraries generated with different codon sets can also be used to develop antibodies that bind to a variety of different epitopes in the target antigen or to provide for isolation of more binders having unique H3 sequences.

In a preliminary analysis to assess the amino acid preferences for each position in H3, a library with diverse H3 was generated using an *NNK* codon set for residues 95-100a of the humanized antibody 4D5 H3 region. The *NNK* codon set encodes all 20 amino acids and stop codons. This library was generated in a Fab phage display format and 400 clones that displayed functionally on phage were identified and sequenced. The amino acid sequence found in H3 regions in the *NNK* library were compared to those found in the Kabat database. A comparison of those amino acids is shown in Figure 23. When the amino acid sequences in the *NNK* library and Kabat database were analyzed, it was determined there was good agreement in most amino acid usage between the library sequences and the sequences of Kabat. Interestingly, there appeared to be more aliphatic/hydrophobic amino acids in the *NNK* library than in the known, naturally occurring sequences. See Figure 23.

Diversity in H3 was also determined using codon sets *DVK* and *XYZ.* The amino acid frequency for each amino acid in the H3 region randomized with *D VK* or *XYZ* was identified and compared to the amino acid frequency of naturally occurring antibodies in the Kabat database as described above. The results are shown in Figure 42. The results show that both the *D VK and XYZ* library had amino acid usage that was generally in good agreement with that of naturally occurring antibodies. The *DVK* library did show decreases in usage of tyrosine, leucine, valine and isoleucine. The *XYZ* library also showed less frequent usage of tyrosine.

.Codon sets are identified by 3 capitol letters in italics(eg. *DVT, NNK, NVT*)*.* These letters refer to generic codon formulas as follows: R is 50%A/50%G; M is 50%A/ 50%C; K is 50%G/ 50%T; S is 50%G/50%C; W is 50%A/ 50%T; H is 33%A/33%T/33%C; B is 33%G/33%T/33%C; V is 33%G/33%A/33%C; D is 33%G/33%A/33%T; N is any nucleic acid; X ix 38%G, 19% A, 26% T, and 17%C; Y is 31% G, 34%A, 17% T, and 18%C; and Z is 24%G/76%C.

Codon sets that resulted in similar amino acid usage in H3 as compared to natural diversity were then used to design libraries containing diversified H3. These codon sets include *NNK, NNS, NVT; DYK and XYZ.* In some embodiments, codon sets encoding all 20 amino acids at a position, such as *NNK or NNS* were utilized as shown in Figure 4.

One of the codon sets *was DVK. DVK* encodes ACDEGKNRSYW and a stop codon. Amino acids that do not occur frequently in known, natural antibody sequences were excluded, for example, LPHQFIMV. Oligonucleotides encoding H3 regions of varying lengths utilizing DVK codons are shown in Figure 4. In one embodiment, a pool of oligonucleotides including F59, F63, F64, F65, F66, and F78 were utilized to form a library of molecules with diversity in H3.

Another codon set used was *NVT. NVT* encodes ACDNGHPRSTY and excludes W (tryptophan) and a stop codon. Tryptophan is favored in phage display and tends to dominate. Stop codons can decrease library diversity. In some instances, the *NVT* design was doped with W by walking W across the residues. Oligonucleotides encoding H3 regions of oligonucleotides including F134, F136, FF137, F138, F142, F155, F156, F157, F158, F160 and F160g were utilized to form a library of molecules with diversity in H3.

In another embodiment, a codon set XYZ was used as shown in the oligonucleotides represented in Figure 4 and Figure 43. The codon set *XYZ* encodes all 20 amino acids.

In terms of which residues to diversify in H3, it was determined that the C-terminus of H3 can be less diversified. In some embodiments, the H3 C terminal was held constant. The C-terminus of H3 had mainly two types of sequences in the Kabat database, the sequences being either:
Y₁₀₀ₐAMD₁₀₁(Y/V)₁₀2 (sometimes Y₁₀₀ₐ can vary slightly)
or F₁₀₀ₐD₁₀₁,(Y/V)₁₀₂.

In humanized antibody 4D5, the C-terminus of H3 is YAMDY. The libraries were designed to keep this part mostly constant, except Y₁₀₀ₐ was varied. Y₁₀₀ₐ was varied using a variety of codon sets such as *DYK, DSG, KSG, NVT and XYZ.* Alternatively, single amino acid substitutions at this residue can be made with substitution of Y with glycine, serine, alanine, tryptophan, valine or aspartic acid.

Libraries were generated using oligonucleotides that encode different sequence lengths in the H3 region. H3 lengths in naturally occurring antibodies range from about 2 amino acids to about 24 amino acids. A series of oligonucleotides was prepared encoding H3 regions of varying lengths from about 7 amino acids to about 19 amino acids as shown in Figure 43. These oligonucleotides also provided for limited variation at the C-terminal end. Less diversity is found at the C-terminal end but the oligonucleotides provide for variation at the C-terminal end of YAMD (Y/V) or FD(Y/V). Variations at the 100a and/or 100b position were also included. The H3 residues that were varied were varied using codon sets *NNK, NNS, XYZ* or mixtures thereof. Other examples including varying lengths of H3 regions are shown in Figure 4. In Figure 4, the HC region varies from about 11 to 13 residues some embodiments have the more constant C-terminal of YAMDY. Some variation is provided at the residue position 100a(Y). The CDRH3 amino acid positions are varied using codon sets such as *DVK, NVT, NNK, XYZ and NNS* or mixtures thereof.

In one embodiment, multiple libraries were prepared with diversity in the H3 in both sequence and length using each of the oligonucleotides shown in Figure 43. These libraries provide diversity in the sequence and length of the H3 region. Diversity was also generated in the H1/H2 regions as described previously. The libraries were pooled and then sorted against target antigen murine or human VEGF. The high affinity and specific binders were isolated and sequenced. Isolating and sequencing the binders allows identification of the optimal length of the H3 for binders to murine or human VEGF was about 11 to 13 amino acids. (Data not shown)

In some designs, framework residue 93 was changed to alanine to reflect the natural consensus (humanized antibody 4D5 has the mouse residue serine). Framework residue 94 (right before the first H3 residue) was designed to be arginine, or arginine and lysine (using codon ARA) to reflect the natural sequence consensus. In addition, in some embodiments, 3 amino acids at the N-terminus of the heavy chain were removed right after the secretion signal.

### EXAMPLE 4

### Library Design: L1,L2,L3

Libraries of antibody variable domains were designed to maximize diversity in the CDR regions of light chains while minimizing structural perturbations in the antibody variable domains. Structural perturbations in antibody variable domains are generally associated with improperly folded antibody domains resulting in low yield, for example when produced in bacterial cells. Low yields decrease the number of binders detected in screening. Diversity in the CDR regions was generated by identifying solvent accessible and highly diverse positions in each CDR for CDRs L1, L2 and L3, and designing an oligonucleotide comprising at least one tailored (i.e., non-random) codon set encoding variant amino acids for the amino acid position corresponding to the position of at least one solvent accessible residue at a highly diverse position in at least one CDR region. A tailored codon set is a degenerate nucleic acid sequence that preferably encodes the most commonly occurring amino acids at the corresponding positions of the solvent accessible residues in known, natural antibodies.

Solvent accessible residues in the CDRs were identified in the antibody variable domain template molecule by analyzing the crystal structure of the template molecule. Humanized antibody 4D5 is efficiently produced and properly folded when produced in a variety of host cells, including bacterial cell culture. The crystal structure for the humanized antibody 4D5 variable region is known and publicly available at www/rcsb/org (accession code IFVC).

The solvent accessible positions in the CDRs of the light chain and CDR1 and CDR2 of the heavy chain were identified using the Insight II program (Accelrys, San Diego, CA).

CDR residues were also analyzed to determine which positions in the CDRs were highly diverse. Highly diverse positions in the CDR regions for the heavy and light chains were identified by examining the sequences of known, naturally occurring antibodies in the Kabat database. The Kabat database is available at immuno/bme/nwu/edu. In the Kabat database, there were about 1540 sequences of the human light chain and 3600 sequences for the human heavy chain. The CDR sites were aligned and numbered as described by Kabat (see immuno/bme/nwu/edu). Highly diverse amino acid positions were identified by lining up and ranking the amino acid usage, from most frequently used to less frequently used for each CDR residue. For example, L3-91 (i.e., residue 91 of the light chain CDR3) was found to be Y (tyrosine) in 849 out of 1582 antibody sequences in the Kabat database, and it is the amino acid found most frequently at this position. Next on the list of frequency serine (occurring in 196 sequences), followed by arginine (169 sequences), alanine (118 sequences), glycine (61 sequences), histidine (41 sequences), with the remaining 35 sequences being one of the remaining amino acids. Illustrative diverse sites, with corresponding diversity list of amino acids, are shown in Figure 1 (for the light chain). The highly diverse positions, as well as the most frequent amino acids at those positions, may change over time as more sequences are incorporated into the database.

Amino acid residues found in a particular position that collectively constitute the most frequently occurring amino acids among the known, natural antibody sequences are selected as the basis for library design. The most frequently occurring amino acids were deemed to be those that most commonly found in the top 90% of the list of diverse amino acids (this group of amino acids is referred to herein as "target group of amino acids"). However, as described herein, the percent cutoff for a target group of amino acids can be varied, as described above, according to the circumstances and purpose of the diversity library that is to be achieved.

For humanized antibody 4D5, the positions identified as solvent accessible and highly diverse were: Light Chain
- CDR1: 28,29,30,31,32
- CDR2: 50,53
- CDR3: 91,92,93,94,96

Examples of amino acids that occur at high frequency in natural diversity (i.e., among known, natural antibody sequences) (referred to as "target group" or "natural diversity" in Figure 3), and the designed diversity of amino acids by DNA codons ("Diversity<DNA codon>") for each of these positions is shown in Figure 3.

Codon sets encoding a specific group of amino acids (Diversity) were designed to include at least a certain percentage of the amino acids in the known, natural sequences (designated as "% covering" in Figure 3). Of the amino acids encoded by a codon set, at least about 40% of the amino acids, more preferably 70%, are target amino acids identified for a particular solvent accessible and highly diverse position (designated as "% good" in Figure 3; amino acids encoded by a codon set that are target amino acids are shown in bold in column 3 of Figure 3). However, as described herein, the % good value can be varied according to circumstance and objectives. The codon sets were selected such that they preferably encoded the amino acids with the highest occurrences at a particular position. The number of non-target amino acids coded by a codon set for a particular position was minimized. Effectiveness of codon set selection/design was evaluated in part based on the "% good" value. A high percentage meant very low non-target amino acids; a high value of "% good" was deemed more important than having more target amino acids among the amino acids coded by a particular codon set. Redundancy was included in calculating the "% good" value. For evaluation purposes, the "% covering" value was also calculated. This value represents the percentage of natural diversity covered by the "good" amino acids (of the amino acids encoded by a particular codon set). For example, for L3-91, when codon set KMT is used, the "good" amino acids are YSA, which is 75% of the YSAD amino acids encoded by the codon set. YSA are amino acids that cover 1190 out of 1580 known, natural antibody sequences at this amino acid position. 1190/1580 equals 75%, which is the "% covering" value. Thus, in one design using KMT at L3-91, 75% of the library covers 75% of the natural diversity in CDRL3 at position 91.

The codon sets were also designed to exclude, when possible, cysteine and stop codons. The presence of cysteine residues tends to cause folding problems and stop codons can decrease the effective library size. In the design of the codon sets, it was also deemed desirable to minimize the number of nontarget amino acids.

The codon sets designed for each solvent accessible and highly diverse residue of humanized antibody 4D5 are shown in Figure 3. At any particular residue, one or more codon sets could be used depending on the target amino acids that were identified. Codon sets *YKG* and *TWT* can each be used to generate diversity at L3-96.

The various codon sets could be used to generate diverse libraries with diversity in one or more CDR regions, including CDRL1, CDRL2 and CDRL3. For example, Figures 5-13 and Figure 24 show various illustrative versions of codon set designs that can be used to generate diversity. Figures 3A and 3B provide a summary of the amino acid coverage of these designs. In general, it is preferable, but not necessary, that the designs narrow the diversity to cover more of the natural diversity and exclude as much as possible the "non-target" amino acids.

### EXAMPLE 5

### Construction, sorting and analysis of scFv libraries

Libraries with diversified CDRs were generated using vectors comprising 4D5 variable domains in the scFv or scFv-zip formats in vector pS1607 as described in Example 1. In total, five libraries were generated and the library characteristics were as follows:

| Library name | Format | CDR Diversity |
|---|---|---|
| ScFv-1 | zipper | H1, H2, H3 |
| ScFv-2 | Zipper | H1,H2,H3,L3 |
| ScFv-3 | Zipper | H3, L3 |
| ScFv-4 | No zipper | H1, H2, H3 |
| ScFv-5 | No zipper | H1 , H2, H3, L3 |

Libraries were constructed using the method of Kunkel (Kunkel et al., Methods Enzymol. (1987),154, 367-382) with previously described methods (Sidhu et al., Methods Enzymol. (2000), 328, 333-363). For each library a "stop template" version of a scFv or scFv-zip display vector was used; in each case, a stop template with TAA stop codons within each of the CDRs to be randomized was used. Mutagenic oligonucleotides with degenerate codons at the positions to be diversified were used to simultaneously introduce CDR diversity and repair the stop codons. For example, for the construction of library scFv-1, three oligonucleotides were simultaneously used to introduce diversity into CDR-H1, CDR-H2, and CDR-H3. Successful incorporation of all the mutagenic oligonucleotides resulted in the introduction of the designed diversity at each position and simultaneously repaired the stop codons, thus generating an open reading frame that encoded either a scFv or scFv-zip fused to the C-terminal domain of the gene-3 minor coat protein. Residues in each CDR were chosen for diversification based on their solvent accessiblity and their level of diversity in the Kabat data base of natural antibodies (as described in Examples 2 and 3). For CDRs H1 and H2, the residues chosen for diversification and the diversity introduced at each position are shown in Figure 24. For CDR-H3, diversity was introduced using an equimolar mixture of four mutagenic oligonucleotides (F59, F63, F64 and F65 in Figure 4).

The mutagenesis reactions were electroporated into E. coli SS320 (Sidhu et al., Methods Enzymol.(2000), 328, 333-363), and the transformed cells were grown overnight in the presence of M13-VCS helper phage to produce phage particles that encapsulated the phagemid DNA and displayed scFv or scFv-zip fragments on their surfaces. Each library contained greater than 2 x 10¹⁰ unique members.

### 1. Selection of Specific Antibodies from the Naïve Libraries.

Phage from each library described above were cycled through rounds of binding selection to enrich for clones binding to targets of interest. Three target proteins were analyzed: Her2, IGF-1, and mVEGF. The binding selections were conducted using previously described methods (Sidhu et al., supra).

NUNC 96-well Maxisorp immunoplates were coated overnight at 4°C with capture target (5 ug/mL) and blocked for 2 h with bovine serum albumin (BSA) (Sigma). After overnight growth at 37 °C, phage were concentrated by precipitation with PEG/NaCl and resuspended in PBS, 0.5% BSA, 0.1% Tween 20 (Sigma), as described previously (Sidhu et al., supra). Phage solutions (10¹³ phage/mL) were added to the coated immunoplates. Following a 2 h incubation to allow for phage binding, the plates were washed 10 times with PBS, 0.05% Tween20. Bound phage were eluted with 0.1 M HCl for 10 min and the eluant was neutralized with 1.0 M Tris base. Eluted phage were amplified in *E*. *coli* XL1-blue and used for further rounds of selection.

The libraries were subjected to two rounds of selection againsts each target protein (Her-2, IGF-1 or mVEGF), followed by a round of selection (round 2a) against an anti-gD epitope antibody to enrich for clones displaying scFv or scFv-zip (there is a gD epitope in the linker between the light chain and heavy chain regions of the scFv). Following the anti-gD enrichment, each library was selected for a third round against the target protein.

Individual clones from each round of selection were grown in a 96-well format in 500 uL of 2YT broth supplemented with carbenicillin and M13-VCS, and the culture supernatants were used directly in phage ELISAs (Sidhu et al., supra) to detect phage-displayed scFvs that bound to plates coated with target protein. The results for each library against each target protein after three rounds of sorting are shown in Figure 25, and it can be seen that each library produced binders against each target protein.

The three scFv-zip libraries (scFv-1, scFv-2 and scFv-3) were subjected to more detailed analysis. Phage clones from round 3 of selections against IGF-1 or mVEGF were analyzed for specific binding to target by doing phage ELISAs against both IGF-1 and mVEGF. Clones that bound only to the target against which they were selected were classified as specific while those that bound both targets were classified as non-specific. The results are shown in Figure 26 which indicates the percentage of clones from each selection that bound targets (Total) and the percentage of clones that bound only the target against which they were selected (specific). All three libraries produced specific binding clones against target, although library scFv-3 produced considerably fewer specific binders than did libraries scFv-1 and scFv-2.

For library scFv-1, clones were also screened from round 2 of selection. From the IGF-1 selection, 140 out of 960 screened clones (15%) were positive. From the mVEGF selection, 24 out of 1152 screened clones (2%) were positive.

For one scFv library (library scFv-4), several hundred clones from round 3 of the IGF-1 or mVEGF binding selections were screened for specific binders. In this case, the IGF-1 selection yielded 35% specific binders while the mVEGF selection yielded 40% specific binders.

Figure 29 shows the sequences and affinities of some positive binders from scFv and ScFv-zip libraries.

### 2. Sequencing of Antigen Specific Binders

Representatives of the specific binding clones characterized above were sequenced using standard methods. The results are shown in Figure 27.

From the IGF-1-specific binders, a total of 255 clones were sequenced and 192 of these were unique sequences. From the mVEGF-specific binders, 202 clones were sequenced and 86 of these were unique sequences. These results confirmed that the methods of the invention for generating CDR diversity and selecting for antigen specific binders resulted in a multiplicity of antigen specific antibody variable domains with different sequences.

The complete sequences from about 450 binding clones were analyzed for amino acid diversity at the heavy chain residues that were diversified in the library designs. The results indicated that all the designed substitutions occurred with a good distribution of amino acid type (data not shown).

Analysis of the sequences of the CDR-H3 region indicated that all four different designs included in the naive libraries (F59, F63, F64 and F65 in Figure 4) were also present in the selected binding clones, as shown in Figure 28. However, the four CDR designs were not equally common among the selected binders, indicating that some CDR designs may be better suited for generating positive binding clones against particular targets. In particular, the design F64 was the most prevalent (52%) while the design F63 was the rarest (5%) (Figure 28).

### EXAMPLE 6

### F(ab')₂ Libraries with L3/H3 Diversity

Libraries with diversity in CDRL3 and H3 were generated using the template Fab-zip 4D5 vector pS 1607 as described in Example 1. For CDRL3, oligonucleotide F61 (F61: GCA ACT TAT TAC TGT CAG CAA NRT NRT RVM NNK CCT TDK ACG TTC GGA CAG GGT ACC; the underlined nucleotides encoding amino acid residues/positions that were diversified; SEQ ID NO: 30). For CDRH3, oligonucleotides used were designated F59, F63, F64, F65, F66, and F78. (See Figure 4). A pair of L3 and H3 oligonucleotides were used per Kunkle mutagenesis reaction, and libraries were made and amplified in E. coli as described in Example 4. In total, six libraries with different H3 diversity (generated with six different oligonucleotides as described above) were made and combined after amplification for sorting on targets. Sorting was done as in Example 4, except that this library underwent two tracks of sorting: with or without presorting on anti-gD before target sorts. The library was either directly sorted on targets (i.e., with no presort) or first on anti-gD as a presorted library before sorting on targets. After one presort, the library was sorted on targets two times and then on anti-gD once before sorting on targets another time. Library without presorting went through targets twice and then was enriched on anti-gD once and then another time on targets.

The hit rate was significantly better with presorted library sorted against mVEGF or IGF-1. For HER2ECD as targets, both presorting or no presorting worked well. The hit rate after two target sorts and one anti-gD sort was about 1-4%. A final sort on target after anti-gD sort resulted in a 90% hit rate. Hit rate was calculated as specific binders found per 100 clones screened. Specific binding clones were defined as in Example 5.

Positive clones were sequenced for their H3 sequences and IC50 (affinity) analyzed as previously described (Sidhu et al, supra). Figure 29 shows the results of analysis of each clone. Clones were obtained with variable sequences and affinity in the range of sub-micromolar and micro-molar. Most binders came from libraries with *DVK* design in H3. In column 2 of Figure 29, underlined residues represent residues that were fixed in the source library of the clones. Most binding clones came from the library that fixed the Y₁₀₀ₐ position.

The binding epitope of some of the clones was also analyzed by competitive binding ELISA. Target (murine VEGF) was coated on 96-well NUNC-maxisorb plate. Phage clones binding to the target in the presence of blocking reagent which bound to a particular epitope on mVEGF was measured. Two mVEGF receptor fragments were used, Flt-D2 (Wiesmann et al., Cell (1997), 91:695-704) or KDR1-7-igg (Fuh et al., J. Biol. Chem. (1998), 273:11197-11204). The results showed that all analyzed binders bound to a similar epitope as KDR1-7-igg since they competed with each other (Figure 30). Flt-D2 (Domain 2 of Flt-1), which has a smaller epitope on mVEGF, only blocked one clone but not the others (Figure 31).

To demonstrate that the binding phage clones did present Fab polypeptide sequences that could become Fab antibody fragments, we generated Fab protein from some of the clones. Phage constructs from binding clones were transformed into E. Coli strain 27C7 that does not have amber stop suppressors, and the bacteria were grown to produce Fab protein. Figure 32 summarizes the characterization of these clones. Fab protein was successfully generated from clones V2, V5 and V8.

### EXAMPLE 7

### Fab and F(ab')₂ Libraries with H1/H2/M3 Diversity

Libraries were generated as described above. Template construct was pV0116b for Fab or pV0116g for F(ab')_{2.} These vectors encode light chain variable domains that change the 4D5 residues at position 30 and 66 to reflect human consensus sequence (N30S; R66S), both of which had phoA promoter and stII signal sequences for both light chain and heavy chain. To make Fab H1/H2/H3 libraries, each mutagenesis reaction used oligonucleotides that coded for H1, H2 and H3 diversity. To ensure the incorporation of all three CDRs in the randomization scheme, a stop codon (TAA) was incorporated in each CDR that was intended to be diversified. Only clones that incorporated all three CDR oligonuceotides would have positive display since the stop codons would have been replaced. Oligonucleotides of different diversity were first combined to use as a source to diversify each CDR.

For this experiment, two H1 oligonucleotides, F151 (GCA GCT TCT GGC TTC ACC ATT AVT RRT WMY KMT ATA CAC TGG GTG CGT CAG; SEQ ID NO: 16) and F152 (GCA GCT TCT GGC TTC ACC ATT AVT RRT WMY KGG ATA CAC TGG GTG CGT CAG; SEQ ID NO: 14) (See also Figure 13) were pooled, and for H2, oligonucleotides F153 (AAG GGC CTG GAA TGG GTT GST DGG ATT WMT CCT DMT RRC GGT DMT ACT DAC TAT GCC GAT AGC GTC AAG GGC; SEQ ID NO: 18) and F154 (AAG GGC CTG GAA TGG GTT GST DHT ATT WMT CCT DMT RRC GGT DMT ACT DAC TAT GCC GAT AGC GTC AAG GGC; SEQ ID NO: 19) (See also Figure 13) were pooled.

For H3, a *DVK* pool of oligonucleotides (F165, F166) and *NVT* pool (F134, F136, F137, F138, F142, F155, F156, F157, F158, F160, F160g) were used. Figure 4 shows H3 positions that were subjected to diversification. Two Fab libraries were generated: one with *DVK* H3 pool and one with *NVT* H3 pool. The two libraries were amplified in E. Coli before being combined for sorting on the targets.

Mutagenized DNA was used to tranform E. Coli strain SS320 by electroporation and size of the libraries were in the range of 10⁹. Transformed bacterial cells were grown up overnight in the presence of helper phage KO7 to produce displaying phage that could still infect other bacterial cells as described in Example 4.

### Sorting on mouse Vascular Endothelial cell growth factor (mVEGF) and human IgG1-Fc (hFc)

*DVK* and *NVT* libraries were pooled for sorting on the targets. Sorting was performed as with other libraries as described above. The combined library was sorted first on target once, next sorted with anti-gD antibody which could get rid of the non-displaying clones, and next with two sorts on targets (S3, S4). About 96 clones from S3, S4 were screened. Positive clones were clones that had above background binding to the targets (binders) and not to other non-relevant proteins (i.e., specific binders). For mVEGF as target, S4 provided the highest hit rate for positive specific binders. For human Fc, S3 and S4 provided high rate of specific binders.

| Library | mVEGF | | hFc | |
|---|---|---|---|---|
| | Total binders | Specific binders | Total binders | Specific binders |
| Fab S3 | 36% | 1-2% | 88% | 83% |
| Fab S4 | 91% | 72% | 99% | 90% |
| | | | | |
| F(ab')₂ S3 | 42% | 3-5% | ND | ND |
| F(ab')₂ S4 | 73% | 72% | ND | ND |

| | | | | |
|---|---|---|---|---|
| ND: Not determined | | | | |

The DNA sequences of the binders and the binding affinity of the unique binders were analyzed. Examples of sequences and binding affinity of binders are shown in Figure 33. For specific hFc binders, many distinct Fab clones, some of which binding at 40nM, 2uM and 5uM individually were obtained. From the F(ab')₂ library, clones with affinities at 41, 47 and 110 nM were obtained. Additional sequences of VEGF binders are shown in Figure 35.

### EXAMPLE 8

### Solution Binding Competition ELISA

To determine a binding affinity for selected anti-Her2 F(ab) and F(ab')2 phage, competition ELISAs were performed as described below. The solution binding competition assay had been previously shown to correlate with Fab protein affinity Kd as shown in Figure 46. The solution binding assay was used as an effficient method for identifying the affinity of clones for a target antigen from the library.

First, the phage were propagated and purified. Ten uls of XL-1 bacteria infected with one of the clones for 30 minutes at 37°C was plated on a carbenicillin plate. A colony was picked and grown in 2 mls (2YT and 50ug/ml carbenicillin) at 37°C for 3-4 hours. Helper phage, KO7 or VCS (VCS is a variant of K07) was added to the culture at a final concentration of 10¹⁰ pfu/ml for another 1 hour at 37°C. Twenty mls of media (2YT/CRAP 50:50 with 50ug/ml carbenicillin was added to the culture for growth overnight at 37°C. The phage was purified as described previously.

Second, the concentration of purified phage that would be optimal for use in the following competition ELISA assay was determined (i.e., approximately 90% of maximal binding capacity on the coated plate). 96-well Nunc Maxisorp plates were coated with Her-2 extracelleular domain(2 ug/ml in PBS) at 4°C overnight or at room temperature for 2 hours. The wells were blocked by adding 65ul 1% BSA for 30 minutes followed by 40ul 1% Tween20 for another 30 minutes. Next, the wells were washed with PBS - 0.05% Tween20 5 times. Various dilutions of F(ab) or F(ab')₂ phage down to 0.1 O.D./ml in ELISA buffer (PBS- 0.1 %BSA and 0.05% Tween20) were added to the wells for 15 minutes at room temperature. The wells were then washed with PBS - 0.05% Tween20 at least three times. 75ul of HRP-conjugated anti-M13 antibody (Amersham, 1/5000 dilution with ELISA buffer) per well was added and incubated at room temperature for 30 minutes. The wells were washed again with PBS - 0.05% Tween20 at least five times. Next, 100ul/well of a 1:1 ratio of 3,3',5,5'-tetramethylbenzidine (TMB) Peroxidase substrate and Peroxidase Solution B (H₂O₂) ((Kirkegaard-Perry Laboratories (Gaithersburg, MD)) was added to the well and incubated for 5 minutes at room temperature. The reaction was stopped by adding 100ul 1M Phosphoric Acid (H₃PO₄) ((Kirkegaard-Perry Laboratories (Gaithersburg, MD)) to each well. The optical density of the color in each well was determined using a standard ELISA plate reader at 450 nm. The dilutions of phage were plotted against the O.D. readings.

Third, a competition ELISA was performed. 96-well Nunc Maxisorp plates were coated with human Her-2 ECD (2ug/ml in PBS) at 4°C overnight or at room temperature for 2 hours. The wells were blocked by adding 65ul % BSA for 30 minutes followed by 40ul 1% Tween20 for another 30 minutes. The wells were washed with PBS - 0.05% Tween20 5 times. Based on the binding assay above, 50ul of the dilution of phage that resulted in about 90% of maximum binding to the coated plate was incubated with 50ul of various concentrations of human Her-2ECD (0.1 to 500nM) in ELISA buffer solution for 1 hour at room temperature in a well. The unbound phage was assayed by transferring 75ul of the well mixture to second 96-well plate pre-coated with human Her-2ECD and incubating at room temperature for 15 minutes. The wells of the second plate were washed with PBS - 0.5% Tween20 at least three times. 75ul of HRP-conjugated anti-M13 antibody (1/5000 dilution with ELISA buffer) per well was added and incubated at room temperature for 30 minutes. The wells were washed again with PBS - 0.05% Tween20 at least five times. Next, 100ul/well of a 1:1 ratio of 3,3',5,5'-tetramethylbenzidine (TMB) Peroxidase substrate and Peroxidase Solution B (H₂O₂) ((Kirkegaard-Perry Laboratories (Gaithersburg, MD)) was added to the well and incubated for 5 minutes at room temperature. The reaction was stopped by adding 100u1 1M Phosphoric Acid (H₃PO₄) ((Kirkegaard-Perry Laboratories (Gaithersburg, MD)) to each well. The optical density of the color in each well was determined using a standard ELISA plate reader at 450 nm. The concentrations of competitor were plotted against the O.D. readings. The IC50, the concentration of Her-2 ECD that inhibits 50% of the F(ab)-phage or F(ab')₂-phage, represents the affinity. See also Figure 19.

### EXAMPLE 9

A combination of different sorting and screening methods can be used for any of the libraries generated as described herein. Assays utilizing target bound to a solid and solution phase assays maybe utilized as described below.

### Selection for Affinity

A combination of sorting methods and high throughput affinity screening assays has been shown effective to select binders with high affinity from a library of either monovalent (Fab) or multivalent F(ab'2) polypeptides. (Figure 45) Different sorting methods can be used to combine with the screening assay as means to find the high affinity clones. Described below is a method of solution sorting method and a high throughput affinity screening assay.

For the first round of sorting, the usual plate-based selection/sorting is preferred because it is more efficient in capturing binding clones. (See also Figure 44.) Add 100u1/well library of phage 1-3 OD (270 nM) in PBS/BSA buffer on target coated Maxisorb wells at room temperature for 1 hour. Wash plate with PBS/0.05% tween20 about five times. Elute with 0.1N HCl for 20 min. at room temperature. Titer and propagate from 4 wells. The washing of the plates is low stringency in order to capture the most binders with a wide range of affinities.

For solution sorting, biotinylated target antigen can be used. (See also Figure 45.) To biotinylate the target protein, target protein should preferably be in amine-free buffer (for e.g., no Tris, no Glycine or any other primary amine containg buffer or free amino acids), preferably at pH higher than 7.0 and protein is >0.5mg/ml concentration (eg. PBS). Buffer exchange can be done with NAP-5 (Amersham Pharmacia Biotech) column if needed. Stock NHS-Biotin reagent (Pierce Biotechnology) in PBS (100X) is preferably prepared fresh before use. Add in about 3:1 molar ratio to target protein and incubate at room temperature for 30 min. to 1hour. Add Tris pH7.5 or ethylamine to 100mM to quench the unreacted NHS for 30 min. Buffer exchange with NAP-5 can be used to remove the free biotin reagent. This is not necessary in most cases since there is very low amount of remaining biotin and the concentration of protein can be calculated from dilution after biotinylation. Biotinylated protein should be tested, if possible, for the ability to bind to natural binding partner, e.g. receptor and/or antibody, to make sure biotinylation did not disrupt the function.

Coat plates with 2-5ug/ml neutravidin (Pierce Biotechnology) in PBS 100ul/well 4°C or room temperature for 2 hours. Block the plate with Superblock (Pierce) in PBS for 30 min., then, add Tween20 to 0.2% and block for another 30 min.

Incubate 0.5-1 OD phage with appropriate concentration of biotinylated target protein in 120-150ul buffer containing Superblock 0.5% and 0.1% tween20 for 0.5-1 hour. Dilute 5-10X with binding buffer and apply 100ul/well to neutravidin coated wells to allow capture of biotinylated target bound with phage for 5 min at room temperature with gentle shaking. Wash plate with PBS/tween20 multiple times, for e.g. eight times. For determination of background binding, phage not incubated with biotinylated target antigen is also captured on neutravidin-coated plate. Another possible control is based on biotinylated target antigen mixed with phage captured in a well not coated with neutravidin. Bound phage are eluted with 0.1N HCl for 20 min, neutralized and titered and propagated to next round.

The concentration of biotinylated reagent to use depends on situation. In one instance, using 100-250nM target concentration is equivalent to plate sorting in terms of stringency. For example, to improve affinity of a 100nM binder, it is good to start with 20nM as first solution sort and progress to 5 and 1nM at the second sort. Multiple concentrations should be included. The concentration that provides for enrichment should be used with the screening assay. For the third sort, adding excess of unbiotinylated target as competitive sorting (described below) may be called for. When low concentration (e.g. < 2nM) of biotinylated target is used, 5X instead of 10X dilution before capture is preferred. Input phage may decrease to 0.1 OD in later round of sorting which will further increase the selection pressure.

Competitive sorting can be done to increase the pressure of selection. After the 30 min. - 1 hour incubation of phage and biotinylated targets (described above), before dilution for capturing, 1000 fold excess of unbiotinylated target may be added and incubated for different length of time at different temperature. Longer time and higher temperatures (e.g., 37°C vs room temperature) increase the pressure of selection. For example, to improve affinity from a 1 nM anti-VEGF binder: In the first round of solution sorting, using 1 nM biotinylated target to select. Before dilution for capturing, adding 1 uM unbiotinylated target at room temperature for 15 min. to compete off high off-rate binders. In the following rounds of solution sorting, using 0.1 nM biotinylated target to select tighter binders, and before dilution for capturing, adding 100nM unbiotinylated target at 37°C for 30 min. or, more stringently, 2 hours to select for the low off-rate binders.

### High throughput affinity screening assay:

A schematic of this assay is shown in Figure 47. Colonies (freshly grown) were picked and grown in 96 well plate (150ul/well) or rack (300ul/tube) overnight at 37°C in 2YT/CRAP (1:1) with 50ug/ml carbenicillin and 1n/ml K07. After growth, the colonies were spun down to obtain the phage supernatant.

The phage supernatent was diluted 1:6-10 if grown from plate or 1:3-5 if grown from rack in ELISA binding buffer (PBS, 0.5%BSA, 0.05% Tween20) with or without target antigen in 150 ul total volume and incubated for 1 hour at room temperature. Final concentration of target in this binding experiment depends on expected/desired the affinity. For screening for sub-nM affinity binders, 10-25nM were used. To screen for 1-100 nM binders, a 100-150nM target concentration is preferred.

Plates were coated with 5ug/ml of target antigen overnight or 2 hours at room temperature and blocked as described above. About 70-100 ul of phage was transferred with or without target antigen to the coated plate, transferring sample (with or without target antigen) into side by side wells simultaneously. The plates were shaken gently for 10-15 minutes to allow the capture of unbound phage to plate. The plates were washed quickly six times with washing buffer. HRP-conjugated anti-M13 antibody in binding buffer (1:5000) was added to the plates and incubated for 30 min., the plates were washed 6 times, and the substrate TMB/H₂O₂ was added for 5-10 min. and absorbance was measured at 450nM.

The % inhibition of binding was calculated by calculating the ratio of OD of samples plus target over samples minus target. The lower the %, the higher the potential affinity of the clones. The graph shows the data from screening about 40 clones. The results show that a great variety of affinities ranging from high affinity (low ratio) to low affinity (high ratio) were present in the 40 clones.

All publications (including patents and patent applications) cited herein are hereby incorporated in their entirety by reference.

The following paragraphs define further aspects and embodiments of the invention:
1. A polypeptide comprising a variant CDRH3 region that comprises an amino acid sequence:

   (X₁)ₙ - X₂ - X₃ - X₄ - X₅ ―X₆

   wherein X₁ is any naturally occurring amino acid encoded by a codon set of *NNK, NNS, DVK, NVT, XYZ* or is tryptophan or glycine or mixtures thereof, and n = 4 to 14;
   X₂ is any naturally occurring amino acid encoded by a codon set of *KSG, DVK, DSG, NVT, KSG, NNS, XYZ, NNK* or is tyrosine, phenylalanine, serine, alanine, glycine, leucine,or tryptophan;
   X₃ is missing when X₂ is phenylalanine or leucine, or if present, is alanine, valine, aspartic acid or glycine;
   X₄ is missing when X₂ is phenyalanine or leucine, or if present, is methionine;
   X₅ is aspartic acid , alanine, valine, or glycine;and
   X₆ is tyrosine or valine.
2. The polypeptide of para 1, wherein the polypeptide is a heavy chain antibody variable domain.
3. The antibody variable domain of para 2, wherein X₃ is alanine, X₄ is methionine, X₅ is aspartic acid and X₆ is tyrosine.
4. The antibody variable domain of para 2, wherein X₂ is tyrosine.
5. The antibody variable domain of para 2, wherein X₂ is leucine or phenylalanine, X₃ and X₄ are missing, X₅ is aspartic acid and X₆ is tyrosine.
6. The antibody variable domain of para 3, wherein n = 6.
7. The antibody variable domain of para 5, wherein n = 6.
8. The antibody variable domain of para 2, wherein n is 6, 7, or 8.
9. The antibody variable domain of para 2, wherein X₁ corresponds to amino acid position 95 and n = 6, X₂ corresponds to amino acid position 100a, and X₃ corresponds to amino acid position 100b in CDRH3 of antibody 4D5.
10. A nucleic acid comprising a polynucleotide molecule that encodes the polypeptide of any of paras 1 to 9.
11. A vector that comprises the nucleic acid of para 10.
12. The vector according to para 11 that is a replicable expression vector.
13. The vector according to para 12, wherein the vector has a promoter region linked to the polypeptide selected from the group consisting of the lac Z promoter system, the alkaline phosphatase pho A promoter (Ap), the bacteriophage λ_{PL} promoter (a temperature sensitive promoter), the tac promoter, the tryptophan promoter, and the bacteriophage T7 promoter.
14. A virus comprising a polypeptide of any of paras 1 to 9 displayed on its surface.
15. A library comprising a plurality of at least 1 x 10⁸ distinct polypeptide sequences of any of paras 1 to 9.
16. A host cell comprising the vector according to para 12.
17. The polypeptide of any of paras 1 to 9, further comprising at least one, two, three, four or five, or all variant CDRs selected from the group consisting of CDRH1, CDRH2, CDRL1, CDRL2 or CDRL3, wherein at least one CDR has a variant amino acid in at least one solvent accessible and highly diverse amino acid position, wherein the variant amino acid is encoded by a nonrandom codon set, and wherein at least 70% of the amino acids encoded by the nonrandom set are target amino acids for that position in known antibody variable domains.
18. The polypeptide of para 17, wherein amino acids encoded by the nonrandom codon set comprise amino acids found at the corresponding position in at least 50% of known antibody variable domains.
19. The polypeptide of any of paras 1 to 9 and 17-18, wherein the variant CDRs are CDRH 1 or CDRH2 or both.
20. The polypeptide of para 19, wherein in the variant CDRH3, X₁ corresponds to amino acid 95 and n = 6, X₂ corresponds to amino acid 100a, X₃ corresponds to amino acid 100b, and X₆ corresponds to amino acid 102 in CDRH3 of antibody 4D5.
21. The polypeptide of para 20, wherein in CDRH3 , X₃ is alanine, X₄ is methionine, X₅ is aspartic acid and X₆ is tyrosine.
22. The polypeptide of para 20, wherein in CDRH3, X₂ is phenylalanine or leucine, X₃ and X₄ are missing, X₅ is aspartic acid and X₆ is tyrosine.
23. The polypeptide of para 19, wherein CDRH 1 comprises a variant amino acid at in at least one, two, three, four or all of amino acid positions 28, 30, 31, 32 and 33; and CDRH2 comprises a variant amino acid in at least one, two, three, four, five or all of amino acid positions 50, 52, 53, 54, 56 and 58.
24. The polypeptide according to para 19, wherein each variant amino acid position in CDRH 1 and CDRH2 has a variant amino acid wherein
   1) in CDRH1,
      a) the variant amino acid at position 28 is encoded by *AVT, WCC* or is threonine;
      b) the variant amino acid at position 30 is encoded by *RVM* or *AVT;*
      c) the variant amino acid at position 31 is encoded by *RVM, RVT or RRT;*
      d) the variant amino acid at position 32 is encoded by codon set *WMY;* and
      e) the variant amino acid at position 33 is encoded by codon set *KVK, RNT, DMT, KMT or KGG* or the combination of codon sets *KMT and KGG;* and
         wherein
   2) in CDRH2,
      a) the variant amino acid at position 50 is encoded by codon set *KDT or DBG* or the combination of codon sets *DGG and DHT;*
      b) the variant amino acid at position 52 is encoded by codon set *DHT or DMT;*
      c) the variant amino acid at position 53 is encoded by codon set *NMT or DMT;*
      d) the variant amino acid at position 54 is encoded by codon set *DMK, DMT or RRC;*
      e) the variant amino acid at position 56 is encoded by codon set *DMK or DMT;* and
      f) the variant amino acid at position 58 is encoded by codon set *DMT or DAC.*
25. The antibody variable domain according to para 24, wherein in the variant CDRH3, X₁ corresponds to amino acid position 95 and n = 6, X₂ corresponds to amino acid position 100a, X₃ corresponds to amino acid position 100b, and X₆ corresponds to amino acid position 102 in CDRH3 of antibody 4D5.
26. A polypeptide comprising at least two antibody variable domains comprising:
   a) a heavy chain antibody variable domain of any of paras 1 to 9, and 17-25; and
   b) a light chain antibody variable domain comprising a variant CDRL1, CDRL2, CDRL3 or mixtures thereof, wherein
      i) at least one variant CDRL1, CDRL2 or CDRL3 or mixtures thereof comprises a variant amino acid in at least one solvent accessible and highly diverse amino acid position, wherein the variant amino acid is encoded by a non-random codon set, and wherein at least 70% of the amino acids encoded by the nonrandom codon set are target amino acids for that position in known antibody variable domains.
27. The polypeptide of para 26, wherein
   CDRL1 comprises variant amino acids in one or more amino acid positions 28, 29, 30, 31 and 32;
   CDRL2 comprises a variant amino acid in one or more amino acid positions 50 and 53; and
   CDRL3 comprises a variant amino acid in one or more amino acid positions 91, 92, 93, 94 and 96.
28. The polypeptide of any of paras 26-27, wherein each variant amino acid position in each CDR has variant amino acid, wherein
   1) in CDRL1,
      a) the variant amino acid at position 28 is encoded by *RDT;*
      b) the variant amino acid at position 29 is encoded by *RKT* or RTT.
      c) the variant amino acid at position 30 is encoded by *RVW;*
      d) the variant amino acid at position 31 is encoded by codon set *RVW or ANW;* and
      e) the variant amino acid at position 32 is encoded by codon set *DHT or THT;*
   2) CDRL2,
      a) the variant amino acid at position 50 is encoded by codon set *KBG;* and
      b) the variant amino acid at position 53 is encoded by codon set *AVC;*
   3) in CDRL3,
      a) the variant amino acid at position 91 is encoded by codon set *KMT or TMT* or a combination of *TMT and SRT;*
      b) the variant amino acid at position 92 is encoded by codon set *DHT or DMC;*
      c) the variant amino acid at position 93 is encoded by codon *RVT or DHT;*
      d) the variant amino acid at position 94 is encoded by *NHT* or *WHT;* and
      e) the variant amino acid at position 96 is encoded by codon set *YHT, HWT, HTT, TDK* or combination of codon sets *YKG* and *TWT.*
29. A polypeptide according to para 28, wherein in the variant CDRH3 X₁ corresponds to amino acid position 95 and n = 6; X₂ corresponds to amino acid position 100a; X₃ corresponds to amino acid position 100b; and X₆ corresponds to amino acid position 102 in CDRH3 of antibody 4D5.
30. A polypeptide according to any of paras 1 to 9 and 17 to 29, further comprising a dimerization domain linked to C-terminal region of the heavy chain antibody variable domain.
31. A polypeptide according to para 30, wherein the dimerization domain comprises a leucine zipper domain or a sequence comprising at least one cysteine residue.
32. A polypeptide according to para 31, wherein the dimerization domain comprises a hinge region from an antibody and leucine zipper.
33. A polypeptide according to para 30, wherein the dimerization domain is a single cysteine.
34. A polypeptide comprising at least one, two, three, four, five or all of CDRs selected from the group consisting of CDRL1, CDRL2, CDRL3, CDRH1, CDRH2 and CDRH3, wherein
   (a) CDRL1 comprises a variant amino acid in at least one, two, three, four or all of amino acid positions 28, 29, 30, 31 and 32;
   (b) CDRL2 comprises a variant amino acid in at least one or both of amino acid positions 50 and 53;
   (c) CDRL3 comprises a variant amino acid in at least one, two, three, four or all of amino acid positions 91, 92, 93, 94 and 96;
   (d) CDRH1 comprises a variant amino acid in at least one, two, three, four or all of amino acid positions 28, 30, 31, 32 and 33;
   (e) CDRH2 comprises a variant amino acid in at least one, two, three, four, five or all of amino acid positions 50, 52, 53, 54, 56 and 58; and
   (f) CDRH3 comprises a variant amino acid in at least one, two, three, four, five, six or all of amino acid positions 95, 96, 97, 98, 99, 100 , 100a, 100b, 100c, 101 and 102;
      wherein the amino acid positions correspond to the Kabat numbering system; and wherein each variant amino acid of (a) to (e) is encoded by a non-random codon set, and wherein at least about 50% up to 90% or all of the amino acids encoded by the non-random codon set are target amino acids for that amino acid position in antibody variable domains; and wherein at least one variant amino acid of (f) is encoded by codon set *NNK, NNS, DVK, XYZ or NYT* or mixtures thereof.
35. The polypeptide of para 34, wherein the polypeptide comprises an antibody heavy chain variable domain or an antibody light chain variable domain or both.
36. A fusion polypeptide comprising:
   a polypeptide according to any of paras 1 to 9 and 17 to 35, wherein the heavy chain variable domain is fused to at least a portion of a viral coat protein.
37. The fusion polypeptide of para 36, wherein the viral coat protein is selected from the group consisting of protein pIII, major coat protein pVIII, Soc, Hoc, gpD, pv1 and variants thereof.
38. The fusion polypeptide of para 36 further comprising a dimerization domain between the heavy chain variable domain and the viral coat protein.
39. The fusion polypeptide of para 35 further comprising a light chain variable domain fused to a peptide tag.
40. The fusion polypeptide of para 39, wherein the peptide tag is selected from the group consisting of gD, c-myc, poly-his, fluorescence protein, and B-galactosidase.
41. A polypeptide of any of paras 1 to 9 and 17 to 40, further comprising FR1, FR2, FR3 or FR4 for each variable domain from a single antibody template.
42. The polypeptide of para 41, wherein each of the FR have the sequence of antibody 4D5 (SEQ ID NO: 1).
43. The polypeptide of para 42, wherein heavy chain FR residues at one, two, three or all amino acid positions 49, 93, 94 or 97 of the heavy chain and amino acid position 66 the light chain of antibody 4D5 have been altered.
44. The polypeptide of para 36, wherein heavy chain FR at position 49 is alanine or glycine, at position 93 is alanine or serine, position 94 is arginine or lysine or threonine, and position 71 is arginine, valine or alanine.
45. A nucleic acid comprising a polynucleotide molecule encoding a polypeptide of any of paras 17-44.
46. A vector that comprises the nucleic acid of para 45.
47. The vector according to para 46 that is a replicable expression vector.
48. The vector according to para 47, wherein the replicable expression vector is M13, fl, fd, Pf3 phage or a derivative thereof, or a lambdoid phage, such as lambda, 21, phi80, phi81, 82, 424, 434, etc., or a derivative thereof.
49. A virus comprising a polypeptide of any of paras 17-44 displayed on its surface.
50. A library compring a plurality of polypeptides of any of paras 17-44, and wherein the library has at least 1 X 10⁸ distinct antibody variable domain sequences.
51. A host cell comprising the vector of para 47.
52. A method of generating a composition comprising a plurality of polypeptides comprising:
   a) generating a plurality of polypeptides comprising at least one variant CDR of CDRH1 or CDRH2 or CDRH3 or mixtures thereof wherein
      i) a plurality of polypeptides comprising variant CDRH3 are formed by substituting one or more amino acid positions in an amino acid sequence of

         (X₁)ₙ - X₂ - X₃ - X₄ - X₅ ―X₆

         wherein X₁ is any naturally occurring amino acid encoded by a codon set of *NNK, NNS, DVK, NVT,* XYZ or is tryptophan or glycine or mixtures thereof, and n = 4 to 14;
         X₂ is any naturally occurring amino acid encoded by a codon set *of KSG, DVK, DSG, NVT, KSG, NNS, XYZ, NNK or* is tyrosine, phenylalanine, serine, alanine, glycine, leucine,or tryptophan;
         X₃ is missing when X₂ is phenylalanine or leucine, or if present, is alanine, valine, aspartic acid or glycine;
         X₄ is missing when X₂ is phenyalanine or leucine, or if present, is methionine;
         X₅ is aspartic acid , alanine, valine, or glycine;and
         X₆ is tyrosine or valine;
      ii) forming a plurality of variant CDRH 1 or CDRH2 or both comprising at least one variant amino acid in at least one solvent accessible position and highly diverse position, wherein the variant amino acid is encoded by a nonrandom codon set and wherein at least 70% of the amino acids encoded by the nonrandom condon set are target amino acids for that position in known antibody variable domains.
53. A method of para 52 further comprising:
   i) generating a plurality of polypeptides comprising a variant CDRL1, CDRL2 or CDRL3 or mixtures thereof, wherein the variant CDRs are formed with at least one variant amino acid in a solvent accessible and highly diverse position; wherein the variant amino acid is encoded by a nonrandom codon set and wherein at least 70% of the amino acids encoded by the nonrandom codon set are target amino acids for that position in known antibody variable domains.
54. A method of selecting for an antigen binding variable domain that binds to a target antigen comprising:
   a) generating a composition with a plurality of polypeptides of any of paras 1 to 9 and 17 to 44;
   b) selecting a polypeptide binder that binds to a target antigen from the composition;
   c) isolating the polypeptide binder from the nonbinders;
   e) identifying high affinity binders from the isolated polypeptide binders.
55. A method of selecting for an antigen binding variable domain that binds to a target antigen from a library of antibody variable domains comprising:
   a) contacting the library of antibody variable domains of any of para s 1 to 9 and 17 to 44 with a target antigen to form binders;
   b) separating the binders from the nonbinders, and eluting the binders from the target antigen and incubating the binders in a solution with decreasing amounts of the target antigen in a concentration from about 0.1 nM to 1000 nM;
   c) selecting the binders that can bind to the lowest concentration of the target antigen and that have an affinity of about 0.1 nM to 200 nM.
56. The method according to para 55, wherein the target antigen is VEGF, IGF-1, or Her-2.
57. The method according to para 55, wherein the concentration of target antigen is about 100 to 250 nM.
58. The method according to para 55, wherein the concentration of target antigen is about 25 to 100 nM.
59. A method of selecting for a polypeptide that binds to a target antigen from a library of polypeptides comprising:
   a) isolating polypeptide binders to a target antigen by contacting a library comprising a plurality of polypeptides of any of paras 1 to 9 and 17 to 44 with an immobilized target antigen under conditions suitable for binding;
   b) separating the polypeptide binders in the library from nonbinders and eluting the binders from the target antigen to obtain a subpopulation enriched for the binders.; and
   c) optionally, repeating steps a-b at least twice, each repetition using the subpopulation of binders obtained from the previous round of selection.
60. The method of selecting of para 59, further comprising:
   f) incubating the subpopulation of polypeptide binders with a concentration of labelled target antigen in the range of 0.1 nM to 1000 nM under conditions suitable for binding to form a mixture;
   g) contacting the mixture with an immobilized agent that binds to the label on the target antigen;
   h) detecting the polypeptide binders bound to labelled target antigens and eluting the polypeptide binders from the labelled target antigen;
   i) optionally, repeating steps f) to i) at least twice, each repetition using the subpopulation of binders obtained from the previous round of selection and using a lower concentration of labelled target antigen than the previous round.
61. The method according to para 60, further comprising adding an excess of unlabelled target antigen to the mixture and incubating for a period of time sufficient to elute low affinity binders from the labelled target antigen.
62. A method of isolating high affinity binders to a target antigen from a library of replicable expression vectors comprising:
   a) contacting a library comprising a plurality of polypeptides of any of para s 1-9 and 17-44 with a target antigen in a concentration of at least about 0.1 nM to 1000 nM to isolate polypeptide binders to the target antigen;
   b) separating the polypeptide binders from the target antigen to obtain a subpopulation enriched for the polypeptide binders;and
   c) optionally , repeating steps a-b at least twice, each repetition using the subpopulation of binders obtained from the previous round of selection and using a decreased concentration of target antigen than the previous round to isolate polypeptide binders that bind to lowest concentration of target antigen.
63. An assay for selecting polypeptide binders from a library comprising a plurality of polypeptides of any of paras 1 to 9 and 17 to 44 comprising:
   a) contacting the library with a concentration of labelled target antigen in a concentration range of 0.1 nM to 1000 nM, under conditions suitable for binding to form a complex of a polypeptide binder and the labelled target antigen;
   b) isolating the complexes and separating the polypeptide binders from the labelled target antigen to obtain a subpopulation enriched for the binders;
   c) optionally, repeating steps a-b at least twice, each time using the subpopulation of binders obtained from the previous round of selection and using a lower concentration of target antigen than the previous round.
64. The method of para 63, further comprising adding an excess of unlabelled target antigen to the complex of the polypeptide binder and target antigen.
65. The method of para 63, wherein the steps are repeated twice and the concentration of target in the first round of selection is about 100 nM to 250 nM, and in the second round of selection is about 25 nM to 100 nM, and in the third round of selection is about 0.1 nM to 25 nM.
66. A method of screening a library comprising a plurality of polypeptides of any of paras 1 to 9 and 17-44 comprising:
   a) incubating a first sample of the library with a concentration of a target antigen under conditions suitable for binding of the polypeptides to the target antigen;
   b) incubating a second sample of the library without a target antigen;
   c) contacting each of the first and second sample with immobilized target antigen under conditions suitable for binding of the polypeptide to the immobilized target antigen;
   d) detecting the polypeptide bound to immobilized target antigen for each sample;
   e) determining the affinity of the polypeptide for the target antigen by calculating the ratio of the amounts of bound polypeptide from the first sample over the amount of bound polypeptide from the second sample.
67. A method comprising:
   a) constructing an expression vector comprising a polynucleotide sequence which encodes a light chain variable domain, a heavy chain variable domain or both of a source antibody comprising at least one, two, three, four, five or all CDRs of the source antibody selected from the group consisting of CDR L1, L2, L3, H I, H2 and H3; and
   b) mutating at least one, two, three, four, five or all CDRs of the source antibody at at least one solvent accessible and highly diverse amino acid position using a non random codon set, wherein at least 50% of the amino acids encoded by the nonrandom codon set are target amino acids for that position in naturally occurring antibodies.
68. The method of para 67, wherein CDRH3 comprises an amino acid sequence:

   (X₁)n-X₂-X₃-X₄-D-X₅

   and is mutated at at least one amino acid position; wherein the mutations are selected from the group consisting of:
   X₁ is any naturally occurring amino acid encoded by a codon set of *NNK, NNS, DVK, NVT, XYZ* or is tryptophan or glycine or mixtures thereof, and n = 4 to 14;
   X₂ is any naturally occurring amino acid encoded by a codon set of *KSG, DVK, DSG, NVT, KSG, NNS, XYZ, NNK* or is tyrosine, phenylalanine, serine, alanine, glycine, leucine,or tryptophan;
   X₃ is missing when X₂ is phenylalanine or leucine, or if present, is alanine, valine, aspartic acid or glycine;
   X₄ is missing when X₂ is phenyalanine or leucine, or if present, is methionine; X₅ is aspartic acid , alanine, valine, or glycine;and
   X₆ is tyrosine or valine.
69. The method of para 68, wherein CDRH1 is mutated at amino acid positions corresponding to amino acid 28, 30, 31, 32 and 33 of antibody 4D5, and CDRH2 is mutated at amino acid positions corresponding to amino acid positions 50, 52, 53, 54, 56 and 58 of antibody 4D5.
70. A method according to para 69, wherein X₁ corresponds to amino acid position 95 in antibody 4D5 and n = 6; X₂ corresponds to amino acid position 100a in antibody 4D5, X₃ corresponds to amino acid position 100b in antibody 4D5, X₄ corresponds to amino acid position 100c in antibody 4D5, and X₅ corresponds to amino acid position 102 in antibody 4D5.
72. A polypeptide comprising a variant CDRH3 region that comprises an amino acid sequence:

   (X₁)ₙ-X₂-A-M-D-Y,

   wherein X₁ is any naturally occurring amino acid encoded by a codon set of *NNK, NNS, DVK, NVT, XYZ* or tryptophan or glycine or mixtures thereof, and n = 4 to 14;
   X₂ is any naturally occurring amino acid encoded by a codon set of *KSG, DVK, DSG, NVT, KSG, NNS, XYZ, NNK* or is tyrosine, glycine, phenylalanine, serine, alanine, leucine,or tryptophan;
   A is alanine; M is methionine; D is aspartic acid; and Y is tyrosine.
73. A polypeptide comprising a variant CDRH3 region that comprises an amino acid sequence:

   (X₁)ₙ - X₂ - X₃ - X₄ - X₅―X₆,

   wherein X₁ is any naturally occurring amino acid encoded by a codon set of *NNK, NNS, DVK, NVT, XYZ* or tryptophan or glycine or mixtures thereof, and n = 4 to 14;
   X₂ is phenylalanine or leucine;
   X₃ is missing;
   X₄ is missing;
   X₅ is aspartic acid, alanine, valine, or glycine;and
   X₆ is tyrosine or valine.
74. The polypeptide of para 73, wherein X₂ is phenylalanine, X₅ is aspartic acid and X₆ is tyrosine.
75. The polypeptide of para 35, wherein position 95 can have a variant amino acid encoded by codon set *DVK, NVT, NNS, XYZ, NNK* or is tryptophan; position 96 can be encoded by *DVK, , NVT, NNS, XYZ,* or is glycine or tryptophan; position 97 can be encoded *by DVK, NVT, NNS, XYZ,* or is tryptophan; position 98 can be encoded by *DVK, NVT, NNS, XYZ,* or is tryptophan; position 99 can be encoded *by DVK, NVT, NNS, XYZ;* position 100 can be encoded by *DVK, NVT, NNS, XYZ,* or is tryptophan; position 100a can be encoded by *DVK, DSG, KSG, NVT,NNS, XYZ,* or can be tyrosine, glycine, serine, alanine, tryptophan, aspartic acid, methionine, phenylalanine, leucine, valine; position 100b can be encoded by *DSG, KSG, XYZ* or is alanine, tyrosine, glycine, valine, aspartic acid, methionine, or phenylalanine; Position 100c can be encoded *by KSG, XYZ,* or is methionine, phenylalanine, leucine, alanine, glycine, valine, tyrosine, aspartic acid; position 101 can be encoded by *KSG, XYZ* or is aspartic acid, methionine, alanine, valine, glycine, tyrosine, phenylalanine, leucine; and position 102 can be encoded by *KSG or XYZ* or is tyrosine, aspartic acid, methionnine, alanine, valine, glycine.

## Claims

1. A library comprising a plurality of polypeptides each comprising a heavy chain antibody variable domain, wherein each heavy chain variable domain comprises a variant CDRH3 region that comprises an amino acid sequence:
(X₁)_{n -} X_{2 -} X_{3 -} X_{4 -} X₅ ―X₆
wherein X₁ is any naturally occurring amino acid encoded by a codon set of *NNK, NNS*, *DVK, NVT, XYZ* or is tryptophan or glycine or mixtures thereof, and n = 4 to 14;
X₂ is any naturally occurring amino acid encoded by a codon set of *KSG, DVK, DSG, NVT, KSG, NNS, XYZ, NNK* or is tyrosine, phenylalanine, serine, alanine, glycine, leucine,or tryptophan;
X₃ is missing when X₂ is phenylalanine or leucine, or if present, is alanine, valine, aspartic acid or glycine;
X₄ is missing when X₂ is phenyalanine or leucine, or if present, is methionine;
X₅ is aspartic acid , alanine, valine, or glycine;and
X₆ is tyrosine or valine; and
a variant CDRH1 and a variant CDRH2, wherein each of the variant CDRH1 and the variant CDRH2 has a variant amino acid at all solvent accessible and highly diverse amino acid positions, wherein each variant amino acid is encoded by a nonrandom codon set, and wherein at least 70% of the amino acids encoded by the nonrandom codon set are target amino acids for each of those positions in known antibody variable domains and 30% or less of the amino acids encoded by the nonrandom codon set are nontarget amino acids.

2. The library of claim 1, wherein:
(1) X₃ is alanine, X₄ is methionine, X₅ is aspartic acid and X₆ is tyrosine; or
(2) X₂ is tyrosine; or
(3) X₂ is leucine or phenylalanine, X₃ and X₄ are missing, X₅ is aspartic acid and X₆ is tyrosine.

3. The library of claim 1, wherein n is 6, 7, or 8.

4. The library of claim 1, wherein X₁ corresponds to amino acid position 95 and n = 6, X₂ corresponds to amino acid position 100a, and X₃ corresponds to amino acid position 100b in CDRH3 of antibody 4D5.

5. A library of any of claims 1 to 4 comprising a plurality of at least 1 x 10⁸ distinct polypeptide sequences.

6. The library of any one of claims 1 to 5, wherein amino acids encoded by the nonrandom codon set comprise amino acids found at the corresponding position in at least 50% of known antibody variable domains.

7. The library of any one of claims 1 to 6, wherein CDRH1 comprises a variant amino acid in all of amino acid positions 28, 30, 31, 32 and 33; and CDRH2 comprises a variant amino acid in all of amino acid positions 50, 52, 53, 54, 56 and 58.

8. The library according to claim 7, wherein
1) in CDRH1,
a) the variant amino acid at position 28 is encoded by *AVT, WCC* or is threonine;
b) the variant amino acid at position 30 is encoded by *RVM* or *AVT;*
c) the variant amino acid at position 31 is encoded by *RVM, RVT or RRT;*
d) the variant amino acid at position 32 is encoded by codon set *WMY;* and
e) the variant amino acid at position 33 is encoded by codon set *KVK, RNT, DMT, KMT or KGG* or the combination of codon sets *KMT and KGG;* and
wherein
2) in CDRH2,
a) the variant amino acid at position 50 is encoded by codon set *KDT or DBG* or the combination of codon sets *DGG and DHT;*
b) the variant amino acid at position 52 is encoded by codon set *DHT or DMT;*
c) the variant amino acid at position 53 is encoded by codon set *NMT or DMT;*
d) the variant amino acid at position 54 is encoded by codon set *DMK, DMT or RRC;*
e) the variant amino acid at position 56 is encoded by codon set *DMK or DMT;* and
f) the variant amino acid at position 58 is encoded by codon set *DMT or DAC.*

9. A library of any one of claims 1 to 8, wherein each polypeptide further comprises a light chain antibody variable domain comprising a variant CDRL2, CDRL2, CDRL3 or mixtures thereof, wherein
i) at least one variant CDRL1, CDRL2 or CDRL3 or mixtures thereof comprises a variant amino acid in at least one solvent accessible and highly diverse amino acid position, wherein the variant amino acid is encoded by a non-random codon set, and wherein at least 70% of the amino acids encoded by the nonrandom codon set are target amino acids for that position in known antibody variable domains.

10. The library of claim 9, wherein
CDRL1 comprises variant amino acids in one or more amino acid positions 28, 29, 30, 31 and 32;
CDRL2 comprises a variant amino acid in one or more amino acid positions 50 and 53; and
CDRL3 comprises a variant amino acid in one or more amino acid positions 91, 92, 93, 94 and 96.

11. The library of any of claims 9-10, wherein each variant amino acid position in each CDR has variant amino acid, wherein
1) in CDRL1,
a) the variant amino acid at position 28 is encoded by *RDT;*
b) the variant amino acid at position 29 is encoded by *RKT* or RTT.
c) the variant amino acid at position 30 is encoded by *RVW;*
d) the variant amino acid at position 31 is encoded by codon set *RVW or ANW;* and
e) the variant amino acid at position 32 is encoded by codon set *DHT or THT;*
2) CDRL2,
a) the variant amino acid at position 50 is encoded by codon set *KBG;* and
b) the variant amino acid at position 53 is encoded by codon set *AVC;*
3) in CDRL3,
a) the variant amino acid at position 91 is encoded by codon set *KMT or TMT* or a combination of *TMT and SRT;*
b) the variant amino acid at position 92 is encoded by codon set *DHT or DMC;*
c) the variant amino acid at position 93 is encoded by codon *RVT or DHT;*
d) the variant amino acid at position 94 is encoded by *NHT* or *WHT; and*
e) the variant amino acid at position 96 is encoded by codon set *YHT, HWT, HTT, TDK* or combination of codon sets *YKG* and *TWT.*

12. The library of claim 4 or any one of claims 5 to 11 as dependent therefrom, wherein position 95 can have a variant amino acid encoded by codon set *DVK, NVT, NNS, XYZ, NNK* or is tryptophan; position 96 can be encoded by *DVK, , NVT, NNS, XYZ,* or is glycine or tryptophan; position 97 can be encoded by *DVK, NVT, NNS, XYZ,* or is tryptophan; position 98 can be encoded by *DVK, NVT, NNS, XYZ,* or is tryptophan; position 99 can be encoded by *DVK, NVT, NNS, XYZ;* position 100 can be encoded by *DVK, NVT, NNS, XYZ,* or is tryptophan; position 100a can be encoded by *DVK, DSG, KSG, NVT, NNS, XYZ,* or can be tyrosine, glycine, serine, alanine, tryptophan, aspartic acid, methionine, phenylalanine, leucine, valine; position 100b can be encoded by *DSG, KSG, XYZ* or is alanine, tyrosine, glycine, valine, aspartic acid, methionine, or phenylalanine; Position 100c can be encoded by *KSG, XYZ,* or is methionine, phenylalanine, leucine, alanine, glycine, valine, tyrosine, aspartic acid; position 101 can be encoded by *KSG, XYZ* or is aspartic acid, methionine, alanine, valine, glycine, tyrosine, phenylalanine, leucine; and position 102 can be encoded by *KSG or XYZ* or is tyrosine, aspartic acid, methionnine, alanine, valine, glycine.

13. A library according to any of claims 1 to 12, further comprising a dimerization domain linked to C-terminal region of the heavy chain antibody variable domain.

14. A library according to claim 13, wherein the dimerization domain:
(1) comprises a leucine zipper domain or a sequence comprising at least one cysteine residue; or
(2) comprises a hinge region from an antibody and leucine zipper; or
(3) is a single cysteine.

15. A library according to any preceding claim, wherein the heavy chain variable domain is fused to at least a portion of a viral coat protein.

16. The library of claim 15, wherein the viral coat protein is selected from the group consisting of protein pIII, major coat protein pVIII, Soc, Hoc, gpD, pv1 and variants thereof.

17. The library of claim 15 further comprising a dimerization domain between the heavy chain variable domain and the viral coat protein.

18. The library of claim 9 or any one of claims 10 to 17 as dependent therefrom, wherein the light chain variable domain is fused to a peptide tag.

19. The library of claim 18, wherein the peptide tag is selected from the group consisting of gD, c-myc, poly-his, fluorescence protein, and B-galactosidase.

20. A library of any preceding claim, further comprising FR1, FR2, FR3 or FR4 for each variable domain from a single antibody template.

21. The library of claim 20, wherein each of the FR have the sequence of antibody 4D5 (SEQ ID NO: 1).

22. The library of claim 21, wherein heavy chain FR residues at one, two, three or all amino acid positions 49, 93, 94 or 97 of the heavy chain and amino acid position 66 the light chain of antibody 4D5 have been altered.

23. The polypeptide of claim 22, wherein heavy chain FR at position 49 is alanine or glycine, at position 93 is alanine or serine, position 94 is arginine or lysine or threonine, and position 71 is arginine, valine or alanine.

24. A library of nucleic acids comprising a plurality of polynucleotides, encoding different polypeptides in the library of any preceding claim.

25. A cell culture comprising the library of claim 24.

26. A method of generating a composition comprising a plurality of polypeptides comprising:
a) generating a library of any one of claims 1 to 23.

27. A method of claim 26 further comprising:
i) generating a plurality of polypeptides comprising a variant CDRL1, CDRL2 or CDRL3 or mixtures thereof, wherein the variant CDRs are formed with at least one variant amino acid in a solvent accessible and highly diverse position; wherein the variant amino acid is encoded by a nonrandom codon set and wherein at least 70% of the amino acids encoded by the nonrandom codon set are target amino acids for that position in known antibody variable domains.

28. A method of selecting:
(1) for an antigen binding variable domain that binds to a target antigen, the method comprising:
a) generating a composition with a library of any of claims 1 to 23;
b) selecting a polypeptide binder that binds to a target antigen from the composition;
c) isolating the polypeptide binder from the nonbinders;
e) identifying high affinity binders from the isolated polypeptide binders; or
(2) for an antigen binding variable domain that binds to a target antigen from a library of antibody variable domains, the method comprising:
a) contacting the library of antibody variable domains of any of claims 1 to 23 with a target antigen to form binders;
b) separating the binders from the nonbinders, and eluting thebinders from the target antigen and incubating the binders in a solution with decreasing amounts of the target antigen in a concentration from about 0.1 nM to 1000 nM;
c) selecting the binders that can bind to the lowest concentration of the target antigen and that have an affinity of about 0.1 nM to 200 nM; or
(3) from a library of polypeptides, a polypeptide that binds to a target antigen, the method comprising:
a) isolating polypeptide binders to a target antigen by contacting a library of any of claims 1 to 23 with an immobilized target antigen under conditions suitable for binding;
b) separating the polypeptide binders in the library from nonbinders and eluting the binders from the target antigen to obtain a subpopulation enriched for the binders.; and
c) optionally, repeating steps a-b at least twice, each repetition using the subpopulation of binders obtained from the previous round of selection.

29. The method according to claim 28 part (2), wherein the target antigen is VEGF, IGF-1, or Her-2.

30. The method according to claim 28 part (2), wherein the concentration of target antigen is either about 100 to 250 nM or about 25 to 100 nM.

31. The method of selecting of claim 28 part (3), further comprising:
f) incubating the subpopulation of polypeptide binders with a concentration of labelled target antigen in the range of 0.1 nM to 1000 nM under conditions suitable for binding to form a mixture;
g) contacting the mixture with an immobilized agent that binds to the label on the target antigen;
h) detecting the polypeptide binders bound to labelled target antigens and eluting the polypeptide binders from the labelled target antigen;
i) optionally, repeating steps f) to i) at least twice, each repetition using the subpopulation of binders obtained from the previous round of selection and using a lower concentration of labelled target antigen than the previous round.

32. The method according to claim 31, further comprising adding an excess of unlabelled target antigen to the mixture and incubating for a period of time sufficient to elute low affinity binders from the labelled target antigen.

33. A method comprising:
a) constructing an expression vector comprising a polynucleotide sequence which encodes a light chain variable domain, a heavy chain variable domain or both of a source antibody comprising at least one, two, three, four, five or all CDRs of the source antibody selected from the group consisting of CDR L1, L2, L3, H1, H2 and H3; and
b) mutating at all heavy chain CDRs of the source antibody at all solvent accessible and highly diverse amino acid positions using a non random codon set, wherein at least 50% of the amino acids encoded by the nonrandom codon set are target amino acids for that position in naturally occurring antibodies.

34. The method of claim 33, wherein CDRH3 comprises an amino acid sequence:
(X₁)ₙ-X₂-X₃-X₄-D-X₅
and is mutated at at least one amino acid position; wherein the mutations are selected from the group consisting of:
X₁ is any naturally occurring amino acid encoded by a codon set of *NNK, NNS, DVK, NVT, XYZ* or is tryptophan or glycine or mixtures thereof, and n = 4 to 14;
X₂ is any naturally occurring amino acid encoded by a codon set *of KSG, DVK, DSG, NVT, KSG, NNS, XYZ, NNK* or is tyrosine, phenylalanine, serine, alanine, glycine, leucine,or tryptophan;
X₃ is missing when X₂ is phenylalanine or leucine, or if present, is alanine, valine, aspartic acid or glycine;
X₄ is missing when X₂ is phenyalanine or leucine, or if present, is methionine;
X₅ is aspartic acid , alanine, valine, or glycine;and
X₆ is tyrosine or valine.

35. The method of claim 34, wherein CDRH1 is mutated at amino acid positions corresponding to amino acid 28, 30, 31, 32 and 33 of antibody 4D5, and CDRH2 is mutated at amino acid positions corresponding to amino acid positions 50, 52, 53, 54, 56 and 58 of antibody 4D5.

36. A method according to claim 35, wherein X₁ corresponds to amino acid position 95 in antibody 4D5 and n = 6; X₂ corresponds to amino acid position 100a in antibody 4D5, X₃ corresponds to amino acid position 100b in antibody 4D5, X₄ corresponds to amino acid position 100c in antibody 4D5, and X₅ corresponds to amino acid position 102 in antibody 4D5.

37. A method for finding original binders from a random library or finding improved binders from a library that was designated to improve affinity of a particular binding clone or group of clones, the method comprising:
contacting a plurality of polypeptides displayed on phage or phagemid particles with a target antigen labelled or fused with a tag molecule; and
separating the phage or phagemid particles that are bound to labelled targets from phage or phagemid particles that do not bind by (1) allowing binding to a molecule that binds the labelled target antigen for a period of 2-5 minutes and (2) eluting the bound particles.


SEQUENCE LISTING

<110>  Genentech, Inc.

<120>  SYNTHETIC ANTIBODY PHAGE LIBRARIES

<130>  11669.140WOU1

<140>  PCT/US03/17545
<141>  2003-06-03

<150>  US 60/385,338
<151>  2002-06-03

<150>  US 60/463,646
<151>  2003-04-16

<160>  174

<170>  PatentIn version 3.3

<210>  1
<211>  109
<212>  PRT
<213>  Artificial Sequence

<220>
<223>  4D5 Light chain variable domain

<400>  1

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Glu Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys Arg Thr
            100                 105

<210>  2
<211>  120
<212>  PRT
<213>  Artificial Sequence

<220>
<223>  4D5 Heavy chain variable domain

<400>  2

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Val Trp Gly Gln
            100                 105                 110

Gly Thr Leu Val Thr Val Ser Ser
        115                 120

<210>  3
<211>  35
<212>  PRT
<213>  Artificial Sequence

<220>
<223>  GNC4 Leucine zipper

<400>  3

Gly Arg Met Lys Gln Leu Glu Asp Lys Val Glu Glu Leu Leu Ser Lys
1               5                   10                  15

Asn Tyr His Leu Glu Asn Glu Val Ala Arg Leu Lys Lys Leu Val Gly
            20                  25                  30

Glu Arg Gly
        35

<210>  4
<211>  5
<212>  PRT
<213>  Artificial Sequence

<220>
<223>  C-terminal of CDRH3

<400>  4

Tyr Ala Met Asp Tyr
1               5

<210>  5
<211>  7
<212>  PRT
<213>  Artificial Sequence

<220>
<223>  Heavy chain CDR3

<400>  5

Ser Arg Asn Ala Trp Ala Phe
1               5


<210>  6
<211>  11
<212>  PRT
<213>  Artificial Sequence

<220>
<223>  Heavy chain CDR3

<400>  6

Ser Arg Asn Leu Ser Glu Asn Ser Tyr Ala Met
1               5                   10


<210>  7
<211>  11
<212>  PRT
<213>  Artificial Sequence

<220>
<223>  Heavy chain CDR3

<400>  7

Ser Arg Ala Gly Trp Ala Gly Trp Tyr Ala Met
1               5                   10


<210>  8
<211>  11
<212>  PRT
<213>  Artificial Sequence

<220>
<223>  Heavy chain CDR3

<400>  8

Ser Arg Ala Ala Lys Ala Gly Trp Tyr Ala Met
1               5                   10


<210>  9
<211>  12
<212>  PRT
<213>  Artificial Sequence

<220>
<223>  Heavy chain CDR3

<400>  9

Ser Arg Ser Asp Gly Arg Asp Ser Ala Tyr Ala Met

1               5                   10


<210>  10
<211>  13
<212>  PRT
<213>  Artificial Sequence

<220>
<223>  Randomized CDRH3


<220>
<221>  MISC_FEATURE
<222>  (3)..(9)
<223>  Xaa is Ala, Cys, Asp, Glu, Gly, Lys, Asn, Arg, Ser, Tyr, Trp or

       Thr

<400>  10

Ser Arg Xaa Xaa Xaa Xaa Xaa Xaa Xaa Ala Met Asp Tyr
1               5                   10


<210>  11
<211>  14
<212>  PRT
<213>  Artificial Sequence

<220>
<223>  Randomized CDRH3


<220>
<221>  MISC_FEATURE
<222>  (3)..(8)
<223>  Xaa is Ala, Cys, Asp, Glu, Gly, Lys, Asn, Arg, Ser, Tyr, Trp or

       Thr

<220>
<221>  MISC_FEATURE
<222>  (9)..(9)
<223>  Xaa is any naturally occurring amino acid

<400>  11

Ser Arg Xaa Xaa Xaa Xaa Xaa Xaa Xaa Tyr Ala Met Asp Tyr
1               5                   10


<210>  12
<211>  13
<212>  PRT
<213>  Artificial Sequence

<220>
<223>  Randomized CDRH3


<220>
<221>  MISC_FEATURE
<222>  (3)..(7)
<223>  Xaa is Ala, Cys, Asp, Glu, Gly, Lys, Asn, Arg, Ser, Tyr, Trp or

       Thr

<220>
<221>  MISC_FEATURE
<222>  (8)..(8)
<223>  Xaa is any naturally occurring amino acid

<400>  12

Ser Arg Xaa Xaa Xaa Xaa Xaa Xaa Tyr Ala Met Asp Tyr
1               5                   10


<210>  13
<211>  13
<212>  PRT
<213>  Artificial Sequence

<220>
<223>  Randomized CDRH3


<220>
<221>  MISC_FEATURE
<222>  (3)..(8)
<223>  Xaa is any naturally occurring amino acid

<400>  13

Ser Arg Xaa Xaa Xaa Xaa Xaa Xaa Tyr Ala Met Asp Tyr
1               5                   10


<210>  14
<211>  51
<212>  DNA
<213>  Artificial Sequence

<220>
<223>  Oligonucleotide encoding randomized CDRH3


<220>
<221>  misc_feature
<222>  (23)..(23)
<223>  n=a, c or g

<220>
<221>  misc_feature
<222>  (25)..(26)
<223>  n=a or g

<220>
<221>  misc_feature
<222>  (28)..(28)
<223>  n=a or t

<220>
<221>  misc_feature
<222>  (29)..(29)
<223>  n=a or c

<220>
<221>  misc_feature
<222>  (30)..(30)
<223>  n=c or t

<220>
<221>  misc_feature
<222>  (31)..(31)

<223>  n=g or t

<220>
<221>  misc_feature
<222>  (32)..(32)
<223>  n=a or c

<400>  14
gcagcttctg gcttcaccat tantnntnnn nntatacact gggtgcgtca g          51


<210>  15
<211>  51
<212>  DNA
<213>  Artificial Sequence

<220>
<223>  Oligonucleotide encoding randomized CDRH3


<220>
<221>  misc_feature
<222>  (23)..(23)
<223>  n=a, c or g

<220>
<221>  misc_feature
<222>  (25)..(26)
<223>  n=a or g

<220>
<221>  misc_feature
<222>  (28)..(28)
<223>  n=a or t

<220>
<221>  misc_feature
<222>  (29)..(29)
<223>  n=a or c

<220>
<221>  misc_feature
<222>  (30)..(30)
<223>  n=c or t

<220>
<221>  misc_feature
<222>  (31)..(31)
<223>  n=g or t

<400>  15
gcagcttctg gcttcaccat tantnntnnn nggatacact gggtgcgtca g          51


<210>  16
<211>  51
<212>  DNA
<213>  Artificial Sequence

<220>
<223>  Oligonucleotide encoding randomized CDRH3


<220>
<221>  misc_feature
<222>  (23)..(23)
<223>  n=a, c or g

<220>
<221>  misc_feature
<222>  (25)..(25)
<223>  n=a or g

<220>
<221>  misc_feature
<222>  (26)..(26)
<223>  n=a, c or g

<220>
<221>  misc_feature
<222>  (27)..(27)
<223>  n=a or c

<220>
<221>  misc_feature
<222>  (28)..(28)
<223>  n=a or t

<220>
<221>  misc_feature
<222>  (29)..(29)
<223>  n=a or c

<220>
<221>  misc_feature
<222>  (30)..(30)
<223>  n=c or t

<220>
<221>  misc_feature
<222>  (31)..(31)
<223>  n=g or t

<220>
<221>  misc_feature
<222>  (32)..(32)
<223>  n=a or c

<400>  16
gcagcttctg gcttcaccat tantnnnnnn nntatacact gggtgcgtca g          51


<210>  17
<211>  51
<212>  DNA
<213>  Artificial Sequence

<220>
<223>  Oligonucleotide encoding randomized CDRH3


<220>
<221>  misc_feature
<222>  (23)..(23)
<223>  n=a, c or g

<220>
<221>  misc_feature
<222>  (25)..(25)
<223>  n=a or g

<220>
<221>  misc_feature
<222>  (26)..(26)
<223>  n=a, c or g

<220>
<221>  misc_feature
<222>  (27)..(27)
<223>  n=a or c

<220>
<221>  misc_feature
<222>  (28)..(28)
<223>  n=a or t

<220>
<221>  misc_feature
<222>  (29)..(29)
<223>  n=a or c

<220>
<221>  misc_feature
<222>  (30)..(30)
<223>  n=c or t

<220>
<221>  misc_feature
<222>  (31)..(31)
<223>  n=g or t

<400>  17
gcagcttctg gcttcaccat tantnnnnnn nggatacact gggtgcgtca g                51


<210>  18
<211>  72
<212>  DNA
<213>  Artificial Sequence

<220>
<223>  Oligonucleotide encoding randomized CDRH3


<220>
<221>  misc_feature
<222>  (20)..(20)
<223>  n=c or g

<220>
<221>  misc_feature
<222>  (22)..(22)
<223>  n=a, g or t

<220>
<221>  misc_feature
<222>  (28)..(28)
<223>  n=a or t

<220>
<221>  misc_feature
<222>  (29)..(29)
<223>  n=a or c

<220>
<221>  misc_feature
<222>  (34)..(34)
<223>  n=a, g or t

<220>
<221>  misc_feature
<222>  (35)..(35)
<223>  n=a or c

<220>
<221>  misc_feature
<222>  (37)..(38)
<223>  n=a or g

<220>
<221>  misc_feature
<222>  (43)..(43)
<223>  n=a, g or t

<220>
<221>  misc_feature
<222>  (44)..(44)
<223>  n=a or c

<220>
<221>  misc_feature
<222>  (49)..(49)
<223>  n=a, g or t

<400>  18
aagggcctgg aatgggttgn tnggattnnt cctnntnncg gcnntactna ctatgccgat     60

agcgtcaagg gc                                                         72


<210>  19
<211>  72
<212>  DNA
<213>  Artificial Sequence

<220>
<223>  Oligonucleotide encoding randomized CDRH3


<220>
<221>  misc_feature
<222>  (20)..(20)
<223>  n=c or g

<220>
<221>  misc_feature
<222>  (22)..(22)
<223>  n=a, g or t

<220>
<221>  misc_feature
<222>  (23)..(23)
<223>  n=a, c or t

<220>
<221>  misc_feature
<222>  (28)..(28)
<223>  n=a or t

<220>
<221>  misc_feature
<222>  (29)..(29)
<223>  n=a or c

<220>
<221>  misc_feature
<222>  (34)..(34)
<223>  n=a, g or t

<220>
<221>  misc_feature
<222>  (35)..(35)

<223>  n=a or c

<220>
<221>  misc_feature
<222>  (37)..(38)
<223>  n=a or g

<220>
<221>  misc_feature
<222>  (43)..(43)
<223>  n=a, g or t

<220>
<221>  misc_feature
<222>  (44)..(44)
<223>  n=a or c

<220>
<221>  misc_feature
<222>  (49)..(49)
<223>  n=a, g or t

<400>  19
aagggcctgg aatgggttgn tnntattnnt cctnntnncg gcnntactna ctatgccgat     60

agcgtcaagg gc                                                         72


<210>  20
<211>  72
<212>  DNA
<213>  Artificial Sequence

<220>
<223>  Oligonucleotide encoding randomized CDRH3


<220>
<221>  misc_feature
<222>  (20)..(20)
<223>  n=c or g

<220>
<221>  misc_feature
<222>  (22)..(22)
<223>  n=a, g or t

<220>
<221>  misc_feature
<222>  (28)..(28)
<223>  n=a, g or t

<220>
<221>  misc_feature
<222>  (29)..(29)
<223>  n=a or c

<220>
<221>  misc_feature
<222>  (34)..(34)
<223>  n=a, c, g or t

<220>
<221>  misc_feature
<222>  (35)..(35)
<223>  n=a or c

<220>

<221>  misc_feature
<222>  (37)..(38)
<223>  n=a or g

<220>
<221>  misc_feature
<222>  (43)..(43)
<223>  n=a, g or t

<220>
<221>  misc_feature
<222>  (44)..(44)
<223>  n=a or c

<220>
<221>  misc_feature
<222>  (49)..(49)
<223>  n=a, g or t

<400>  20
agggccctgg aatgggttgn tnggattnnt cctnntnncg gcnntactna ctatgccgat     60

agcgtcaagg gc                                                         72


<210>  21
<211>  72
<212>  DNA
<213>  Artificial Sequence

<220>
<223>  Oligonucleotide encoding randomized CDRH3


<220>
<221>  misc_feature
<222>  (20)..(20)
<223>  n=c or g

<220>
<221>  misc_feature
<222>  (22)..(22)
<223>  n=a, g or t

<220>
<221>  misc_feature
<222>  (23)..(23)
<223>  n=a, c or t

<220>
<221>  misc_feature
<222>  (28)..(28)
<223>  n=a, g or t

<220>
<221>  misc_feature
<222>  (29)..(29)
<223>  n=a or c

<220>
<221>  misc_feature
<222>  (34)..(34)
<223>  n=a, c, g or t

<220>
<221>  misc_feature
<222>  (35)..(35)
<223>  n=a or c

<220>
<221>  misc_feature
<222>  (37)..(38)
<223>  n=a or g

<220>
<221>  misc_feature
<222>  (43)..(43)
<223>  n=a, g or t

<220>
<221>  misc_feature
<222>  (44)..(44)
<223>  n=a or c

<220>
<221>  misc_feature
<222>  (49)..(49)
<223>  n=a, g or t

<400>  21
aagggcctgg aatgggttgn tnntattnnt cctnntnncg gcnntactna ctatgccgat      60

agcgtcaagg gc                                                          72


<210>  22
<211>  33
<212>  DNA
<213>  Artificial Sequence

<220>
<223>  Oligonucleotide encoding randomized CDRH3


<220>
<221>  misc_feature
<222>  (3)..(3)
<223>  n=a or g

<400>  22
cgnttcacta taagccgtga cacatccaaa aac                                   33


<210>  23
<211>  1441
<212>  DNA
<213>  Artificial Sequence

<220>
<223>  Single chain Fv

<400>  23
gaaatgagct gttgacaatt aatcatcggc tcgtataatg tgtggaattg tgagcggata      60

acaatttcac acaggaaaca gccagtccgt ttaggtgttt tcacgagcac ttcaccaaca     120

aggaccatag attatgaaaa taaaaacagg tgcacgcatc ctcgcattat ccgcattaac     180

gacgatgatg ttttccgcct cggcttatgc atccgatatc cagatgaccc agtccccgag     240

ctccctgtcc gcctctgtgg gcgatagggt caccatcacc tgccgtgcca gtcaggatgt     300

gaatactgct gtagcctggt atcaacagaa accaggaaaa gctccgaagc ttctgattta     360

ctcggcatcc ttcctctact ctggagtccc ttctcgcttc tctggtagcc gttccgggac     420

ggatttcact ctgaccatca gcagtctgca gccggaagac ttcgcaactt attactgtca 480

gcaacattat actactcctc ccacgttcgg acagggtacc aaggtggaga tcaaatcgga 540

tatgccgatg gctgatccga accgtttccg cggtaagaac ctggtttttc attctgagat 600

ctccgaggtt cagctggtgg agtctggcgg tggcctggtg cagccagggg gctcactccg 660

tttgtcctgt gcagcttctg gcttcaacat taaagacacc tatatacact gggtgcgtca 720

ggccccgggt aagggcctgg aatgggttgc aaggatttat cctacgaatg gttatactag 780

atatgccgat agcgtcaagg gccgtttcac tataagcgca gacacatcca aaaacacagc 840

ctacctacaa atgaacagct taagagctga ggacactgcc gtctattatt gtagccgctg 900

gggaggggac ggcttctatg ctatggacta ctggggtcaa ggaacactag tcaccgtctc 960

cagcagtggc ggtggctctg gttccggtga ttttgattat gaaaagatgg caaacgctaa 1020

taagggggct atgaccgaaa atgccgatga aaacgcgcta cagtctgacg ctaaaggcaa 1080

acttgattct gtcgctactg attacggtgc tgctatcgat ggtttcattg gtgacgtttc 1140

cggccttgct aatggtaatg gtgctactgg tgattttgct ggctctaatt cccaaatggc 1200

tcaagtcggt gacggtgata attcaccttt aatgaataat ttccgtcaat atttaccttc 1260

cctccctcaa tcggttgaat gtcgcccttt tgtctttagc gctggtaaac catatgaatt 1320

ttctattgat tgtgacaaaa taaacttatt ccgtggtgtc tttgcgtttc ttttatatgt 1380

tgccaccttt atgtatgtat tttctacgtt tgctaacata ctgcgtaata aggagtctta 1440

a 1441

<210> 24
<211> 1588
<212> DNA
<213> Artificial Sequence

<220>
<223> Single chain Fv with zipper domain

<400> 24
gaaatgagct gttgacaatt aatcatcggc tcgtataatg tgtggaattg tgagcggata 60

acaatttcac acaggaaaca gccagtccgt ttaggtgttt tcacgagcac ttcaccaaca 120

aggaccatag attatgaaaa taaaaacagg tgcacgcatc ctcgcattat ccgcattaac 180

gacgatgatg ttttccgcct cggcttatgc atccgatatc cagatgaccc agtccccgag 240

ctccctgtcc gcctctgtgg gcgatagggt caccatcacc tgccgtgcca gtcaggatgt 300

gaatactgct gtagcctggt atcaacagaa accaggaaaa gctccgaagc ttctgattta 360

ctcggcatcc ttcctctact ctggagtccc ttctcgcttc tctggtagcc gttccgggac 420

ggatttcact ctgaccatca gcagtctgca gccggaagac ttcgcaactt attactgtca 480

gcaacattat actactcctc ccacgttcgg acagggtacc aaggtggaga tcaaatcgga 540

tatgccgatg gctgatccga accgtttccg cggtaagaac ctggtttttc attctgagat 600

ctccgaggtt cagctggtgg agtctggcgg tggcctggtg cagccagggg gctcactccg 660

tttgtcctgt gcagcttctg gcttcaacat taaagacacc tatatacact gggtgcgtca 720

ggccccgggt aagggcctgg aatgggttgc aaggatttat cctacgaatg gttatactag 780

atatgccgat agcgtcaagg gccgtttcac tataagcgca gacacatcca aaaacacagc 840

ctacctacaa atgaacagct taagagctga ggacactgcc gtctattatt gtagccgctg 900

gggaggggac ggcttctatg ctatggacta ctggggtcaa ggaacactag tcaccgtctc 960

cagcacatgc ccgccgtgcc cagcaccaga actgctgggc ggccgcatga aacagctaga 1020

ggacaaggtc gaagagctac tctccaagaa ctaccaccta gagaatgaag tggcaagact 1080

caaaaaactt gtcggggagc gcggaaagct tagtggcggt ggctctggtt ccggtgattt 1140

tgattatgaa aagatggcaa acgctaataa gggggctatg accgaaaatg ccgatgaaaa 1200

cgcgctacag tctgacgcta aaggcaaact tgattctgtc gctactgatt acggtgctgc 1260

tatcgatggt ttcattggtg acgtttccgg ccttgctaat ggtaatggtg ctactggtga 1320

ttttgctggc tctaattccc aaatggctca agtcggtgac ggtgataatt cacctttaat 1380

gaataatttc cgtcaatatt taccttccct ccctcaatcg gttgaatgtc gcccttttgt 1440

ctttagcgct ggtaaaccat atgaattttc tattgattgt gacaaaataa acttattccg 1500

tggtgtcttt gcgtttcttt tatatgttgc cacctttatg tatgtatttt ctacgtttgc 1560

taacatactg cgtaataagg agtcttaa 1588

<210> 25
<211> 2239
<212> DNA
<213> Artificial Sequence

<220>
<223> Fab fragment

<400> 25
gaaatgagct gttgacaatt aatcatcggc tcgtataatg tgtggaattg tgagcggata 60

acaatttcac acaggaaaca gccagtccgt ttaggtgttt tcacgagcac ttcaccaaca 120

aggaccatag attatgaaaa taaaaacagg tgcacgcatc ctcgcattat ccgcattaac 180

gacgatgatg ttttccgcct cggcttatgc atccgatatc cagatgaccc agtccccgag 240

ctccctgtcc gcctctgtgg gcgatagggt caccatcacc tgccgtgcca gtcaggatgt 300

gaatactgct gtagcctggt atcaacagaa accaggaaaa gctccgaagc ttctgattta 360

ctcggcatcc ttcctctact ctggagtccc ttctcgcttc tctggtagcc gttccgggac 420

ggatttcact ctgaccatca gcagtctgca gccggaagac ttcgcaactt attactgtca 480

gcaacattat actactcctc ccacgttcgg acagggtacc aaggtggaga tcaaacgaac 540

tgtggctgca ccatctgtct tcatcttccc gccatctgat gagcagttga aatctggaac 600

tgcctctgtt gtgtgcctgc tgaataactt ctatcccaga gaggccaaag tacagtggaa 660

ggtggataac gccctccaat cgggtaactc ccaggagagt gtcacagagc aggacagcaa 720

ggacagcacc tacagcctca gcagcaccct gacgctgagc aaagcagact acgagaaaca 780

caaagtctac gcctgcgaag tcacccatca gggcctgagc tcgcccgtca caaagagctt 840

caacagggga gagtgtggtg ccagctccgg tatggctgat ccgaaccgtt tccgcggtaa 900

ggacctggca taactcgagg ctgatcctct acgccggacg catcgtggcc ctagtacgca 960

agttcacgta aaaagggtaa ctagaggttg aggtgatttt atgaaaaaga atatcgcatt 1020

tcttcttgca tctatgttcg ttttttctat tgctacaaac gcgtacgctg agatctccga 1080

ggttcagctg gtggagtctg gcggtggcct ggtgcagcca gggggctcac tccgtttgtc 1140

ctgtgcagct tctggcttca acattaaaga cacctatata cactgggtgc gtcaggcccc 1200

gggtaagggc ctggaatggg ttgcaaggat ttatcctacg aatggttata ctagatatgc 1260

cgatagcgtc aagggccgtt tcactataag cgcagacaca tccaaaaaca cagcctacct 1320

acaaatgaac agcttaagag ctgaggacac tgccgtctat tattgtagcc gctggggagg 1380

ggacggcttc tatgctatgg actactgggg tcaaggaacc ctggtcaccg tctcctcggc 1440

ctccaccaag ggcccatcgg tcttccccct ggcaccctcc tccaagagca cctctggggg 1500

cacagcggcc ctgggctgcc tggtcaagga ctacttcccc gaaccggtga cggtgtcgtg 1560

gaactcaggc gccctgacca gcggcgtgca caccttcccg gctgtcctac agtcctcagg 1620

actctactcc ctcagcagcg tggtgaccgt gccctccagc agcttgggca cccagaccta 1680

catctgcaac gtgaatcaca agcccagcaa caccaaggtc gacaagaaag ttgagcccaa 1740

atcttgtgac aaaactcacc tcagtggcgg tggctctggt tccggtgatt ttgattatga 1800

aaagatggca aacgctaata agggggctat gaccgaaaat gccgatgaaa acgcgctaca 1860

gtctgacgct aaaggcaaac ttgattctgt cgctactgat tacggtgctg ctatcgatgg 1920

tttcattggt gacgtttccg gccttgctaa tggtaatggt gctactggtg attttgctgg 1980

ctctaattcc caaatggctc aagtcggtga cggtgataat tcacctttaa tgaataattt 2040

ccgtcaatat ttaccttccc tccctcaatc ggttgaatgt cgcccttttg tctttagcgc 2100

tggtaaacca tatgaatttt ctattgattg tgacaaaata aacttattcc gtggtgtctt 2160

tgcgtttctt ttatatgttg ccacctttat gtatgtattt tctacgtttg ctaacatact 2220

gcgtaataag gagtcttaa 2239

<210> 26
<211> 2383
<212> DNA
<213> Artificial Sequence

<220>
<223> Fab fragment with zipper domain

<400> 26
gaaatgagct gttgacaatt aatcatcggc tcgtataatg tgtggaattg tgagcggata 60

acaatttcac acaggaaaca gccagtccgt ttaggtgttt tcacgagcac ttcaccaaca 120

aggaccatag attatgaaaa taaaaacagg tgcacgcatc ctcgcattat ccgcattaac 180

gacgatgatg ttttccgcct cggcttatgc atccgatatc cagatgaccc agtccccgag 240

ctccctgtcc gcctctgtgg gcgatagggt caccatcacc tgccgtgcca gtcaggatgt 300
gaatactgct gtagcctggt atcaacagaa accaggaaaa gctccgaagc ttctgattta 360
ctcggcatcc ttcctctact ctggagtccc ttctcgcttc tctggtagcc gttccgggac 420
ggatttcact ctgaccatca gcagtctgca gccggaagac ttcgcaactt attactgtca 480
gcaacattat actactcctc ccacgttcgg acagggtacc aaggtggaga tcaaacgaac 540
tgtggctgca ccatctgtct tcatcttccc gccatctgat gagcagttga aatctggaac 600
tgcctctgtt gtgtgcctgc tgaataactt ctatcccaga gaggccaaag tacagtggaa 660
ggtggataac gccctccaat cgggtaactc ccaggagagt gtcacagagc aggacagcaa 720
ggacagcacc tacagcctca gcagcaccct gacgctgagc aaagcagact acgagaaaca 780
caaagtctac gcctgcgaag tcacccatca gggcctgagc tcgcccgtca caaagagctt 840
caacagggga gagtgtggtg ccagctccgg tatggctgat ccgaaccgtt tccgcggtaa 900
ggacctggca taactcgagg ctgatcctct acgccggacg catcgtggcc ctagtacgca 960
agttcacgta aaaagggtaa ctagaggttg aggtgatttt atgaaaaaga atatcgcatt 1020
tcttcttgca tctatgttcg ttttttctat tgctacaaac gcgtacgctg agatctccga 1080
ggttcagctg gtggagtctg gcggtggcct ggtgcagcca gggggctcac tccgtttgtc 1140
ctgtgcagct tctggcttca acattaaaga cacctatata cactgggtgc gtcaggcccc 1200
gggtaagggc ctggaatggg ttgcaaggat ttatcctacg aatggttata ctagatatgc 1260
cgatagcgtc aagggccgtt tcactataag cgcagacaca tccaaaaaca cagcctacct 1320
acaaatgaac agcttaagag ctgaggacac tgccgtctat tattgtagcc gctggggagg 1380
ggacggcttc tatgctatgg actactgggg tcaaggaacc ctggtcaccg tctcctcggc 1440
ctccaccaag ggcccatcgg tcttccccct ggcaccctcc tccaagagca cctctggggg 1500
cacagcggcc ctgggctgcc tggtcaagga ctacttcccc gaaccggtga cggtgtcgtg 1560
gaactcaggc gccctgacca gcggcgtgca caccttcccg gctgtcctac agtcctcagg 1620
actctactcc ctcagcagcg tggtgaccgt gccctccagc agcttgggca cccagaccta 1680
catctgcaac gtgaatcaca agcccagcaa caccaaggtc gacaagaaag ttgagcccaa 1740
atcttgtgac aaaactcaca catgcccgcc gtgcccagca ccagaactgc tgggcggccg 1800
catgaaacag ctagaggaca aggtcgaaga gctactctcc aagaactacc acctagagaa 1860
tgaagtggca agactcaaaa aacttgtcgg ggagcgcgga aagcttagtg gcggtggctc 1920
tggttccggt gattttgatt atgaaaagat ggcaaacgct aataaggggg ctatgaccga 1980
aaatgccgat gaaaacgcgc tacagtctga cgctaaaggc aaacttgatt ctgtcgctac 2040
tgattacggt gctgctatcg atggtttcat tggtgacgtt tccggccttg ctaatggtaa 2100
tggtgctact ggtgattttg ctggctctaa ttcccaaatg gctcaagtcg gtgacggtga 2160
taattcacct ttaatgaata atttccgtca atatttacct tccctccctc aatcggttga 2220
atgtcgccct tttgtcttta gcgctggtaa accatatgaa ttttctattg attgtgacaa 2280

aataaactta ttccgtggtg tctttgcgtt tcttttatat gttgccacct ttatgtatgt 2340

attttctacg tttgctaaca tactgcgtaa taaggagtct taa 2383

<210> 27
<211> 12
<212> PRT
<213> Artificial Sequence

<220>
<223> Hinge sequence from full length IgG1 antibody

<400> 27

Thr Cys Pro Pro Cys Pro Ala Pro Glu Leu Leu Gly
1               5                   10

<210> 28
<211> 7060
<212> DNA
<213> Artificial Sequence

<220>
<223> Fab phagemid vector pV0350-2b

<400> 28
gaattcaact tctccatact ttggataagg aaatacagac atgaaaaatc tcattgctga 60

gttgttattt aagcttgccc aaaaagaaga agagtcgaat gaactgtgtg cgcaggtaga 120

agctttggag attatcgtca ctgcaatgct tcgcaatatg gcgcaaaatg accaacagcg 180

gttgattgat caggtagagg gggcgctgta cgaggtaaag cccgatgcca gcattcctga 240

cgacgatacg gagctgctgc gcgattacgt aaagaagtta ttgaagcatc ctcgtcagta 300

aaaagttaat cttttcaaca gctgtcataa agttgtcacg gccgagactt atagtcgctt 360

tgtttttatt ttttaatgta tttgtaacta gtacgcaagt tcacgtaaaa agggtatgta 420

gaggttgagg tgattttatg aaaaagaata tcgcatttct tcttgcatct atgttcgttt 480

tttctattgc tacaaatgcc tatgcagata tccagatgac ccagtccccg agctccctgt 540

ccgcctctgt gggcgatagg gtcaccatca cctgccgtgc cagtcaggat gtgtccactg 600

ctgtagcctg gtatcaacag aaaccaggaa aagctccgaa gcttctgatt tactcggcat 660

ccttcctcta ctctggagtc ccttctcgct tctctggtag cggttccggg acggatttca 720

ctctgaccat cagcagtctg cagccggaag acttcgcaac ttattactgt cagcaatctt 780

atactactcc tcccacgttc ggacagggta ccaaggtgga gatcaaacga actgtggctg 840

caccatctgt cttcatcttc ccgccatctg atgagcagtt gaaatctgga actgcctctg 900

ttgtgtgcct gctgaataac ttctatccca gagaggccaa agtacagtgg aaggtggata 960

acgccctcca atcgggtaac tcccaggaga gtgtcacaga gcaggacagc aaggacagca 1020

cctacagcct cagcagcacc ctgacgctga gcaaagcaga ctacgagaaa cacaaagtct 1080

acgcctgcga agtcacccat cagggcctga gctcgcccgt cacaaagagc ttcaacaggg 1140

gagagtgtgg tgccagctcc ggtatggctg atccgaaccg tttccgcggt aaggacctgg 1200

cataactcga ggctgatcct ctacgccgga cgcatcgtgg ccctagtacg caagttcacg 1260
taaaaagggt aactagaggt tgaggtgatt ttatgaaaaa gaatatcgca tttcttcttg 1320
catctatgtt cgttttttct attgctacaa acgcgtacgc tgaggttcag ctggtggagt 1380
ctggcggtgg cctggtgcag ccagggggct cactccgttt gtcctgtgca gcttctggct 1440
tcaacattaa agacacctat atacactggg tgcgtcaggc cccgggtaag ggcctggaat 1500
gggttgcaag gatttatcct acgaatggtt atactagata tgccgatagc gtcaagggcc 1560
gtttcactat aagcgcagac acatccaaaa acacagccta cctacaaatg aacagcttaa 1620
gagctgagga cactgccgtc tattattgta gccgctgggg aggggacggc ttctatgcta 1680
tggactactg gggtcaagga acactagtca ccgtctcctc ggcctccacc aagggcccat 1740
cggtcttccc cctggcaccc tcctccaaga gcacctctgg gggcacagcg gccctgggct 1800
gcctggtcaa ggactacttc cccgaaccgg tgacggtgtc gtggaactca ggcgccctga 1860
ccagcggcgt gcacaccttc ccggctgtcc tacagtcctc aggactctac tccctcagca 1920
gcgtggtgac cgtgccctcc agcagcttgg gcacccagac ctacatctgc aacgtgaatc 1980
acaagcccag caacaccaag gtcgacaaga aagttgagcc caaatcttgt gacaaaactc 2040
acctcagtgg cggtggctct ggttccggtg attttgatta tgaaaagatg gcaaacgcta 2100
ataagggggc tatgaccgaa aatgccgatg aaaacgcgct acagtctgac gctaaaggca 2160
aacttgattc tgtcgctact gattacggtg ctgctatcga tggtttcatt ggtgacgttt 2220
ccggccttgc taatggtaat ggtgctactg gtgattttgc tggctctaat tcccaaatgg 2280
ctcaagtcgg tgacggtgat aattcacctt taatgaataa tttccgtcaa tatttacctt 2340
ccctccctca atcggttgaa tgtcgccctt ttgtctttag cgctggtaaa ccatatgaat 2400
tttctattga ttgtgacaaa ataaacttat tccgtggtgt ctttgcgttt cttttatatg 2460
ttgccacctt tatgtatgta ttttctacgt ttgctaacat actgcgtaat aaggagtctt 2520
aatcatgcca gttcttttgg ctagcgccgc cctatacctt gtctgcctcc ccgcgttgcg 2580
tcgcggtgca tggagccggg ccacctcgac ctgaatggaa gccggcggca cctcgctaac 2640
ggattcacca ctccaagaat tggagccaat caattcttgc ggagaactgt gaatgcgcaa 2700
accaaccctt ggcagaacat atccatcgcg tccgccatct ccagcagccg cacgcggcgc 2760
atctcgggca gcgttgggtc ctggccacgg gtgcgcatga tcgtgctcct gtcgttgagg 2820
acccggctag gctggcgggg ttgccttact ggttagcaga atgaatcacc gatacgcgag 2880
cgaacgtgaa gcgactgctg ctgcaaaacg tctgcgacct gagcaacaac atgaatggtc 2940
ttcggtttcc gtgtttcgta aagtctggaa acgcggaagt cagcgccctg caccattatg 3000
ttccggatct gcatcgcagg atgctgctgg ctaccctgtg gaacacctac atctgtatta 3060
acgaagcgct ggcattgacc ctgagtgatt tttctctggt cccgccgcat ccataccgcc 3120
agttgtttac cctcacaacg ttccagtaac cgggcatgtt catcatcagt aacccgtatc 3180
gtgagcatcc tctctcgttt catcggtatc attaccccca tgaacagaaa ttccccctta 3240

cacggaggca tcaagtgacc aaacaggaaa aaaccgccct taacatggcc cgctttatca 3300
gaagccagac attaacgctt ctggagaaac tcaacgagct ggacgcggat gaacaggcag 3360
acatctgtga atcgcttcac gaccacgctg atgagcttta ccgcaggatc cggaaattgt 3420
aaacgttaat attttgttaa aattcgcgtt aaatttttgt taaatcagct cattttttaa 3480
ccaataggcc gaaatcggca aaatccctta taaatcaaaa gaatagaccg agatagggtt 3540
gagtgttgtt ccagtttgga acaagagtcc actattaaag aacgtggact ccaacgtcaa 3600
agggcgaaaa accgtctatc agggctatgg cccactacgt gaaccatcac cctaatcaag 3660
ttttttgggg tcgaggtgcc gtaaagcact aaatcggaac cctaaaggga gcccccgatt 3720
tagagcttga cggggaaagc cggcgaacgt ggcgagaaag gaagggaaga aagcgaaagg 3780
agcgggcgct agggcgctgg caagtgtagc ggtcacgctg cgcgtaacca ccacacccgc 3840
cgcgcttaat gcgccgctac agggcgcgtc cggatcctgc ctcgcgcgtt tcggtgatga 3900
cggtgaaaac ctctgacaca tgcagctccc ggagacggtc acagcttgtc tgtaagcgga 3960
tgccgggagc agacaagccc gtcagggcgc gtcagcgggt gttggcgggt gtcggggcgc 4020
agccatgacc cagtcacgta gcgatagcgg agtgtatact ggcttaacta tgcggcatca 4080
gagcagattg tactgagagt gcaccatatg cggtgtgaaa taccgcacag atgcgtaagg 4140
agaaaatacc gcatcaggcg ctcttccgct tcctcgctca ctgactcgct gcgctcggtc 4200
gttcggctgc ggcgagcggt atcagctcac tcaaaggcgg taatacggtt atccacagaa 4260
tcaggggata acgcaggaaa gaacatgtga gcaaaaggcc agcaaaaggc caggaaccgt 4320
aaaaaggccg cgttgctggc gtttttccat aggctccgcc cccctgacga gcatcacaaa 4380
aatcgacgct caagtcagag gtggcgaaac ccgacaggac tataaagata ccaggcgttt 4440
ccccctggaa gctccctcgt gcgctctcct gttccgaccc tgccgcttac cggatacctg 4500
tccgcctttc tcccttcggg aagcgtggcg ctttctcata gctcacgctg taggtatctc 4560
agttcggtgt aggtcgttcg ctccaagctg ggctgtgtgc acgaaccccc cgttcagccc 4620
gaccgctgcg ccttatccgg taactatcgt cttgagtcca acccggtaag acacgactta 4680
tcgccactgg cagcagccac tggtaacagg attagcagag cgaggtatgt aggcggtgct 4740
acagagttct tgaagtggtg gcctaactac ggctacacta gaaggacagt atttggtatc 4800
tgcgctctgc tgaagccagt taccttcgga aaaagagttg gtagctcttg atccggcaaa 4860
caaaccaccg ctggtagcgg tggttttttt gtttgcaagc agcagattac gcgcagaaaa 4920
aaaggatctc aagaagatcc tttgatcttt tctacggggt ctgacgctca gtggaacgaa 4980
aactcacgtt aagggatttt ggtcatgaga ttatcaaaaa ggatcttcac ctagatcctt 5040
ttaaattaaa aatgaagttt taaatcaatc taaagtatat atgagtaaac ttggtctgac 5100
agttaccaat gcttaatcag tgaggcacct atctcagcga tctgtctatt tcgttcatcc 5160
atagttgcct gactccccgt cgtgtagata actacgatac gggagggctt accatctggc 5220
cccagtgctg caatgatacc gcgagaccca cgctcaccgg ctccagattt atcagcaata 5280

aaccagccag ccggaagggc cgagcgcaga agtggtcctg caactttatc cgcctccatc 5340

cagtctatta attgttgccg ggaagctaga gtaagtagtt cgccagttaa tagtttgcgc 5400

aacgttgttg ccattgctgc aggcatcgtg gtgtcacgct cgtcgtttgg tatggcttca 5460

ttcagctccg gttcccaacg atcaaggcga gttacatgat cccccatgtt gtgcaaaaaa 5520

gcggttagct ccttcggtcc tccgatcgtt gtcagaagta agttggccgc agtgttatca 5580

ctcatggtta tggcagcact gcataattct cttactgtca tgccatccgt aagatgcttt 5640

tctgtgactg gtgagtactc aaccaagtca ttctgagaat agtgtatgcg gcgaccgagt 5700

tgctcttgcc cggcgtcaac acgggataat accgcgccac atagcagaac tttaaaagtg 5760

ctcatcattg gaaaacgttc ttcggggcga aaactctcaa ggatcttacc gctgttgaga 5820

tccagttcga tgtaacccac tcgtgcaccc aactgatctt cagcatcttt tactttcacc 5880

agcgtttctg ggtgagcaaa aacaggaagg caaaatgccg caaaaaaggg aataagggcg 5940

acacggaaat gttgaatact catactcttc ctttttcaat attattgaag catttatcag 6000

ggttattgtc tcatgagcgg atacatattt gaatgtattt agaaaaataa acaaataggg 6060

gttccgcgca catttccccg aaaagtgcca cctgacgtct aagaaaccat tattatcatg 6120

acattaacct ataaaaatag gcgtatcacg aggccctttc gtcttcaata caggtagacc 6180

tttcgtagag atgtacagtg aaatccccga aattatacac atgactgaag gaagggagct 6240

cgtcattccc tgccgggtta cgtcacctaa catcactgtt actttaaaaa agtttccact 6300

tgacactttg atccctgatg gaaaacgcat aatctgggac agtagaaagg gcttcatcat 6360

atcaaatgca acgtacaaag aaatagggct tctgacctgt gaagcaacag tcaatgggca 6420

tttgtataag acaaactatc tcacacatcg acaaaccaat acaatacagg tagacctttc 6480

gtagagatgt acagtgaaat ccccgaaatt atacacatga ctgaaggaag ggagctcgtc 6540

attccctgcc gggttacgtc acctaacatc actgttactt taaaaaagtt tccacttgac 6600

actttgatcc ctgatggaaa acgcataatc tgggacagta gaaagggctt catcatatca 6660

aatgcaacgt acaaagaaat agggcttctg acctgtgaag caacagtcaa tgggcatttg 6720

tataagacaa actatctcac acatcgacaa accaatacaa tctacaggta gacctttcgt 6780

agagatgtac agtgaaatcc ccgaaattat acacatgact gaaggaaggg agctcgtcat 6840

tccctgccgg gttacgtcac ctaacatcac tgttacttta aaaaagtttc cacttgacac 6900

tttgatccct gatggaaaac gcataatctg ggacagtaga aagggcttca tcatatcaaa 6960

tgcaacgtac aaagaaatag ggcttctgac ctgtgaagca acagtcaatg ggcatttgta 7020

taagacaaac tatctcacac atcgacaaac caatacaatc 7060

<210> 29
<211> 7171
<212> DNA
<213> Artificial Sequence

<220>
<223> F(ab)'2 phagemid vector pV0350-4

<400> 29
gaattcaact tctccatact ttggataagg aaatacagac atgaaaaatc tcattgctga 60
gttgttattt aagcttgccc aaaaagaaga agagtcgaat gaactgtgtg cgcaggtaga 120
agctttggag attatcgtca ctgcaatgct tcgcaatatg gcgcaaaatg accaacagcg 180
gttgattgat caggtagagg gggcgctgta cgaggtaaag cccgatgcca gcattcctga 240
cgacgatacg gagctgctgc gcgattacgt aaagaagtta ttgaagcatc ctcgtcagta 300
aaaagttaat cttttcaaca gctgtcataa agttgtcacg gccgagactt atagtcgctt 360
tgtttttatt ttttaatgta tttgtaacta gtacgcaagt tcacgtaaaa agggtatgta 420
gaggttgagg tgattttatg aaaaagaata tcgcatttct tcttgcatct atgttcgttt 480
tttctattgc tacaaatgcc tatgcatccg atatccagat gacccagtcc ccgagctccc 540
tgtccgcctc tgtgggcgat agggtcacca tcacctgccg tgccagtcag gatgtgtcca 600
ctgctgtagc ctggtatcaa cagaaaccag gaaaagctcc gaagcttctg atttactcgg 660
catccttcct ctactctgga gtcccttctc gcttctctgg tagcggttcc gggacggatt 720
tcactctgac catcagcagt ctgcagccgg aagacttcgc aacttattac tgtcagcaat 780
cttatactac tcctcccacg ttcggacagg gtaccaaggt ggagatcaaa cgaactgtgg 840
ctgcaccatc tgtcttcatc ttcccgccat ctgatgagca gttgaaatct ggaactgcct 900
ctgttgtgtg cctgctgaat aacttctatc ccagagaggc caaagtacag tggaaggtgg 960
ataacgccct ccaatcgggt aactcccagg agagtgtcac agagcaggac agcaaggaca 1020
gcacctacag cctcagcagc accctgacgc tgagcaaagc agactacgag aaacacaaag 1080
tctacgcctg cgaagtcacc catcagggcc tgagctcgcc cgtcacaaag agcttcaaca 1140
ggggagagtg tggtgccagc tccggtatgg ctgatccgaa ccgtttccgc ggtaaggacc 1200
tggcataact cgaggctgat cctctacgcc ggacgcatcg tggccctagt acgcaagttc 1260
acgtaaaaag ggtaactaga ggttgaggtg attttatgaa aaagaatatc gcatttcttc 1320
ttgcatctat gttcgttttt tctattgcta caaacgcgta cgctgaggtt cagctggtgg 1380
agtctggcgg tggcctggtg cagccagggg gctcactccg tttgtcctgt gcagcttctg 1440
gcttcaacat taaagacacc tatatacact gggtgcgtca ggccccgggt aagggcctgg 1500
aatgggttgc aaggatttat cctacgaatg gttatactag atatgccgat agcgtcaagg 1560
gccgtttcac tataagcgca gacacatcca aaaacacagc ctacctacaa atgaacagct 1620
taagagctga ggacactgcc gtctattatt gtagccgctg gggaggggac ggcttctatg 1680
ctatggacta ctggggtcaa ggaacactag tcaccgtctc ctcggcctcc accaagggcc 1740
catcggtctt ccccctggca ccctcctcca agagcacctc tgggggcaca gcggccctgg 1800
gctgcctggt caaggactac ttccccgaac cggtgacggt gtcgtggaac tcaggcgccc 1860
tgaccagcgg cgtgcacacc ttcccggctg tcctacagtc ctcaggactc tactccctca 1920
gcagcgtggt gaccgtgccc tccagcagct tgggcaccca gacctacatc tgcaacgtga 1980

atcacaagcc cagcaacacc aaggtcgaca agaaagttga gcccaaatct tgtgacaaaa 2040
ctcacggccg catgaaacag ctagaggaca aggtcgaaga gctactctcc aagaactacc 2100
acctagagaa tgaagtggca agactcaaaa aacttgtcgg ggagcgcgga aagcttagtg 2160
gcggtggctc tggttccggt gattttgatt atgaaaagat ggcaaacgct aataaggggg 2220
ctatgaccga aaatgccgat gaaaacgcgc tacagtctga cgctaaaggc aaacttgatt 2280
ctgtcgctac tgattacggt gctgctatcg atggtttcat tggtgacgtt tccggccttg 2340
ctaatggtaa tggtgctact ggtgattttg ctggctctaa ttcccaaatg gctcaagtcg 2400
gtgacggtga taattcacct ttaatgaata atttccgtca atatttacct tccctccctc 2460
aatcggttga atgtcgccct tttgtcttta gcgctggtaa accatatgaa ttttctattg 2520
attgtgacaa aataaactta ttccgtggtg tctttgcgtt tcttttatat gttgccacct 2580
ttatgtatgt attttctacg tttgctaaca tactgcgtaa taaggagtct taatcatgcc 2640
agttcttttg gctagcgccg ccctatacct tgtctgcctc cccgcgttgc gtcgcggtgc 2700
atggagccgg gccacctcga cctgaatgga agccggcggc acctcgctaa cggattcacc 2760
actccaagaa ttggagccaa tcaattcttg cggagaactg tgaatgcgca aaccaaccct 2820
tggcagaaca tatccatcgc gtccgccatc tccagcagcc gcacgcggcg catctcgggc 2880
agcgttgggt cctggccacg ggtgcgcatg atcgtgctcc tgtcgttgag gacccggcta 2940
ggctggcggg gttgccttac tggttagcag aatgaatcac cgatacgcga gcgaacgtga 3000
agcgactgct gctgcaaaac gtctgcgacc tgagcaacaa catgaatggt cttcggtttc 3060
cgtgtttcgt aaagtctgga aacgcggaag tcagcgccct gcaccattat gttccggatc 3120
tgcatcgcag gatgctgctg gctaccctgt ggaacaccta catctgtatt aacgaagcgc 3180
tggcattgac cctgagtgat ttttctctgg tcccgccgca tccataccgc cagttgttta 3240
ccctcacaac gttccagtaa ccgggcatgt tcatcatcag taacccgtat cgtgagcatc 3300
ctctctcgtt tcatcggtat cattaccccc atgaacagaa attccccctt acacggaggc 3360
atcaagtgac caaacaggaa aaaaccgccc ttaacatggc ccgctttatc agaagccaga 3420
cattaacgct tctggagaaa ctcaacgagc tggacgcgga tgaacaggca gacatctgtg 3480
aatcgcttca cgaccacgct gatgagcttt accgcaggat ccggaaattg taaacgttaa 3540
tattttgtta aaattcgcgt taaatttttg ttaaatcagc tcatttttta accaataggc 3600
cgaaatcggc aaaatccctt ataaatcaaa agaatagacc gagatagggt tgagtgttgt 3660
tccagtttgg aacaagagtc cactattaaa gaacgtggac tccaacgtca aagggcgaaa 3720
aaccgtctat cagggctatg gcccactacg tgaaccatca ccctaatcaa gttttttggg 3780
gtcgaggtgc cgtaaagcac taaatcggaa ccctaaaggg agcccccgat ttagagcttg 3840
acggggaaag ccggcgaacg tggcgagaaa ggaagggaag aaagcgaaag gagcgggcgc 3900
tagggcgctg gcaagtgtag cggtcacgct gcgcgtaacc accacacccg ccgcgcttaa 3960
tgcgccgcta cagggcgcgt ccggatcctg cctcgcgcgt ttcggtgatg acggtgaaaa 4020

cctctgacac atgcagctcc cggagacggt cacagcttgt ctgtaagcgg atgccgggag 4080
cagacaagcc cgtcagggcg cgtcagcggg tgttggcggg tgtcggggcg cagccatgac 4140
ccagtcacgt agcgatagcg gagtgtatac tggcttaact atgcggcatc agagcagatt 4200
gtactgagag tgcaccatat gcggtgtgaa ataccgcaca gatgcgtaag gagaaaatac 4260
cgcatcaggc gctcttccgc ttcctcgctc actgactcgc tgcgctcggt cgttcggctg 4320
cggcgagcgg tatcagctca ctcaaaggcg gtaatacggt tatccacaga atcaggggat 4380
aacgcaggaa agaacatgtg agcaaaaggc cagcaaaagg ccaggaaccg taaaaaggcc 4440
gcgttgctgg cgtttttcca taggctccgc ccccctgacg agcatcacaa aaatcgacgc 4500
tcaagtcaga ggtggcgaaa cccgacagga ctataaagat accaggcgtt tccccctgga 4560
agctccctcg tgcgctctcc tgttccgacc ctgccgctta ccggatacct gtccgccttt 4620
ctcccttcgg gaagcgtggc gctttctcat agctcacgct gtaggtatct cagttcggtg 4680
taggtcgttc gctccaagct gggctgtgtg cacgaacccc ccgttcagcc cgaccgctgc 4740
gccttatccg gtaactatcg tcttgagtcc aacccggtaa gacacgactt atcgccactg 4800
gcagcagcca ctggtaacag gattagcaga gcgaggtatg taggcggtgc tacagagttc 4860
ttgaagtggt ggcctaacta cggctacact agaaggacag tatttggtat ctgcgctctg 4920
ctgaagccag ttaccttcgg aaaaagagtt ggtagctctt gatccggcaa acaaaccacc 4980
gctggtagcg gtggtttttt tgtttgcaag cagcagatta cgcgcagaaa aaaaggatct 5040
caagaagatc ctttgatctt ttctacgggg tctgacgctc agtggaacga aaactcacgt 5100
taagggattt tggtcatgag attatcaaaa aggatcttca cctagatcct tttaaattaa 5160
aaatgaagtt ttaaatcaat ctaaagtata tatgagtaaa cttggtctga cagttaccaa 5220
tgcttaatca gtgaggcacc tatctcagcg atctgtctat ttcgttcatc catagttgcc 5280
tgactccccg tcgtgtagat aactacgata cgggagggct taccatctgg ccccagtgct 5340
gcaatgatac cgcgagaccc acgctcaccg gctccagatt tatcagcaat aaaccagcca 5400
gccggaaggg ccgagcgcag aagtggtcct gcaactttat ccgcctccat ccagtctatt 5460
aattgttgcc gggaagctag agtaagtagt tcgccagtta atagtttgcg caacgttgtt 5520
gccattgctg caggcatcgt ggtgtcacgc tcgtcgtttg gtatggcttc attcagctcc 5580
ggttcccaac gatcaaggcg agttacatga tcccccatgt tgtgcaaaaa agcggttagc 5640
tccttcggtc ctccgatcgt tgtcagaagt aagttggccg cagtgttatc actcatggtt 5700
atggcagcac tgcataattc tcttactgtc atgccatccg taagatgctt ttctgtgact 5760
ggtgagtact caaccaagtc attctgagaa tagtgtatgc ggcgaccgag ttgctcttgc 5820
ccggcgtcaa cacgggataa taccgcgcca catagcagaa ctttaaaagt gctcatcatt 5880
ggaaaacgtt cttcggggcg aaaactctca aggatcttac cgctgttgag atccagttcg 5940
atgtaaccca ctcgtgcacc caactgatct tcagcatctt ttactttcac cagcgtttct 6000
gggtgagcaa aaacaggaag gcaaaatgcc gcaaaaaagg gaataagggc gacacggaaa 6060

tgttgaatac tcatactctt cctttttcaa tattattgaa gcatttatca gggttattgt 6120

ctcatgagcg gatacatatt tgaatgtatt tagaaaaata aacaaatagg ggttccgcgc 6180

acatttcccc gaaaagtgcc acctgacgtc taagaaacca ttattatcat gacattaacc 6240

tataaaaata ggcgtatcac gaggcccttt cgtcttcaat acaggtagac ctttcgtaga 6300

gatgtacagt gaaatccccg aaattataca catgactgaa ggaagggagc tcgtcattcc 6360

ctgccgggtt acgtcaccta acatcactgt tactttaaaa aagtttccac ttgacacttt 6420

gatccctgat ggaaaacgca taatctggga cagtagaaag ggcttcatca tatcaaatgc 6480

aacgtacaaa gaaatagggc ttctgacctg tgaagcaaca gtcaatgggc atttgtataa 6540

gacaaactat ctcacacatc gacaaaccaa tacaatacag gtagaccttt cgtagagatg 6600

tacagtgaaa tccccgaaat tatacacatg actgaaggaa gggagctcgt cattccctgc 6660

cgggttacgt cacctaacat cactgttact ttaaaaaagt ttccacttga cactttgatc 6720

cctgatggaa aacgcataat ctgggacagt agaaagggct tcatcatatc aaatgcaacg 6780

tacaaagaaa tagggcttct gacctgtgaa gcaacagtca atgggcattt gtataagaca 6840

aactatctca cacatcgaca aaccaataca atctacaggt agacctttcg tagagatgta 6900

cagtgaaatc cccgaaatta tacacatgac tgaaggaagg gagctcgtca ttccctgccg 6960

ggttacgtca cctaacatca ctgttacttt aaaaaagttt ccacttgaca ctttgatccc 7020

tgatggaaaa cgcataatct gggacagtag aaagggcttc atcatatcaa atgcaacgta 7080

caaagaaata gggcttctga cctgtgaagc aacagtcaat gggcatttgt ataagacaaa 7140

ctatctcaca catcgacaaa ccaatacaat c 7171

<210> 30
<211> 57
<212> DNA
<213> Artificial Sequence

<220>
<223> CDR Oligonucleotide


<220>
<221> misc_feature
<222> (22)..(22)
<223> n=a, c, t or g

<220>
<221> misc_feature
<222> (23)..(23)
<223> n=a or g

<220>
<221> misc_feature
<222> (25)..(25)
<223> n=a, c, t or g

<220>
<221> misc_feature
<222> (26)..(26)
<223> n=a or g

<220>
<221>  misc_feature
<222>  (28)..(28)
<223>  n=a or g

<220>
<221>  misc_feature
<222>  (29)..(29)
<223>  n=a, c or g

<220>
<221>  misc_feature
<222>  (30)..(30)
<223>  n=a or c

<220>
<221>  misc_feature
<222>  (31)..(32)
<223>  n=a, c, g or t

<220>
<221>  misc_feature
<222>  (33)..(33)
<223>  n=g or t

<220>
<221>  misc_feature
<222>  (38)..(38)
<223>  n=a, g or t

<220>
<221>  misc_feature
<222>  (39)..(39)
<223>  n=g or t

<400>  30
gcaacttatt actgtcagca anntnntnnn nnnccttnna cgttcggaca gggtacc        57


<210>  31
<211>  13
<212>  PRT
<213>  Artificial Sequence

<220>
<223>  CDRH3

<400>  31

Ser Arg Trp Gly Gly Asp Gly Phe Tyr Ala Met Asp Tyr
1               5                   10


<210>  32
<211>  10
<212>  PRT
<213>  Artificial Sequence

<220>
<223>  Randomized CDRH3


<220>
<221>  MISC_FEATURE
<222>  (3)..(7)
<223>  Xaa is Ala, Cys, Asp, Glu, Gly, Lys, Asn, Arg, Ser, Tyr, Trp

       or Thr

<400>  32

Ser Arg Xaa Xaa Xaa Xaa Xaa Phe Asp Tyr
1               5                   10


<210>  33
<211>  13
<212>  PRT
<213>  Artificial Sequence

<220>
<223>  Randomized CDRH3


<220>
<221>  MISC_FEATURE
<222>  (2)..(2)
<223>  Xaa is Arg or Lys

<220>
<221>  MISC_FEATURE
<222>  (3)..(7)
<223>  Xaa is Ala, Cys, Asp, Glu, Gly, Lys, Asn, Arg, Ser, Tyr, Trp or

       Thr

<220>
<221>  MISC_FEATURE
<222>  (8)..(8)
<223>  Xaa is any naturally occurring amino acid

<400>  33

Ala Xaa Xaa Xaa Xaa Xaa Xaa Xaa Tyr Ala Met Asp Tyr
1               5                   10


<210>  34
<211>  13
<212>  PRT
<213>  Artificial Sequence

<220>
<223>  Randomized CDRH3


<220>
<221>  MISC_FEATURE
<222>  (2)..(2)
<223>  Xaa is Arg or Lys

<220>
<221>  MISC_FEATURE
<222>  (4)..(4)
<223>  Xaa is Ala, Cys, Asp, Gly, His, Asn, Pro, Arg, Ser, Thr or Tyr

<220>
<221>  MISC_FEATURE
<222>  (5)..(8)
<223>  Xaa is Lys, Asn, Thr, Arg, Ser, Glu, Asp, Ala, Gly, Tyr, Trp or

       Cys

<220>
<221>  MISC_FEATURE
<222>  (9)..(9)

<223>  Xaa is Ala, Gly, Arg, Ser, Thr or Trp

<400>  34

Ala Xaa Trp Xaa Xaa Xaa Xaa Xaa Xaa Ala Met Asp Tyr
1               5                   10


<210>  35
<211>  13
<212>  PRT
<213>  Artificial Sequence

<220>
<223>  Randomized CDRH3


<220>
<221>  MISC_FEATURE
<222>  (2)..(2)
<223>  Xaa is Arg, Lys or Thr

<220>
<221>  MISC_FEATURE
<222>  (3)..(8)
<223>  Xaa is Ala, Cys, Asp, Gly, His, Asn, Pro, Arg, Ser, Thr or Tyr

<400>  35

Ala Xaa Xaa Xaa Xaa Xaa Xaa Xaa Tyr Ala Met Asp Tyr
1               5                   10


<210>  36
<211>  14
<212>  PRT
<213>  Artificial Sequence

<220>
<223>  Randomized CDRH3


<220>
<221>  MISC_FEATURE
<222>  (2)..(2)
<223>  Xaa is Arg, Lys or Thr

<220>
<221>  MISC_FEATURE
<222>  (4)..(9)
<223>  Xaa is Ala, Cys, Asp, Gly, His, Asn, Pro, Arg, Ser, Thr or Tyr

<400>  36

Ala Xaa Trp Xaa Xaa Xaa Xaa Xaa Xaa Tyr Ala Met Asp Tyr
1               5                   10


<210>  37
<211>  14
<212>  PRT
<213>  Artificial Sequence

<220>
<223>  Randomized CDRH3


<220>

<221>  MISC_FEATURE
<222>  (2)..(2)
<223>  Xaa is Arg, Lys or Thr

<220>
<221>  MISC_FEATURE
<222>  (3)..(3)
<223>  Xaa is Ala, Cys, Asp, Gly, His, Asn, Pro, Arg, Ser, Thr or Tyr

<220>
<221>  MISC_FEATURE
<222>  (5)..(9)
<223>  Xaa is Ala, Cys, Asp, Gly, His, Asn, Pro, Arg, Ser, Thr or Tyr

<400>  37

Ala Xaa Xaa Trp Xaa Xaa Xaa Xaa Xaa Tyr Ala Met Asp Tyr
1               5                   10


<210>  38
<211>  14
<212>  PRT
<213>  Artificial Sequence

<220>
<223>  Randomized CDRH3


<220>
<221>  MISC_FEATURE
<222>  (2)..(2)
<223>  Xaa is Arg, Lys or Thr

<220>
<221>  MISC_FEATURE
<222>  (3)..(4)
<223>  Xaa is Ala, Cys, Asp, Gly, His, Asn, Pro, Arg, Ser, Thr or Tyr

<220>
<221>  MISC_FEATURE
<222>  (6)..(9)
<223>  Xaa is Ala, Cys, Asp, Gly, His, Asn, Pro, Arg, Ser, Thr or Tyr

<400>  38

Ala Xaa Xaa Xaa Trp Xaa Xaa Xaa Xaa Tyr Ala Met Asp Tyr
1               5                   10


<210>  39
<211>  14
<212>  PRT
<213>  Artificial Sequence

<220>
<223>  Randomized CDRH3


<220>
<221>  MISC_FEATURE
<222>  (2)..(2)
<223>  Xaa is Arg, Lys or Thr

<220>
<221>  MISC_FEATURE
<222>  (3)..(8)
<223>  Xaa is Ala, Cys, Asp, Gly, His, Pro, Arg, Ser, Thr, Tyr or Asn

<400>  39

Ala Xaa Xaa Xaa Xaa Xaa Xaa Xaa Trp Tyr Ala Met Asp Tyr
1               5                   10


<210>  40
<211>  13
<212>  PRT
<213>  Artificial Sequence

<220>
<223>  Randomized CDRH3


<220>
<221>  MISC_FEATURE
<222>  (2)..(2)
<223>  Xaa is Arg or Lys

<220>
<221>  MISC_FEATURE
<222>  (4)..(9)
<223>  Xaa is Ala, Cys, Asp, Gly, His, Asn, Pro, Arg, Ser, Thr or Tyr

<220>
<221>  MISC_FEATURE
<222>  (10)..(10)
<223>  Xaa is Gly, Ser, Ala or Trp

<400>  40

Ala Xaa Trp Xaa Xaa Xaa Xaa Xaa Xaa Xaa Ala Met Tyr
1               5                   10


<210>  41
<211>  14
<212>  PRT
<213>  Artificial Sequence

<220>
<223>  Randomized CDRH3


<220>
<221>  MISC_FEATURE
<222>  (2)..(2)
<223>  Xaa is Arg or Lys

<220>
<221>  MISC_FEATURE
<222>  (3)..(3)
<223>  Xaa is Ala, Cys, Asp, Gly, His, Asn, Pro, Arg, Ser, Thr or Tyr

<220>
<221>  MISC_FEATURE
<222>  (5)..(9)
<223>  Xaa is Ala, Cys, Asp, Gly, His, Asn, Pro, Arg, Ser, Thr or Tyr

<220>
<221>  MISC_FEATURE
<222>  (10)..(10)
<223>  Xaa is Gly, Ser, Ala or Trp

<400>  41

Ala Xaa Xaa Trp Xaa Xaa Xaa Xaa Xaa Xaa Ala Met Asp Tyr
1               5                   10

<210>  42
<211>  14
<212>  PRT
<213>  Artificial Sequence

<220>
<223>  Randomized CDRH3

<220>
<221>  MISC_FEATURE
<222>  (2)..(2)
<223>  Xaa is Arg or Lys

<220>
<221>  MISC_FEATURE
<222>  (3)..(4)
<223>  Xaa is Ala, Cys, Asp, Gly, His, Asn, Pro, Arg, Ser, Thr or Tyr

<220>
<221>  MISC_FEATURE
<222>  (6)..(9)
<223>  Xaa is Ala, Cys, Asp, Gly, His, Asn, Pro, Arg, Ser, Thr or Tyr

<220>
<221>  MISC_FEATURE
<222>  (10)..(10)
<223>  Xaa is Gly, Ser, Ala or Trp

<400>  42

Ala Xaa Xaa Xaa Trp Xaa Xaa Xaa Xaa Xaa Ala Met Asp Tyr
1               5                   10

<210>  43
<211>  14
<212>  PRT
<213>  Artificial Sequence

<220>
<223>  Randomized CDRH3

<220>
<221>  MISC_FEATURE
<222>  (2)..(2)
<223>  Xaa is Arg or Lys

<220>
<221>  MISC_FEATURE
<222>  (3)..(5)
<223>  Xaa is Ala, Cys, Asp, Gly, His, Asn, Pro, Arg, Ser, Thr or Tyr

<220>
<221>  MISC_FEATURE
<222>  (7)..(9)
<223>  Xaa is Ala, Cys, Asp, Gly, His, Asn, Pro, Arg, Ser, Thr or Tyr

<220>
<221>  MISC_FEATURE
<222>  (10)..(10)
<223>  Xaa is Gly, Ser, Ala or Trp

<400>  43

Ala Xaa Xaa Xaa Xaa Trp Xaa Xaa Xaa Xaa Ala Met Asp Tyr
1               5                   10


<210>  44
<211>  14
<212>  PRT
<213>  Artificial Sequence

<220>
<223>  Randomized CDRH3


<220>
<221>  MISC_FEATURE
<222>  (2)..(2)
<223>  Xaa is Arg or Lys

<220>
<221>  MISC_FEATURE
<222>  (3)..(7)
<223>  Xaa is Ala, Cys, Asp, Gly, His, Asn, Pro, Arg, Ser, Thr or Tyr

<220>
<221>  MISC_FEATURE
<222>  (9)..(9)
<223>  Xaa is Ala, Cys, Asp, Gly, His, Asn, Pro, Arg, Ser, Thr or Tyr

<220>
<221>  MISC_FEATURE
<222>  (10)..(10)
<223>  Xaa is Gly, Ser, Ala or Trp

<400>  44

Ala Xaa Xaa Xaa Xaa Xaa Xaa Trp Xaa Xaa Ala Met Asp Tyr
1               5                   10


<210>  45
<211>  14
<212>  PRT
<213>  Artificial Sequence

<220>
<223>  Randomized CDRH3


<220>
<221>  MISC_FEATURE
<222>  (2)..(2)
<223>  Xaa is Arg or Lys

<220>
<221>  MISC_FEATURE
<222>  (3)..(8)
<223>  Xaa is Ala, Cys, Asp, Gly, His, Asn, Pro, Arg, Ser, Thr or Tyr

<220>
<221>  MISC_FEATURE
<222>  (10)..(10)
<223>  Xaa is Gly, Ser, Ala or Trp

<400>  45

Ala Xaa Xaa Xaa Xaa Xaa Xaa Xaa Trp Xaa Ala Met Asp Tyr

1               5                   10


<210>  46
<211>  13
<212>  PRT
<213>  Artificial Sequence

<220>
<223>  Randomized CDRH3


<220>
<221>  MISC_FEATURE
<222>  (2)..(2)
<223>  Xaa is Arg or Lys

<220>
<221>  MISC_FEATURE
<222>  (3)..(8)
<223>  Xaa is Ala, Cys, Asp, Glu, Gly, Lys, Asn, Arg, Ser, Tyr, Trp or

       Thr

<220>
<221>  MISC_FEATURE
<222>  (9)..(9)
<223>  Xaa is Trp, Ser, Ala or Gly

<400>  46

Ala Xaa Xaa Xaa Xaa Xaa Xaa Xaa Xaa Ala Met Asp Tyr
1               5                   10


<210>  47
<211>  14
<212>  PRT
<213>  Artificial Sequence

<220>
<223>  Randomized CDRH3


<220>
<221>  MISC_FEATURE
<222>  (3)..(9)
<223>  Xaa is Ala, Cys, Asp, Glu, Gly, Lys, Asn, Arg, Ser, Tyr, Trp or

       Thr

<400>  47

Ala Arg Xaa Xaa Xaa Xaa Xaa Xaa Xaa Tyr Ala Met Asp Tyr
1               5                   10


<210>  48
<211>  14
<212>  PRT
<213>  Artificial Sequence

<220>
<223>  Randomized CDRH3


<220>
<221>  MISC_FEATURE

<222>  (3)..(9)
<223>  Xaa is Ala, Cys, Asp, Glu, Gly, Lys, Asn, Arg, Ser, Tyr, Trp or

       Thr

<220>
<221>  MISC_FEATURE
<222>  (10)..(10)
<223>  Xaa is Ala, Gly, Arg, Ser, Thr or Trp

<400>  48

Ala Arg Xaa Xaa Xaa Xaa Xaa Xaa Xaa Xaa Ala Met Asp Tyr
1               5                   10


<210>  49
<211>  15
<212>  PRT
<213>  Artificial Sequence

<220>
<223>  Randomized CDRH3


<220>
<221>  MISC_FEATURE
<222>  (3)..(10)
<223>  Xaa is Ala, Cys, Asp, Glu, Gly, Lys, Asn, Arg, Ser, Tyr, Trp or

       Thr

<400>  49

Ala Arg Xaa Xaa Xaa Xaa Xaa Xaa Xaa Xaa Tyr Ala Met Asp Tyr
1               5                   10                  15


<210>  50
<211>  15
<212>  PRT
<213>  Artificial Sequence

<220>
<223>  Randomized CDRH3


<220>
<221>  MISC_FEATURE
<222>  (3)..(10)
<223>  Xaa is Ala, Cys, Asp, Glu, Gly, Lys, Asn, Arg, Ser, Tyr, Trp or

       Thr

<220>
<221>  MISC_FEATURE
<222>  (11)..(11)
<223>  Xaa is Ala, Gly, Arg, Ser, Thr or Trp

<400>  50

Ala Arg Xaa Xaa Xaa Xaa Xaa Xaa Xaa Xaa Xaa Ala Met Asp Tyr
1               5                   10                  15


<210>  51
<211>  13
<212>  PRT

<213>  Artificial Sequence

<220>
<223>  Randomized CDRH3


<220>
<221>  MISC_FEATURE
<222>  (2)..(2)
<223>  Xaa is Arg or Lys

<220>
<221>  MISC_FEATURE
<222>  (4)..(9)
<223>  Xaa is Ala, Cys, Asp, Gly, His, Asn, Pro, Arg, Ser, Thr or Tyr

<220>
<221>  MISC_FEATURE
<222>  (10)..(10)
<223>  Xaa is Gly, Ser, Ala or Trp

<400>  51

Ala Xaa Trp Xaa Xaa Xaa Xaa Xaa Xaa Xaa Ala Met Tyr
1               5                   10


<210>  52
<211>  13
<212>  PRT
<213>  Artificial Sequence

<220>
<223>  Randomized CDRH3


<220>
<221>  MISC_FEATURE
<222>  (2)..(2)
<223>  Xaa is Arg or Lys

<220>
<221>  MISC_FEATURE
<222>  (4)..(8)
<223>  Xaa is Ala, Cys, Asp, Gly, His, Asn, Pro, Arg, Ser, Thr or Tyr

<220>
<221>  MISC_FEATURE
<222>  (9)..(9)
<223>  Xaa is Ala, Gly, Arg, Ser, Thr or Trp

<400>  52

Ala Xaa Trp Xaa Xaa Xaa Xaa Xaa Xaa Ala Met Asp Tyr
1               5                   10


<210>  53
<211>  13
<212>  PRT
<213>  Artificial Sequence

<220>
<223>  Randomized CDRH3


<220>
<221>  MISC_FEATURE

<222>  (2)..(2)
<223>  Xaa is Arg, Lys or Thr

<220>
<221>  MISC_FEATURE
<222>  (4)..(9)
<223>  Xaa is Ala, Cys, Asp, Gly, His, Asn, Pro, Arg, Ser, Thr or Tyr

<400>  53

Ala Xaa Trp Xaa Xaa Xaa Xaa Xaa Xaa Ala Met Asp Tyr
1               5                   10


<210>  54
<211>  15
<212>  PRT
<213>  Artificial Sequence

<220>
<223>  Randomized CDRH3


<220>
<221>  MISC_FEATURE
<222>  (3)..(10)
<223>  Xaa is Ala, Cys, Asp, Gly, His, Asn, Pro, Arg, Ser, Thr or Tyr

<400>  54

Ser Arg Xaa Xaa Xaa Xaa Xaa Xaa Xaa Xaa Tyr Ala Met Asp Tyr
1               5                   10                  15


<210>  55
<211>  13
<212>  PRT
<213>  Artificial Sequence

<220>
<223>  Randomized CDRH3


<220>
<221>  MISC_FEATURE
<222>  (3)..(8)
<223>  Xaa is any naturally occurring amino acid

<400>  55

Ala Arg Xaa Xaa Xaa Xaa Xaa Xaa Tyr Ala Met Asp Tyr
1               5                   10


<210>  56
<211>  13
<212>  PRT
<213>  Artificial Sequence

<220>
<223>  Randomized CDRH3


<220>
<221>  MISC_FEATURE
<222>  (3)..(8)
<223>  Xaa is any naturally occurring amino acid

<220>
<221>  MISC_FEATURE
<222>  (9)..(9)
<223>  Xaa is Ala, Gly, Arg, Ser, Thr or Trp

<400>  56

Ala Arg Xaa Xaa Xaa Xaa Xaa Xaa Xaa Ala Met Asp Tyr
1               5                   10


<210>  57
<211>  12
<212>  PRT
<213>  Artificial Sequence

<220>
<223>  Randomized CDRH3


<220>
<221>  MISC_FEATURE
<222>  (3)..(7)
<223>  Xaa is any naturally occurring amino acid

<220>
<221>  MISC_FEATURE
<222>  (9)..(9)
<223>  Xaa is Ala, Gly or Val

<400>  57

Ala Arg Xaa Xaa Xaa Xaa Xaa Tyr Xaa Met Asp Tyr
1               5                   10


<210>  58
<211>  12
<212>  PRT
<213>  Artificial Sequence

<220>
<223>  Randomized CDRH3


<220>
<221>  MISC_FEATURE
<222>  (3)..(7)
<223>  Xaa is any naturally occurring amino acid

<220>
<221>  MISC_FEATURE
<222>  (8)..(8)
<223>  Xaa is Trp, Ser, Ala or Gly

<220>
<221>  MISC_FEATURE
<222>  (9)..(9)
<223>  Xaa is Ala, Gly or Val

<400>  58

Ala Arg Xaa Xaa Xaa Xaa Xaa Xaa Xaa Met Asp Tyr
1               5                   10


<210>  59
<211>  11

<212>  PRT
<213>  Artificial Sequence

<220>
<223>  Randomized CDRH3


<220>
<221>  MISC_FEATURE
<222>  (3)..(6)
<223>  Xaa is any naturally occurring amino acid

<220>
<221>  MISC_FEATURE
<222>  (8)..(8)
<223>  Xaa is Ala, Gly or Val

<400>  59

Ala Arg Xaa Xaa Xaa Xaa Tyr Xaa Met Asp Tyr
1               5                   10


<210>  60
<211>  11
<212>  PRT
<213>  Artificial Sequence

<220>
<223>  Randomized CDRH3


<220>
<221>  MISC_FEATURE
<222>  (3)..(6)
<223>  Xaa is any naturally occurring amino acid

<220>
<221>  MISC_FEATURE
<222>  (7)..(7)
<223>  Xaa is Trp, Ser, Ala or Gly

<220>
<221>  MISC_FEATURE
<222>  (8)..(8)
<223>  Xaa is Ala, Gly or Val

<400>  60

Ala Arg Xaa Xaa Xaa Xaa Xaa Xaa Met Asp Tyr
1               5                   10


<210>  61
<211>  13
<212>  PRT
<213>  Artificial Sequence

<220>
<223>  Randomized CDRH3


<220>
<221>  MISC_FEATURE
<222>  (3)..(8)
<223>  Xaa is any naturally occurring amino acid

<220>

<221>  MISC_FEATURE
<222>  (10)..(10)
<223>  Xaa is Ala, Gly or Val

<400>  61

Ala Arg Xaa Xaa Xaa Xaa Xaa Xaa Tyr Xaa Met Asp Tyr
1               5                   10


<210>  62
<211>  13
<212>  PRT
<213>  Artificial Sequence

<220>
<223>  Randomized CDRH3


<220>
<221>  MISC_FEATURE
<222>  (3)..(8)
<223>  Xaa is any naturally occurring amino acid

<220>
<221>  MISC_FEATURE
<222>  (9)..(9)
<223>  Xaa is Trp, Ser, Ala or Gly

<220>
<221>  MISC_FEATURE
<222>  (10)..(10)
<223>  Xaa is Ala, Gly or Val

<400>  62

Ala Arg Xaa Xaa Xaa Xaa Xaa Xaa Xaa Xaa Met Asp Tyr
1               5                   10


<210>  63
<211>  14
<212>  PRT
<213>  Artificial Sequence

<220>
<223>  Randomized CDRH3


<220>
<221>  MISC_FEATURE
<222>  (3)..(9)
<223>  Xaa is any naturally occurring amino acid

<220>
<221>  MISC_FEATURE
<222>  (10)..(10)
<223>  Xaa is Trp, Ser, Ala or Gly

<220>
<221>  MISC_FEATURE
<222>  (11)..(11)
<223>  Xaa is Ala, Gly or Val

<400>  63

Ala Arg Xaa Xaa Xaa Xaa Xaa Xaa Xaa Xaa Xaa Met Asp Tyr
1               5                   10

<210>  64
<211>  14
<212>  PRT
<213>  Artificial Sequence

<220>
<223>  Randomized CDRH3


<220>
<221>  MISC_FEATURE
<222>  (3)..(9)
<223>  Xaa is any naturally occurring amino acid

<220>
<221>  MISC_FEATURE
<222>  (11)..(11)
<223>  Xaa is Ala, Gly or Val

<400>  64

Ala Arg Xaa Xaa Xaa Xaa Xaa Xaa Xaa Tyr Xaa Met Asp Tyr
1               5                   10


<210>  65
<211>  15
<212>  PRT
<213>  Artificial Sequence

<220>
<223>  Randomized CDRH3


<220>
<221>  MISC_FEATURE
<222>  (3)..(10)
<223>  Xaa is any naturally occurring amino acid

<220>
<221>  MISC_FEATURE
<222>  (12)..(12)
<223>  Xaa is Ala, Gly or Val

<400>  65

Ala Arg Xaa Xaa Xaa Xaa Xaa Xaa Xaa Xaa Tyr Xaa Met Asp Tyr
1               5                   10                  15


<210>  66
<211>  15
<212>  PRT
<213>  Artificial Sequence

<220>
<223>  Randomized CDRH3


<220>
<221>  MISC_FEATURE
<222>  (3)..(10)
<223>  Xaa is any naturally occurring amino acid

<220>
<221>  MISC_FEATURE

<222>  (11)..(11)
<223>  Xaa is Trp, Ser, Ala or Gly

<220>
<221>  MISC_FEATURE
<222>  (12)..(12)
<223>  Xaa is Ala, Gly or Val

<400>  66

Ala Arg Xaa Xaa Xaa Xaa Xaa Xaa Xaa Xaa Xaa Xaa Met Asp Tyr
1               5                   10                  15


<210>  67
<211>  10
<212>  PRT
<213>  Artificial Sequence

<220>
<223>  Randomized CDRH3


<220>
<221>  MISC_FEATURE
<222>  (2)..(2)
<223>  Xaa is Arg or Lys

<220>
<221>  MISC_FEATURE
<222>  (3)..(7)
<223>  Xaa is any naturally occurring amino acid

<400>  67

Ala Xaa Xaa Xaa Xaa Xaa Xaa Phe Asp Tyr
1               5                   10


<210>  68
<211>  11
<212>  PRT
<213>  Artificial Sequence

<220>
<223>  Randomized CDRH3


<220>
<221>  MISC_FEATURE
<222>  (2)..(2)
<223>  Xaa is Arg or Lys

<220>
<221>  MISC_FEATURE
<222>  (3)..(8)
<223>  Xaa is any naturally occurring amino acid

<400>  68

Ala Xaa Xaa Xaa Xaa Xaa Xaa Xaa Phe Asp Tyr
1               5                   10


<210>  69
<211>  12
<212>  PRT
<213>  Artificial Sequence

<220>
<223>  Embodiment of oligonucleotide for randomized CDRH3


<220>
<221>  MISC_FEATURE
<222>  (2)..(2)
<223>  Xaa is Arg or Lys

<220>
<221>  MISC_FEATURE
<222>  (3)..(9)
<223>  Xaa is any naturally occurring amino acid

<400>  69

Ala Xaa Xaa Xaa Xaa Xaa Xaa Xaa Xaa Phe Asp Tyr
1               5                   10


<210>  70
<211>  9
<212>  PRT
<213>  Artificial Sequence

<220>
<223>  Randomized CDRH3


<220>
<221>  MISC_FEATURE
<222>  (2)..(2)
<223>  Xaa is Arg or Lys

<220>
<221>  MISC_FEATURE
<222>  (3)..(6)
<223>  Xaa is any naturally occurring amino acid

<400>  70

Ala Xaa Xaa Xaa Xaa Xaa Phe Asp Tyr
1               5


<210>  71
<211>  12
<212>  PRT
<213>  Artificial Sequence

<220>
<223>  Randomized CDRH3


<220>
<221>  MISC_FEATURE
<222>  (2)..(2)
<223>  Xaa is Arg or Lys

<220>
<221>  MISC_FEATURE
<222>  (3)..(9)
<223>  Xaa is any naturally occurring amino acid

<400>  71

Ala Xaa Xaa Xaa Xaa Xaa Xaa Xaa Xaa Phe Asp Tyr

1                   5                        10


<210>  72
<211>  13
<212>  PRT
<213>  Artificial Sequence

<220>
<223>  Randomized CDRH3


<220>
<221>  MISC_FEATURE
<222>  (2)..(2)
<223>  Xaa is Arg or Lys

<220>
<221>  MISC_FEATURE
<222>  (3)..(10)
<223>  Xaa is any naturally occurring amino acid

<400>  72

Ala Xaa Xaa Xaa Xaa Xaa Xaa Xaa Xaa Xaa Phe Asp Tyr
1               5                   10


<210>  73
<211>  16
<212>  PRT
<213>  Artificial Sequence

<220>
<223>  Randomized CDRH3


<220>
<221>  MISC_FEATURE
<222>  (2)..(2)
<223>  Xaa is Arg or Lys

<220>
<221>  MISC_FEATURE
<222>  (3)..(11)
<223>  Xaa is any naturally occurring amino acid

<400>  73

Ala Xaa Xaa Xaa Xaa Xaa Xaa Xaa Xaa Xaa Xaa Tyr Ala Met Asp Tyr
1               5                   10                  15


<210>  74
<211>  15
<212>  PRT
<213>  Artificial Sequence

<220>
<223>  Embodiment of oligonucleotide for randomized CDRH3


<220>
<221>  MISC_FEATURE
<222>  (2)..(2)
<223>  Xaa is Arg or Lys

<220>

<221>  MISC_FEATURE
<222>  (3)..(10)
<223>  Xaa is any naturally occurring amino acid

<220>
<221>  MISC_FEATURE
<222>  (12)..(12)
<223>  Xaa is Ala, Val or Gly

<400>  74

Ala Xaa Xaa Xaa Xaa Xaa Xaa Xaa Xaa Xaa Tyr Xaa Met Asp Tyr
1               5                   10                  15


<210>  75
<211>  17
<212>  PRT
<213>  Artificial Sequence

<220>
<223>  Randomized CDRH3


<220>
<221>  MISC_FEATURE
<222>  (2)..(2)
<223>  Xaa is Arg or Lys

<220>
<221>  MISC_FEATURE
<222>  (3)..(12)
<223>  Xaa is any naturally occurring amino acid

<220>
<221>  MISC_FEATURE
<222>  (14)..(14)
<223>  Xaa is Ala, Val or Gly

<400>  75

Ala Xaa Xaa Xaa Xaa Xaa Xaa Xaa Xaa Xaa Xaa Xaa Tyr Xaa Met Asp
1               5                   10                  15


Tyr




<210>  76
<211>  7
<212>  PRT
<213>  Artificial Sequence

<220>
<223>  4D5 Heavy chain CDR

<400>  76

Trp Gly Gly Asp Gly Phe Tyr
1               5


<210>  77
<211>  11
<212>  PRT
<213>  Artificial Sequence

<220>
<223>  CDRH3

<400>  77

Ser Arg Trp Lys Tyr Ala Thr Arg Tyr Ala Met
1               5                   10


<210>  78
<211>  11
<212>  PRT
<213>  Artificial Sequence

<220>
<223>  CDRH3

<400>  78

Ser Arg Ser Arg Gly Trp Trp Thr Ala Ala Met
1               5                   10


<210>  79
<211>  11
<212>  PRT
<213>  Artificial Sequence

<220>
<223>  CDRH3

<400>  79

Ser Arg Ala Ser Arg Asp Trp Tyr Gly Ala Met
1               5                   10


<210>  80
<211>  5
<212>  PRT
<213>  Artificial Sequence

<220>
<223>  CDRH1

<400>  80

Thr Thr Ser Asn Gly
1               5


<210>  81
<211>  8
<212>  PRT
<213>  Artificial Sequence

<220>
<223>  CDRH2

<400>  81

Ala Tyr Ser Ser Asn Tyr Tyr Arg
1               5


<210>  82
<211>  9
<212>  PRT

<213>  Artificial Sequence

<220>
<223>  CDRH3

<400>  82

Ala Arg Trp Ser Arg Ala Ser Phe Tyr
1               5


<210>  83
<211>  5
<212>  PRT
<213>  Artificial Sequence

<220>
<223>  CDRH1

<400>  83

Thr Thr Gly Thr Asp
1               5


<210>  84
<211>  8
<212>  PRT
<213>  Artificial Sequence

<220>
<223>  CDRH2

<400>  84

Ala Ile Thr Tyr Asp Ser Tyr Arg
1               5


<210>  85
<211>  9
<212>  PRT
<213>  Artificial Sequence

<220>
<223>  CDRH3

<400>  85

Ala Lys Ala Gly Asp Arg Glu Gly Tyr
1               5


<210>  86
<211>  5
<212>  PRT
<213>  Artificial Sequence

<220>
<223>  CDRH1

<400>  86

Thr Thr Asp Ser Gly
1               5


<210>  87

<211>  8
<212>  PRT
<213>  Artificial Sequence

<220>
<223>  CDRH2

<400>  87

Gly Arg Ser Tyr Ser Ser Asn Arg
1               5


<210>  88
<211>  9
<212>  PRT
<213>  Artificial Sequence

<220>
<223>  CDRH3

<400>  88

Ala Lys Trp Pro Trp Tyr Asn Ala Trp
1               5


<210>  89
<211>  5
<212>  PRT
<213>  Artificial Sequence

<220>
<223>  CDRH1

<400>  89

Thr Asn Asn Tyr Trp
1               5


<210>  90
<211>  7
<212>  PRT
<213>  Artificial Sequence

<220>
<223>  CDRH2

<400>  90

Gly Tyr Ser Tyr Gly Thr Arg
1               5


<210>  91
<211>  9
<212>  PRT
<213>  Artificial Sequence

<220>
<223>  CDRH3


<220>
<221>  MISC_FEATURE
<222>  (4)..(4)
<223>  Xaa is unknown

<400>  91

Ala Lys Ala Xaa Lys Gly Ser Leu Tyr
1               5


<210>  92
<211>  5
<212>  PRT
<213>  Artificial Sequence

<220>
<223>  CDRH1

<400>  92

Thr Thr Gly Asn Ala
1               5


<210>  93
<211>  5
<212>  PRT
<213>  Artificial Sequence

<220>
<223>  CDRH1

<400>  93

Thr Asn Asp Tyr Tyr
1               5


<210>  94
<211>  5
<212>  PRT
<213>  Artificial Sequence

<220>
<223>  CDRH1

<400>  94

Thr Ser Asn Thr Gly
1               5


<210>  95
<211>  5
<212>  PRT
<213>  Artificial Sequence

<220>
<223>  CDRH1

<400>  95

Thr Thr Ser Tyr Gly
1               5


<210>  96
<211>  7
<212>  PRT
<213>  Artificial Sequence

<220>
<223>  CDRH2

<400>  96

Ala Ser Ser Tyr Ser Tyr Arg
1               5


<210>  97
<211>  9
<212>  PRT
<213>  Artificial Sequence

<220>
<223>  CDRH3


<220>
<221>  MISC_FEATURE
<222>  (4)..(4)
<223>  Xaa is unknown

<220>
<221>  MISC_FEATURE
<222>  (9)..(9)
<223>  Xaa is unknown

<400>  97

Ala Lys Tyr Xaa Ala Arg Glu Gly Xaa
1               5


<210>  98
<211>  5
<212>  PRT
<213>  Artificial Sequence

<220>
<223>  CDRH1

<400>  98

Thr Asn Asn Asn Ser
1               5


<210>  99
<211>  7
<212>  PRT
<213>  Artificial Sequence

<220>
<223>  CDRH2

<400>  99

Gly Tyr Asn Ser Gly Ser Arg
1               5


<210>  100
<211>  9
<212>  PRT
<213>  Artificial Sequence

<220>

<223>  CDRH3

<400>  100

Ala Lys Trp Arg Thr Ser Trp Lys Tyr
1               5


<210>  101
<211>  5
<212>  PRT
<213>  Artificial Sequence

<220>
<223>  CDRH1

<400>  101

Thr Ser Ser Ser Ala
1               5


<210>  102
<211>  7
<212>  PRT
<213>  Artificial Sequence

<220>
<223>  CDRH2

<400>  102

Ala Trp Ser Asn Gly Ser Arg
1               5


<210>  103
<211>  9
<212>  PRT
<213>  Artificial Sequence

<220>
<223>  CDRH3


<220>
<221>  MISC_FEATURE
<222>  (2)..(2)
<223>  Xaa is unknown

<400>  103

Ala Xaa Thr Ala Gly Gly Ala Lys Tyr
1               5


<210>  104
<211>  5
<212>  PRT
<213>  Artificial Sequence

<220>
<223>  CDRH1

<400>  104

Thr Thr Asn Thr Trp
1               5

<210>  105
<211>  7
<212>  PRT
<213>  Artificial Sequence

<220>
<223>  CDRH2

<400>  105

Gly Asp Tyr Asp Gly Tyr Arg
1               5


<210>  106
<211>  9
<212>  PRT
<213>  Artificial Sequence

<220>
<223>  CDRH3


<220>
<221>  MISC_FEATURE
<222>  (2)..(2)
<223>  Xaa is unknown

<400>  106

Ala Xaa Trp Arg Trp Trp Gly Arg Tyr
1               5


<210>  107
<211>  5
<212>  PRT
<213>  Artificial Sequence

<220>
<223>  CDRH1

<400>  107

Thr Asn Gly Asn Tyr
1               5


<210>  108
<211>  7
<212>  PRT
<213>  Artificial Sequence

<220>
<223>  CDRH2

<400>  108

Gly Trp Ser Asn Gly Tyr Arg
1               5


<210>  109
<211>  9
<212>  PRT
<213>  Artificial Sequence

<220>
<223>  CDRH3

<400>  109

Ala Arg Tyr Ser Gly Gly Arg Arg Tyr
1               5


<210>  110
<211>  5
<212>  PRT
<213>  Artificial Sequence

<220>
<223>  CDRH1

<400>  110

Thr Ser Asn Asn Ala
1               5


<210>  111
<211>  7
<212>  PRT
<213>  Artificial Sequence

<220>
<223>  CDRH2

<400>  111

Gly Arg Ser Tyr Asn Tyr Arg
1               5


<210>  112
<211>  9
<212>  PRT
<213>  Artificial Sequence

<220>
<223>  CDRH3


<220>
<221>  MISC_FEATURE
<222>  (2)..(2)
<223>  Xaa is unknown

<220>
<221>  MISC_FEATURE
<222>  (4)..(4)
<223>  Xaa is unknown

<400>  112

Ala Xaa Gly Xaa Thr Ser Gly Gly Tyr
1               5


<210>  113
<211>  5
<212>  PRT
<213>  Artificial Sequence

<220>
<223>  CDRH1

<400>  113

Thr Thr Ser Asn Asp
1               5


<210>  114
<211>  7
<212>  PRT
<213>  Artificial Sequence

<220>
<223>  CDRH2

<400>  114

Ala Trp Ser Tyr Asn Tyr Arg
1               5


<210>  115
<211>  9
<212>  PRT
<213>  Artificial Sequence

<220>
<223>  CDRH3

<400>  115

Ala Arg Arg Ser Arg Trp Ser Arg Ala
1               5


<210>  116
<211>  5
<212>  PRT
<213>  Artificial Sequence

<220>
<223>  CDRH1

<400>  116

Thr Gly Asn Ser Trp
1               5


<210>  117
<211>  6
<212>  PRT
<213>  Artificial Sequence

<220>
<223>  CDRH2

<400>  117

Val Ala Thr Tyr Tyr Asn
1               5


<210>  118
<211>  7
<212>  PRT

<213>  Artificial Sequence

<220>
<223>  CDRH3

<400>  118

Trp Gly Ala Lys Gly Thr Trp
1               5


<210>  119
<211>  5
<212>  PRT
<213>  Artificial Sequence

<220>
<223>  CDRH1

<400>  119

Asn Ala Asp Ser Ala
1               5


<210>  120
<211>  6
<212>  PRT
<213>  Artificial Sequence

<220>
<223>  CDRH2

<400>  120

Tyr Ala Tyr Asp Tyr Tyr
1               5


<210>  121
<211>  7
<212>  PRT
<213>  Artificial Sequence

<220>
<223>  CDRH3

<400>  121

Trp Gly Trp Thr Thr Asn Gly
1               5


<210>  122
<211>  5
<212>  PRT
<213>  Artificial Sequence

<220>
<223>  CDRH1

<400>  122

Asn Asp Asn Thr Ala
1               5


<210>  123

<211>  6
<212>  PRT
<213>  Artificial Sequence

<220>
<223>  CDRH2

<400>  123

Val Ser His Asp Thr Tyr
1               5


<210>  124
<211>  7
<212>  PRT
<213>  Artificial Sequence

<220>
<223>  CDRH3

<400>  124

Trp Gly Trp Glu Thr Asp Gly
1               5


<210>  125
<211>  6
<212>  PRT
<213>  Artificial Sequence

<220>
<223>  CDRH2

<400>  125

Leu Asp Ser Ser Tyr Asp
1               5


<210>  126
<211>  7
<212>  PRT
<213>  Artificial Sequence

<220>
<223>  CDRH3

<400>  126

Ser Arg Ala Gly Tyr Thr Tyr
1               5


<210>  127
<211>  5
<212>  PRT
<213>  Artificial Sequence

<220>
<223>  CDRH1

<400>  127

Asn Gly Lys Ser Ser
1               5

<210>  128
<211>  6
<212>  PRT
<213>  Artificial Sequence

<220>
<223>  CDRH2

<400>  128

Trp Ser Tyr Glu Ala Ala
1               5


<210>  129
<211>  7
<212>  PRT
<213>  Artificial Sequence

<220>
<223>  CDRH3

<400>  129

Thr Ser Trp Ser Lys Pro Tyr
1               5


<210>  130
<211>  5
<212>  PRT
<213>  Artificial Sequence

<220>
<223>  CDRH1

<400>  130

Asn Thr Ala Tyr Gly
1               5


<210>  131
<211>  6
<212>  PRT
<213>  Artificial Sequence

<220>
<223>  CDRH2

<400>  131

Val Thr Tyr Asp Asp Thr
1               5


<210>  132
<211>  7
<212>  PRT
<213>  Artificial Sequence

<220>
<223>  CDRH3

<400>  132

Trp Gly Trp Glu Ala Asn Trp

1               5


<210>  133
<211>  5
<212>  PRT
<213>  Artificial Sequence

<220>
<223>  CDRH1

<400>  133

Thr Gly Gly Ser Trp
1               5


<210>  134
<211>  6
<212>  PRT
<213>  Artificial Sequence

<220>
<223>  CDRH2

<400>  134

Val Tyr Thr Tyr Tyr Asp
1               5


<210>  135
<211>  7
<212>  PRT
<213>  Artificial Sequence

<220>
<223>  CDRH3

<400>  135

Trp Gly Ala Gly Gly Thr Trp
1               5


<210>  136
<211>  6
<212>  PRT
<213>  Artificial Sequence

<220>
<223>  CDRH2

<400>  136

Val Ser Asp Tyr Tyr Asp
1               5


<210>  137
<211>  7
<212>  PRT
<213>  Artificial Sequence

<220>
<223>  CDRH3

<400>  137

Trp Gly Ser Gly Tyr Thr Trp
1               5


<210>  138
<211>  5
<212>  PRT
<213>  Artificial Sequence

<220>
<223>  CDRH1

<400>  138

Ser Ala Gly Tyr Asp
1               5


<210>  139
<211>  6
<212>  PRT
<213>  Artificial Sequence

<220>
<223>  CDRH2

<400>  139

Leu Ala Tyr Ala Tyr Asn
1               5


<210>  140
<211>  7
<212>  PRT
<213>  Artificial Sequence

<220>
<223>  CDRH3

<400>  140

Ala Ala Ala Trp Ala Ser Tyr
1               5


<210>  141
<211>  5
<212>  PRT
<213>  Artificial Sequence

<220>
<223>  CDRH1

<400>  141

Thr Thr Glu Ser Gly
1               5


<210>  142
<211>  6
<212>  PRT
<213>  Artificial Sequence

<220>
<223>  CDRH2

<400>  142

Val Tyr His Asp Lys Tyr
1               5


<210>  143
<211>  7
<212>  PRT
<213>  Artificial Sequence

<220>
<223>  CDRH3

<400>  143

Trp Trp Tyr Ser Trp Asn Trp
1               5


<210>  144
<211>  11
<212>  PRT
<213>  Artificial Sequence

<220>
<223>  Randomized CDRH3


<220>
<221>  MISC_FEATURE
<222>  (2)..(2)
<223>  Xaa is Arg or Lys

<220>
<221>  MISC_FEATURE
<222>  (3)..(6)
<223>  Xaa is any naturally occurring amino acid

<400>  144

Ala Xaa Xaa Xaa Xaa Xaa Tyr Ala Met Asp Tyr
1               5                   10


<210>  145
<211>  11
<212>  PRT
<213>  Artificial Sequence

<220>
<223>  Randomized CDRH3


<220>
<221>  MISC_FEATURE
<222>  (2)..(2)
<223>  Xaa is Arg or Lys

<220>
<221>  MISC_FEATURE
<222>  (3)..(6)
<223>  Xaa is any naturally occurring amino acid

<220>
<221>  MISC_FEATURE
<222>  (7)..(7)

<223>  Xaa is Trp, Ser, Ala or Gly

<400>  145

Ala Xaa Xaa Xaa Xaa Xaa Xaa Ala Met Asp Tyr
1               5                   10


<210>  146
<211>  13
<212>  PRT
<213>  Artificial Sequence

<220>
<223>  Randomized CDRH3


<220>
<221>  MISC_FEATURE
<222>  (2)..(2)
<223>  Xaa is Arg, His, Ser or Asn

<220>
<221>  MISC_FEATURE
<222>  (3)..(8)
<223>  Xaa is any naturally occurring amino acid

<220>
<221>  MISC_FEATURE
<222>  (9)..(9)
<223>  Xaa is Trp, Ser, Ala or Gly

<220>
<221>  MISC_FEATURE
<222>  (10)..(10)
<223>  Xaa is Ala, Val, Gly or Asp

<400>  146

Ala Xaa Xaa Xaa Xaa Xaa Xaa Xaa Xaa Xaa Met Asp Tyr
1               5                   10


<210>  147
<211>  13
<212>  PRT
<213>  Artificial Sequence

<220>
<223>  Randomized CDRH3


<220>
<221>  MISC_FEATURE
<222>  (2)..(2)
<223>  Xaa is Arg, His, Ser or Asn

<220>
<221>  MISC_FEATURE
<222>  (3)..(8)
<223>  Xaa is any naturally occurring amino acid

<220>
<221>  MISC_FEATURE
<222>  (10)..(10)
<223>  Xaa is Ala, Val, Gly or Asp

<400>  147

Ala Xaa Xaa Xaa Xaa Xaa Xaa Xaa Tyr Xaa Met Asp Tyr
1               5                   10


<210>  148
<211>  14
<212>  PRT
<213>  Artificial Sequence

<220>
<223>  Randomized CDRH3


<220>
<221>  MISC_FEATURE
<222>  (3)..(11)
<223>  Xaa is any naturally occurring amino acid

<220>
<221>  MISC_FEATURE
<222>  (12)..(12)
<223>  Xaa is Phe or Leu

<400>  148

Ala Arg Xaa Xaa Xaa Xaa Xaa Xaa Xaa Xaa Xaa Xaa Asp Tyr
1               5                   10


<210>  149
<211>  14
<212>  PRT
<213>  Artificial Sequence

<220>
<223>  Randomized CDRH3


<220>
<221>  MISC_FEATURE
<222>  (2)..(2)
<223>  Xaa is Arg or Lys

<220>
<221>  MISC_FEATURE
<222>  (3)..(9)
<223>  Xaa is any naturally occurring amino acid

<220>
<221>  MISC_FEATURE
<222>  (10)..(10)
<223>  Xaa is Trp, Ser, Ala or Gly

<400>  149

Ala Xaa Xaa Xaa Xaa Xaa Xaa Xaa Xaa Xaa Ala Met Asp Tyr
1               5                   10


<210>  150
<211>  14
<212>  PRT
<213>  Artificial Sequence

<220>
<223>  Randomized CDRH3

<220>
<221>  MISC_FEATURE
<222>  (2)..(2)
<223>  Xaa is Arg, His, Ser or Asn

<220>
<221>  MISC_FEATURE
<222>  (3)..(9)
<223>  Xaa is any naturally occurring amino acid

<220>
<221>  MISC_FEATURE
<222>  (10)..(10)
<223>  Xaa is Trp, Ser, Ala or Gly

<220>
<221>  MISC_FEATURE
<222>  (11)..(11)
<223>  Xaa is Ala, Val, Gly or Asp

<400>  150

Ala Xaa Xaa Xaa Xaa Xaa Xaa Xaa Xaa Xaa Xaa Met Asp Tyr
1               5                   10


<210>  151
<211>  14
<212>  PRT
<213>  Artificial Sequence

<220>
<223>  Randomized CDRH3


<220>
<221>  MISC_FEATURE
<222>  (2)..(2)
<223>  Xaa is Arg, His, Ser or Asn

<220>
<221>  MISC_FEATURE
<222>  (3)..(9)
<223>  Xaa is any naturally occurring amino acid

<220>
<221>  MISC_FEATURE
<222>  (11)..(11)
<223>  Xaa is Ala, Val, Gly or Asp

<400>  151

Ala Xaa Xaa Xaa Xaa Xaa Xaa Xaa Xaa Tyr Xaa Met Asp Tyr
1               5                   10


<210>  152
<211>  15
<212>  PRT
<213>  Artificial Sequence

<220>
<223>  Randomized CDRH3


<220>
<221>  MISC_FEATURE

<222>  (3)..(12)
<223>  Xaa is any naturally occurring amino acid

<220>
<221>  MISC_FEATURE
<222>  (13)..(13)
<223>  Xaa is Phe or Leu

<400>  152

Ala Arg Xaa Xaa Xaa Xaa Xaa Xaa Xaa Xaa Xaa Xaa Xaa Asp Tyr
1               5                   10                  15


<210>  153
<211>  15
<212>  PRT
<213>  Artificial Sequence

<220>
<223>  Randomized CDRH3


<220>
<221>  MISC_FEATURE
<222>  (2)..(2)
<223>  Xaa is Arg or Lys

<220>
<221>  MISC_FEATURE
<222>  (3)..(10)
<223>  Xaa is any naturally occurring amino acid

<400>  153

Ala Xaa Xaa Xaa Xaa Xaa Xaa Xaa Xaa Xaa Tyr Ala Met Asp Tyr
1               5                   10                  15


<210>  154
<211>  15
<212>  PRT
<213>  Artificial Sequence

<220>
<223>  Randomized CDRH3


<220>
<221>  MISC_FEATURE
<222>  (2)..(2)
<223>  Xaa is Arg or Lys

<220>
<221>  MISC_FEATURE
<222>  (3)..(10)
<223>  Xaa is any naturally occurring amino acid

<220>
<221>  MISC_FEATURE
<222>  (11)..(11)
<223>  Xaa is Trp, Ser, Ala or Gly

<400>  154

Ala Xaa Xaa Xaa Xaa Xaa Xaa Xaa Xaa Xaa Xaa Ala Met Asp Tyr
1               5                   10                  15

<210>  155
<211>  18
<212>  PRT
<213>  Artificial Sequence

<220>
<223>  Randomized CDRH3


<220>
<221>  MISC_FEATURE
<222>  (3)..(13)
<223>  Xaa is any naturally occurring amino acid

<220>
<221>  MISC_FEATURE
<222>  (14)..(14)
<223>  Xaa is Trp, Ser, Ala or Gly

<220>
<221>  MISC_FEATURE
<222>  (15)..(15)
<223>  Xaa is Ala, Val or Gly

<400>  155

Ala Arg Xaa Xaa Xaa Xaa Xaa Xaa Xaa Xaa Xaa Xaa Xaa Xaa Xaa Met
1               5                   10                  15


Asp Tyr



<210>  156
<211>  18
<212>  PRT
<213>  Artificial Sequence

<220>
<223>  Randomized CDRH3


<220>
<221>  MISC_FEATURE
<222>  (3)..(13)
<223>  Xaa is any naturally occurring amino acid

<220>
<221>  MISC_FEATURE
<222>  (15)..(15)
<223>  Xaa is Ala, Val or Gly

<400>  156

Ala Arg Xaa Xaa Xaa Xaa Xaa Xaa Xaa Xaa Xaa Xaa Xaa Tyr Xaa Met
1               5                   10                  15


Asp Tyr



<210>  157
<211>  19
<212>  PRT
<213>  Artificial Sequence

<220>
<223>  Randomized CDRH3


<220>
<221>  MISC_FEATURE
<222>  (3)..(14)
<223>  Xaa is any naturally occurring amino acid

<220>
<221>  MISC_FEATURE
<222>  (15)..(15)
<223>  Xaa is Trp, His, Ala or Gly

<220>
<221>  MISC_FEATURE
<222>  (16)..(16)
<223>  Xaa is Ala, Val or Gly

<400>  157

Ala Arg Xaa Xaa Xaa Xaa Xaa Xaa Xaa Xaa Xaa Xaa Xaa Xaa Xaa Xaa
1               5                   10                  15


Met Asp Tyr




<210>  158
<211>  19
<212>  PRT
<213>  Artificial Sequence

<220>
<223>  Randomized CDRH3


<220>
<221>  MISC_FEATURE
<222>  (3)..(14)
<223>  Xaa is any naturally occurring amino acid

<220>
<221>  MISC_FEATURE
<222>  (16)..(16)
<223>  Xaa is Ala, Val or Gly

<400>  158

Ala Arg Xaa Xaa Xaa Xaa Xaa Xaa Xaa Xaa Xaa Xaa Xaa Xaa Tyr Xaa
1               5                   10                  15


Met Asp Tyr




<210>  159
<211>  20
<212>  PRT
<213>  Artificial Sequence

<220>
<223>  Randomized CDRH3

<220>
<221>  MISC_FEATURE
<222>  (3)..(15)
<223>  Xaa is any naturally occurring amino acid

<220>
<221>  MISC_FEATURE
<222>  (16)..(16)
<223>  Xaa is Trp, Ser, Ala or Gly

<220>
<221>  MISC_FEATURE
<222>  (17)..(17)
<223>  Xaa is Ala, Val or Gly

<400>  159

Ala Arg Xaa Xaa Xaa Xaa Xaa Xaa Xaa Xaa Xaa Xaa Xaa Xaa Xaa Xaa
1               5                   10                  15

Xaa Met Asp Tyr
            20

<210>  160
<211>  20
<212>  PRT
<213>  Artificial Sequence

<220>
<223>  Randomized CDRH3

<220>
<221>  MISC_FEATURE
<222>  (3)..(15)
<223>  Xaa is any naturally occurring amino acid

<220>
<221>  MISC_FEATURE
<222>  (17)..(17)
<223>  Xaa is Ala, Val or Gly

<400>  160

Ala Arg Xaa Xaa Xaa Xaa Xaa Xaa Xaa Xaa Xaa Xaa Xaa Xaa Xaa Tyr
1               5                   10                  15

Xaa Met Asp Tyr
            20

<210>  161
<211>  21
<212>  PRT
<213>  Artificial Sequence

<220>
<223>  Randomized CDRH3

<220>
<221>  MISC_FEATURE
<222>  (3)..(16)
<223>  Xaa is any naturally occurring amino acid

<220>
<221>  MISC_FEATURE
<222>  (18)..(18)
<223>  Xaa is Ala, Val or Gly

<400>  161

Ala Arg Xaa Xaa Xaa Xaa Xaa Xaa Xaa Xaa Xaa Xaa Xaa Xaa Xaa Xaa
1               5                   10                  15

Tyr Xaa Met Asp Tyr
            20

<210>  162
<211>  13
<212>  PRT
<213>  Artificial Sequence

<220>
<223>  Embodiment of oligonucleotide for CDRH3

<220>
<221>  MISC_FEATURE
<222>  (5)..(9)
<223>  Xaa is Ala, Cys, Asp, Glu, Gly, Lys, Asn, Arg, Ser, Tyr, Trp or

       Thr

<400>  162

Ser Arg Trp Gly Xaa Xaa Xaa Xaa Xaa Ala Met Asp Tyr
1               5                   10

<210>  163
<211>  5
<212>  PRT
<213>  Artificial Sequence

<220>
<223>  C-Terminal of CDRH3

<220>
<221>  MISC_FEATURE
<222>  (5)..(5)
<223>  Xaa is Tyr or Val

<400>  163

Tyr Ala Met Asp Xaa
1               5

<210>  164
<211>  19
<212>  PRT
<213>  Artificial Sequence

<220>
<223>  CDRH3

<220>
<221>  MISC_FEATURE

<222>  (1)..(14)
<223>  Xaa is any naturally occurring amino acid and 10 may be absent

<220>
<221>  MISC_FEATURE
<222>  (15)..(15)
<223>  Xaa is any naturally occurring amino acid

<400>  164

Xaa Xaa Xaa Xaa Xaa Xaa Xaa Xaa Xaa Xaa Xaa Xaa Xaa Xaa Xaa Ala
1               5                   10                  15

Met Asp Tyr

<210>  165
<211>  16
<212>  PRT
<213>  Artificial Sequence

<220>
<223>  Randomized CDRH3

<220>
<221>  MISC_FEATURE
<222>  (2)..(2)
<223>  Xaa is Arg or Lys

<220>
<221>  MISC_FEATURE
<222>  (3)..(11)
<223>  Xaa is any amino acid

<220>
<221>  MISC_FEATURE
<222>  (12)..(12)
<223>  Xaa is Trp, Ser, Ala or Gly

<220>
<221>  MISC_FEATURE
<222>  (13)..(13)
<223>  Xaa is Ala, Val or Gly

<400>  165

Ala Xaa Xaa Xaa Xaa Xaa Xaa Xaa Xaa Xaa Xaa Xaa Xaa Met Asp Tyr
1               5                   10                  15

<210>  166
<211>  16
<212>  PRT
<213>  Artificial Sequence

<220>
<223>  Randomized CDRH3

<220>
<221>  MISC_FEATURE
<222>  (2)..(2)
<223>  Xaa is Arg or Lys

<220>

<221>  MISC_FEATURE
<222>  (3)..(11)
<223>  Xaa is any amino acid

<220>
<221>  MISC_FEATURE
<222>  (12)..(12)
<223>  Xaa is Trp, Ser, Ala or Gly

<220>
<221>  MISC_FEATURE
<222>  (13)..(13)
<223>  Xaa is Ala, Val or Gly

<400>  166

Ala Xaa Xaa Xaa Xaa Xaa Xaa Xaa Xaa Xaa Xaa Xaa Xaa Met Asp Tyr
1               5                   10                  15


<210>  167
<211>  15
<212>  PRT
<213>  Artificial Sequence

<220>
<223>  Randomized CDRH3


<220>
<221>  MISC_FEATURE
<222>  (2)..(2)
<223>  Xaa is Arg or Lys

<220>
<221>  MISC_FEATURE
<222>  (3)..(10)
<223>  Xaa is any amino acid

<220>
<221>  MISC_FEATURE
<222>  (12)..(12)
<223>  Xaa is Ala, Val or Gly

<400>  167

Ala Xaa Xaa Xaa Xaa Xaa Xaa Xaa Xaa Xaa Tyr Xaa Met Asp Tyr
1               5                   10                  15


<210>  168
<211>  9
<212>  PRT
<213>  Artificial Sequence

<220>
<223>  Randomized CDRH3


<220>
<221>  MISC_FEATURE
<222>  (2)..(2)
<223>  Xaa is Arg or Lys

<220>
<221>  MISC_FEATURE
<222>  (3)..(6)
<223>  Xaa is any amino acid

<220>
<221>  MISC_FEATURE
<222>  (8)..(8)
<223>  Xaa is Asp or Ala

<400>  168

Ala Xaa Xaa Xaa Xaa Xaa Phe Xaa Tyr
1               5


<210>  169
<211>  9
<212>  PRT
<213>  Artificial Sequence

<220>
<223>  Randomized CDRH3


<220>
<221>  MISC_FEATURE
<222>  (2)..(2)
<223>  Xaa is Arg, His, Ser or Asn

<220>
<221>  MISC_FEATURE
<222>  (3)..(6)
<223>  Xaa is any amino acid

<220>
<221>  MISC_FEATURE
<222>  (7)..(7)
<223>  Xaa is Phe or Leu

<220>
<221>  MISC_FEATURE
<222>  (8)..(8)
<223>  Xaa is Gly, Val, Asp or Ala

<400>  169

Ala Xaa Xaa Xaa Xaa Xaa Xaa Xaa Tyr
1               5


<210>  170
<211>  10
<212>  PRT
<213>  Artificial Sequence

<220>
<223>  Randomized CDRH3


<220>
<221>  MISC_FEATURE
<222>  (2)..(2)
<223>  Xaa is Arg, His, Ser or Asn

<220>
<221>  MISC_FEATURE
<222>  (3)..(7)
<223>  Xaa is any amino acid

<220>
<221>  MISC_FEATURE

<222>  (8)..(8)
<223>  Xaa is Phe or Leu

<220>
<221>  MISC_FEATURE
<222>  (9)..(9)
<223>  Xaa is Gly, Val, Asp or Ala

<400>  170

Ala Xaa Xaa Xaa Xaa Xaa Xaa Xaa Xaa Tyr
1               5                   10


<210>  171
<211>  10
<212>  PRT
<213>  Artificial Sequence

<220>
<223>  Randomized CDRH3


<220>
<221>  MISC_FEATURE
<222>  (2)..(2)
<223>  Xaa is Arg, His, Ser or Asn

<220>
<221>  MISC_FEATURE
<222>  (3)..(7)
<223>  Xaa is any amino acid

<220>
<221>  MISC_FEATURE
<222>  (8)..(8)
<223>  Xaa is Phe or Leu

<220>
<221>  MISC_FEATURE
<222>  (9)..(9)
<223>  Xaa is Asp or Ala

<400>  171

Ala Xaa Xaa Xaa Xaa Xaa Xaa Xaa Xaa Tyr
1               5                   10


<210>  172
<211>  11
<212>  PRT
<213>  Artificial Sequence

<220>
<223>  Randomized CDRH3


<220>
<221>  MISC_FEATURE
<222>  (2)..(2)
<223>  Xaa is Arg or Lys

<220>
<221>  MISC_FEATURE
<222>  (3)..(8)
<223>  Xaa is any amino acid

<220>
<221>  MISC_FEATURE
<222>  (10)..(10)
<223>  Xaa is Asp or Ala

<400>  172

Ala Xaa Xaa Xaa Xaa Xaa Xaa Xaa Phe Xaa Tyr
1               5                   10


<210>  173
<211>  12
<212>  PRT
<213>  Artificial Sequence

<220>
<223>  Randomized CDRH3


<220>
<221>  MISC_FEATURE
<222>  (2)..(2)
<223>  Xaa is Arg or Lys

<220>
<221>  MISC_FEATURE
<222>  (3)..(9)
<223>  Xaa is any amino acid

<220>
<221>  MISC_FEATURE
<222>  (10)..(10)
<223>  Xaa is Phe or Leu

<220>
<221>  MISC_FEATURE
<222>  (11)..(11)
<223>  Xaa is Gly, Val, Asp or Ala

<400>  173

Ala Xaa Xaa Xaa Xaa Xaa Xaa Xaa Xaa Xaa Xaa Tyr
1               5                   10


<210>  174
<211>  6
<212>  PRT
<213>  Artificial Sequence

<220>
<223>  Randomized CDRH3


<220>
<221>  MISC_FEATURE
<222>  (1)..(1)
<223>  Xaa is any amino acid, n = 4 to 14

<220>
<221>  MISC_FEATURE
<222>  (2)..(2)
<223>  Xaa is any amino acid or is Tyr, Phe, Gly, Ser, Ala, Leu or Trp

<220>
<221>  MISC_FEATURE
<222>  (3)..(3)

<223>  Xaa is missing when Xaa (2)...(2) is Phe or Leu or is Ala, Val,
       Asp or Gly

<220>
<221>  MISC_FEATURE
<222>  (4)..(4)
<223>  Xaa is missing when Xaa (2)...(2) is Phe or Leu or is Met

<220>
<221>  MISC_FEATURE
<222>  (5)..(5)
<223>  Xaa is Asp, Ala, Val or Gly

<220>
<221>  MISC_FEATURE
<222>  (6)..(6)
<223>  Xaa is Tyr or Val

<400>  174

Xaa Xaa Xaa Xaa Xaa Xaa
1               5